# EUROPEAN PATENT SPECIFICATION

(11) **EP 3 535 267 B1**
(45) Date of publication and mention of the grant of the patent: **30.12.2020**
(21) Application number: 17792066.7
(22) Date of filing: 31.10.2017
(51) Int. Cl.: C07D 487/04, C07D 519/00, A61K 31/519, A61P 25/00

(54) **[1,2,4]TRIAZOLO[1,5-A]PYRIMIDINE DERIVATIVES AS PDE2 INHIBITORS**
[1,2,4]TRIAZOLO[1,5-A]PYRIMIDIN-DERIVATE ALS PDE2-INHIBITOREN
DÉRIVÉS DE [1,2,4]TRIAZOLO[1,5-A]PYRIMIDINE COMME INHIBITEURS DE PDE2

(30) Priority: 02.11.2016 EP 16196924
(43) Date of publication of application: 11.09.2019
(73) Proprietor: Janssen Pharmaceutica NV, 2340 Beerse (BE)
(72) Inventor: VAN ROOSBROECK, Yves, Emiel, Maria, 2340 Beerse (BE); VAN DEN KEYBUS, Frans, Alfons, Maria, 2340 Beerse (BE); TRESADERN, Gary, John, 28042 Madrid (ES); BUIJNSTERS, Peter, Jacobus, Johannes, Antonius, 2340 Beerse (BE); VELTER, Adriana, Ingrid, 2340 Beerse (BE); JACOBY, Edgar, 2340 Beerse (BE); MACDONALD, Gregor, James, 2340 Beerse (BE); GIJSEN, Henricus, Jacobus, Maria, 2340 Beerse (BE); AHNAOU, Abdellah, 2340 Beerse (BE); DRINKENBURG, Wilhelmus, Helena, Ignatius, Maria, 2340 Beerse (BE)
(74) Representative: Garcia Prieto, Maria
(86) International application number: PCT/EP2017/077910
(87) International publication number: WO 2018/083098

(56) References cited:
- WO-A1-2015/164508
- WO-A1-2017/076900

## Description

### FIELD OF THE INVENTION

The present invention relates to novel [1,2,4]triazolo[1,5-a]pyrimidin-yl derivatives as inhibitors of phosphodiesterase 2 (PDE2). The invention is also directed to pharmaceutical compositions comprising the compounds, to processes for preparing such compounds and compositions, and to the use of such compounds and compositions for the prevention and treatment of disorders in which PDE2 is involved, such as neurological and psychiatric disorders.

### BACKGROUND OF THE INVENTION

Phosphodiesterases (PDEs) are a family of enzymes encoded by 21 genes and subdivided into 11 distinct families according to structural and functional properties. These enzymes metabolically inactivate widely occurring intracellular second messengers, 3',5'-cyclic adenosine monophosphate (cAMP) and 3',5'-cyclic guanosine monophosphate (cGMP). These two messengers regulate a wide variety of biological processes, including pro-inflammatory mediator production and action, ion channel function, muscle contraction, learning, differentiation, apoptosis, lipogenesis, glycogenolysis, and gluconeogenesis. They do this by activation of protein kinase A (PKA) and protein kinase G (PKG), which in turn phosphorylate a wide variety of substrates including transcription factors and ion channels that regulate innumerable physiological responses. In neurons, this includes the activation of cAMP and cGMP-dependent kinases and subsequent phosphorylation of proteins involved in acute regulation of synaptic transmission as well as in neuronal differentiation and survival. Intracellular concentrations of cAMP and cGMP are strictly regulated by the rate of biosynthesis by cyclases and by the rate of degradation by PDEs. PDEs are hydrolases that inactivate cAMP and cGMP by catalytic hydrolysis of the 3'-ester bond, forming the inactive 5'-monophosphate (Scheme A).

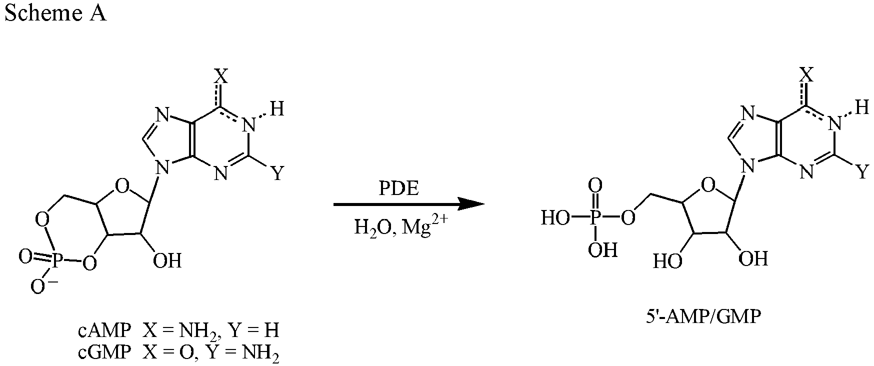

On the basis of substrate specificity, the PDE families can be divided into three groups: i) the cAMP-specific PDEs, which include PDE4, 7 and 8; ii) the cGMP-selective enzymes PDE5, 6 and 9; and iii) the dual-substrate PDEs, PDE1, 2 and 3, as well as PDE10 and 11.
Furthermore, PDEs are expressed differentially throughout the organism, including the central nervous system. Different PDE isozymes therefore may have different physiological functions. Compounds that inhibit selectively PDE families or isozymes may display particular therapeutic activity, fewer side effects, or both.

Phosphodiesterase 2A (PDE2A) inactivates intracellular signalling mechanisms reliant on cyclic nucleotide signalling mediated by cAMP and cGMP via their degradation (by hydrolizing the biologically relevant second messengers cAMP and cGMP into nonsignalling AMP and GMP, respectively). Such signalling pathways are known to play a role in the regulation of genes involved in the induction of synaptic plasticity. The pharmacological inhibition of PDE2 therefore causes increased levels of synaptic plasticity (an underlying correlate of learning and memory), suggesting that PDE2A modulation may be a target for alleviating cognitive deficits seen in people suffering from disorders such as for example, schizophrenia, Alzheimer's disease, Parkinson's disease and other CNS disorders associated with cognitive dysfunction.

Phosphodiesterase 2A (PDE2A) is more abundantly expressed in the brain relative to peripheral tissues. The high expression of PDE2 in the limbic system (isocortex, hippocampus, amygdala, habenula, basal ganglia) suggests that PDE2 may modulate neuronal signalling involved in emotion, perception, concentration, learning and memory. Additionally, PDE2 is expressed in the nucleus accumbens, the olfactory bulb, the olfactory tubercle and the amygdala, supporting the suggestion that PDE2 may also be involved in anxiety and depression. (see for instance, Lakics, V. et al. (2010) Quantitative comparison of phosphodiesterase mRNA distribution in human brain and peripheral tissues. Neuropharmacol. 59, 367-374).
Additionally, PDE2 inhibitors have been shown to be beneficial in the reduction of oxidative stress-induced anxiety, supporting their use in the treatment of anxiety in neuropsychiatric and neurodegenerative disorders that involve oxidative stress, such as Alzheimer's disease, Parkinson's disease and multiple sclerosis.

PDE2 inhibitors have been shown to enhance long term potentiation of synaptic transmission and to improve memory acquisition and consolidation in the object recognition and in the social recognition tests in rats. Furthermore, PDE2 inhibitors have been shown to reverse the MK-801 induced working memory deficit in the T-maze in mice. PDE2 inhibitors have also been shown to display activity in forced swim test and light/dark box models; and to show anxiolytic-like effects in elevated plus-maze, hole-board and open-field tests and to prevent stress-induced changes in apoptosis and behaviour.

Thus, PDE2 inhibitors may be useful in the treatment of memory deficiency, cognitive disorders, anxiety, bipolar disorder and depression.

WO2015/164508 (Dart Neuroscience, LLC) discloses substituted [1,2,4]triazolo[1,5-a]pyrimidin-yl compounds as PDE2 inhibitors. WO2017/076900 (Janssen Pharmaceutica NV) discloses [(3S,4R)-3-[5-(difluoromethyl)-[1,2,4]triazolo[1,5-a]pyrimidin-7-yl]-4-methyl-1-piperidyl]-(2,6-dimethyl-4-pyridyl)methanone.

There is still a need for PDE2 inhibitor compounds with an advantageous balance of properties, for example with improved potency, better selectivity against PDE3 and/or PDE10, and/or better chemical or metabolic stability.

### SUMMARY OF THE INVENTION

It is the object of the present invention to provide novel inhibitors of PDE2 that may be potentially useful in the treatment of diseases related to PDE2 enzyme activity.

Thus, the present invention is directed to compounds of Formula (I) 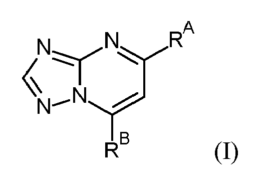 and the stereoisomeric forms thereof, wherein
R^{A} is selected from the group consisting of H, CH₃, CN, and CHF₂;
R^{B} is a radical selected from the group consisting of (a), (b) and (c): 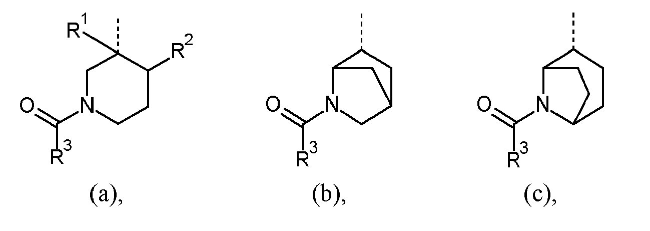 wherein
R¹ is H, F or CH₃;
R² is H or C₁₋₄alkyl, in particular methyl or n-butyl; with the proviso that when R² is H, then R¹ is F or CH₃;
R³ is Ar, Het, or Ar-C₂₋₄alkenyl; wherein
Ar represents phenyl or naphthyl, each optionally substituted with 1, 2 or 3 substituents, each independently selected from the group consisting of halo; CN; NR^{2A}R^{2B} wherein R^{2A} and R^{2B} are each independently selected from H and CH₃; OH; C₁₋₆alkyl optionally substituted with 1, 2 or 3 independently selected halo substituents; C₁₋₆alkyl substituted with CN; C₃₋₆cycloalkyl; C₁₋₆alkyloxy optionally substituted with 1, 2 or 3 independently selected halo substituents; and pyrazolyl; Het represents
   (i) a 5-membered heteroaryl selected from the group consisting of 1H-pyrrolyl; thienyl; furanyl; 1H-pyrazolyl; 1H-imidazolyl; 1,2-oxazolyl; 1,3-oxazolyl; and thiazolyl; each of which may be optionally substituted with 1, 2 or 3 substituents each independently selected from the group consisting of halo; C₁₋₄alkyl optionally substituted with 1, 2, or 3 independently selected halo substituents; NR^{3A}R^{3B} wherein R^{3A} and R^{3B} are each independently selected from H and CH₃; and furan-2-yl; or
   (ii) a 6-membered heteroaryl selected from the group consisting of pyridyl, pyrimidinyl, pyrazinyl, and pyridazinyl; each of which may be optionally substituted with 1, 2 or 3 substituents each independently selected from the group consisting of halo; OH; CN; NR^{4A}R^{4B} wherein R^{4A} and R^{4B} are each independently selected from H and CH₃; C₁₋₄alkyl optionally substituted with 1, 2 or 3 independently selected halo substituents; C₁₋₄alkyl substituted with OH; C₃₋₆cycloalkyl; C₃₋₆cycloalkyloxy; C₁₋₄alkyloxy optionally substituted with 1, 2 or 3 independently selected halo substituents; and C₁₋₄alkyloxyC₁₋₄alkyl; or
   (iii) a 8- to 10-membered bicyclic partially unsaturated heterocyclyl selected from the group consisting of 2,3-dihydro-1-benzofuranyl; 2H-chromenyl; 3,4-dihydro-2H-chromenyl; 2,3-dihydro-1H-indolyl optionally substituted at the 1-position with C₁₋₄alkyl, methylsulfonyl, 1-acetyl, or fluoroacetyl; 2,2-difluoro-1,3-benzodioxolyl; 1,3-benzodioxolyl optionally substituted with a methyl substituent; 3,4-dihydro-2H-1,4-benzoxazinyl optionally substituted with C₁₋₄alkyl; 5,6,7,8-tetrahydroimidazo[1,2-a]pyridinyl; 5,6,7,8-tetrahydroquinolinyl optionally substituted with a halo substituent; and 2,3-dihydropyrazolo[5,1-b][1,3]oxazolyl; or
   (iv) a 9- to 10-membered bicyclic heteroaryl selected from the group consisting of 1-benzofuranyl; 1-benzothiophenyl; 1H-indolyl; 1,3-benzoxazolyl; 1,3-benzothiazolyl; indolizinyl; 1H-benzimidazolyl; imidazo[1,2-a]pyridinyl; pyrazolo[1,5-a]pyridinyl; 1H-thieno[2,3-c]pyrazolyl; imidazo[2,1-b]thiazolyl; pyrrolo[2,3-c]pyridinyl; thieno[3,2-b]pyridinyl; quinolinyl; isoquinolinyl; quinoxalinyl; 1,8-naphthyridinyl; and 1,6-naphthyridinyl; each of which may be optionally substituted with 1 or 2 substituents each independently selected from the group consisting of halo; OH; NR^{5A}R^{5B} wherein R^{5A} and R^{5B} are each independently selected from H and CH₃; C₁₋₄alkyl optionally substituted with 1, 2 or 3 independently selected halo substituents; and C₁₋₄alkyloxy optionally substituted with 1, 2 or 3 independently selected halo substituents;
   with the proviso that the compound is not 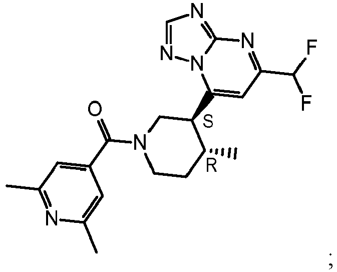 and the N-oxides, and the pharmaceutically acceptable salts and the solvates thereof.

Illustrative of the invention is a pharmaceutical composition comprising a pharmaceutically acceptable carrier and a compound of Formula (I) as described herein, or a pharmaceutically acceptable salt or a solvate thereof. An illustration of the invention is a pharmaceutical composition made by mixing a compound of Formula (I) as described herein, or a pharmaceutically acceptable salt or a solvate thereof, and a pharmaceutically acceptable carrier. Illustrating the invention is a process for making a pharmaceutical composition comprising mixing a compound of Formula (I) as described herein, or a pharmaceutically acceptable salt or a solvate thereof, and a pharmaceutically acceptable carrier.

Further illustrative of the invention are methods to enhance neuronal plasticity comprising administering to a subject in need thereof a therapeutically effective amount of a compound of Formula (I) as described herein, or a pharmaceutically acceptable salt or a solvate thereof, or pharmaceutical compositions described herein.

Exemplifying the invention are methods of treating a disorder mediated by the PDE2 enzyme, comprising administering to a subject in need thereof a therapeutically effective amount of a compound of Formula (I) as described herein, or a pharmaceutically acceptable salt or a solvate thereof, or pharmaceutical compositions described herein.

Further exemplifying the invention are methods of inhibiting the PDE2 enzyme, comprising administering to a subject in need thereof a therapeutically effective amount of a compound of Formula (I) as described herein, or a pharmaceutically acceptable salt or a solvate thereof, or pharmaceutical compositions described herein.

An example of the invention is a method of treating a disorder selected from the group consisting of neurological and psychiatric disorders, comprising administering to a subject in need thereof, a therapeutically effective amount of a compound of Formula (I) as described herein, or a pharmaceutically acceptable salt or a solvate thereof, or pharmaceutical compositions described herein.

An example of the invention is a method of treating a disorder selected from the group of neurological and psychiatric disorders selected from psychotic disorders and conditions; anxiety disorders; movement disorders; drug abuse; mood disorders; neurodegenerative disorders; disorders or conditions comprising as a symptom a deficiency in attention and/or cognition; stroke; and autistic disorders, comprising administering to a subject in need thereof, a therapeutically effective amount of a compound of Formula (I) as described herein, or a pharmaceutically acceptable salt or a solvate thereof, or a pharmaceutically acceptable salt or a solvate thereof or pharmaceutical compositions described herein.

An example of the invention is a method of treating a disorder selected from the group consisting of neurological and psychiatric disorders comprising administering to a subject in need thereof, a therapeutically effective amount of a compound of Formula (I) as described herein or a pharmaceutically acceptable salt or a solvate thereof, or pharmaceutical compositions described herein.

An example of the invention is a method of treating a disorder selected from the group of neurological and psychiatric disorders selected from psychotic disorders and conditions; anxiety disorders; movement disorders; drug abuse; mood disorders; neurodegenerative disorders; disorders or conditions comprising as a symptom a deficiency in attention and/or cognition; disorders related to memory acquisition and consolidation; stroke; and autistic disorders, comprising administering to a subject in need thereof, a therapeutically effective amount of a compound of Formula (I) or a salt or a solvate thereof, or pharmaceutical compositions described herein.

Also exemplifying the invention is a compound of Formula (I) or a salt or a solvate thereof, or a pharmaceutical composition described herein, for use as a medicament.

Further exemplifying the invention is a compound of Formula (I) or a salt or a solvate thereof, or a pharmaceutical composition according to the invention for use in the treatment, prevention, amelioration, control or reduction of the risk of various neurological and psychiatric disorders associated with phosphodiesterase 2 dysfunction in a mammal, including a human, the treatment or prevention of which is affected or facilitated by the inhibition of phosphodiesterase 2.

An example of the invention is a compound of Formula (I) or a pharmaceutically acceptable salt or a solvate thereof according to the present invention or a pharmaceutical composition according to the invention for use in the treatment, prevention, amelioration, control or reduction of the risk of various disorders selected from psychotic disorders and conditions; anxiety disorders; movement disorders; drug abuse; mood disorders; neurodegenerative disorders; disorders or conditions comprising as a symptom a deficiency in attention and/or cognition; disorders related to memory acquisition and consolidation; stroke; and autistic disorder.

An example of the invention is a method of treating a disorder selected from the group consisting of Alzheimer's disease, mild cognitive impairment, senility, dementia, dementia with Lewy bodies, Down's syndrome, dementia associated with stroke, dementia associated with Parkinson's disease and dementia associated with beta-amyloid, preferably Alzheimer's disease, comprising administering to a subject in need thereof, a therapeutically effective amount of a compound of Formula (I) or a pharmaceutically acceptable salt or a solvate thereof, or pharmaceutical compositions described herein.

Another example of the invention is a compound of Formula (I) or a pharmaceutically acceptable salt or a solvate thereof described herein for use in treating: (a) Alzheimer's Disease, (b) mild cognitive impairment, (c) senility, (d) dementia, (e) dementia with Lewy bodies, (f) Down's syndrome, (g) dementia associated with stroke, (h) dementia associated with Parkinson's disease, (i) dementia associated with beta-amyloid, (j) depressive disorders and (k) anxiety disorders, in a subject in need thereof.

### DESCRIPTION OF THE FIGURES

Figure 1 shows the effect of compound 110 on weak HFS-induction of long term potentiation (LTP) at the mossy fiber synapse.
Figure 2 shows the experimental design for theta-burst stimulation.
Figure 3 shows the effect of compound 70 on weak HFS-induction of long term potentiation (LTP) at the mossy fiber synapse.
Figure 4 shows the effect of compound 25a on weak HFS-induction of long term potentiation (LTP) at the mossy fiber synapse.
Figure 5 shows the effect of compound 220 on weak HFS-induction of long term potentiation (LTP) at the mossy fiber synapse.
Figures 6 to 15 (compounds 220, 110, 93, 32, 70, 25a, 281, 295, 356, and 335, respectively) show measurement of cGMP levels in CSF in Marshall Beagle dogs. In

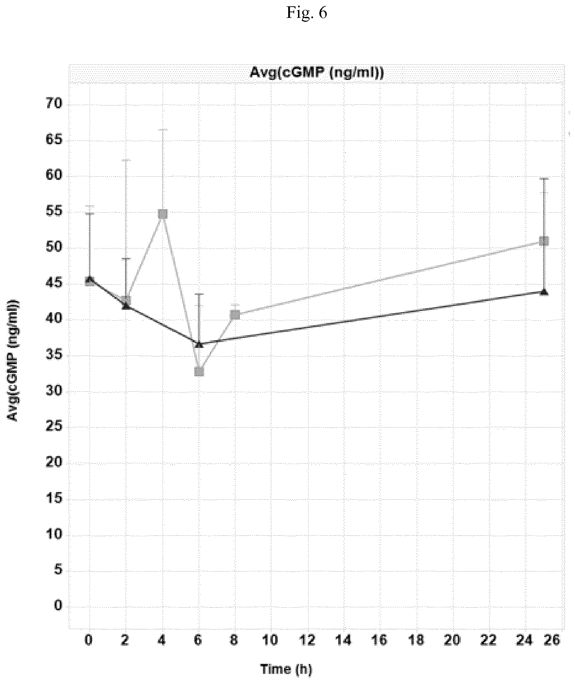

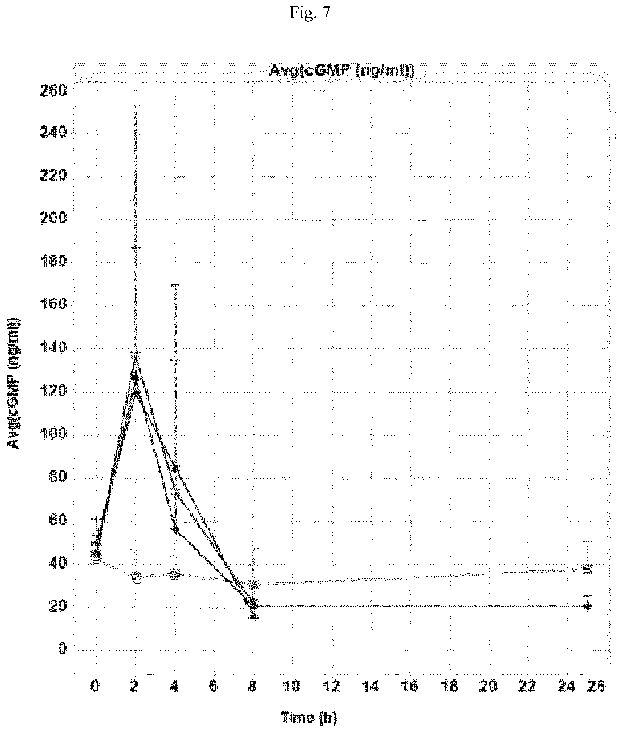

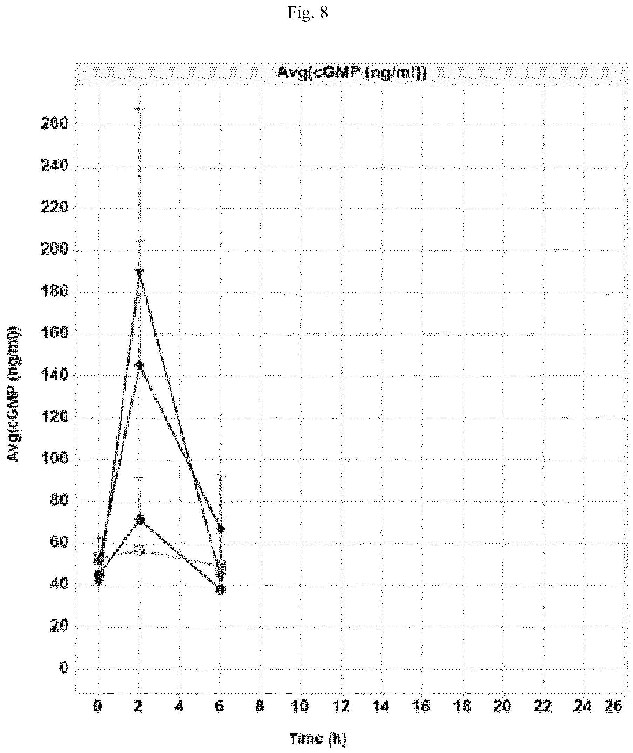

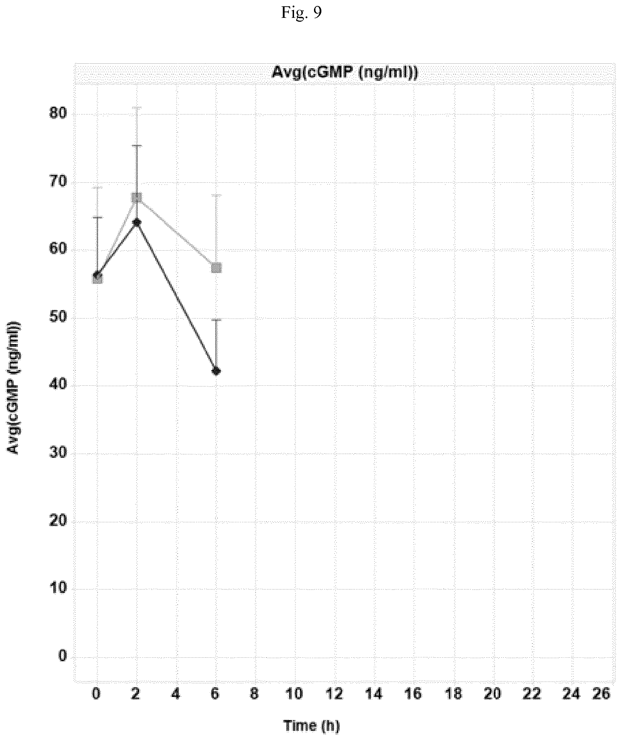

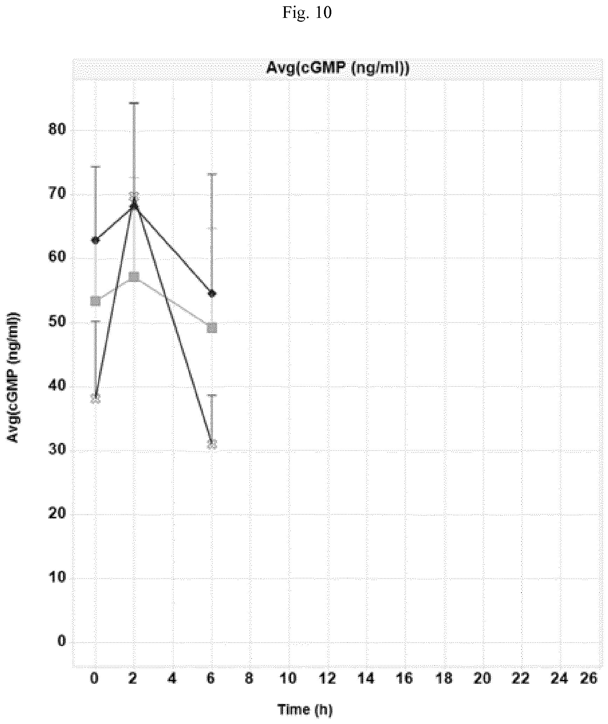

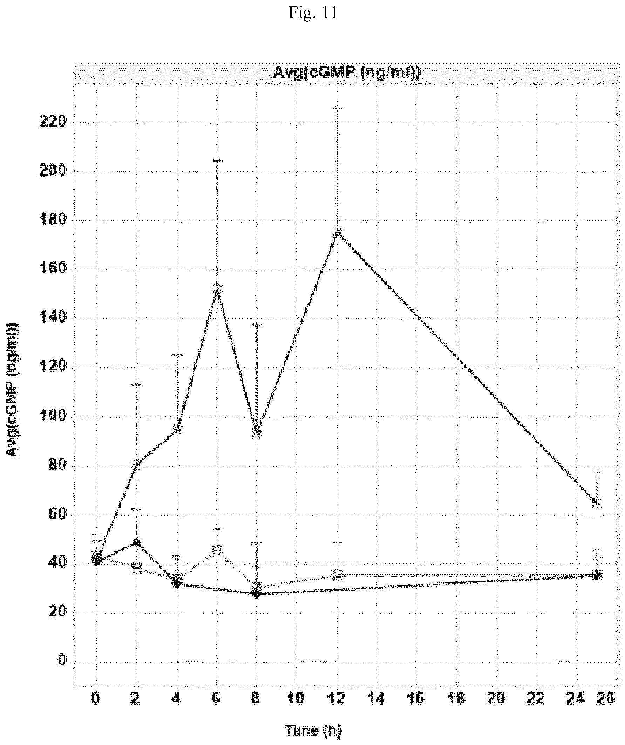

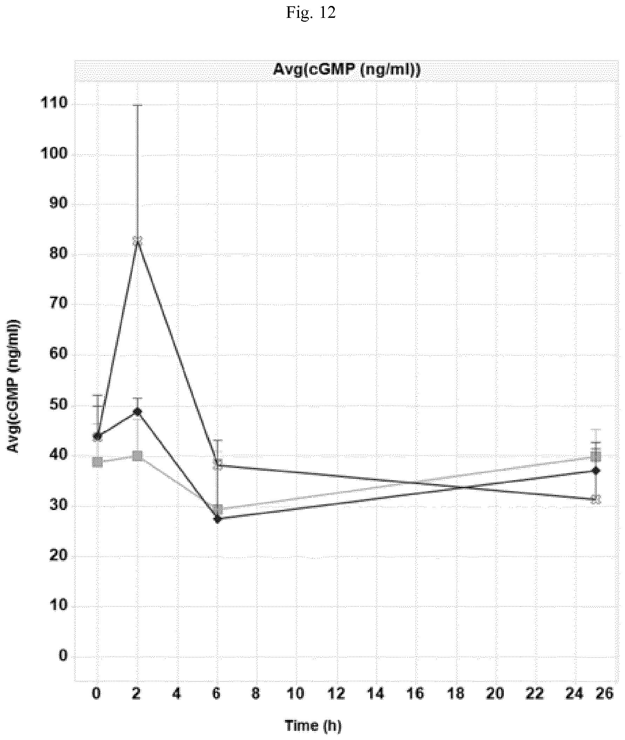

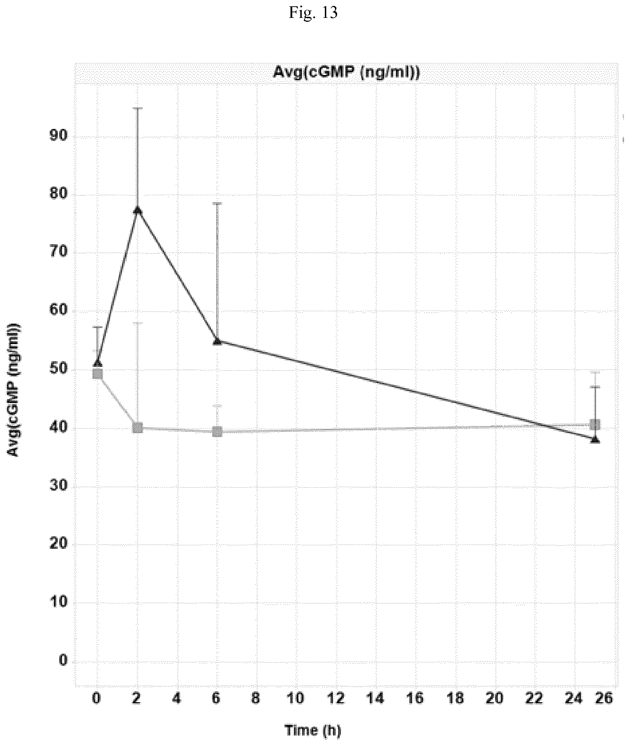

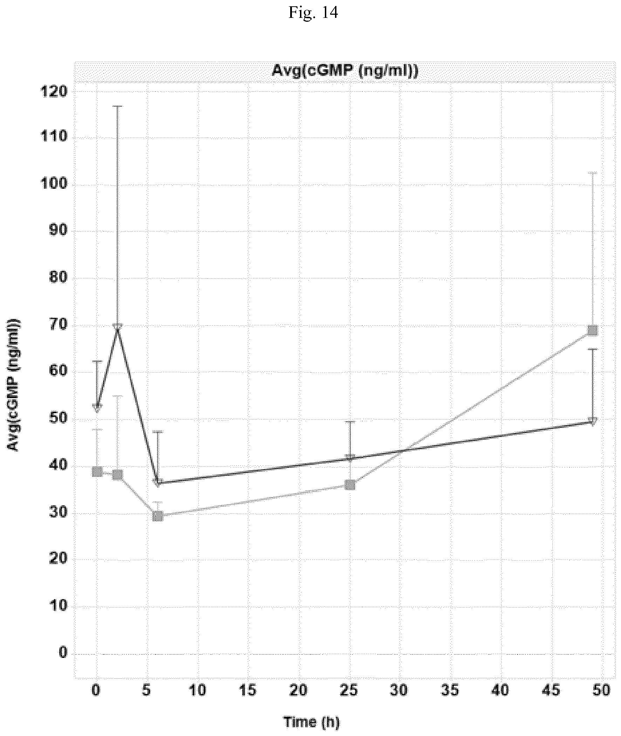

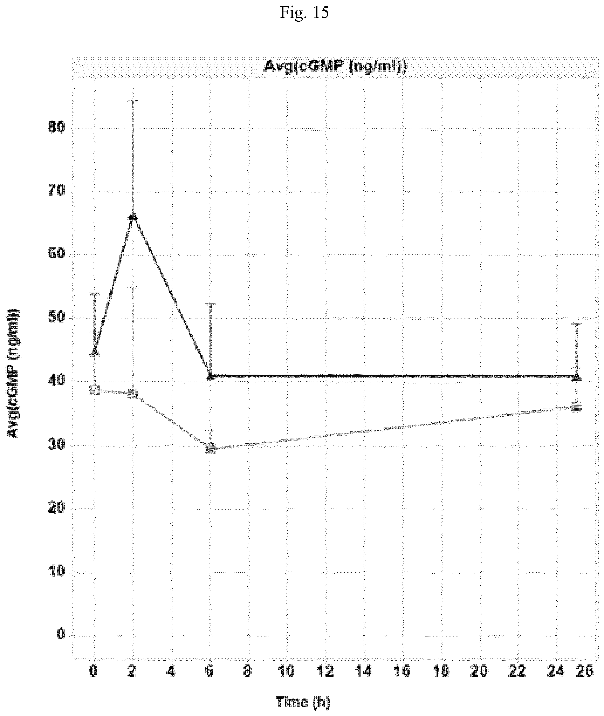

Figures 6 to 15, the different doses of the test compounds are represented as follows: ■ 0 mg/kg; ● 0.5 mg/kg; ◆ 0.75 mg/kg ▲ 1 mg/kg; ▼ 1.25 mg/kg; × 1.5 mg/kg; ∇ 2.5 mg/kg.

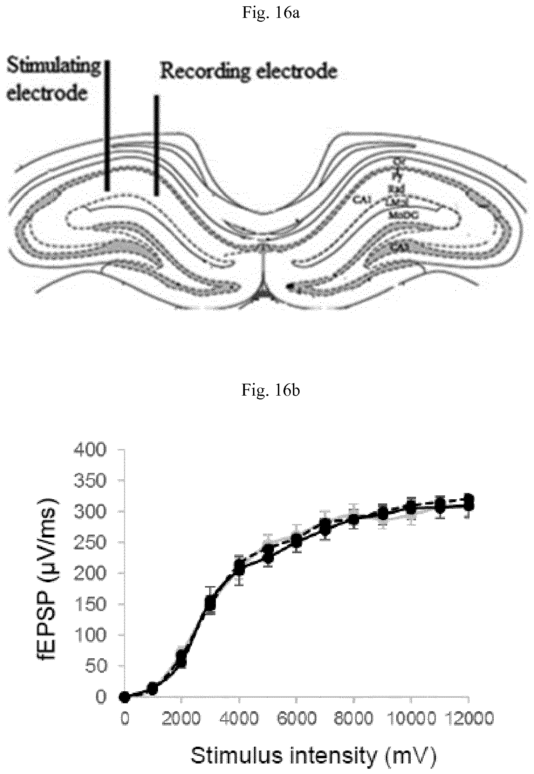

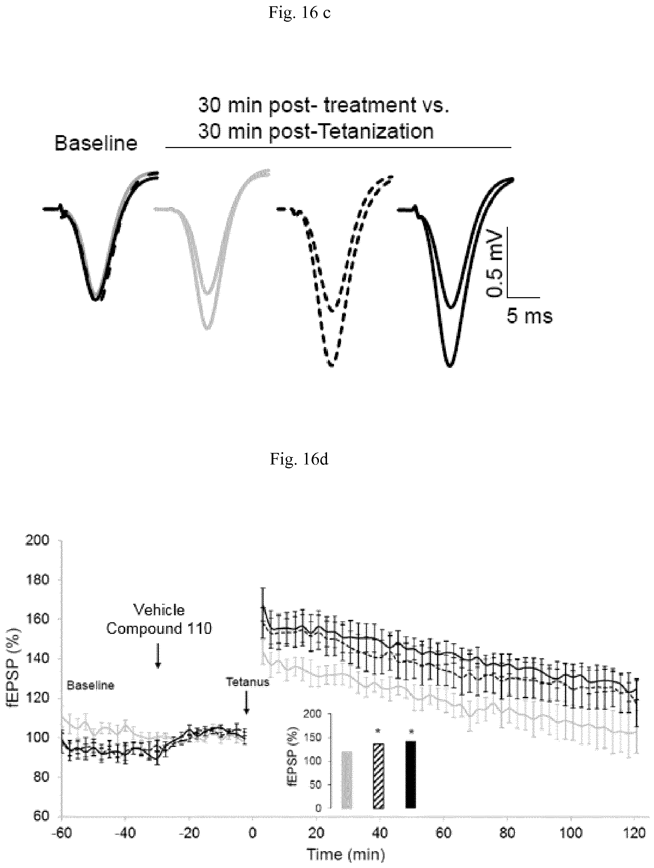

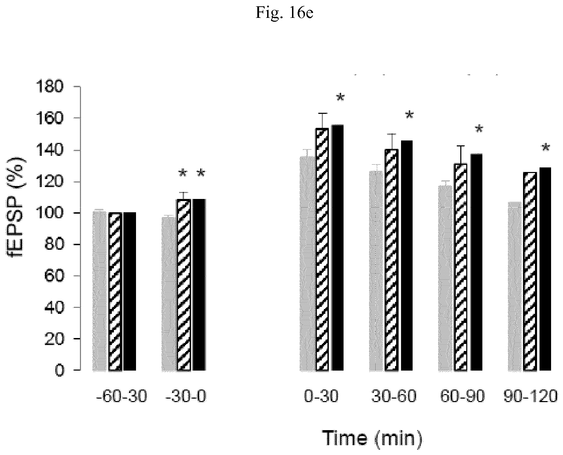

Figure 16 shows the acute effects of compound 110 on synaptic function in vivo at the Schaffer Collateral (SC)-CA1 synapse. Fig. 16a shows a schematic representation of the location of the bipolar stimulating electrode in the Schaffer Collateral path and of the monopolar recording electrode in the stratum radiatum of the *Cornu Ammonis* CA1 area of the hippocampus; Fig. 16b shows a summary of I/O curves generated by applying stimuli of increasing intensity and measuring the initial slopes of the resulting fEPSP revealed no difference in basic excitability of the SC-CA1 pathway; Fig. 16c shows response morphologies during baseline, 30 min post-treatment and 30 min post-tetanic HFS protocol. Note similarity in the magnitude of fEPSP responses during baseline recordings between treatment groups, which increased above baseline and vehicle condition in the compound 110-treated group; Fig. 16d shows the time course of fEPSP slope after 30 min of baseline recording, 30 min after the administration of compound 110, and tetanic HFS protocol was applied and slope of fEPSP was recorded for other 2 hours. Note that compound 110 at the dose of 20 and 40 mg/kg enhanced basal synaptic transmission prior the tetanic HFS protocol and the slope of the fEPSPs throughout the 2-hours post-HFS protocol (Inset plot); Fig. 16e shows that when fEPSPs were averaged in 30 min periods, compound 110 (40 mg/kg) enhanced the magnitude of synaptic transmission and the difference with the vehicle-treated group remained significant. Values are expressed as a percentage of the values recorded before HFS and results are presented as means ± SEM. One-way analysis of variance (ANOVA) and least significant difference (LSD) post-hoc analysis tests were applied for group comparisons.

In Fig. 16b and c the patterns have the following meanings: 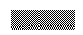 Vehicle (n = 10); ■ ■ compound 110 (mg/kg) (20) n = 9; 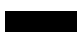 compound 110 (mg/kg) (40) n = 8; in Fig. 16d and 16e the patterns have the following meanings: ■ vehicle (n = 10); 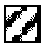 compound 110 (mg/kg), (20) n = 9; ■ compound 110 (mg/kg) (40) n = 8

### DETAILED DESCRIPTION OF THE INVENTION

### DEFINITIONS

"C₁₋₄alkyl" as used herein alone or as part of another group, defines a saturated, straight or branched, hydrocarbon radical having, 1, 2, 3 or 4 carbon atoms, such as methyl, ethyl, 1-propyl, 1-methyl, butyl, 1-methyl-propyl, 2-methyl-1-propyl, 1,1-dimethylethyl and the like. The term "C₁₋₆alkyl" as used herein as a group or part of a group represents a straight or branched chain saturated hydrocarbon radical having from 1 to 6 carbon atoms such as the groups defined for C₁₋₄alkyl and *n*-pentyl, *n*-hexyl, 2-methylbutyl and the like. "C₁₋₄alkyloxy" shall denote an ether radical wherein C₁₋₄alkyl is as defined herein. "C₁₋₆alkyloxy" shall denote an ether radical wherein C₁₋₆alkyl is as defined herein. "Halo" shall denote fluoro, chloro and bromo. "C₃₋₆cycloalkyl" shall denote cyclopropyl, cyclobutyl, cyclopentyl, and cyclohexyl. "C₃₋₆cycloalkyloxy" shall denote an ether radical wherein C₃₋₆cycloalkyl is as defined herein.

Whenever the term "substituted" is used in the present invention, it is meant, unless otherwise indicated or is clear from the context, to indicate that one or more hydrogens, preferably from 1 to 3 hydrogens, or from 1 to 2 hydrogens, or 1 hydrogen, on the atom or radical indicated in the expression using "substituted" is replaced with a selection from the indicated group, provided that the normal valency is not exceeded, and that the substitution results in a chemically stable compound, i.e. a compound that is sufficiently robust to survive isolation to a useful degree of purity from a reaction mixture, and formulation into a therapeutic agent.

The N-oxide forms of the compounds according to Formula (I) are meant to comprise those compounds of Formula (I) wherein one or several nitrogen atoms are oxidized to the so called N-oxide, particularly those N-oxides wherein a nitrogen atom in a pyridinyl radical is oxidized. N-oxides can be formed following procedures known to the skilled person. The N-oxidation reaction may generally be carried out by reacting the starting material of Formula (I) with an appropriate organic or inorganic peroxide. Appropriate inorganic peroxides comprise, for example, hydrogen peroxide, alkali metal or alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chloroperoxybenzoic acid (or 3-chloroperbenzoic acid), peroxoalkanoic acids, e.g. peroxoacetic acid, alkylhydroperoxides, e.g. tert-butyl hydroperoxide. Suitable solvents, e.g are for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

Thus, in a particular embodiment, the invention relates to a compound of Formula (I) wherein R³ is Het, and Het is an oxide of an optionally substituted pyridyl radical as described herein, i.e. an optionally substituted pyridiniumyl oxide radical.

The term "subject" as used herein, refers to an animal, preferably a mammal, most preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.
Hereinbefore and hereinafter, the term "compound of formula (I)" is meant to include the addition salts, the solvates and the stereoisomers thereof.
The terms "stereoisomers" or "stereochemically isomeric forms" hereinbefore or hereinafter are used interchangeably.

The invention includes all stereoisomers of the compound of Formula (I) either as a pure stereoisomer or as a mixture of two or more stereoisomers.
Enantiomers are stereoisomers that are non-superimposable mirror images of each other. A 1: 1 mixture of a pair of enantiomers is a racemate or racemic mixture. Diastereomers (or diastereoisomers) are stereoisomers that are not enantiomers, i.e. they are not related as mirror images. Therefore, the invention includes enantiomers, diastereomers, racemates.
In the compounds according to the invention, bonds shown with a wedge of parallel lines ( 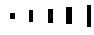 ) represent bonds projected below the plane of the drawing, while bonds shown with a bold wedge ( 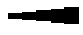 ) represent bonds projected above the plane of the drawing.
The absolute configuration is specified according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer.

Furthermore, some of the crystalline forms for the compounds of the present invention may exist as polymorphs and as such are intended to be included in the present invention. In addition, some of the compounds of the present invention may form solvates with water (i.e., hydrates) or common organic solvents, and such solvates are also intended to be encompassed within the scope of this invention.

For use in medicine, the salts of the compounds of this invention refer to nontoxic "pharmaceutically acceptable salts". Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

Representative acids which may be used in the preparation of pharmaceutically acceptable salts include, but are not limited to, the following: acetic acid, 2,2-dichloroacetic acid, acylated amino acids, adipic acid, alginic acid, ascorbic acid, L-aspartic acid, benzenesulfonic acid, benzoic acid, 4- acetamidobenzoic acid, (+)-camphoric acid, camphorsulfonic acid, capric acid, caproic acid, caprylic acid, cinnamic acid, citric acid, cyclamic acid, ethane-1,2-disulfonic acid, ethanesulfonic acid, 2-hydroxyethanesulfonic acid, formic acid, fumaric acid, galactaric acid, gentisic acid, glucoheptonic acid, D-gluconic acid, D-glucoronic acid, L-glutamic acid, beta-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5- disulfonic acid, 1-hydroxy-2-naphthoic acid, nicotinic acid, nitric acid, oleic acid, orotic acid, oxalic acid, palmitic acid, pamoic acid, phosphoric acid, L- pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, trifluoromethylsulfonic acid, and undecylenic acid. Representative bases which may be used in the preparation of pharmaceutically acceptable salts include, but are not limited to, the following: ammonia, L-arginine, benethamine, benzathine, calcium hydroxide, choline, dimethylethanolamine, diethanolamine, diethylamine, 2-(diethylamino)-ethanol, ethanolamine, ethylenediamine, *N*-methyl-glucamine, hydrabamine, 1*H*-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The names of the compounds of the present invention were generated according to the nomenclature rules agreed upon by the Chemical Abstracts Service (CAS) using Advanced Chemical Development, Inc., software (ACD/Name product version 10.01; Build 15494, 1 Dec 2006 or ACD/ChemSketch product version 12.5; Build 47877, 20 Apr 2011) or according to the nomenclature rules agreed upon by the International Union of Pure and Applied Chemistry (IUPAC) using Advanced Chemical Development, Inc., software (ACD/Name product version 10.01.0.14105, October 2006). In case of tautomeric forms, the name of the depicted tautomeric form of the structure was generated. The other non-depicted tautomeric form is also included within the scope of the present invention.

The present invention is directed to compounds of Formula (I) as defined hereinbefore and pharmaceutically acceptable salts and solvates thereof.

In an embodiment, the present invention is directed to compounds of Formula (I) 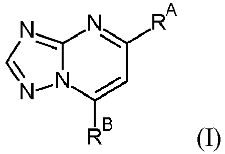 and the stereoisomeric forms thereof, wherein
R^{A} is selected from the group consisting of H, CH₃, CN, and CHF₂;
R^{B} is a radical selected from the group consisting of (a), (b) and (c): 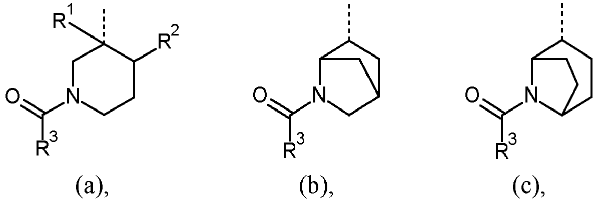 wherein
   R¹ is H, F or CH₃;
   R² is H or C₁₋₄alkyl, in particular methyl or n-butyl; with the proviso that when R² is H, then R¹ is F or CH₃;
   R³ is Ar or Het; wherein
Ar represents phenyl optionally substituted with 1, 2 or 3 substituents, each independently selected from the group consisting of halo; CN; OH; C₁₋₆alkyl optionally substituted with 1, 2 or 3 independently selected halo substituents; C₁₋₆alkyl substituted with CN; C₃₋₆cycloalkyl; and C₁₋₆alkyloxy optionally substituted with 1, 2 or 3 independently selected halo substituents;
Het represents
   (i) a 5-membered heteroaryl selected from the group consisting of 1H-pyrrolyl; thienyl; furanyl; 1H-pyrazolyl; 1H-imidazolyl; 1,2-oxazolyl; 1,3-oxazolyl; and thiazolyl; each of which may be optionally substituted with 1, 2 or 3 substituents each independently selected from the group consisting of halo; C₁₋₄alkyl optionally substituted with 1, 2, or 3 independently selected halo substituents; NR^{3A}R^{3B} wherein R^{3A} and R^{3B} are each independently selected from H and CH₃; and furan-2-yl; or
   (ii) a 6-membered heteroaryl selected from the group consisting of pyridyl, pyrimidinyl, pyrazinyl, and pyridazinyl; each of which may be optionally substituted with 1, 2 or 3 substituents each independently selected from the group consisting of halo; OH; CN; NR^{4A}R^{4B} wherein R^{4A} and R^{4B} are each independently selected from H and CH₃; C₁₋₄alkyl optionally substituted with 1, 2 or 3 independently selected halo substituents; C₃₋₆cycloalkyl; C₃₋₆cycloalkyloxy; and C₁₋₄alkyloxy optionally substituted with 1, 2 or 3 independently selected halo substituents; or
   (iii) a 8- to 10-membered bicyclic partially unsaturated heterocyclyl selected from the group consisting of 2,3-dihydro-1-benzofuranyl; 2H-chromenyl; 3,4-dihydro-2H-chromenyl; 2,3-dihydro-1H-indolyl optionally substituted at the 1-position with C₁₋₄alkyl, methylsulfonyl, 1-acetyl, or fluoroacetyl; 2,2-difluoro-1,3-benzodioxolyl; 1,3-benzodioxolyl optionally substituted with a methyl substituent; 5,6,7,8-tetrahydroimidazo[1,2-a]pyridinyl; 5,6,7,8-tetrahydroquinolinyl optionally substituted with a halo substituent; and 2,3-dihydropyrazolo[5,1-b][1,3]oxazolyl; or
   (iv) a 9- to 10-membered bicyclic heteroaryl selected from the group consisting of 1-benzofuranyl; 1-benzothiophenyl; 1H-indolyl; 1,3-benzoxazolyl; 1,3-benzothiazolyl; indolizinyl; 1H-benzimidazolyl; imidazo[1,2-a]pyridinyl; pyrazolo[1,5-a]pyridinyl; 1H-thieno[2,3-c]pyrazolyl; thieno[3,2-b]pyridinyl; quinolinyl; 1,8-naphthyridinyl; and 1,6-naphthyridinyl; each of which may be optionally substituted with 1 or 2 substituents each independently selected from the group consisting of halo; OH; NR^{5A}R^{5B} wherein R^{5A} and R^{5B} are each independently selected from H and CH₃; C₁₋₄alkyl optionally substituted with 1, 2 or 3 independently selected halo substituents; and C₁₋₄alkyloxy optionally substituted with 1, 2 or 3 independently selected halo substituents;
   with the proviso that the compound is not 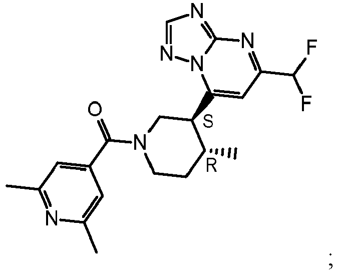 and the pharmaceutically acceptable salts and the solvates thereof.

In a particular embodiment, R^{A} is CH₃ or CHF₂; and the rest of variables are as defined herein.

In a particular embodiment, R^{B} is selected from the group consisting of (a) and (c) and the rest of variables are as defined herein.

In a particular embodiment, R^{A} is CH₃ or CHF₂; R^{B} is selected from the group consisting of (a) and (c) and the rest of variables are as defined herein.

In a particular embodiment, R¹ is H and R² is C₁₋₄alkyl, in particular methyl or n-butyl; and the rest of variables are as defined herein.

In a particular embodiment, R³ is Het and the rest of variables are as defined herein.

In a particular embodiment, R³ is
(i) a 6-membered heteroaryl selected from the group consisting of pyridyl, pyrimidinyl, pyrazinyl, and pyridazinyl; each of which may be optionally substituted with 1, 2 or 3 substituents each independently selected from the group consisting of halo; OH; CN; NR^{4A}R^{4B} wherein R^{4A} and R^{4B} are each independently selected from H and CH₃; C₁₋₄alkyl optionally substituted with 1, 2 or 3 independently selected halo substituents; C₃₋₆cycloalkyl; C₃₋₆cycloalkyloxy; and C₁₋₄alkyloxy optionally substituted with 1, 2 or 3 independently selected halo substituents; or
(ii) a 9- to 10-membered bicyclic heteroaryl selected from the group consisting of 1-benzofuranyl; 1-benzothiophenyl; 1H-indolyl; 1,3-benzoxazolyl; 1,3-benzothiazolyl; indolizinyl; 1H-benzimidazolyl; imidazo[1,2-a]pyridinyl; pyrazolo[1,5-a]pyridinyl; 1H-thieno[2,3-c]pyrazolyl; thieno[3,2-b]pyridinyl; quinolinyl; 1,8-naphthyridinyl; and 1,6-naphthyridinyl; each of which may be optionally substituted with 1 or 2 substituents each independently selected from the group consisting of halo; OH; NR^{5A}R^{5B} wherein R^{5A} and R^{5B} are each independently selected from H and CH₃; C₁₋₄alkyl optionally substituted with 1, 2 or 3 independently selected halo substituents; and C₁₋₄alkyloxy optionally substituted with 1, 2 or 3 independently selected halo substituents;
and the rest of variables are as defined herein.

In a further embodiment, R³ is
(i) a 6-membered heteroaryl selected from the group consisting of pyridyl, pyrimidinyl, pyrazinyl, and pyridazinyl; each of which may be optionally substituted with 1, 2 or 3 substituents each independently selected from the group consisting of halo; NR^{4A}R^{4B} wherein R^{4A} and R^{4B} are each independently selected from H and CH₃;
   C₁₋₄alkyl optionally substituted with 1, 2 or 3 independently selected halo substituents; C₃₋₆cycloalkyl; C₃₋₆cycloalkyloxy; and C₁₋₄alkyloxy optionally substituted with 1, 2 or 3 independently selected halo substituents; or
(ii) a 9- to 10-membered bicyclic heteroaryl selected from the group consisting of 1-benzofuranyl; 1-benzothiophenyl; 1H-indolyl; 1,3-benzoxazolyl; 1,3-benzothiazolyl; indolizinyl; 1H-benzimidazolyl; imidazo[1,2-a]pyridinyl; pyrazolo[1,5-a]pyridinyl; 1H-thieno[2,3-c]pyrazolyl; thieno[3,2-b]pyridinyl; quinolinyl; 1,8-naphthyridinyl; and 1,6-naphthyridinyl; each of which may be optionally substituted with 1 or 2 substituents each independently selected from the group consisting of halo; NR^{5A}R^{5B} wherein R^{5A} and R^{5B} are each independently selected from H and CH₃;
C₁₋₄alkyl optionally substituted with 1, 2 or 3 independently selected halo substituents; and C₁₋₄alkyloxy optionally substituted with 1, 2 or 3 independently selected halo substituents;
and the rest of variables are as defined herein.

In an embodiment, the compound of Formula (I) as defined herein, is in particular a compound of Formula (I-a) or (I-b) 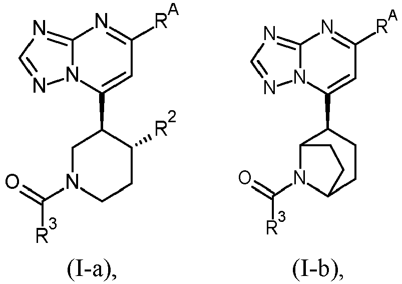 wherein all variables are as defined herein.

More in particular, the compound of Formula (I) as defined herein, has the Formula (I-b 1) 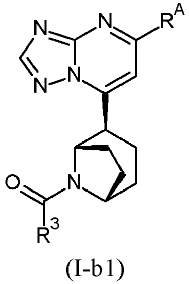 wherein all variables are as defined herein.

In a particular embodiment, R³ is a radical selected from the group consisting of (3-a), (3-b) and (3-c) 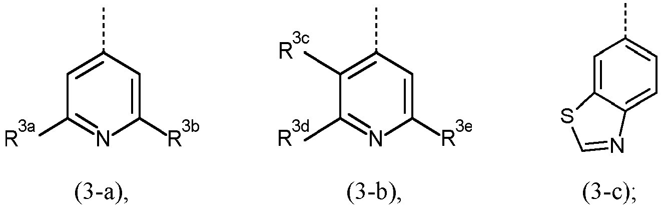 wherein
R^{3A} and R^{3b} are each independently selected from the group consisting of hydrogen; halo; CN; NR^{4a}R^{4b} wherein R^{4a} and R^{4b} are each independently selected from H and CH₃; C₁₋₄alkyl optionally substituted with 1, 2 or 3 independently selected halo substituents; C₃₋₆cycloalkyl; and C₁₋₄alkyloxy optionally substituted with 1, 2 or 3 independently selected halo substituents; and
R^{3c} is selected from the group consisting of F, Cl, C₁₋₃alkyl, cyclopropyl, C₁₋₃alkyloxy, cyclopropyloxy, and CF₃; and R^{3d} and R^{3e} are each independently selected from the group consisting of hydrogen, Cl, CN, C₁₋₃alkyl, cyclopropyl, C₁₋₃alkyloxy, cyclopropyloxy, CHF₂, CF₃, OCHF₂, and OCF₃; with the proviso that R^{3d} and R^{3e} are not simultaneously hydrogen.

In particular, R^{3a} are R^{3b} are each independently selected from the group consisting of hydrogen; chloro; -NH₂; C₁₋₄alkyl; -CF₃; cyclopropyl; -OCH₃; -OCH(CH₃)₂; -OCHF₂; and -OCF₃.

More in particular, R^{3a} are R^{3b} are each independently selected from the group consisting of chloro; C₁₋₄alkyl; -CF₃; cyclopropyl; -OCH₃; -OCH(CH₃)₂; -OCHF₂; and -OCF₃.

In particular, R^{3a} is Cl or CH₃ and R^{3b} is selected from the group consisting of CH₃, -OCH₃, -CF₃, and cyclopropyl.

In an additional embodiment, R^{3a} is Cl or CH₃ and R^{3b} is CH₃ or -OCH₃.

In particular R^{3c} is selected from the group consisting of F, CH₃, and -OCH₃; R^{3d} is selected from the group consisting of hydrogen, CH₃, cyclopropyl, and -OCH₃; and R^{3e} is selected from the group consisting of Cl, CH₃, cyclopropyl, -OCH₃; -OCH(CH₃)₂; cyclopropyloxy; CHF₂, and OCHF₂. In an additional embodiment R^{3c} is F.

In an additional embodiment, the compound according to the invention is a compound having Formula (I-a), wherein R² is C₁₋₄alkyl, in particular methyl or n-butyl.

### PREPARATION OF THE COMPOUNDS

### EXPERIMENTAL PROCEDURE 1

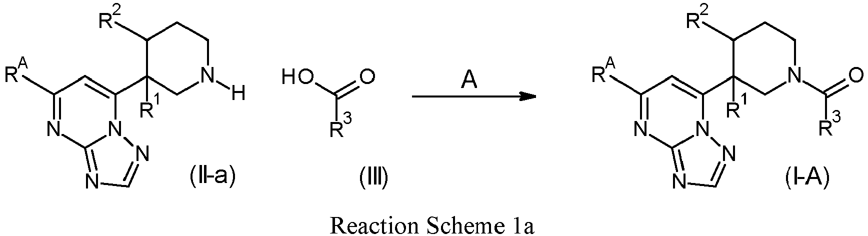

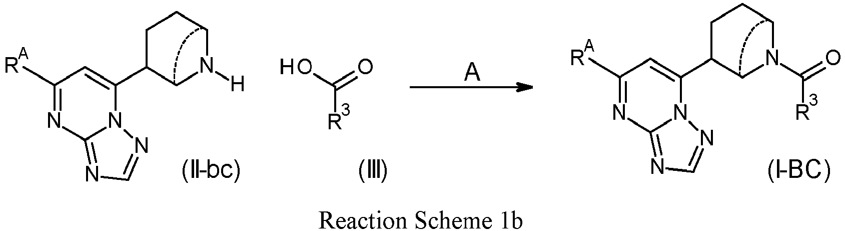

A: amide coupling
   Final compounds wherein R^{B} is a radical of Formula (a) or of Formulae (b) or (c) herein referred to as compounds of Formula (I-A) and Formula (I-BC) whereinrepresents CH₂ or CH₂CH₂, respectively, may be conveniently prepared by reaction with an appropriate carboxylic acid (III) following art-known coupling procedures (reaction step A). Said conversion may conveniently be conducted by treatment of the piperidine type functionality in the intermediates of Formula (II-a) or (II-b) with a coupling agent, such as HBTU or EDCI, in the presence of a base, such as DIPEA and triethylamine in a suitable reaction-inert solvent such as, for example DCM or DMF and the like, at a suitable temperature, for example, at room temperature; alternatively, the intermediates of Formula (II-a) or (II-b) may be reacted with a phosphonic anhydride, such as 1-propane phosphonic anhydride, in the presence of an appropriate base, such as triethylamine, in a suitable reaction-inert solvent, such as, for example, dry ACN. Carboxylic acids of Formula (III) are either commercially available or can be prepared according to art-known procedures.

### EXPERIMENTAL PROCEDURE 2

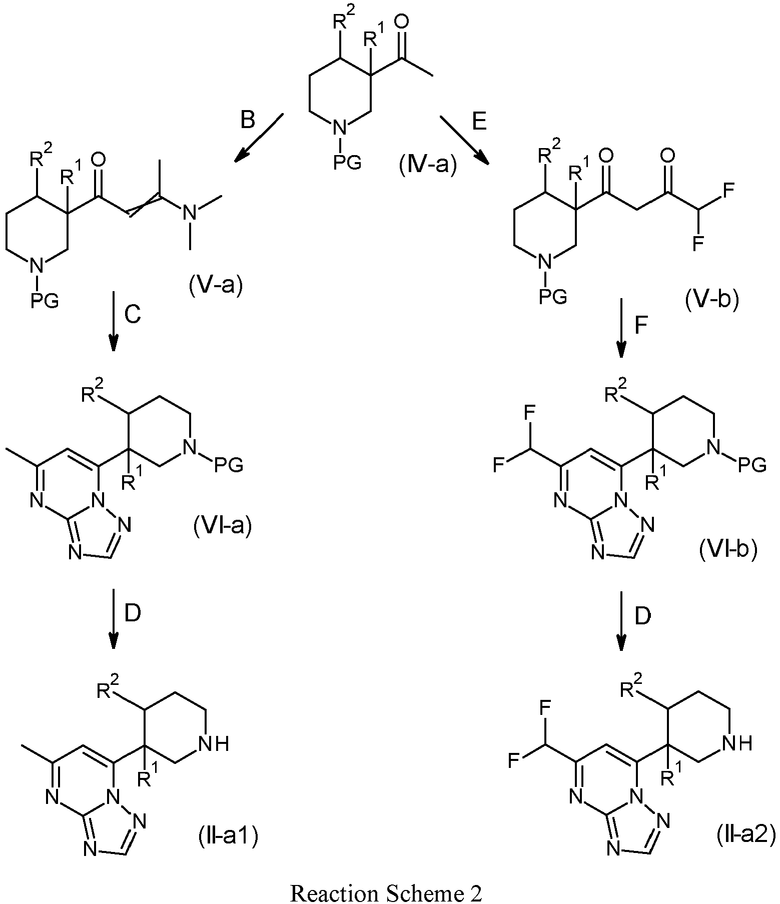

B: reaction with N,N-dimethylacetamide dimethyl acetal
C, F: reaction with 1H-1,2,4-triazol-3-amine hydrochloride
D: protecting group cleavage
E: reaction with 2,2,-difluoro-acetic acid ethyl ester

Formation of intermediates of Formula (II-a), for instance wherein R^{A} is methyl or CHF₂, herein referred to as intermediates of Formula (II-a1) and (II-a2), respectively can be prepared from intermediates of Formula (IV-a), wherein PG is a suitable amino protecting group, such as for example, tert-butyloxycarbonyl (Boc).

The reaction with N,N-dimethylacetamide dimethyl acetal can be performed neat, under thermal conditions, such as for example, heating at 100 °C.

The reaction with 2,2-difluoroacetic acid ethyl ester can be performed in the presence of a base, such as KO^{t}Bu, in a reaction-inert solvent, such as toluene, at an appropriate temperature, such as 0-5 °C, then at RT.

The bicyclic core can be formed by reaction of intermediates (V-a) or (V-b) with 1H-1,2,4-triazol-3-amine hydrochloride in a reaction-inert solvent, such as for example DMF, under thermal conditions, such as for example, heating at 80 °C. In the case of (V-b) to (VI-b), the protecting group may be labile and a subsequent protection step may be required.

The cleavage of the protecting group in intermediates (VI-a) or (VI-b) can be performed according to art-known procedures, for instance, when the protecting group is Boc, the cleavage can be performed under acidic conditions, such as for example HCl in MeOH at RT, or TFA in DCM.

### EXPERIMENTAL PROCEDURE 3

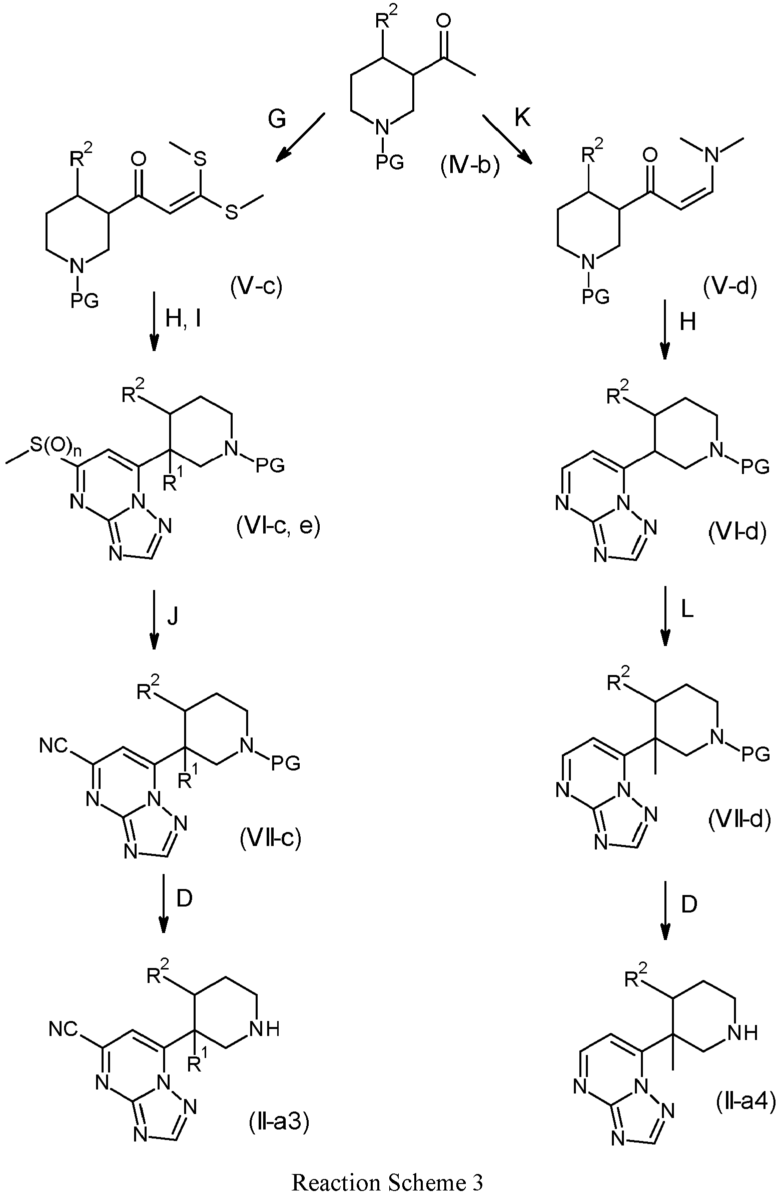

G: reaction with carbon disulphide and methyl iodide
H, L: reaction with 1H-1,2,4-triazol-3-amine hydrochloride
I: oxidation
J: reaction with NaCN
K: reaction with N,N-dimethylformamide dimethyl acetal
L: methylation
D: protecting group cleavage

Intermediates of Formula (II-a), wherein R^{A} is cyano herein referred to as (II-a3) or wherein R^{A} is hydrogen and R¹ is methyl, herein referred to as (II-a4), can be made according to a series of synthetic steps from intermediates of Formula (IV-b), which are either commercially available or made according to art-known procedures (e.g. R² = H or methyl, PG = Boc)

Thus, reaction with carbon disulphide followed by methyl iodide in the presence of a base such as NaH and a reaction-inert solvent such as THF at 0 °C provides (V-c), which can then be reacted with 1H-1,2,4-triazol-5-amine hydrochloride under conditions described herein thereby providing (VI-c, n = 0), followed by oxidation with an appropriate peroxiacid, such as for example mCPBA, in the presence of a reaction-inert solvent, such as for example DCM or CHCl₃, to yield (VI-e, n = 2). Displacement of the methylsulfone group with a source of cyanide, such as for example NaCN in DMSO, affords (VII-c), which can be subjected to cleavage of the protecting group under conditions as described herein.

The reaction of (IV-b) with N,N-dimethylformamide dimethyl acetal can be performed neat under thermal conditions, such as heating at reflux. Reaction with 1H-1,2,4-triazol-3-amine hydrochloride under conditions described herein affords (VI-d), which can be methylated under art-known conditions, such as for example by reacting it with methyl iodide in the presence of a base such as NaH in a reaction-inert solvent such as DMF. Subsequent protecting group cleavage under conditions as described herein, afford intermediate (II-a4).

### EXPERIMENTAL PROCEDURE 4

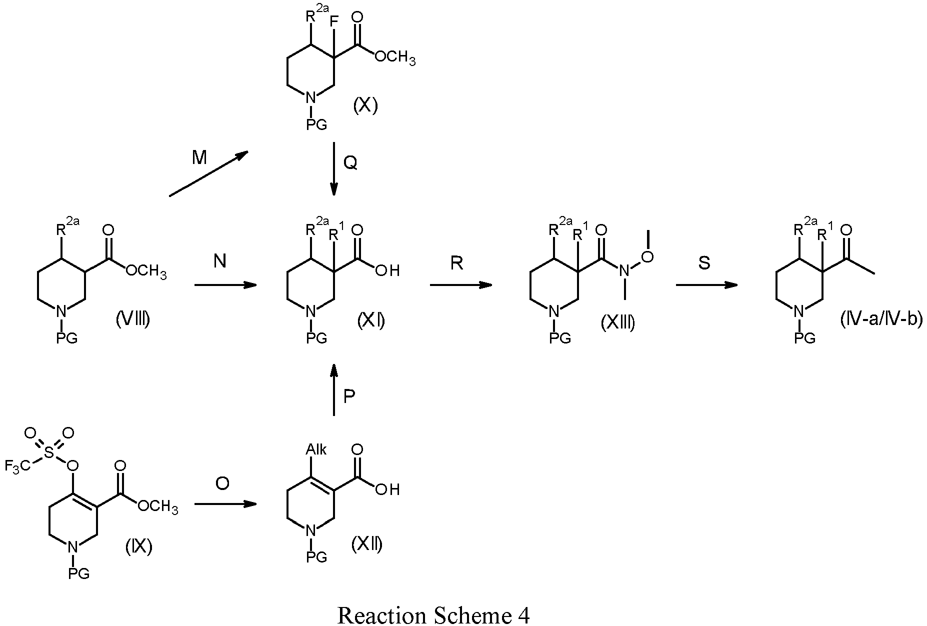

M: fluorination
N: methylation and/or saponification
X: reduction
O: Suzuki (alkylation) and saponification
P: hydrogenation
Q: saponification
R: Weinreb amide formation
S: amide to ketone conversion (e.g. Grignard)

The formation of intermediates (IV-a) or (IV-b) can be performed by a series of functional group interconversions, starting from intermediates (VIII), (IX) or (X) which are either commercially available, or can be prepared for example, according to procedures such as those described herein.

Compounds of Formula (VIII), wherein R^{2a} is hydrogen or methyl, and PG is Boc are either commercially available or made according to a series of known procedures, such as those described herein. They can be fluorinated or alkylated according to art-known procedures, such as by reaction with N-fluorobenzensulfonimide in the presence of a base such as LDA in a reaction-inert solvent such as THF, or by alkylation with alkyl iodide after treatment with a base such as LiHMDS; optionally, subsequent saponification under conditions known in the art, afford (XI).

Compounds of Formula (IX) are also known in the art, and can be alkylated, by means of Suzuki-type procedures, using conditions known to the skilled person, such as the use of a boronic acid/ester, in the presence of a catalyst, such as Pd(PPh₃)₄, in a reaction-inert solvent, such as 1,4-dioxane, under thermal conditions, such as heating. Subsequent saponification and hydrogenation under conditions analogous to those described herein, yield intermediate of Formula (XI).

Subsequent Weinreb amide formation and amide to ketone conversion with Grignard, as described herein, afford the desired intermediates (IV-a) or (IV-b).

### EXPERIMENTAL PROCEDURE 5

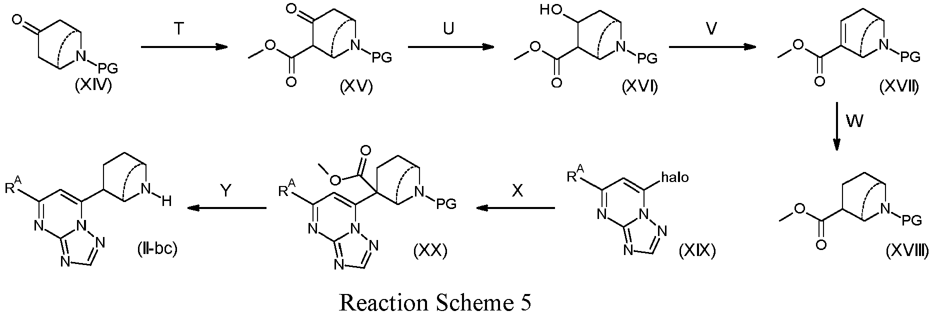

T: reaction with methyl cyanoformate
U: reduction
V: dehydration
W: hydrogenation
X: coupling
Y: decarboxylation and protecting group cleavage

Formation of intermediates of Formula (II-bc), wherein ---- represents CH₂ or CH₂CH₂ can be prepared by a series of synthetic steps starting from commercially available starting materials of Formula (XIV), such as N-Boc-nortropinone [185099-67-6] or 6-Boc-3-oxo-6-azabicyclo[3.1.1]heptane [1246281-86-6]. Reaction with methyl cyanoformate in the presence of a base such as nBuLi and NHⁱPr₂ in a reaction-inert solvent, such as THF at an appropriate temperature, such as at -78 °C, affords ketoester (XV), which then can be reduced under art-known conditions with NaBH₄, for example in MeOH at about 0 °C and subsequently dehydrated with for example, trifluoroacetic anhydride in the presence of a base such as triethylamine and DMAP in a reaction inert solvent such as DCM, keeping the temperature below 60 °C. Hydrogenation under art-known conditions, such as for example in the presence of palladium on carbon catalyst in MeOH affords intermediate (XVIII), which can then be reacted with intermediates of Formula (XIX) which are either commercially available or made according to art-known procedures, in the presence of a base such as for example LDA, in a reaction-inert solvent, such as THF at a temperature between -78 to -60 °C. Reaction with concentrated HCl under thermal conditions, such as for example, heating at 150 °C renders intermediate (II-bc) with concomitant cleavage of the protecting group, when acid labile, such as for example, Boc. An alternative manner of making an intermediate of Formula (II-bc) wherein R^{A} is CHF₂ and --- is CH₂CH₂ from a commercially available starting material, is described herein in the examples.

### PHARMACOLOGY

The compounds according to the invention inhibit PDE2 enzyme activity, in particular PDE2A, and hence raise the levels of cAMP or cGMP within cells that express PDE2. Accordingly, inhibition of PDE2 enzyme activity may be useful in the treatment of diseases caused by deficient amounts of cAMP or cGMP in cells. PDE2 inhibitors may also be of benefit in cases in which raising the amount of cAMP or cGMP above normal levels results in a therapeutic effect. Inhibitors of PDE2 may be used to treat neurological and psychiatric disorders.

Hence, the present invention relates to a compound of Formula (I) or a pharmaceutically acceptable salt or a solvate thereof according to the present invention, for use as a medicine, as well as to the use of a compound of Formula (I) or a pharmaceutically acceptable salt or a solvate thereof according to the invention or a pharmaceutical composition according to the invention for the manufacture of a medicament. The present invention also relates to a compound of Formula (I) or a pharmaceutically acceptable salt or a solvate thereof according to the present invention or a pharmaceutical composition according to the invention for use in the treatment or prevention of, in particular treatment of, a condition in a mammal, including a human, the treatment or prevention of which is affected or facilitated by the inhibition of phosphodiesterase 2 enzyme. The present invention also relates to the use of a compound of Formula (I) or a pharmaceutically acceptable salt or a solvate thereof according to the present invention or a pharmaceutical composition according to the invention for the manufacture of a medicament for the treatment or prevention of, in particular treatment of, a condition in a mammal, including a human, the treatment or prevention of which is affected or facilitated by the inhibition of phosphodiesterase 2 enzyme.

The present invention also relates to a compound of Formula (I) or a pharmaceutically acceptable salt or a solvate thereof according to the invention, or a pharmaceutical composition according to the invention for use in the treatment, prevention, amelioration, control or reduction of the risk of various neurological and psychiatric disorders associated with phosphodiesterase 2 dysfunction in a mammal, including a human, the treatment or prevention of which is affected or facilitated by the inhibition of phosphodiesterase 2.

Also, the present invention relates to the use of a compound of Formula (I) or a pharmaceutically acceptable salt or a solvate thereof according to the invention or a pharmaceutical composition according to the invention for the manufacture of a medicament for treating, preventing, ameliorating, controlling or reducing the risk of various neurological and psychiatric disorders associated with phosphodiesterase 2 dysfunction in a mammal, including a human, the treatment or prevention of which is affected or facilitated by the inhibition of phosphodiesterase 2.

Where the invention is said to relate to the use of a compound of Formula (I) or a pharmaceutically acceptable salt or a solvate thereof or composition according to the invention for the manufacture of a medicament for e.g. the treatment of a subject, e.g. a mammal, it is understood that such use is to be interpreted in certain jurisdictions as a method of e.g. treatment of a subject, comprising administering to a subject in need of such e.g. treatment, an effective amount of a compound of Formula (I) or a pharmaceutically acceptable salt or a solvate thereof or composition according to the invention.

In particular, the indications that may be treated with PDE2 inhibitors, either alone or in combination with other drugs, include, but are not limited to, those diseases thought to be mediated in part by the basal ganglia, prefrontal cortex and hippocampus.

These indications include neurological and psychiatric disorders selected from psychotic disorders and conditions; anxiety disorders; movement disorders; drug abuse; mood disorders; neurodegenerative disorders; disorders or conditions comprising as a symptom a deficiency in attention and/or cognition; disorders related to memory acquisition and consolidation; stroke; and autistic disorder or autism.

In particular, the psychotic disorders and conditions associated with PDE2 dysfunction include one or more of the following conditions or diseases: schizophrenia, for example of the paranoid, disorganized, catatonic, undifferentiated or residual type; schizophreniform disorder; schizoaffective disorder, such as delusional or depressive type; delusional disorder; substance-induced psychotic disorder such as psychosis induced by alcohol, amphetamine, cannabis, cocaine, hallucinogens, inhalants, opioids, or phencyclidine; personality disorders of the paranoid type; and personality disorder of the schizoid type.

In particular, the anxiety disorders include panic disorder; agoraphobia; specific phobia; social phobia; obsessive-compulsive disorder; post-traumatic stress disorder; acute stress disorder; and generalized anxiety disorder.

In particular, movement disorders include Huntington's disease and dyskinesia; Parkinson's disease; restless leg syndrome and essential tremor. Additionally, Tourette's syndrome and other tic disorders can be included.

In particular, the central nervous system disorder is a substance-related disorder selected from the group of alcohol abuse; alcohol dependence; alcohol withdrawal; alcohol withdrawal delirium; alcohol-induced psychotic disorder; amphetamine dependence; amphetamine withdrawal; cocaine dependence; cocaine withdrawal; nicotine dependence; nicotine withdrawal; opioid dependence and opioid withdrawal.

In particular, mood disorders and mood episodes include depression, mania and bipolar disorders. Preferably, the mood disorder is selected from the group of bipolar disorders (I and II); cyclothymic disorder; depression; dysthymic disorder; major depressive disorder; treatment-resistant depression; and substance-induced mood disorder.

In particular, neurodegenerative disorders include Parkinson's disease; Huntington's disease; dementia such as for example Alzheimer's disease; multi-infarct dementia; AIDS-related dementia or frontotemporal dementia. The neurodegenerative disorder or condition comprises dysfunction of striatal medium spiny neurons responses.

In particular, disorders or conditions comprising as a symptom a deficiency in attention and/or cognition include dementia, such as Alzheimer's disease; multi-infarct dementia; dementia due to Lewy body disease; alcoholic dementia or substance-induced persisting dementia; dementia associated with intracranial tumours or cerebral trauma; dementia associated with Huntington's disease; dementia associated with Parkinson's disease; AIDS-related dementia; dementia due to Pick's disease; dementia due to Creutzfeldt-Jakob disease; other diseases include delirium; amnestic disorder; post-traumatic stress disorder; stroke; progressive supranuclear palsy; mental retardation; a learning disorder; attention-deficit/hyperactivity disorder (ADHD); mild cognitive disorder; Asperger's syndrome; age-related cognitive impairment; and cognitive impairment related to perception, concentration, learning or memory.

In particular, disorders related to memory acquisition and consolidation include, memory disorders, such as age-associated memory losses, memory deficiency.

Preferably, the psychotic disorder is selected from the group of schizophrenia, delusional disorder, schizoaffective disorder, schizophreniform disorder and substance-induced psychotic disorder.

Preferably, the central nervous system disorder is a personality disorder selected from the group of obsessive-compulsive personality disorder and schizoid, schizotypal disorder.

Preferably, the central nervous system disorder is a mood disorder selected from the group of bipolar disorders (I & II), cyclothymic disorder, depression, dysthymic disorder, major depressive disorder; treatment-resistant depression; and substance-induced mood disorder.

Preferably, the central nervous system disorder is attention-deficit/hyperactivity disorder.

Preferably, the central nervous system disorder is a cognitive disorder selected from the group of delirium, substance-induced persisting delirium, dementia, dementia due to HIV disease, dementia due to Huntington's disease, dementia due to Parkinson's disease, dementia of the Alzheimer's type, substance-induced persisting dementia and mild cognitive impairment.

Preferably the disorders treated by the compounds of formula (I) or a pharmaceutically acceptable salt or a solvate thereof of the present invention are selected from schizophrenia; obsessive-compulsive disorder; generalized anxiety disorder; Huntington's disease; dyskinesia; Parkinson's disease; depression; bipolar disorders; dementia such as Alzheimer's disease; attention-deficit/hyperactivity disorder; drug abuse; stroke; and autism.

Preferably, the disorders treated by the compounds of formula (I) or a pharmaceutically acceptable salt or a solvate thereof of the present invention are schizophrenia, including positive and negative symptoms thereof, and cognitive deficits, such as impaired attention or memory.

Of the disorders mentioned above, the treatment of anxiety, obsessive-compulsive disorder, post-traumatic stress disorder; generalized anxiety disorder, schizophrenia, depression, attention-deficit/hyperactivity disorder, Alzheimer's disease, dementia due to Huntington's disease, dementia due to Parkinson's disease, dementia of the Alzheimer's type, substance-induced persisting dementia and mild cognitive impairment are of particular importance.

Of the disorders mentioned above, the treatment of anxiety, obsessive-compulsive disorder, schizophrenia, depression, attention-deficit/hyperactivity disorder, and Alzheimer's disease are of particular importance.

Other central nervous system disorders include schizoanxiety disorder, and comorbid depression and anxiety, in particular major depressive disorder with comorbid generalized anxiety disorder, social anxiety disorder, or panic disorder; it is understood that comorbid depression and anxiety may also be referred to by the terms anxious depression, mixed anxiety depression, mixed anxiety-depressive disorder, or major depressive disorder with anxiety symptoms, which are used indistinctively herein.

At present, the fourth edition of the Diagnostic & Statistical Manual of Mental Disorders (DSM-IV) of the American Psychiatric Association provides a diagnostic tool for the identification of the disorders described herein. The person skilled in the art will recognize that alternative nomenclatures, nosologies, and classification systems for neurological and psychiatric disorders described herein exist, and that these evolve with medical and scientific progresses. For example, the "American Psychiatric Association: Diagnostic and Statistical Manual of Mental Disorders, Fifth Edition. Arlington, VA, American Psychiatric Association, 2013" (DSM-5™) utilizes terms such as depressive disorders, in particular, major depressive disorder, persistent depressive disorder (dysthymia), substance-medication-induced depressive disorder; neurocognitive disorders (NCDs) (both major and mild), in particular, neurocognitive disorders due to Alzheimer's disease, vascular NCD (such as vascular NCD present with multiple infarctions), NCD due to HIV infection, NCD due to traumatic brain injury (TBI), NCD due to Parkinson's disease, NCD due to Huntington's disease, frontotemporal NCD, NCD due to prion disease, and substance/medication-induced NCD; neurodevelopmental disorders, in particular, intellectual disability, specific learning disorder, neurodevelopmental motor disorder, communication disorder, and attention-deficit/hyperactivity disorder (ADHD); substance-related disorders and addictive disorders, in particular, alcohol use disorder, amphetamine use disorder, cannabis use disorder, cocaine use disorder, other hallucinogen use disorder, tobacco use disorder, opiod use disorder, and phencyclidine use disorder; schizophrenia spectrum and other psychotic disorders, in particular, schizophrenia, schizophreniform disorder, schizoaffective disorder, delusional disorder, brief psychotic disorder, substance/medication-induced psychotic disorder; and cyclothymic disorder (which under DSM-5™ falls under the bipolar and related disorders category). Such terms may be used by the skilled person as an alternative nomenclature for some of the diseases or conditions referred to herein. An additional neurodevelopmental disorder includes autism spectrum disorder (ASD), which encompasses according to the DSM-5™, disorders previously known by the terms early infantile autism, childhood autism, Kanner's autism, high-functioning autism, atypical autism, pervasive developmental disorder not otherwise specified, childhood disintegrative disorder, and Asperger's disorder.

Therefore, the invention also relates to a compound of Formula (I) or a pharmaceutically acceptable salt or a solvate thereof according to the invention, for use in the treatment of any one of the diseases mentioned hereinbefore.

The invention also relates to a compound of Formula (I) or a pharmaceutically acceptable salt or a solvate thereof according to the invention for use in treating any one of the diseases mentioned hereinbefore.

The invention also relates to a compound of Formula (I) or a pharmaceutically acceptable salt or a solvate thereof according to the invention, for the treatment or prevention, in particular treatment, of any one of the diseases mentioned hereinbefore.

The invention also relates to the use of a compound of Formula (I) or a pharmaceutically acceptable salt or a solvate thereof according to the invention, for the manufacture of a medicament for the treatment or prevention of any one of the disease conditions mentioned hereinbefore.

The invention also relates to the use of a compound of Formula (I) or a pharmaceutically acceptable salt or a solvate thereof according to the invention for the manufacture of a medicament for the treatment of any one of the disease conditions mentioned hereinbefore.

The compound of Formula (I) or a pharmaceutically acceptable salt or a solvate thereof of the present invention can be administered to mammals, preferably humans, for the treatment or prevention of any one of the diseases mentioned hereinbefore.

In view of the utility of the compounds of Formula (I), and the pharmaceutically acceptable salts and the solvates thereof, according to the invention, there is provided a method of treating a disorder or disease mentioned hereinbefore, comprising administering to a subject in need thereof, a therapeutically effective amount of a compound of Formula (I) or a pharmaceutically acceptable salt or a solvate thereof or pharmaceutical compositions described herein.

Said methods comprise the administration, i.e. the systemic or topical administration, preferably oral administration, of a therapeutically effective amount of a compound of Formula (I) or a pharmaceutically acceptable salt or a solvate thereof according to the invention to warm-blooded animals, including humans.

Therefore, the invention also relates to a method for the prevention and/or treatment of any one of the diseases mentioned hereinbefore comprising administering a therapeutically effective amount of a compound of Formula (I) or a pharmaceutically acceptable salt or a solvate thereof according to the invention to a patient in need thereof.

The PDE2 inhibitor described herein can be used alone, in combination or in combination with other pharmaceutical agents such as other agents used in the treatment of psychoses, such as schizophrenia and bipolar disorder, obsessive-compulsive disorder, Parkinson's disease, cognitive impairment and/or memory loss, e.g. nicotinic α-7 agonists, PDE4 inhibitors (Rolipram, GEBR-7b, GSK356278, GSK256066, Apremilast, MK-0952, Roflumilast, AN2898, AN2728, Ariflo Cilomilast, Dotraverine, Ronomilast Elbimilast, Revamilast, Tetomilast, E6005, GDP-1116, HT0712, MK-0873), PDE5 inhibitors (Sildenafil, Vardenafil, Tadalafil, Udenafil, Avanafil, Mirodenafil, Lodenafil, Dasantafil, PF-00489791), PDE9 (PF-04447943), other PDE2 inhibitors (Bay 60-7550, PF-999, ND-7001), PDE10 inhibitors (PF-02545920, AMG579), PDE2 and 10 inhibitors, calcium channel blockers, muscarinic m1 and m2 modulators, adenosine receptor modulators, ampakines, NMDA-R modulators, mGluR modulators, dopamine modulators, serotonin modulators, cannabinoid modulators, HDAC inhibitors (Vorinostat SAHA, Panobinostat, Quisinostat, Valproic acid) and cholinesterase inhibitors (e.g. donepezil, rivastigmine, and galantamine). In such combinations, the compound of Formula (I) or a pharmaceutically acceptable salt or a solvate thereof of the present invention may be utilized in combination with one or more other drugs in the treatment, prevention, control, amelioration, or reduction of risk of diseases or conditions for which the compound of Formula (I) or the other drugs may have utility, where the combination of the drugs together are safer or more effective than either drug alone.

One skilled in the art will recognize that a therapeutically effective amount of the PDE2 inhibitor of the present invention is the amount sufficient to inhibit the PDE2 enzyme and that this amount varies *inter alia,* depending on the type of disease, the concentration of the compound in the therapeutic formulation, and the condition of the patient. Generally, an amount of PDE2 inhibitor to be administered as a therapeutic agent for treating diseases in which inhibition of the PDE2 enzyme is beneficial, such as the disorders described herein, will be determined on a case by case by an attending physician.

Generally, a suitable dose is one that results in a concentration of the PDE2 inhibitor at the treatment site in the range of 0.5 nM to 200 µM, and more usually 5 nM to 50 µM. To obtain these treatment concentrations, a patient in need of treatment likely will be administered between 0.001 mg/kg to 15 mg/kg body weight, in particular from 0.01 mg/kg to 2.50 mg/kg body weight, in particular, from 0.01 to 1.5 mg/kg body weight, in particular from 0.1 mg/kg to 0.50 mg/kg body weight. The amount of a compound according to the present invention, also referred to here as the active ingredient, which is required to achieve a therapeutical effect will, of course vary on case-by-case basis, vary with the particular compound, the route of administration, the age and condition of the recipient, and the particular disorder or disease being treated. A method of treatment may also include administering the active ingredient on a regimen of between one and four intakes per day. In these methods of treatment the compound according to the invention is preferably formulated prior to admission. As described herein below, suitable pharmaceutical formulations are prepared by known procedures using well known and readily available ingredients.

### PHARMACEUTICAL COMPOSITIONS

The present invention also provides compositions for preventing or treating diseases in which inhibition of PDE2 is beneficial, such as neurological and psychiatric disorders. Said compositions comprising a therapeutically effective amount of a compound of Formula (I) and a pharmaceutically acceptable carrier or diluent.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy. A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Depending on the mode of administration, the pharmaceutical composition will comprise from 0.05 to 99 % by weight, preferably from 0.1 to 70 % by weight, more preferably from 0.1 to 50 % by weight of the active ingredient, and, from 1 to 99.95 % by weight, preferably from 30 to 99.9 % by weight, more preferably from 50 to 99.9 % by weight of a pharmaceutically acceptable carrier, all percentages being based on the total weight of the composition.

The present compound can be used for systemic administration such as oral, percutaneous or parenteral administration; or topical administration such as via inhalation, a nose spray, eye drops or via a cream, gel, shampoo or the like. The compound is preferably orally administered.

The exact dosage and frequency of administration depends on the compound, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compound of the instant invention.

The amount of the compound of Formula (I) that can be combined with a carrier material to produce a single dosage form will vary depending upon the disease treated, the mammalian species, and the particular mode of administration. However, as a general guide, suitable unit doses for the compound of the present invention can, for example, preferably contain between 0.1 mg to about 1000 mg of the active compound. A preferred unit dose is between 1 mg to about 500 mg. A more preferred unit dose is between 1 mg to about 300 mg. An even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

For the compositions, methods and kits provided above, one of skill in the art will understand that the preferred compound for use in each is the compound noted herein.

### EXPERIMENTAL PART

As used herein, the term "ACN" means acetonitrile, "AcOH" or "TFA" means acetic acid, "Boc" means tert-butyloxycarbonyl, "Boc₂O" means di-tert-butyl decarbonate, "d" means day(s), "DMAP" 4-dimethylaminopyridine, "DSC" means differential scanning calorimetry, "LCMS" means liquid chromatography/mass spectrometry, "HPLC" means high-performance liquid chromatography, "RP HPLC" means reverse phase high-performance liquid chromatography, "aq." means aqueous, "DCM" means dichloromethane, "DIPE" means diisopropyl ether, "DIPEA" means diisopropylethyl amine, "DMF" means *N*,*N*-dimethylformamide, "DMSO" means dimethyl sulfoxide, "EDCI" means 1-ethyl-3-(3-dimethylaminopropyl)carbodiimide, "EtOH" means ethanol, "Et₂O" means diethylether, "EtOAc" means ethyl acetate, "Et₃N" or "TEA" means triethylamine, "HATU" means 1-[Bis(dimethylamino)methylene]-1H-1,2,3-triazolo[4,5-b]pyridinium 3-oxid hexafluorophosphate, "HBTU" means O-(benzotriazol-1-yl)-N,N,N'N,'-tetramethyluroniumhexafluoro-phosphate, "LiHMDS" means Lithium bis(trimethylsilyl)amide, "THF" means tetrahydrofuran, "min" means minutes, "h" means hours, "mCPBA" means 3-chloroperbenzoic acid, "MeOH" means methanol, "MTBE" means methyl tert-butyl ether, "Pd/C" means Palladium on carbon, "Pd(PPh₃)₄" means tetrakis(triphenylphosphine)palladium(0), "iPrOH" means 2-propanol, "RM" or "rm" means reaction mixture, "RT" or "rt" means room temperature, "OL" means organic layer, "Rₜ" means retention time (in minutes), "quant." means quantitative, "sat." means saturated, "SFC" means supercritical fluid chromatography, "sol." means solution, "m.p." means melting point, "q.s." means quantum sufficit.

Thin layer chromatography (TLC) was carried out on silica gel 60 F254 plates (Merck) using reagent grade solvents. Open column chromatography was performed on silica gel, mesh 230-400 particle size and 60 Å pore size (Merck) under standard techniques. Automated flash column chromatography was performed using ready-to-connect cartridges from Merck, on irregular silica gel, particle size 15-40 µm (normal phase disposable flash columns) on an SPOT or LAFLASH system from Armen Instrument.

When a stereocenter is indicated with 'RS' this means that a racemic mixture was obtained at the indicated centre, unless otherwise indicated. The stereochemical configuration for centres in some compounds may be designated "*R*" or "*S*" when the mixture(s) was separated; for some compounds, the stereochemical configuration at indicated centres has been designated as "**R*" or "**S*" when the absolute stereochemistry is undetermined although the compound itself has been isolated as a single stereoisomer and is enantiomerically/diastereomerically pure.

The absolute stereochemical configuration for some of the compounds was determined using vibrational circular dichroism (VCD). They were measured on a Bruker Equinox 55 equipped with a PMA 37, in a KBr liquid cell using CD₂Cl₂ as solvent (PEM: 1350cm-1, LIA: 1mV, resolution: 4 cm⁻¹). A description on the use of VCD for the determination of absolute configuration can be found in Dyatkin A.B. et. al, Chirality, 14:215-219 (2002). Ab initio calculations: A thorough conformational search was performed at molecular mechanics level using Macromodel to do a mixed torsional/low-mode sampling with the OPLS-2005 force field. The located minima were optimized using Jaguar on the B3LYP/6-31G** level with a Poisson-Boltzmann continuum solvation model to mimic a dichloromethane solvent. All conformations within 10 kJ/mol interval were used to simulate VCD and IR spectrum. Dipole and rotational strengths were calculated at the same B3LYP/6-31G** level, using Jaguar. The calculated VCD spectra, generated after scaling the frequencies with a factor of 0.97, converting to a Lorentzian bandshape, and summing up the contribution of each conformer assuming a Boltzmann ensemble, were visually compared with the experimental spectra for assigning the correct stereo chemistry.

As understood by a person skilled in the art, compounds synthesised using the protocols as indicated may exist as a solvate e.g. hydrate, and/or contain residual solvent or minor impurities. Compounds isolated as a salt form, may be integer stoichiometric i.e. mono- or di-salts, or of intermediate stoichiometry.

The following examples are intended to illustrate but not to limit the scope of the present invention. Unless otherwise noted, all starting materials were obtained from commercial suppliers and used without further purification.

### A. SYNTHESIS OF INTERMEDIATES

### INTERMEDIATE 1

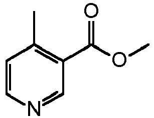

Procedure a: 4-Methyl-3-pyridinecarboxylic acid hydrochloride (1:1) (40 g, 230.4 mmol) was added to a refluxing mixture of sulphuric acid (20 mL) and MeOH (400 mL). The mixture was refluxed overnight, then it was evaporated and the resulting slurry was added to a cold solution of NaHCO₃ (64 g) in water (360 mL). The product was extracted with DCM and the OL was dried over MgSO₄, filtered and evaporated, yielding intermediate 1 (28.70 g, 83%).
Procedure b: A metal reactor was charged with 3-bromo-4-methyl-pyridine (200 g, 0.116 mol) and a mixture of DMF/MeOH (1 L/1L). To this was added Et₃N (400 g, 0.395 mol), palladium (II) acetate (8 g, 0.036 mol) and 1,1'-bis(diphenylphosphino)ferrocene (16 g, 0.029 mol). The reactor was closed and pressurized with CO gas (3 MPa) and the reaction mixture was stirred and heated overnight at 140 °C. The RM was cooled, filtered and concentrated *in vacuo.* The residue was purified by flash column chromatography over silica gel (gradient eluent: EtOAc/Petroleum ether from 1/1 to 1/0). The product fractions were collected and the solvent was evaporated to afford the desired intermediate 1 (90 g, 51%).

### INTERMEDIATE 2

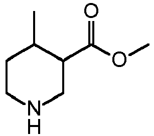

Procedure a: A hydrogenation flask was charged with AcOH (500 mL) and then PtO₂ (15.02 g, 66.2 mmol) was added. Intermediate 1 (50 g, 330.8 mmol) was added and the mixture was hydrogenated at 50 °C for 7 days. The RM was filtered over dicalite® and the filtrate was evaporated to yield intermediate 2 (52 g), which was used in the next step without further purification.
Procedure b: Platinum oxide (5 g, 0.022 mol) was added to a solution of intermediate 1 (90 g, 0.595 mol) and AcOH (1 L). The r.m. was stirred and hydrogenated for 5 days at 50°C under a pressure of 3.5 kPa. The cooled RM was concentrated *in vacuo* to give intermediate 2 as the acetic acid salt (140 g, 97%, 90% purity determined by ¹H-NMR).

### INTERMEDIATE 3

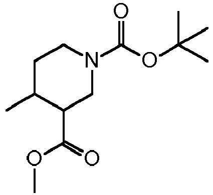

Procedure a: To a solution of intermediate 2 (52 g, 330.8 mmol) in DCM (869 mL), DIPEA (85.5 g, 661.5 mmol) and DMAP (4.04 g, 33.08 mmol) were added. Then di-tert-butyl dicarbonate (72.19 g, 330.8 mmol) was added to this solution in small portions and the reaction was stirred at RT for 1 h. The RM was washed with water and brine and the organic layer was dried over MgSO₄, filtered and evaporated. The product was purified by column chromatograph (silica gel, eluent: DCM, 1% MeOH in DCM, 2%, 4%). The desired fractions were evaporated, yielding intermediate 3 (64.1 g, 75%).
Procedure b: To a stirred and cooled (0 °C) solution of intermediate 2 (140 g, 0.595 mol) in DCM (1.5 L) was added sequentially di-tert-butyl dicarbonate (130 g, 0.596 mol), Et₃N (225 g, 1.74 mol) and DMAP (10 g, 0.082 mol) and stirring was continued at RT for 2 h. The reaction mixture was poured onto H₂O (500 mL) and extracted with DCM (2x 100 mL). The organic layers were separated, dried (Na₂SO₄), and the solvent was evaporated to give crude intermediate 3 (150 g, 90%, 90% purity determined by ¹H-NMR) which was used as such in the next.

### INTERMEDIATE 4

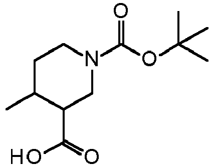

Procedure a: Intermediate 3 (64.1 g, 249.1 mmol) was stirred in MeOH (500 mL) at RT. NaOH (2 M, 747.3 mL) was added and the mixture was stirred for 2 h at RT. The RM was acidified with HCl 1N and the product was extracted with Et₂O. The OL was washed with brine and dried over MgSO₄, filtered and evaporated, yielding intermediate 4 (59.70 g) as a white solid.
Procedure b: To a stirred solution of intermediate 3 (150 g, 90% pure, 0.524 mol) in MeOH (0.9L) was added a solution of a 2M NaOH solution (1.8 mol). After 14 h at RT, the RM was extracted with MTBE (2 x 0.8 L). The aqueous layer was acidified with 10% citric acid and then extracted with EtOAc (4 x 1 L). The combined organic layers were dried over Na₂SO₄, filtered and concentrated *in vacuo* to give crude intermediate 4 (142 g, 90% purity determined by ¹H-NMR, 100%) which was used as such in the next step.

### INTERMEDIATE 5

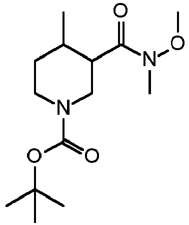

Procedure a: To a solution of intermediate 4 (59.7 g, 0.25 mol) in THF (800 mL), was added di-1H-imidazol-1-yl-methanone (54 g, 0.33 mol) and the mixture was stirred at RT for 1 h. In another flask, to a suspension of N-methoxy-methanamine hydrochloride (1:1) (32.93 g, 0.34 mol) in ACN (500 mL), was added trimethylamine (35.75 g, 0.35 mol). Both mixtures were combined and stirred at 50 °C while monitoring. The intermediate product crystallized out of the RM and did not react with N-methoxy-methanamine to form the desired product. DCM was added until the intermediate dissolved. The reaction was left stirring for 1 week at 80 °C. The solvents were evaporated. The residue was dissolved in DCM and washed with water, 20% AcOH solution and finally with a saturated NaHCO₃ solution. The OL was dried over MgSO₄, filtered and evaporated. The product was purified by column chromatography (silica gel, eluent: 2% MeOH in DCM, 4%). The pure fractions were evaporated, yielding intermediate 5 (70 g, quantitative).
Procedure b: To a stirred and ice-cooled solution of intermediate 4 (140 g, 0.518 mol) in DCM (2 L) was added N,O-dimethylhydroxylamine (113 g, 1.16 mol) and Et₃N (113 g, 1.79 mol). Then HATU (235 g, 0.618 mol) was added and stirring was continued for 14 h. The solvent was evaporated and a NaHCO₃ solution (0.5 L) was added and then extracted with DCM (3 x 1 L). The combined organic layers were separated, dried over Na₂SO₄, filtered and concentrated *in vacuo.* The residue was purified by silica gel flash chromatography eluting with 1-10% EtOAc in petroleum ether to afford intermediate 5 (152 g, 100%).

### INTERMEDIATE 6

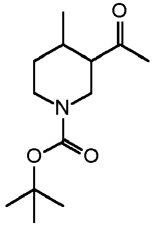

Procedure a: Intermediate 5 (70 g, 244.4 mmol) in THF (250 mL) was charged in a flask under N₂ and cooled to -15°C. Methylmagnesium bromide (1.4 M in toluene/THF 75/25, 206 mL) was added dropwise, with the temperature not exceeding 0 °C. After addition, the RM was stirred at RT for 1 h. Then the RM was poured on ice with 20 mL AcOH. The product was extracted with Et₂O and the OL was washed with a 5% NaHCO₃ solution. The OL was dried over MgSO₄, filtered and evaporated to give intermediate 6 (53.35 g, 90%).
Procedure b: To a stirred and cooled solution (0 °C) of intermediate 5 (150 g, 0.524 mol) in THF (2 L) was added dropwise a 3M methylmagnesium bromide solution in THF (0.75 L, 2.25 mol) and stirring was continued at RT for 2 h. The reaction mixture was poured onto aqueous NH₄Cl solution and extracted with DCM. The combined organic layers were dried over Na₂SO₄, filtered and concentrated *in vacuo.* The residue was purified by silica gel chromatography eluting with 1-5% EtOAc in petroleum ether to afford intermediate 6 (120 g, 95%).

### INTERMEDIATE 7

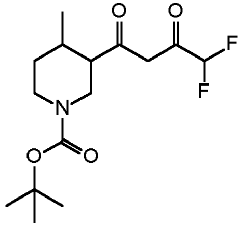

Intermediate 6 (53.35 g, 0.22 mol) was stirred in toluene (1500 mL) at 0 °C under N₂. Potassium tert-butoxide (34.14 g) was added at 0-5 °C and 2,2-difluoro-acetic acid ethyl ester (33.01 g, 0.27 mol) was added dropwise at 0-5 °C. The RM was stirred at RT for 2 h, then washed with 10% H₂SO₄ in water and the OL was dried on MgSO₄, filtered and evaporated, yielding intermediate 7 (70.50 g, quantitative).

### INTERMEDIATE 8

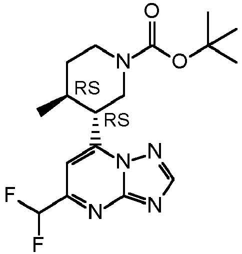

Intermediate 7 (70.5 g, 220.8 mmol), 1*H*-1,2,4-triazol-5-amine hydrochloride (1:1) (53.22 g, 441.52 mmol) and DMF (1500 mL) were stirred at 80 °C for 24 h. Et₃N (20 g) and di-tert-butyl dicarbonate (20 g) were added. The mixture was stirred for 30 min, evaporated and then dissolved in EtOAc, washed with water and brine. The OL was dried over MgSO₄, filtered and evaporated. Four isomers were observed. The first fraction crystallized from Et₂O. The crystals were filtered off and dried, yielding intermediate 8 (24.60 g, 30%). The mother liquor yielded a second fraction of the compound. The crystals were filtered off and dried, yielding intermediate 8 (2.53 g, 3%). N.B. "RS" means the intermediate is a racemic mixture of two enantiomers of *trans* relative configuration.

### INTERMEDIATES 9, 9A AND 9B

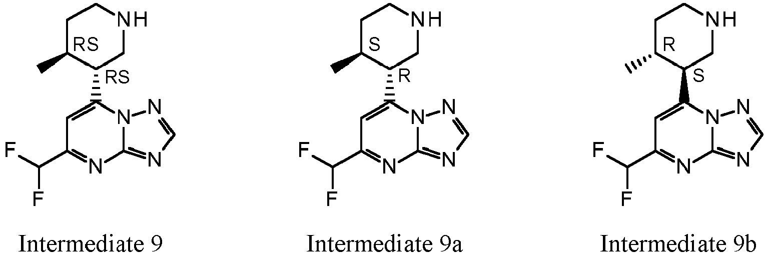

To a solution of intermediate 8 (24.6 g, 67 mmol) in MeOH (350 mL), was added HCl-iPrOH (350 mL) and the RM was stirred for 2 h at RT. The RM was evaporated and the product was crystallized from EtOH. The crystals were filtered off and dried, yielding 20.33 g of a crude, to which water, Na₂CO₃ and DCM were added. The OL was dried over MgSO₄, filtered and evaporated, yielding 12.80 g of intermediate 9. This free base was separated into enantiomers 9a and 9b by purification by Prep SFC (Stationary phase: Chiralpak Diacel AD 30 x 250 mm;, mobile phase: CO₂, ((MeOH - iPrOH 50/50) with 0.4% iPrNH₂), yielding intermediate 9a (5g, 19%, Rt = 7.57 min) and intermediate 9b (5.13 g, 19%, Rt = 9.36 min).

Intermediates 9a and 9b were isolated as free bases or alternatively, they were dissolved in MeOH, followed by addition of HCl/i-PrOH and the mixture evaporated. The hydrochloride salts (in each instance, .HCl) were crystallized from ACN, filtered off and dried.

### INTERMEDIATE 10

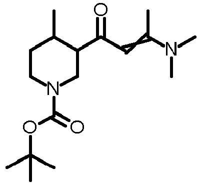

A stirred mixture of I-6 (7.3 g, 0.03 mol) in N,N-dimethylacetamide dimethyl acetal (20 mL, 0.91 g/mL, 0.14 mol) was heated at 100 °C for 4 h. The RM was concentrated *in vacuo,* co-evaporated with toluene (2 x 20 mL) to yield 1-7 as a brown residue (9.4 g, yield 100.1%) which was used as such in the next step.

### INTERMEDIATE 11 (I-11)

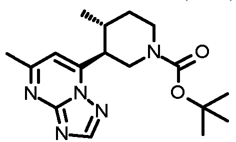

To a mixture of I-10 (9.4 g, 0.03 mol) in AcOH (50 mL, 1.05 g/mL, 1.75 mol) was added a mixture of 3-amino-1,2,4-triazole (2.68 g, 0.03 mol) in HOAc (50 mL, 1.05 g/mL, 1.75 mol) and the ensuing RM was heated on a Drysyn® metal heating block of 130°C for 15 min. The RM was cooled, concentrated *in vacuo,* diluted with DCM (0.2 L) and treated with 1 NaOH until pH∼8. The layers were separated and the aqueous layer was extracted with DCM (2 x 50 mL). The combined organic layers were dried (MgSO₄), filtered and concentrated *in vacuo* to give a dark brown oil which was purified by silica gel chromatography using a Redisep® 120 g Flash column eluting with a gradient of 0-3% 7N NH₃/MeOH in DCM to afford intermediate 11 as a tan oil, in a ∼1:4 = cis:trans mixture (2.15 g, yield 21.42%).

### INTERMEDIATE 12

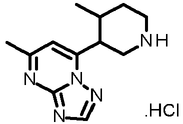

A stirred mixture of I-11 (2.15 g, 0.0065 mol) in MeOH (50 mL, 0.79 g/mL, 1.23 mol) was treated with HCl (6M in iPrOH) (50 mL, 6 M, 0.3 mol) and after 16 h at RT the RM was concentrated *in vacuo* to give an off white solid. This was triturated with a mixture of Et₂O (200 mL) and ACN (30 mL) for 16 h. The solid was collected by filtration and dried to afford intermediate 12 as an off white solid as a cis/trans mixture (18%/82%) (1.7 g, yield 97.87%).

### INTERMEDIATE 12A AND INTERMEDIATE 12B

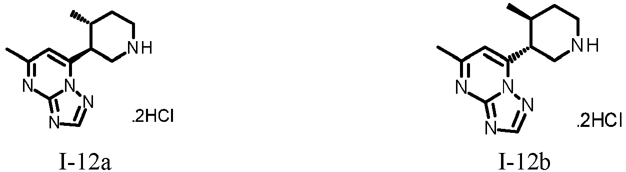

A stirred mixture of I-11 (23 g, 0.0694 mol) in MeOH (165 mL) was treated with HCl (6M in iPrOH) (165 mL, 6 M, 0.986 mol) and after 16 h at RT the RM was concentrated *in vacuo* to give an off white solid. This was diluted with water and DCM and treated with Na₂CO₃. The OL was dried over MgSO₄, filtered and concentrated *in vacuo* to afford a residue which was purified using SCF (Stationary phase: Chiralpak® Diacel AD 20 x 250 mm, mobile phase: CO₂, MeOH-iPrOH (50-50) + 0.4% iPrNH₂) to afford intermediates 12a and 12b and. These were dissolved in MeOH (100 mL) and treated with HCl (6M in iPrOH) (100 mL) at 0 °C for 2 h. The volatiles were evaporated under reduced pressure and the resulting residues were stirred at 0 °C in Et₂O to give intermediate 12a (9.25 g, 43%, Rt = 3.54 min, [α]²⁰_{D} = -17.47 ° (c 0.54, DMF)) and intermediate 12b (8.8 g, 42%, Rt = 3.24 min, [α]²⁰_{D} = +16.5 °(c 0.52, DMF)).

### INTERMEDIATE 13

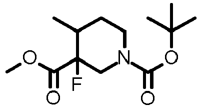

To a solution of LDA (2 M in THF/heptane/ethylbenzene, 42.7 mL, 2 M, 85.493 mmol) in 100 ml THF (245 mL) was added dropwise a solution of intermediate 3 (20 g, 77.721 mmol) in THF (50 mL) at 0°C. The solution was stirred for 30 min and then transferred to a solution of N-fluorobenzenesulfonimide (30.6 g, 97.1 mmol) in 100 ml THF at 0°C. The RM was stirred for 15 min at 0°C and then at rt overnight. The RM was evaporated, EtOAc was added and the RM was washed subsequently with water, 0.1N HCl-solution, saturated NaHCO₃ solution and brine. The OL was dried on MgSO₄, filtered and evaporated. The product was purified on silica gel, eluent: DCM -> 1% MeOH in DCM. The pure fractions were evaporated to yield intermediate 13 (21.4 g, quantitative).

### INTERMEDIATE 14

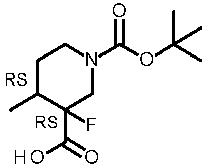

A solution of intermediate 13 (21.4 g, 77.728 mmol) was stirred in MeOH (500 mL) at RT. NaOH (486 mL, 2 M, 973 mmol) was added and the mixture was stirred for 2 h at rt. The RM was acidified with HCl 1N and the product was extracted with Et2O. The OL was washed with brine and dried on MgSO₄, filtered and evaporated. The product was purified on silicagel, eluent: DCM -> 5% MeOH in DCM. The pure fractions were evaporated to yield 14 (15 g, 74%).

### INTERMEDIATE 15

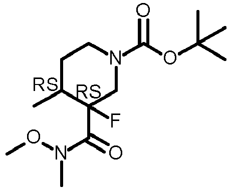

Intermediate 14 (15 g, 57.407 mmol) was dissolved in DCM (1000 mL). Then N,O-dimethylhydroxylamine hydrochloride (11.2 g, 114.8 mmol) and Et₃N (17.4 g, 172 mmol) were added. The reaction mixture was cooled to 0°C. Then HATU (24.0 g, 63.1 mmol) was added. The reaction mixture was stirred at rt for 2 h. The RM was poured into aq. NaHCO₃ (100 mL). The OL was separated, dried with MgSO₄, and the solvent was evaporated. The residue was purified by flash column chromatography over silica gel eluent: DCM -> 1% MeOH in DCM. The product fractions were collected and the solvent was evaporated to give the desired product 15 (8.49, 49% g).

### INTERMEDIATE 16

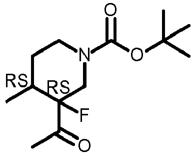

Intermediate 15 (8.49, 27.9 mmol) in THF (60 mL) was brought in a flask under N₂ and cooled to -15°C. Methyl magnesium bromide (16.3 mL, 3M, 48.8 mmol) was added dropwise, temperature not exceeding 0°C. After addition, the RM was stirred for 1 h at RT. Then the RM was poured on ice with 20 mL AcOH. The product was extracted with Et₂O and the OL was washed with a 5% NaHCO₃ solution. The OL was dried on MgSO₄, filtered and evaporated and purified on silicagel, eluent: DCM. The pure fractions were evaporated to give intermediate 16 (3.20 g, 44%).

### INTERMEDIATE 17

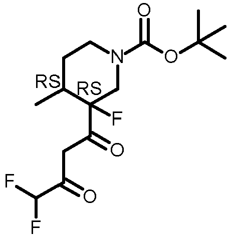

Intermediate 16 (3.2 g, 0.0123 mol) was stirred in toluene (150 mL) at 0°C under N₂. Potassium tert-butoxide (1.94 g, 17.3 mmol) was added at 0-5°C, ethyldifluoroacetate (1.84 g, 0.0149 mol) was added dropwise at 0-5°C. RM was stirred at RT for 2 hr. The RM was washed with 10% H₂SO₄ in water and the OL was dried on MgSO₄, filtered and evaporated to yield intermediate 17 (4.16 g, 99%).

### INTEMEDIATE 18

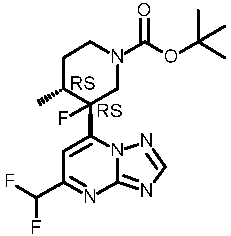

Intermediate 17 (4.16 g, 12.3 mmol) and 1H-1,2,4-triazol-5-amine hydrochloride (2.97 g, 24.7 mmol) in DMF (40 mL) were stirred at 80°C for 16 h. The RM was evaporated, DCM was added and 2 g (Boc)₂O and 2 mL of Et₃N was added. The mixture was stirred for 30 min, washed with water, the OL was dried on MgSO₄, filtered and evaporated. The product (4 isomers) was purified on silicagel, eluent: DCM -> 2% MeOH in DCM. The fractions were evaporated, yielding 3.55 g of a crude that was purified via Prep HPLC (stationary phase: Uptisphere® C18 ODB - 10µm, 200g, 5cm, mobile phase: 0.25% NH₄HCO₃ solution in water, CH₃CN) to afford intermediate 18 (730 mg, 15%).

### INTERMEDIATE 19

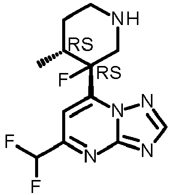

To intermediate 18 (0.73 g, 1.894 mmol) in MeOH (20 mL) was added HCl (6M in iPrOH) (20 mL, 6 M, 120 mmol) and this was stirred at RT overnight. The solvents were evaporated to yield intermediate 19 (600 mg, 99%).

### ALTERNATIVE PROCEDURE TO INTERMEDIATE 19 AND SEPARATION INTO INTERMEDIATES 19A AND 19B

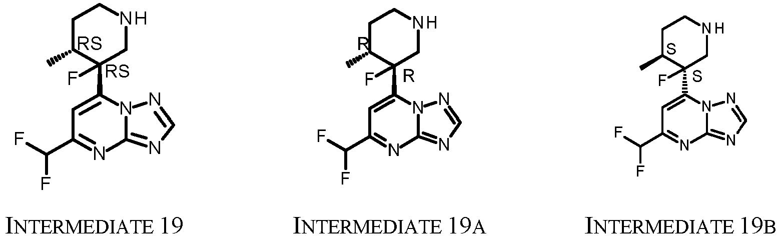

To intermediate 18 (4.5 g, 11.7 mmol) in DCM (52 mL) was added trifluoroacetic acid (TFA) (6M in iPrOH) (5.4 mL, 6 M, 70 mmol) and this was stirred at RT for 1 h. The solvents were evaporated and the resulting residue was dissolved in DCM and washed with a saturated aqueous solution of NaHCO₃. The OL was separated and the aqueous layer back-extracted 2xDCM. The combined OL were dried over MgSO₄, filtered and evaporated under reduced pressure. The resulting residue was purified via flash column chromatography on silica gel using as eluent a gradient DCM/NH₃(MeOH), 99/1 to 93/7, to give intermediate 19 (2.2 g, 66% yield) as a racemic mixture. This was separated in enantiomers by prep SFC (Stationary phase: Chiralpak Diacel AD 20 x 250 mm, mobile phase: CO₂, EtOH + 0.4% iPrNH₂) to afford intermediates 19a (955 mg, 29%, Rt = 2.36 min) and 19b (970 mg, 29%, Rt =2.99 min).

### INTERMEDIATE 20

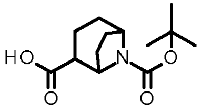

8-Azabicyclo[3.2.1]octane-2,8-dicarboxylic acid, 8-(1,1-dimethylethyl) 2-methyl ester [1033820-28-8] (4.77 g, 17.71 mmol) was stirred in MeOH (41.608 mL) at RT. NaOH (106 mL, 1 M, 106 mmol) was added and the mixture was stirred overnight at rt. The MeOH was evaporated. The RM was acidified with HCl 1N and the product was extracted with chloroform. The OL was dried on MgSO₄, filtered and evaporated to give intermediate 20 (4.52 g, 100%).

### INTERMEDIATE 21

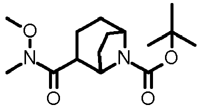

Intermediate 20 (4.52 g, 17.704 mmol) was dissolved in DCM (200 mL). Then N,O-dimethylhydroxylamine hydrochloride (3.454 g, 35.407 mmol) and Et₃N (5.37 g, 53.1 mmol) were added. The reaction mixture was cooled to 0°C. Then HATU (7.41 g, 19.5 mmol) was added. The reaction mixture was stirred at RT for 2 h. The reaction mixture was poured into aq. NaHCO₃ (100 mL). The OL was separated, dried with MgSO₄, and the solvent was evaporated. The residue was purified by flash column chromatography over silica gel eluent: DCM -> 1% MeOH in DCM. The product fractions were collected and the solvent was evaporated to give intermediate 21 (3.03 g, 57%).

### INTERMEDIATE 22

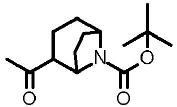

Intermediate 21 (3.03 g, 10.2 mmol) in THF (50 mL) was brought in a flask under N₂ and cooled to -15°C. Methylmagnesium bromide (12.7 mL, 1.4 M, 17.8 mmol) was added dropwise, temperature not exceeded 0°C. After addition, the RM was stirred for 1 h at RT. Then the RM was poured on ice with AcOH (20 mL). The product was extracted with Et₂O and the OL was washed with a 5% NaHCO₃ solution. The OL was dried on MgSO₄, filtered and evaporated and purified on silicagel, eluent: DCM. The pure fractions were evaporated to give intermediate 22 (2.57 g, 100%).

### INTERMEDIATE 23

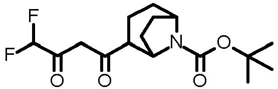

Intermediate 22 (2.57 g, 0.0101 mol) was stirred in toluene (150 mL) at 0°C under N₂. Potasssium tert.-butoxide (1.59 g, 14.2 mmol) was added at 0-5°C, ethyldifluoroacetate (1.52 g, 0.0122 mol) was added dropwise at 0-5°C. RM was stirred at RT for 2 h. The RM was washed with 10% H₂SO₄ in water and the OL was dried on MgSO₄, filtered and evaporated to yield intermediate 23 (3.34 g, 99%).

### INTERMEDIATE 24

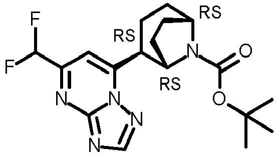

Intermediate 23 (3.34 g, 10.1 mmol) and 1H-1,2,4-triazol-5-amine hydrochloride (2.43 g, 20.2 mmol) in DMF (30 mL) were stirred at 80°C for 16 h. The RM was evaporated, DCM was added and 2 g (Boc)₂O and Et₃N (2 mL) was added. The mixture was stirred for 30 min, washed with water, the OL was dried on MgSO₄, filtered and evaporated. The product (4 isomers) was purified on silica gel, eluent: DCM -> 2% MeOH in DCM. The fractions were evaporated, yielding 3.07 g of a crude that was purified via Prep HPLC (stationary phase: Uptisphere® C18 ODB - 10µm, 200g, 5cm I.D., mobile phase: 0.25% NH₄HCO₃ solution in water, MeOH) to give intermediate 24 (1.07 g, 28%).

### INTERMEDIATE 25

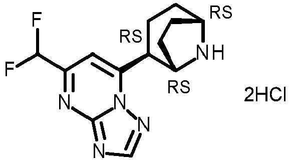

To intermediate 24 (1.07 g, 2.82 mmol) in MeOH (30 mL) was added HCl (6M in iPrOH 30 mL, 6 M, 179 mmol) and the reaction mixture was stirred at RT overnight. The solvents were evaporated and the product was crystallized from ether. Crystals were filtered off and dried to yield intermediate 25 as the hydrochloric acid salt (1.12 g, 112%) .

### ALTERNATIVE PROCEDURE TO INTERMEDIATE 25

### STEP 1. INTERMEDIATE 43

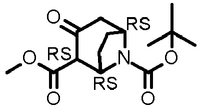

n-Butyllithium (nBuLi, 106.5 mL, 266.3 mmol, 2.5 M in hexanes) was added at 0 °C to a solution of diisopropylamine (26.95 g, 266.3 mmol) in THF (500 mL). The reaction mixture was stirred at 0 °C for 30 min, then it was cooled to -78 °C and treated with a solution of N-Boc-nortropinone (50 g, 221.9 mmol) in THF (75 mL). The resulting mixture was stirred at -78 °C for 90 min, then methyl cyanoformate (22.9 mL, 288.5 mmol) was added. The RM was allowed to warm to RT and stirred overnight. The reaction mixture was quenched with a saturated aqueous NH₄Cl solution, then it was diluted with EtOAc. The organic layer was separated, then washed with water and brine, dried over MgSO₄, filtered and concentrated under reduced pressure. The resulting residue was purified by flash column chromatography on silica gel using as eluent a gradient: 100% DCM to 1% MeOH in DCM, to provide intermediate 43 (60.25 g, 95.8%).

### STEP 2. INTERMEDIATE 44

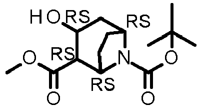

Sodium borohydride (16.02 g, 423.5 mmol) was added to a cold (ice-bath) solution of intermediate 43 (60 g, 211.8 mmol) in methanol (700 mL) and the reaction mixture allowed to stir for 5 h. The completed reaction was quenched with a saturated aqueous NH₄Cl solution, then the solvent was evaporated to dryness under reduced pressure. The resulting residue was dissolved in water and extracted 3xDCM. The combined organic layers were dried over MgSO₄, filtered and concentrated in vacuo, to afford intermediate 44 (59 g, 97.6%) which was used as such without further purification.

### STEP 3. INTERMEDIATE 45

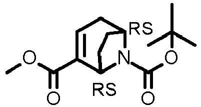

Trifluoroacetic anhydride (57.5 mL, 413.5 mmol) was added dropwise very slowly (CAUTION! Exothermic) to a solution of intermediate 44 (59 g, 206.8 mmol), triethylamine (115 mL, 827.1 mmol) and 4-(dimethylamino) pyridine (2.52 g, 20.7 mmol) in DCM (800 mL), while keeping the temperature below 60 °C. The reaction mixture was stirred at RT overnight, then it was cooled in an ice bath and quenched with water. The pH of the resulting solution was adjusted to 8-8.5 using a saturated aqueous NaHCO₃ solution and allowed to stir at RT for 1 hour. The aqueous was then extracted 3xDCM, then the combined organic layers were dried over MgSO₄, filtered and concentrated in vacuo. The resulting residue was purified by flash column chromatography on silica gel, using as eluent a gradient 100% DCM to 2% MeOH in DCM, to afford Intermediate 45 (33.7 g, 61%).

### STEP 4. INTERMEDIATE 46

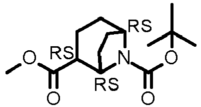

MeOH (59 mL) was added to a flask containing intermediate 45 (2.44 g, 9.13 mmol) and Pd/C (10%) (0.971 g, 0.913 mmol) under a nitrogen atmosphere. This was evacuated and backfilled with hydrogen gas and stirred at RT overnight. The reaction mixture was filtered over a pad of Celite®, then the solvent was removed in vacuo. The resulting residue was purified by flash column chromatography on silica gel, using as eluent a gradient 100% DCM to 2% MeOH in DCM, to afford intermediate 46 (2 g, 81%).

### STEP 5. INTERMEDIATE 47

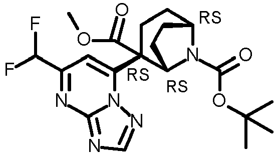

LDA (54.3 mL, 108.7 mmol, 2 M in cyclohexane/ethylbenzene/THF) was added dropwise at -78 °C to -60 °C to a solution of intermediate 46 (24.4 g, 90.6 mmol) in anhydrous THF (363 mL), under an atmosphere of nitrogen. After the addition, the reaction mixture was warmed to -30 °C and stirred for 10 min, before being cooled back to -78 °C. 7-Chloro-5-(difluoromethyl)-[1,2,4]triazolo[1,5-*a*]pyrimidine ([1340394-63-9], 18.5 g, 90.6 mmol) was dissolved in a minimum amount of THF and added dropwise with a syringe. The reaction mixture was stirred at -60 °C for 1 h, then the temperature was raised to RT and the reaction was quenched with a saturated aqueous NH₄Cl solution. The volatiles were evaporated in vacuo and the resulting residue partitioned between EtOAc and water. The layers were separated, then the aqueous layer was extracted 2xEtOAc. The combined OL were washed with brine, dried over MgSO₄, filtered and concentrated under reduced pressure. The resulting residue was crystallized from diethylether, to afford intermediate 47 (23.8 g, 60%). The filtrate was evaporated and the resulting residue was purified by flash column chromatography on silica gel, using as eluent a gradient 100% DCM to 2% MeOH in DCM, to afford intermediate 47 (2 g, 5%), after recrystallization from diethylether.

### Step 6. INTERMEDIATE 25, INTERMEDIATE 25A AND INTERMEDIATE 25B

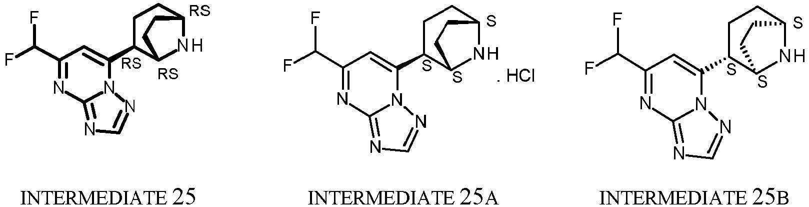

Intermediate 47 (25.8 g, 58.9 mmol) was stirred in a concentrated HCl solution (266 mL, 3.19 mol, 37% in H₂O) at 150 °C overnight. The volatiles were evaporated in vacuo and the resulting residue was co-evaporated twice with toluene. The product was treated with a saturated aqueous NaHCO₃ solution until basic pH. The aqueous layer was extracted with DCM, then the organic layer was dried on MgSO₄, filtered and evaporated under reduced pressure. The resulting residue was purified by flash column chromatography on silica gel, using as eluent a gradient 100% DCM to 8% MeOH/NH₃ in DCM, to afford the intermediate 25 as a racemic mixture (13 g, 79%). This material was purified via Prep SFC (Stationary phase: Chiralpak Diacel AD 20 x 250 mm, Mobile phase: CO₂, EtOH + 0.4 iPrNH₂). The 1*S*,4*S*,5*S*-enantiomer was converted to its HCl salt and recrystallized from CH₃CN to provide intermediate 25a (5.58 g, 30%, Rt = 2.91 min, [α]²⁰_{D} = -66.6 (c = 0.48, DMF)).

### INTERMEDIATE 26

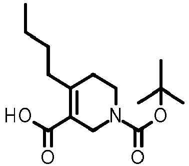

A mixture of 1-tert-butyl 3-methyl 4-(((trifluoromethyl)sulfonyl)oxy)-5,6-dihydropyridine-1,3(2H)-dicarboxylate [161491-25-4] (15 g, 38.53 mmol, prepared according to Angew. Chem. Int. Ed. 2015, 54, 12942-12946), N-butylboronic acid [4426-47-5] (5.89 g, 57.8 mmol), Pd(PPh₃)₄ [14221-01-3] (1.78 g, 1.54 mmol) and Na₂CO₃ [497-19-8] (8.167 g, 77.052 mmol) in 1,4-dioxane [123-91-1] (300 mL) was stirred and heated at 90°C overnight. The volatiles were evaporated in vacuo and the resulting residue treated with an aqueous 1N HCl solution. The aqueous layer was extracted with DCM, then the combined OL was dried over MgSO₄, filtered and evaporated in vacuo. The resulting residue was purified via flash column chromatography on silica gel, using as eluent a gradient DCM-MeOH (9:1, v/v)/DCM, 0/100 to 30/70). This afforded 3.65 g of 1-tert-butyl-5-methyl-4-butyl-3,6-dihydro-2H-pyridine-1,5-dicarboxylate,which was dissolved in a mixture of methanol and aqueous 1 N NaOH solution (1:1, v/v, 200 mL) and stirred overnight at RT. The volatiles were evaporated under reduced pressure, and the resulting residue placed in ice and acidified with an aqueous 1 N HCl solution. The aqueous layer was extracted with chloroform, then the combined OL was dried over MgSO₄, filtered and evaporated under reduced pressure. The resulting residue was purified via flash column chromatography on silica gel, using as eluent a gradient 1% MeOH in DCM to 2% MeOH in DCM, to afford intermediate 26 (1.69 g, 15.5% yield).

### INTERMEDIATE 27

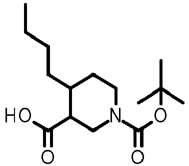

Intermediate 26 (1.47 g, 5.188 mmol) was added to a suspension of Pd/C (10%) (668 mg, 0.63 mmol) in MeOH [67-56-1] (134 mL). The mixture was then placed under an atmosphere of H₂ and stirred for 36 h. After this time, the catalyst was filtered over a pad of Celite and the solvent evaporated under reduced pressure to provide intermediate 27 (1.45 g, 98%).

### INTERMEDIATE 28

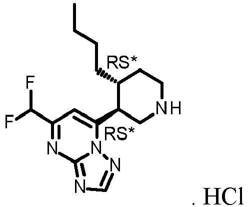

By following a procedure similar to the one reported for the synthesis of intermediate 8, intermediate 28 was synthesised starting from intermediate 27 was obtained intermediate 28 (200 mg).

### INTERMEDIATES 29, 29A AND 29B

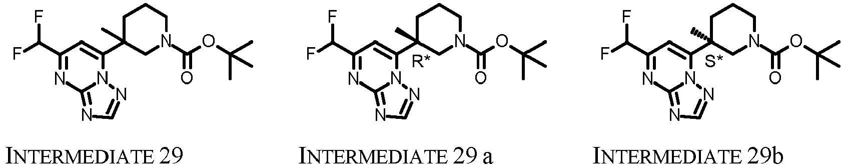

NaH (60% dispersion in mineral oil, 1.5 g, 39.05 mmol) was added to a solution of tert-butyl 3-[5-(difluoromethyl)-[1,2,4]triazolo[1,5-a]pyrimidin-7-yl]piperidine-1-carboxylate which was prepared analogously to intermediate 8 and 11, starting from 3-pyridine carboxylic acid (11.5 g, 32.54 mmol) in DMF (500 mL) at RT and under a flow of N₂. The reaction mixture was stirred for 30 min at RT, then methyl iodide (5.54 g, 39.05 mmol) was added dropwise and the mixture was stirred overnight at RT. Water was added and the product was extracted with EtOAc. The OL was washed with brine, dried over MgSO₄, filtered and evaporated. The resulting residue was purified via prep HPLC (stationary phase: Uptisphere® C18 ODB - 10µm, 200g, 5cm I.D., mobile phase: 0.25% NH₄HCO₃ solution in water, MeOH) to give intermediate 29 (4.32 g, 36%) as a racemic mixture. This was separated in enantiomers by prep SFC (Stationary phase: Chiralcel Diacel OJ 20 x 250 mm, mobile phase: CO₂, iPrOH + 0.2% iPrNH₂) to afford intermediates 29a (2 g, 17%) and 29b (2 g, 17%).

### INTERMEDIATE 30A

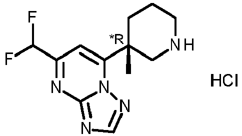

To intermediate 29a (500 mg, 1.36 mmol) in MeOH (20 mL) was added HCl (6M in iPrOH 20 mL, 6 M, 120 mmol) and the reaction mixture was stirred at RT overnight. The solvents were evaporated to give intermediate 30a as the hydrochloric acid salt (400 mg, 97%).

### INTERMEDIATE 30B

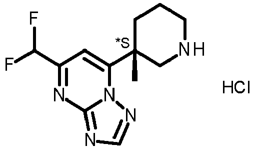

Intermediate 30b was obtained in the same manner as described for intermediate 30a, starting from 29b (400 mg, 97%).

### ALTERNATIVE PROCEDURE TO INTERMEDIATE 29

### Step 1. INTERMEDIATE 31

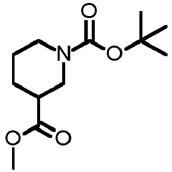

K₂CO₃ (33.15 g, 0.24 mol) was added to a stirred solution of 1-(tert-butoxycarbonyl)piperidine-3-carboxylic acid [84358-12-3] (50.0 g, 0.22 mol) in DMF (600 mL) at RT. Methyl iodide (34.05 g, 0.24 mol) was added after 30 minutes and the reaction mixture was stirred for another 2.5 h at rt. The volatiles were evaporated in vacuo and the resulting residue placed in water and extracted with DIPE. The combined OL was dried over MgSO₄, filtered and evaporated to provide 54.75 g of intermediate 31, which was used as such in the next step.

### Step 2. INTERMEDIATE 32

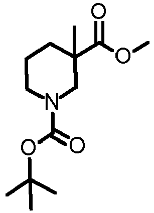

LiHMDS (450 mL, 0.45 mol, 1 M) was added dropwise to a solution of intermediate 31 (54.75 g, 0.22 mmol) in THF (1 L) at -78 °C and under an atmosphere of N₂. The solution was stirred at -78 °C for 1 hour and then methyl iodide (63.9 g, 0.45 mmol) was added dropwise at this temperature. After the addition was completed, the reaction mixture was allowed to warm to RT. The RM was then treated with a saturated aqueous NH₄Cl solution. The aqueous layer was extracted with Et₂O. The combined OL were dried over MgSO₄, filtered and evaporated. The resulting residue was purified via flash column chromatography, using as eluent a 20% solution of EtOAc in heptane, to provide intermediate 32 (55.5 g, 96% yield).

### INTERMEDIATE 29

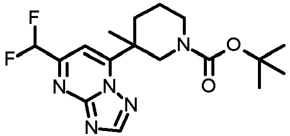

By following a procedure similar to the one reported for the synthesis of intermediate 8, starting from intermediate 32 intermediate 29 (36.7 g, 43.5% yield) was obtained.

### INTERMEDIATE 33

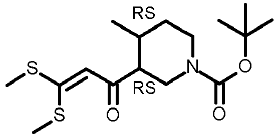

NaH (60% dispersion in mineral oil, 0.97 g, 24.2 mmol) was added to intermediate 6 (5 g, 20.7 mmol) in anhydrous THF (35 mL), in a 250 mL four-necked RBF, at 0 °C under N2 flow. After 10 minutes, carbon disulfide (1.46 mL, 24.1 mmol) was added dropwise and then methyl iodide (2.71 mL, 43.5 mmol) was added dropwise. The mixture was stirred overnight at RT. Water was added and the product was extracted with EtOAc. The OL was washed with brine, dried on MgSO₄, filtered and evaporated yielding intermediate 33 (8.2 g, 92%) which was used as such in the next step.

### INTERMEDIATE 34

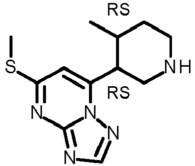

Intermediate 33 (1 g, 2.89 mmol) and 1H-1,2,4-triazol-5-amine hydrochloride (0.35 g, 2.89 mmol) were stirred at 150°C for 3 hours in a melt reaction. The RM was cooled to RT and dissolved in DCM. The OL was washed with satd aq soln NaHCO₃, water, brine, dried on MgSO₄, filtered and evaporated. The product was purified on silicagel, eluent DCM/MeOH, 100/0 to 98/2 yielding intermediate 34 (200 mg, 26%).

### INTERMEDIATE 35

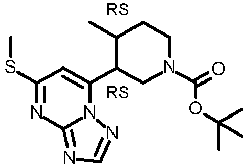

To intermediate 34 (200 mg, 0.76 mmol) in DCM (3.3 mL) was added (Boc)₂O (199 mg, 0.91 mmol), Et₃N (127 uL, 0.91 mmol) and the RM stirred at RT overnight. The solvent was evaporated, then the residue placed in DCM and washed with water (3x), then brine. The OL was separated, dried (MgSO4), filtered and evaporated, to give intermediate 35 (199 mg, 72%) which was used withouth further purification in the next step.

### INTERMEDIATE 36

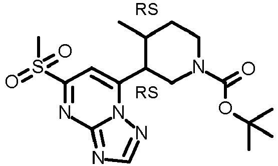

To intermediate 35 (347 mg, 0.95 mmol) in CCl₃ (12 mL) was added mCPBA (659 mg, 3.82 mmol) and the RM was refluxed for 2 hours. The RM was diluted with CHCl₃, washed with NaOH IN, dried on MgSO₄, filtered and evaporated, yielding intermediate 36 (301 mg, 79%) which was used as such in the next step.

### INTERMEDIATE 37

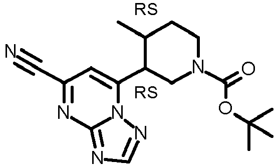

Intermediate 36 (799 mg, 2.02 mmol) and sodium cyanide (202 mg, 4.04 mmol) were stirred in DMSO (5 mL) at RT for 30 minutes. The RM was poured in water and extracted with EtOAc. The OL was washed with brine, dried on MgSO₄, filtered and evaporated to give intermediate 37 (550 mg, 80%) which used as such in the next step.

### INTERMEDIATE 38

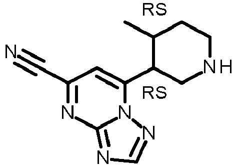

To intermediate 37 (520 mg, 1.52 mmol) in DCM (3.0 mL) was added TFA (6 mL) and the RM was stirred for 1 hours at 0 °C. The RM was poured onto saturated Na₂CO₃ solution, then the OL was washed again with saturated Na₂CO₃, brine, dried on MgSO₄, filtered and evaporated, to give intermediate 38 (273 mg, 73%).

### INTERMEDIATE 39

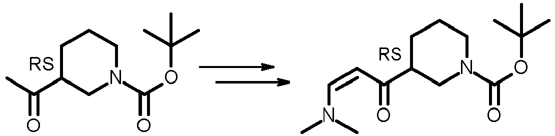

Intermediate 6' (obtained from intermediate 31 following the same transformations as for intermediates 3-6) (50 g, 0.22) was added to N,N-dimethylformamide dimethyl acetal (110 mL) and the mixture was refluxed for 4 days. The reaction mixture was evaporated and additional N,N-dimethylformamide dimethyl acetal was added and the mixture was refluxed for an additional 4 hours. The solvent was evaporated and the product was purified on silicagel, eluent: 1% MeOH in DCM, 2%. The pure fractions were evaporated, yielding intermediate 39 (60 g, 97%).

### INTERMEDIATE 40

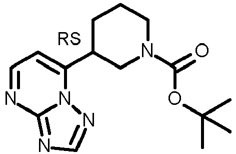

A solution of intermediate 39 (60 g, 0.21 mol) and 1H-1,2,4-triazol-5-amine hydrochloride (22.3 g, 0.27 mol) in acetic acid (53 mL) was stirred at reflux during 1 hour. Water was added and the product was extracted with ether. The OL was washed with brine and dried on MgSO₄, filtered and evaporated, yielding intermediate 40 (62 g, 96%).

### INTERMEDIATE 41

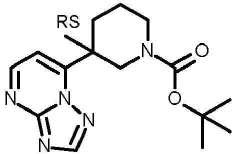

Intermediate 40 (500 mg, 1.65 mmol) was stirred in DMF (50 mL) at RT under N₂ flow. NaH (60% dispersion in mineral oil, 72 mg, 1.8 mmol) was added and the mixture stirred for 30 min. Methyl iodide (2.57 mg, 1.8 mmol) was added and the mixture was stirred for 2 hours at RT. The reaction was quenched with water and evaporated. Water was added and the product was extracted with EtOAc. The OL was washed with brine, dried on MgSO₄, filtered and evaporated. This was purified by Prep HPLC on (RP Vydac Denali C18 - 10µm, 200g, 5 cm; mobile phase (0.25% NH₄HCO₃ solution in water, MeOH), yielding intermediate 41 (250 mg, 48%).

### INTERMEDIATE 42

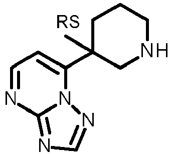

Intermediate 41 (200 mg, 0.85 mmol) was stirred in MeOH (20 mL) and 6N HCl in i-PrOH (23 mL) was added. The mixture was stirred for 1 hour. The RM was evaporated, yielding intermediate 42 (250 mg, 100%).

### INTERMEDIATE 48

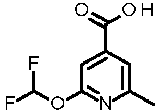

### Step 1. INTERMEDIATE 49

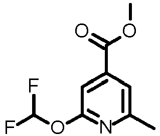

NaH (1.37 g, 34.4 mmol, 60% dispersion in mineral oil) was added to a slurry of methyl 2-hydroxy-6-methylisonicotinate (2.3 g, 13.76 mmol) in ACN (150 mL) at 0 °C and under an atmosphere of N₂. The rm was allowed to reach rt and stirred for 45 min. 2,2-Difluoro-2-(fluorosulfonyl)acetic acid (3.16 g, 17.76 mmol) was then added dropwise to the reaction mixture, which was further stirred at rt for 15 hours. After this time it was quenched with a saturated aqueous solution of NH₄Cl, and the resulting mixture extracted with DCM (100 mL x 3). The combined organic extracts were dried over MgSO₄, filtered and concentrated under reduced pressure. The resulting residue was purified by flash column chromatography using as eluent a gradient heptane/EtOAc, 100/0 to 50/50, to afford intermediate 49 (2.21 g, 73.9%).

### Step 2. INTERMEDIATE 48

An aqueous solution of NaOH (15 mL, 15 mmol, 1 M) was added to a solution of intermediate 49 (2.11 g, 9.72 mmol) in ethanol (15 mL). The resulting mixture was stirred for 1 h, then it was treated with an aqueous solution of HCl (15 mL, 15 mmol, 1 N). A white precipitate was formed and it was filtered, washed with cold water, then dried in the vacuum oven overnight to give intermediate 48 (1.75 g, 88.6%).

### INTERMEDIATE 50

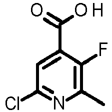

### Step 1. INTERMEDIATE 51

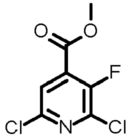

Concentrated H₂SO₄ (0.791 mL, 4.85 mmol, 1.84 g/mL) was added to a solution of 2,6-dichloro-3-fluoro-isonicotinic acid ([149468-00-8], 5.5 g, 26.19 mmol) in MeOH (99 mL), and the resulting mixture was heated to 85 °C for 12 h. After this time, more H₂SO₄ (0.791 mL, 14.846 mmol, 1.84 g/mL) was added and the rm stirred at 90 °C for 24 h. The reaction was cooled to rt, the solvent evaporated and ice was added to the resulting residue. A precipitate formed and it was filtered, washed with ice cold water and dried in the vacuum oven for 2 d, to provide intermediate 51 (4.35 g, 19.4 mmol,) as a beige solid.

### Step 2. INTERMEDIATES 52A AND 52B

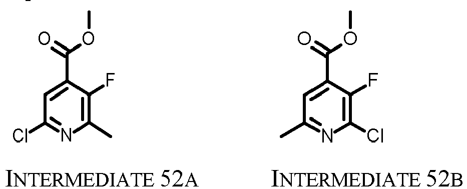

A solution of intermediate 51 (3.95 g, 17.6 mmol) and trimethylboroxine (1.66 mL, 5.82 mmol, 3.5 M in THF) in anhydrous 1,4-dioxane (128 mL) was degassed for 15 minutes. Palladium (II) acetate (198 mg, 0.88 mmol), tricyclophenylphosphine (494 mg, 1.76 mmol) and potassium phosphate tribasic (11.23 g, 53.0 mmol) were added sequentially and the resulting mixture was degassed, then stirred and heated at 110 °C for 18 h in a pressure tube. The rm was cooled to rt and the solvent evaporated in vacuo. The resulting residue was partitioned between water and EtOAc. The resulting biphasic mixture was separated and the aqueous layer extracted with EtOAc (3x). The combined organic layers were washed with brine (1 x), dried over MgSO₄, filtered and the solvent evaporated in vacuo. The resulting residue was purified via flash column chromatography on silicagel, using as eluent a gradient heptane/EtOAc, 100/0 to 90/10, to provide intermediate 52a (980 mg, 27%) and its regioisomer 52b (460 mg, 12.8%).

### Step 3. INTERMEDIATE 50

A solution of LiOH (277 mg, 11.5 mmol) in water (10 mL) was added to a stirred solution of intermediate 52a (785 mg, 3.85 mmol) in THF (10 mL). The rm was stired at rt for 1.5 h, then the volatiles were evaporated in vacuo. The pH of the resulting aqueous residue was brought to ∼2 by treatment with an aqueous HCl (1 M) solution. A white precipitate was formed and it was filtered, washed with ice cold water and dried in the vaccum oven for 2 d, to give intermediate 50 (491mg, 67%) as a white solid.

### INTERMEDIATE 53

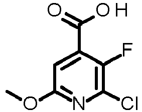

### Step 1. INTERMEDIATE 54

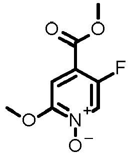

Methyltrioxorhenium (VII) (0.443 g, 1.78 mmol) was added in one portion to a cold (0 °C) solution of methyl 5-fluoro-2-methoxyisonicotinate (4 g, 21.6 mmol) in DCM (71 mL), followed by dropwise addition of hydrogen peroxide (64.3 mL, 0.735 mol, 35% in water). The rm was left to warm to rt, and then heated to 35 °C. After 24 h, more methyltrioxorhenium (VII) (0.2 g, 0.80 mmol) was added and the rm was left stirring at 35 °C for 2 d. Portions of methyltrioxorhenium (VII) (0.2 g, 0.802 mmol) and hydrogen peroxide (30 mL, 0.343 mol, 35% in water) were added 3x over 8 h, and the rm stirred at 27 °C for 16 h. The rm was cooled in an ice bath, then it was quenched by portionwise addition of manganese dioxide (CAUTION! Strong gas and heat evolution) until gas evolution ceased, giving a black mixture. This was combined with another reaction mixture batch resulting from a reaction starting with 1g of methyl 5-fluoro-2-methoxyisonicotinate. The combined reaction mixtures were filtered through a Celite® plug, which was washed with DCM. The biphasic filtrate was separated and the aqueous phase was extracted with DCM (3 x 20 mL). The combined organic layers were dried over MgSO₄, filtered and evaporated in vacuo. The resulting residue (4.9 g) was purified via flash column chromatography on silica gel, using as eluent a gradient heptane/EtOAc, 100/0 to 0/100, to intermediate 54 (585 mg).

### Step 2. INTERMEDIATE 55

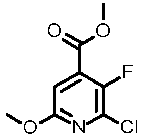

A suspension of intermediate 54 (585 mg, 2.91 mmol) in phosphorus oxychloride (4.1 mL, 43.6 mmol) was stirred and warmed to 105 °C in an oil bath. After 1.5 h, the solvent was removed in vacuo. Ice (10 mL) was added to the resulting residue and the aqueous mixture was extracted with DCM (3x 20 mL). The combined organic layers were washed with brine, dried over MgSO₄ and evaporated in vacuo. The resulting residue (580 mg) was purified by column chromatography on silica gel, using as eluent a gradient heptane/DCM, 100/0 to 50/50, to provide intermediate 55 (522 mg, 72.7%,)

### STEP 3. INTERMEDIATE 53

A solution of LiOH (152 mg mg, 6.35 mmol) in water (5.7 mL) was added to a stirred solution of intermediate 55 (522 mg, 2.12 mmol) in THF (5.7 mL). The rm was stirred at rt for 1.5 h, then the volatiles were evaporated in vacuo. The pH of the resulting aqueous residue was brought to ∼3 by treatment with an aqueous HCl (1 M) solution. A white precipitate was formed and it was filtered, washed with ice cold water and dried in the vaccum oven to give intermediate 53 (172 mg, 39%) as a white solid. The filtrate was evaporated to dryness under reduced pressure, to give the lithium salt of compound 53 (366 mg).

### B-SYNTHESIS OF FINAL COMPOUNDS

### COMPOUND 1A AND COMPOUND 1B

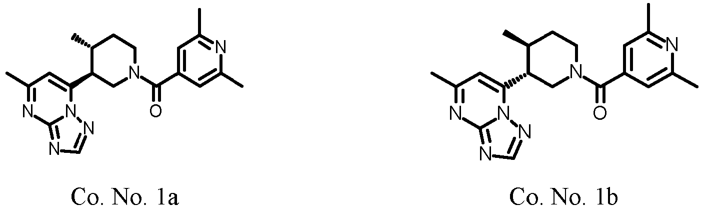

A mixture of I-12 (0.27 g, 0.001 mol) and 2,6-dimethylisonicotinic acid (0.16 g, 0.001 mol) in DCM (10 mL) was treated with DIPEA (0.69 mL, 0.75 g/mL, 0.004 mol) and HBTU (0.38 g, 0.001 mol). Stirring was continued for 16 h. The RM was diluted with water (5 mL), acidified with 1 M HCl until pH∼3 and the layers were separated and the OL was washed with 1 M NaOH until pH∼9, water, then dried over MgSO₄, filtered and concentrated *in vacuo* to give an oil (0.8 g). A purification was performed via Prep HPLC (stationary phase: RP XBridge® Prep C18 OBD-10µm, 50 x150mm, mobile phase: 0.25% NH₄HCO₃ solution in water, MeOH) yielding two fractions. A purification was performed using Prep SFC (stationary phase: Chiralpak® Diacel AD 20 x 250 mm, mobile phase: CO₂, EtOH with 0.4% iPrNH₂) yielding 4 fractions of which two afforded compounds 1b (64 mg, 18%) and 1a (70 mg, 19%).

Compounds 2a to 3b were prepared in an analogous manner to compounds 1a and 1b from the indicated starting material:

| Co. No. | Structure | Prepared from | Yield (%) |
|---|---|---|---|
| 2a | 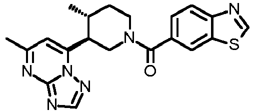 | I-12a | 82 |
| 2b | 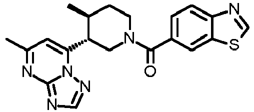 | | 82 |
| 3a | 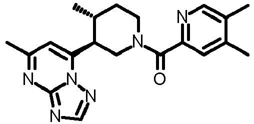 | I-12a [802256-42-4] | 12 |
| 3b | 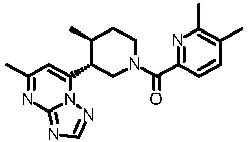 | I-12b | |

### COMPOUND 4

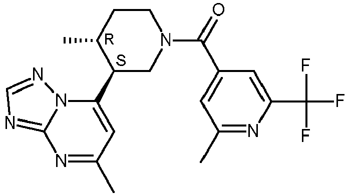

2-Methyl-6-(trifluoromethyl)isonicotinic acid (101 mg, 0.493 mmol) was stirred in DCM (20 mL). DIPEA (0.34 mL, 0.75 g/mL, 1.97 mmol) and HBTU (206 mg, 0.542 mmol) were added, stirring was continued for 20 min at RT. I-13a (150 mg, 0.493 mmol) was added and stirring was continued overnight at RT. The RM was quenched with water, stirred 20 min then the OL was separated. The aqueous layer was back extracted 2x DCM. The combined organic layers were washed with brine, then separated and dried over MgSO₄, filtered and concentrated *in vacuo.* This material was purified by silicagel flash chromatography eluting with 0-5% MeOH in DCM to afford crude compound 4 (120 mg, yield 52.931%). A purification was performed via Prep HPLC (stationary phase: RP Vydac Denali C18 - 10µm, 200g, 5cm I.D., mobile phase: 0.25% NH₄HCO₃ solution in water, MeOH) yielding, after co-evaporation with MeOH and drying in the vacuum oven overnight, compound 4 (74 mg, 36%).

Compounds 5 to 23 were prepared in a similar way as compound 6 using enantiopure intermediate I-13a.

| Co. No. | Structure | Yield (%) |
|---|---|---|
| 5 | 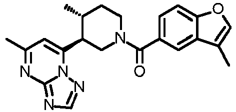 | 54 |
| 6 | 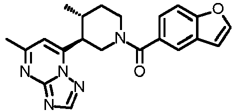 | 61 |
| 7 | 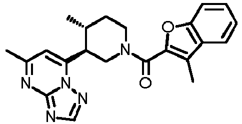 | 56 |
| 8 | 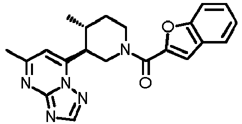 | 48 |
| 9 | 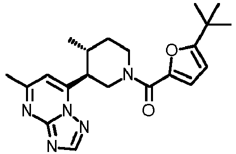 | 58 |
| 10 | 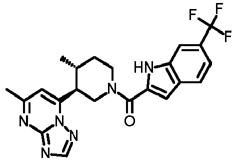 | 8 |
| 11 | 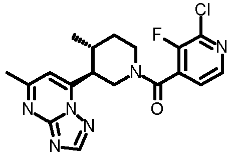 | 10 |
| 12 | 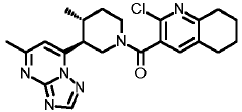 | 57 |
| 13 | 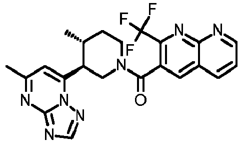 | 55 |
| 14 | 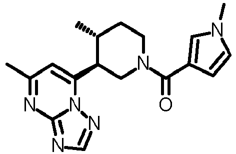 | 45 |
| 15 | 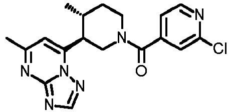 | 11 |
| 16 | 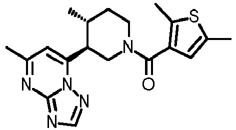 | 48 |
| 17 | 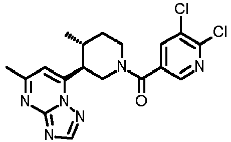 | 7.8 |
| 18 | 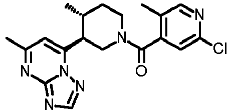 | 19 |
| 19 | 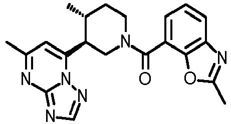 | 55 |
| 20 | 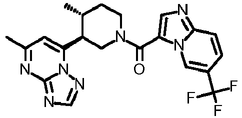 | 50 |
| 21 | 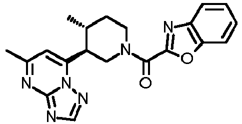 | 42 |
| 22 | 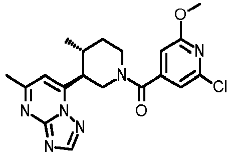 | 58 |
| 23 | 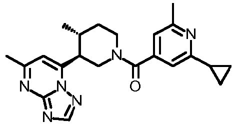 | 52 |

### COMPOUND 24A AND COMPOUND 24B

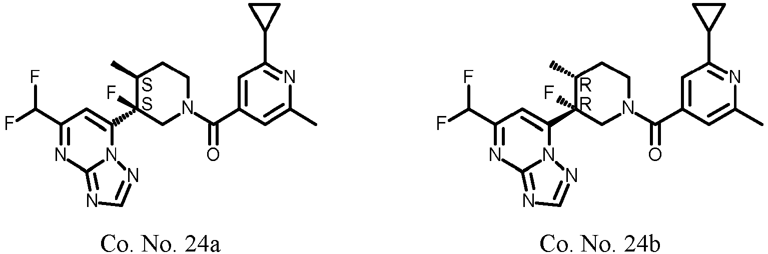

To a stirred mixture of 2-cyclopropyl-6-methyl-pyridine-4-carboxylic acid (0.16 g, 0.00075 mol) in DCM (20 mL, 1.33 g/mL, 0.31 mol) was added HBTU (0.28 g, 0.00075 mol) and DIPEA (0.54 mL, 0.75 g/mL, 0.0031 mol). After the mixture was stirred 30 min, 1-19 (0.2 g, 0.00062 mol) was added in one portion. The RM was left stirring for 1 h and then 1 M NaOH (5 mL) was added, the layers were separated and the OL was washed with water (10 mL), dried over MgSO₄, filtered and concentrated in vacuo to afford an oil. This was purified by flash chromatography using a 24 g Redisep® Flash column eluting with a gradient of 0-5% MeOH in DCM to afford an oil was which crystallized from DIPE (10 mL) to afford a white solid (0.23 g, yield 83.2%). A purification was performed via Prep SFC (stationary phase: Chiralpak® Diacel AD 30 x 250 mm, mobile phase: CO₂, iPrOH with 0.2% iPrNH₂) to afford the two fractions. Both fractions were transferred to tubes and dried in a vacuum oven (45°C, 16 h) and this afforded amorphous solids Co. No. 24a (0.11 g, yield 39.7%) and Co. No. 24b (0.11 g, yield 38.3%).

### COMPOUNDS 25A AND 25B

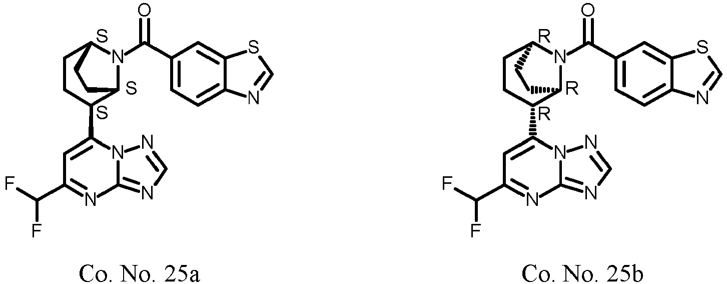

Benzothiazole-6-carboxylic acid (142 mg, 0.792 mmol) was stirred in DCM (15 mL), DIPEA (0.82 mL, 4.8 mmol) and HBTU (300 mg, 0.792 mmol) were added. Stirring was continued for 0.5 h at RT. Intermediate 25 (250 mg, 0.792 mmol) was added to the solution and stirring was continued for 2 h at RT. NaOH solution (IN, 1 mL) was added and stirred for 5 min. The product filtered on an extrelute filter and the filtrate was evaporated. The product was purified on silica gel, eluent: DCM -> 4% MeOH in DCM. The pure fractions were evaporated to give a mixture of compounds 25a and 25b (340 mg). This was purified via Prep SFC (Stationary phase: Chiralcel® Diacel OD 20 x 250 mm, Mobile phase: CO₂, EtOH + 0.4 iPrNH2) to give both products which were crystallized from Et₂O and afforded Co. No. 25a (121 mg, 35%) and Co. No. 25b (128 mg, 37%).

### COMPOUNDS 26A AND 26B

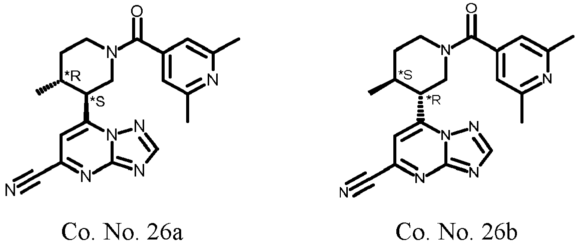

2,6-Dimethylisonicotinic acid (154.845 mg, 1.0 mmol) was stirred DCM (10 mL). DIPEA (0.53 mL, 3.1 mmol) and HBTU (427 mg, 1.1 mmol) were added, stirring was continued for 0.5 hours at RT. Intermediate 38 (273 mg, 1.1 mmol) was dissolved in DCM (5 mL) and this mixture was added to the solution, which was stirred was continued for 3.5 hours at room temperature. The RM was quenched with water, then the two layers were separated and the WL back-extracted with DCM. The OL was dried on MgSO₄, filtered and evaporated. The product was purified on silica gel, eluent: DCM/MeOH, 100/0 to 97/3 to 94/6 which afforded the two pairs of diastereomers as a mixture. A purification was performed via Prep SFC (Stationary phase: Chiralcel® Diacel OD 20 x 250 mm, Mobile phase: CO₂, EtOH + 0.4 iPrNH₂) which afforded the two trans enantiomers. A purification was performed via Prep HPLC (Stationary phase: RP XBridge Prep C18 ODB- 5µm,30x250mm, Mobile phase: 0.25% NH₄HCO₃ solution in water, CH₃CN to give compound 26a (26 mg, yield 6.761%) and 26b (20 mg, yield 5.201%).

### COMPOUND 321

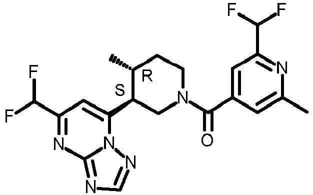

Intermediate 9b (. 2HCl, 1.2 g, 3.527 mmol), 2-(difluoromethyl)-6-methyl-4-pyridinecarboxylic acid (660 mg, 3.527 mmol), EDCI (1.352 g, 7.053 mmol), and DIPEA (1.823 g, 14.107 mmol) in DCM (71.4 mL) were stirred at RT for 4 h. The RM was washed with NaOH IN, the OL was dried over MgSO₄, filtered and evaporated. The product was purified on silicagel, eluent MeOH/DCM 0/100 to 3/97. The pure fractions were evaporated and crystallized from DIPE. The crystals were filtered off and dried to give compound 321 (920 mg, 60%).

### COMPOUND 333

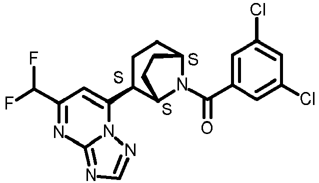

To a stirred solution of intermediate 25a (100 mg, 0.317 mmol) in dry ACN (5 mL), was added 3,5-dichlorobenzoic acid (72.593 mg, 0.38 mmol). TEA (0.22 mL, 1.58 mmol) was added, followed by 1-propanephosphonic anhydride ([68957-94-8], 0.28 mL, 0.48 mmol) and the mixture was stirred at RT for 3 h, giving a white precipitate in a brown solution. The solvent was removed in vacuo, and the crude was partitioned between DCM (10 mL) and a saturated aqueous NaHCO₃ solution (10 mL). The OL was washed with a saturated aqueous Na₂CO₃ solution (1x15 mL), dried (MgSO₄), filtered, the solvent was removed in vacuo, and coevaporated with toluene (1x20 mL), giving a white precipitate in a brown solution, which was dried overnight to give brown and white crystals. Recrystallisation from Et₂O yielded off white crystals, which were oven dried overnight in a 40°C vaccum oven, giving compound 333 (88 mg, 61%), as off white crystals.

Table 1 below lists additional compounds that were prepared by analogy to the above Examples. In case no salt form is indicated, the compound was obtained as a free base. 'Co. No.' means compound number. Reagents used in the synthesis of the compounds are either commercially available or can be made by procedures known to the skilled person. Compounds made by analogy to compound 321 are indicated (N.B. EDCI coupling); compounds made by analogy to compound 333 are indicated (N.B. phosphonic acid anhydride) in the reagent column.

**TABLE 1**

| Co. No. | Structure | Prepared from | Yield (%) |
|---|---|---|---|
| 27 | 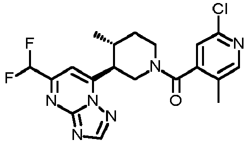 | I-9b | 78 |
| 28 | 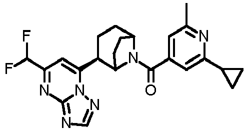 | 1-25 | 35 |
| 29 | 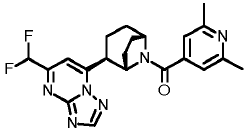 | 1-25 | 39 |
| 30 | 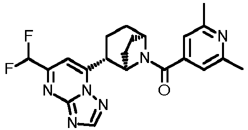 | 1-25 | 38 |
| 31 | 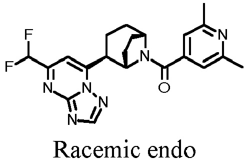 | 1-25 | 26 |
| 32 | 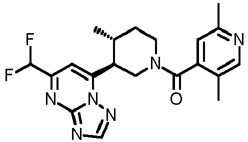 | I-9b | 55 |
| 33 | 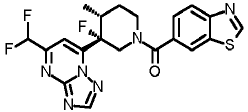 | I-19 | 44 |
| 34 | 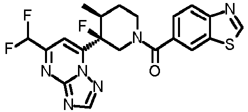 | I-19 | 41 |
| 35 | 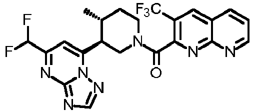 | I-9b | 68 |
| 36 | 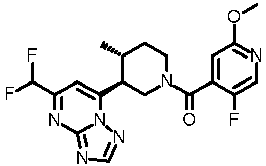 | I-9b | 73 |
| 37 | 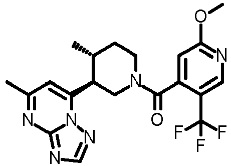 | I-12a | 52 |
| 38 | 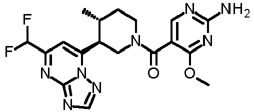 | I-9b | 41 |
| 39 | 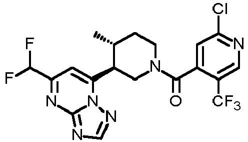 | I-9b | 21 |
| 40 | 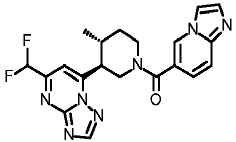 | I-9b | 78 |
| 41 | 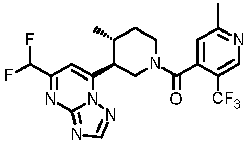 | I-9b | 38 |
| 42 | 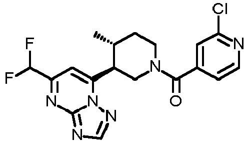 | I-9b | 60 |
| 43 | 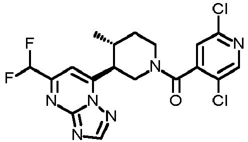 | I-9b | 55 |
| 44 | 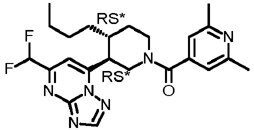 | 1-28 | 29 |
| | trans mixture | | |
| 45 | 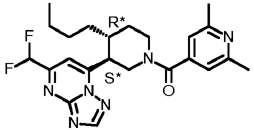 | I-28 | 12 |
| | pure enantiomer, trans | | |
| 46 | 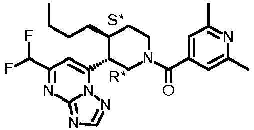 | I-28 | 12 |
| | pure enantiomer, trans | | |
| 47 | 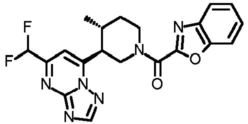 | I-9b | 44 |
| 48 | 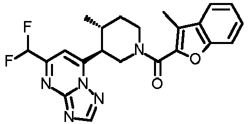 | I-9b | 77 |
| 49 | 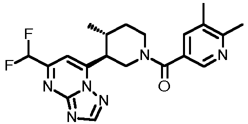 | I-9b | 62 |
| 50 | 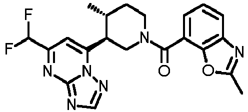 | I-9b | 68 |
| 51 | 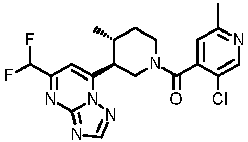 | I-9b | 31 |
| 52 | 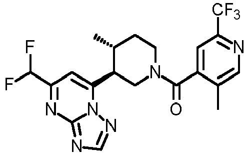 | I-9b | 6 |
| 53 | 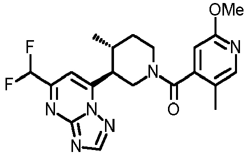 | I-9b | 17 |
| 54 | 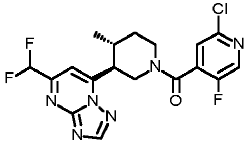 | I-9b | 53 |
| 55 | 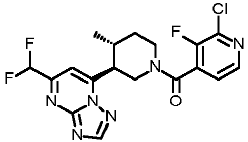 | I-9b | 63 |
| 56 | 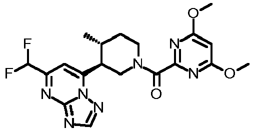 | I-9b | 51 |
| 57 | 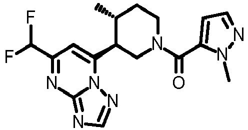 | I-9b | 61 |
| 58 | 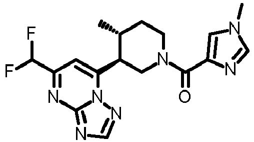 | I-9b | 75 |
| 59 | 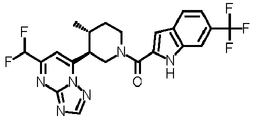 | I-9b | 72 |
| 60 | 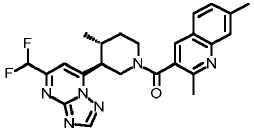 | I-9b | 64 |
| 61 | 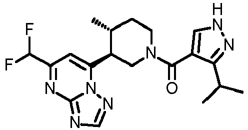 | I-9b | 73 |
| 62 | 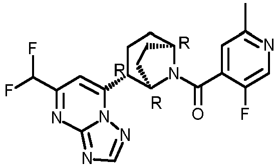 | I-25 | 42 |
| 63 | 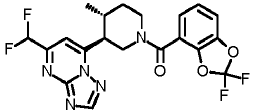 | I-9b | 40 |
| 64 | 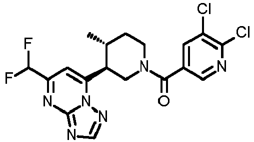 | I-9b | 60 |
| 65 | 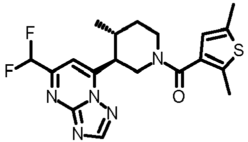 | I-9b | 49 |
| 66 | 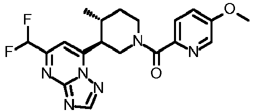 | I-9b | 57 |
| 67 | 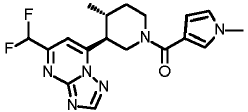 | I-9b | 63 |
| 68 | 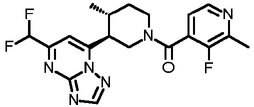 | I-9b | 48 |
| 69 | 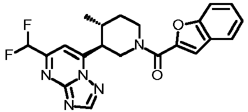 | I-9b | 78 |
| 70 | 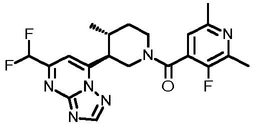 | I-9b | 58 |
| 71 | 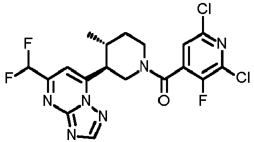 | I-9b | 73 |
| 72 | 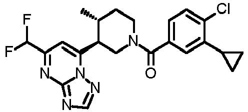 | I-9b | 38 |
| 73 | 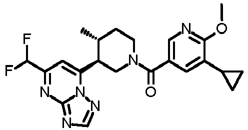 | I-9b | 39 |
| 74 | 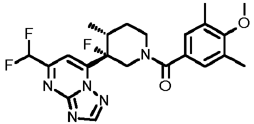 | I-19 | 30 |
| 75 | 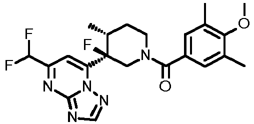 | I-19 | 32 |
| 76 | 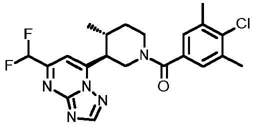 | I-9b | 36 |
| 77 | 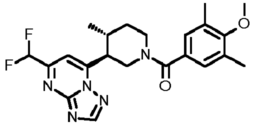 | I-9b | 42 |
| 78 | 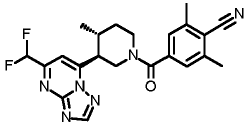 | I-9b | 58 |
| 79 | 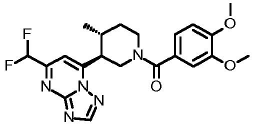 | I-9b | 74 |
| 80 | 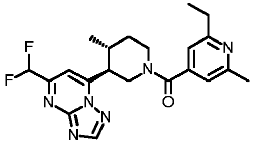 | I-9b | 54 |
| 81 | 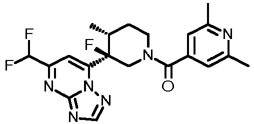 | I-19 | 30 |
| 82 | 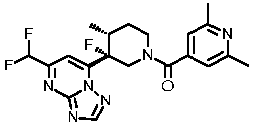 | I-19 | 31 |
| 83 | 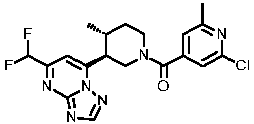 | I-9b | 81 |
| 84 | 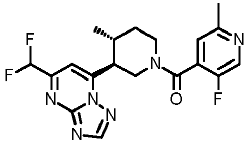 | I-9b | 30 |
| 85 | 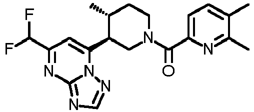 | I-9b | 22 |
| 86 | 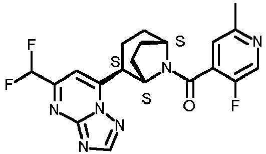 | I-25 | 41 |
| 87 | 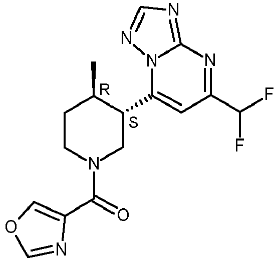 | I-9b | 65 |
| 88 | 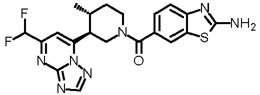 | I-9b [332898-48-3] | 27 |
| 89 | 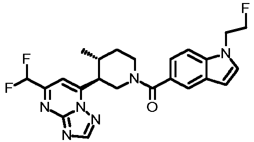 | I-9b | 46 |
| 90 | 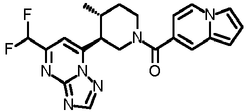 | I-9b [1533853-53-0] | 73 |
| 91 | 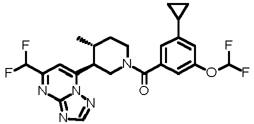 | I-9b | 33 |
| 92 | 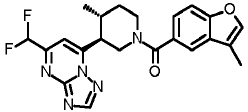 | I-9b [501892-99-5] | 61 |
| 93 | 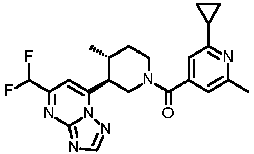 | I-9b | 15 |
| 94 | 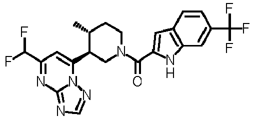 | I-9b [327-20-8] | 25 |
| 95 | 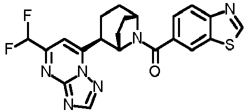 | I-25 | 36 |
| 96 | 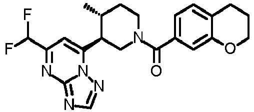 | I-9b [103203-84-5] | 83 |
| 97 | 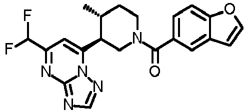 | I-9b [90721-27-0] | |
| 98 | 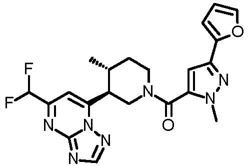 | I-9b [859851-00-6] | 60 |
| 99 | 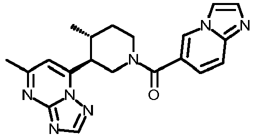 | I-9b [139022-25-6] | 24 |
| 100 | 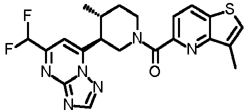 | I-9b [1372924-05-4] | 53 |
| 101 | 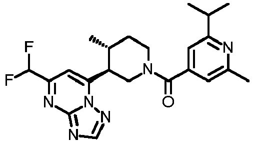 | I-9b [1011264-07-5] | 28 |
| 102 | 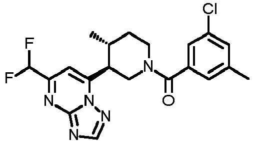 | I-9b [1427392-05-9] | 61 |
| 103 | 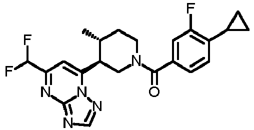 | I-9b [1247451-23-5] | 48 |
| 104 | 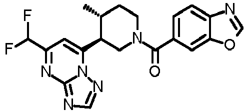 | I-9b [154235-77-5] | 21 |
| 105 | 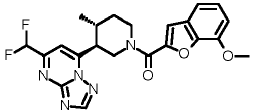 | I-9b [4790-79-8] | 60 |
| 106 | 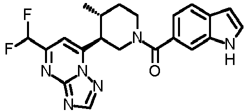 | I-9b [1670-82-2] | 76 |
| 107 | 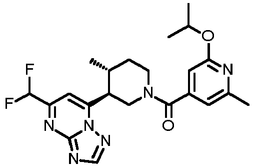 | I-9b [1011264-06-4] | 47 |
| 108 | 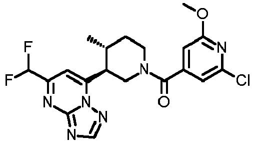 | I-9b [15855-06-8] | 49 |
| 109 | 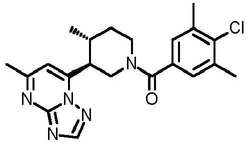 | I-9b [90649-78-8] | 21 |
| 110 | 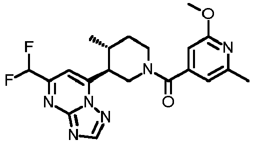 | I-9b [1803602-19-8] | 51 |
| 111 | 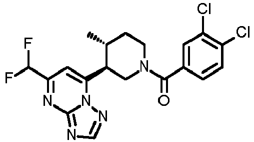 | I-9b [51-44-5] | 9 |
| 112 | 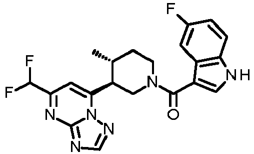 | I-9b [23077-43-2] | 41 |
| 113 | 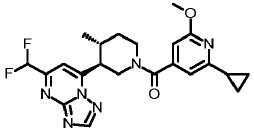 | I-9b [1256820-02-6] | 42 |
| 114 | 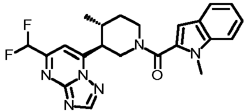 | I-9b [16136-58-6] | 64 |
| 115 | 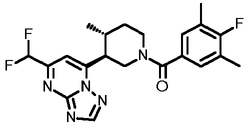 | I-9b [120512-59-6] | 64 |
| 116 | 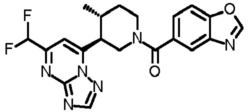 | I-9b [15112-41-1] | 73 |
| 117 | 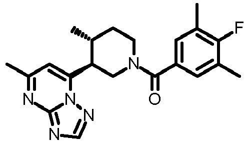 | I-12a [120512-59-6] | 27 |
| 118 | 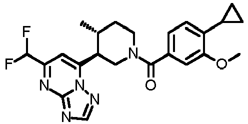 | I-9b [1248462-73-8] | 46 |
| 119 | 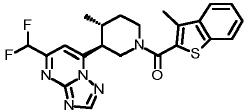 | I-9b [3133-78-6] | 67 |
| 120 | 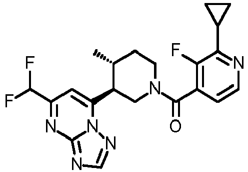 | I-9b | 45 |
| 121 | 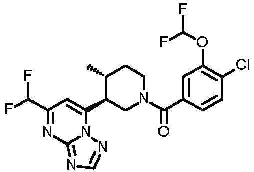 | I-9b [1427355-37-0] | 65 |
| 122 | 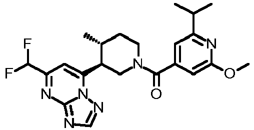 | I-9b [1256790-25-6] | 36 |
| 123 | 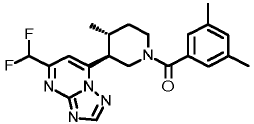 | I-9b [6613-44-1] | 38 |
| 124 | 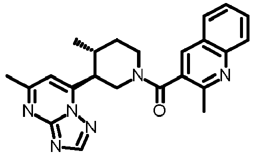 | I-9b [470702-35-3] | 60 |
| 125 | 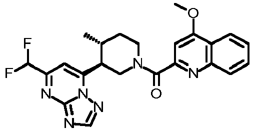 | I-9b [15733-83-2] | 74 |
| 126 | 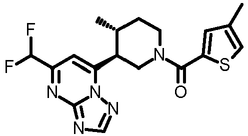 | I-9b [14282-78-1] | 35 |
| 127 | 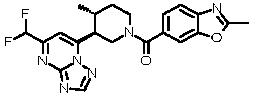 | I-9b [13452-14-7] | 29 |
| 128 | 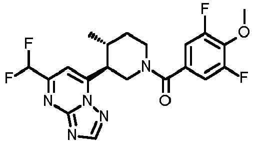 | I-9b [319-60-8] | 63 |
| 129 | 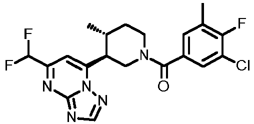 | I-9b [1427392-05-9] | 44 |
| 130 | 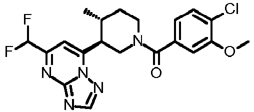 | I-9b [85740-98-3] | 63 |
| 131 | 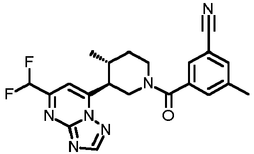 | I-9b [78621-81-5] | 46 |
| 132 | 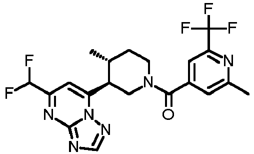 | I-9b [1211590-99-6] | 45 |
| 133 | 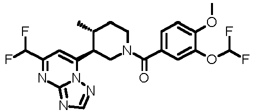 | I-9b [162401-65-2] | 61 |
| 134 | 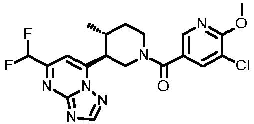 | I-9b [884494-85-3] | 59 |
| 135 | 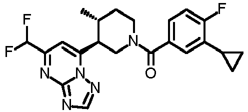 | I-9b | 53 |
| 136 | 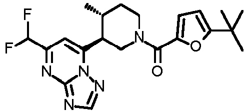 | I-9b [56311-39-8] | 53 |
| 137 | 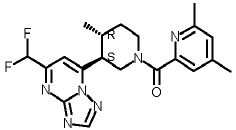 | I-9b 4,6-dimethylpyridine-2-carboxylic acid [18088-10-3] | 34 |
| 138 | 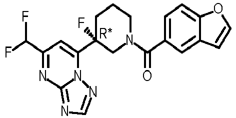 | I-19 Benzofuran-5-carboxylic acid [90721-27-0] | 25 couplin g, 40% SFC) |
| 139 | 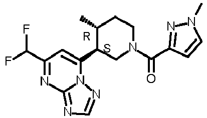 | I-9b 1-methyl-1H-pyrazole-3-carboxylic acid [25016-20-0] | 67 |
| 140 | 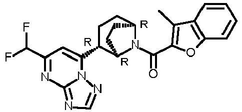 | I-25 3-methylbenzofuran-2-carbonyl chloride [2256-86-2] | 36 |
| 141 | 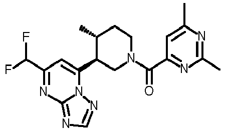 | I-9b 2,6-dimethylpyrimidine-4-carboxylic acid [54198-74-2] | 14 |
| 142 | 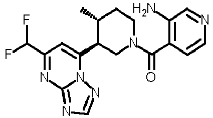 | I-9b 3-aminopyridine-4-carboxylic acid [7529-20-6] | 59 |
| 143 | 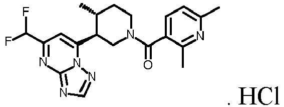 | I-9b 2,6-dimethyl-nicotinic acid [5860-71-9] | 44 |
| 144 | 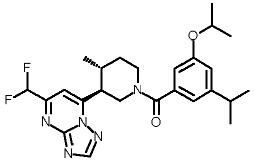 | I-9b 5-(tert-butyl)isoxazole-3-carboxylic acid [90607-21-9] | 52 |
| 145 | 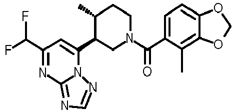 | I-9b 4-methyl-2H-1,3-benzodioxole-5-carboxylic acid [162506-58-3] | 60 |
| 146 | 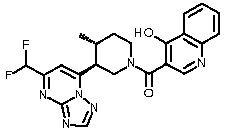 | I-9b 4-hydroxyquinoline-3-carboxylic acid [34785-11-0] | 19 |
| 147 | 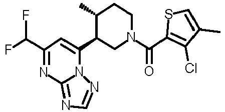 | I-9b 3-chloro-4-methylthiophene-2-carboxylic acid [229342-86-3] | 52 |
| 148 | 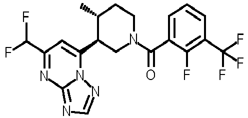 | I-9b 2-fluoro-3-(trifluoromethyl)benz oic acid [115029-22-6] | 63 |
| 149 | 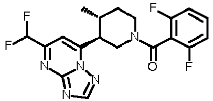 | I-9b 2,6-difluorobenzoic acid [385-00-2] | 54 |
| 150 | 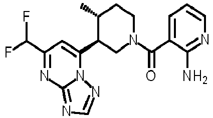 | I-9b 2-aminonicotinic acid [5345-47-1] | 67 |
| 151 | 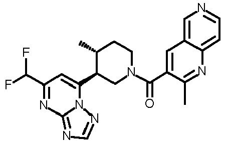 | I-9b 2-methyl-1,6-naphthydrine-3-carboxylic acid [387350-63-2] | 75 |
| 152 | 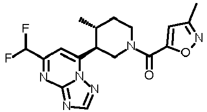 | I-9b 3-methylisoxazole-5-carboxylic acid [4857-42-5] | 63 |
| 153 | 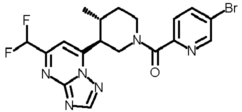 | I-9b 5-bromopyridine-2-carboxylic acid [30766-11-1] | 45 |
| 154 | 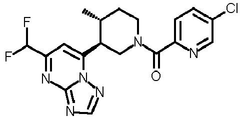 | I-9b 5-chloropyridine-2-carboxylic acid [36070-80-1] | 65 |
| 155 | 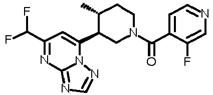 | I-9b 3-fluoro-4-pyridinecarboxylic acid [131307-35-2] | 60 |
| 156 | 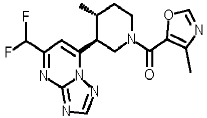 | I-9b 4-methyloxazole-5-carboxylic acid [2510-32-9] | 65 |
| 157 | 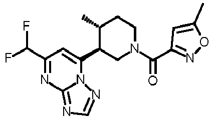 | I-9b 5-methylisoxazole-3-carboxylic acid [3405-77-4] | 65 |
| 158 | 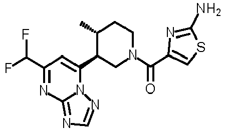 | I-9b 2-aminothiazole-4-carboxylic acid [112539-08-9] | 68 |
| 159 | 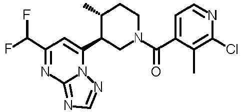 | I-9b 2-chloro-3-methylisonicotinic acid [133928-73-1] | 54 |
| 160 | 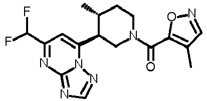 | I-9b 4-methylisoxazole-5-carboxylic acid [261350-46-3] | 52 |
| 161 | 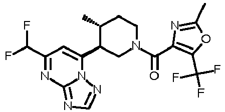 | I-9b 2-methyl-5-(trifluoromethyl)-oxazole-4-carboxylic acid [18955-88-9] | 68 |
| 162 | 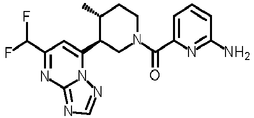 | I-9b 6-amino-pyridine- 2-carboxylic acid [23628-31-1] | 53 |
| 163 | 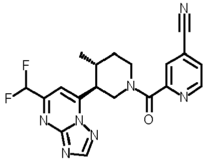 | I-9b 4-cyanopyridine-2-carboxylic acid [640296-19-1] | 67 |
| 164 | 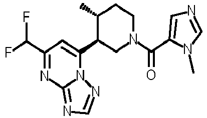 | I-9b 1-methyl-1H-imidazole-5-carboxylic acid [41806-40-0] | 69 |
| 165 | 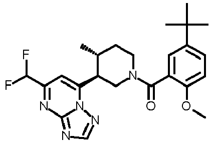 | I-9b 5-tert-butyl-2-methoxybenzoic acid [73469-54-2] | 61 |
| 166 | 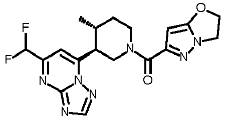 | I-9b 2H, 3H-pyrazolo[3,2-B][1,3]oxazole-6-carboxylic acid [1239722-75-8] | 62 |
| 167 | 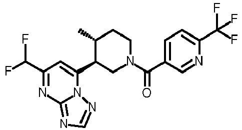 | I-9b 6-(trifluoromethyl)nicot inic acid [231291-22-8] | 48 |
| 168 | 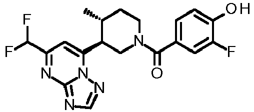 | I-9b [350-29-8] | 25 |
| 169 | 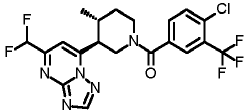 | I-9b [1737-36-6] | 67 |
| 170 | 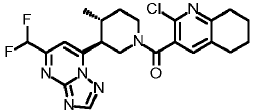 | I-9b [99058-34-1] | 69 |
| 171 | 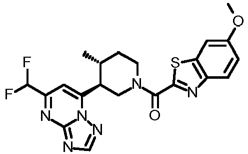 | I-9b [946-13-4] | 40 |
| 172 | 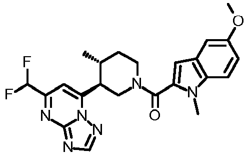 | I-9b [59908-54-2] | 58 |
| 173 | 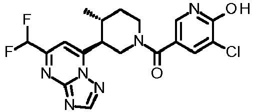 | I-9b [54127-63-8] | 31 |
| 174 | 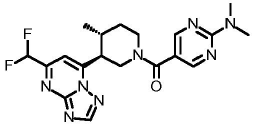 | I-9b [180283-66-3] | 64 |
| 175 | 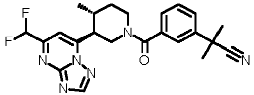 | I-9b [872091-00-4] | 59 |
| 176 | 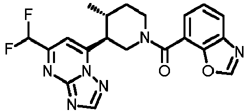 | I-9b [208772-24-1] | 59 |
| 177 | 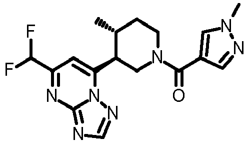 | I-9b [5952-92-1] | 75 |
| 178 | 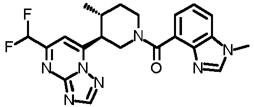 | I-9b [1187732-69-9] | 66 |
| 179 | 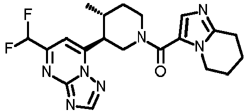 | I-9b [55365-04-3] | 68 |
| 180 | 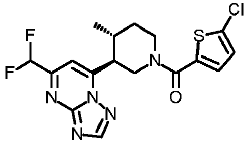 | I-9b [24065-33-6] | 66 |
| 181 | 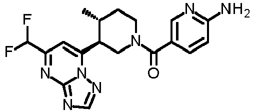 | I-9b [3167-49-5] | 63 |
| 182 | 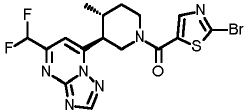 | I-9b [54045-76-0] | 65 |
| 183 | 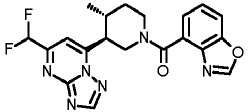 | I-9b [208772-23-0] | 48 |
| 184 | 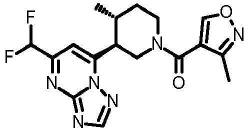 | I-9b [17153-20-7] | 67 |
| 185 | 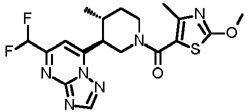 | I-9b [126909-38-4] | 56 |
| 186 | 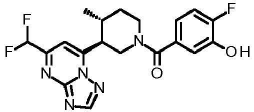 | I-9b [51446-31-2] | 24 |
| 187 | 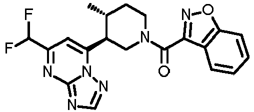 | I-9b [28691-47-6] | 58 |
| 188 | 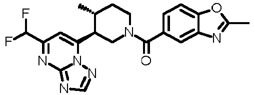 | I-9b [90322-32-0] | 58 |
| 189 | 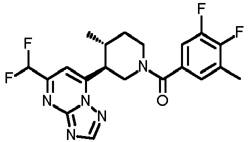 | I-9b [1017778-60-7] | 26 |
| 190 | 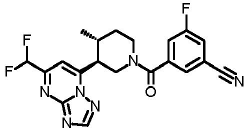 | I-9b [327056-74-6] | 62 |
| 191 | 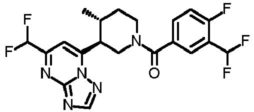 | I-9b | 60 |
| 192 | 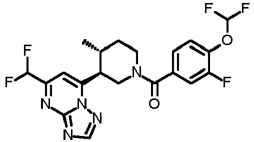 | I-9b [886496-49-7] | 21 |
| 193 | 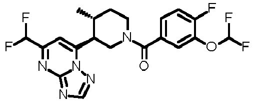 | I-9b [1214383-15-9] | 57 |
| 194 | 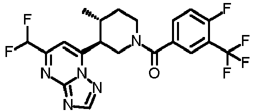 | I-9b [67515-55-3] | 25 |
| 195 | 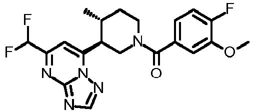 | I-9b [82846-18-2] | 76 |
| 196 | 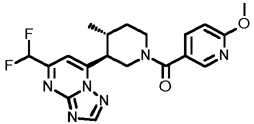 | I-9b [26218-80-4] | 45 |
| 197 | 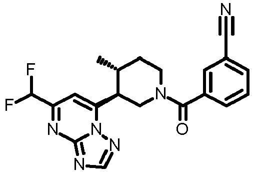 | I-9b [1877-72-1] | 22 |
| 198 | 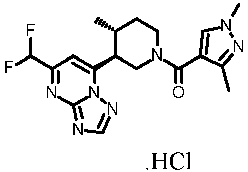 | I-9b [113100-61-1] | 49 |
| 199 | 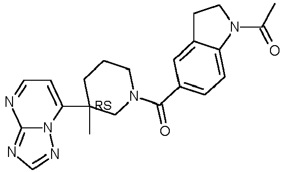 | I-42 | 8.7 |
| 200 | 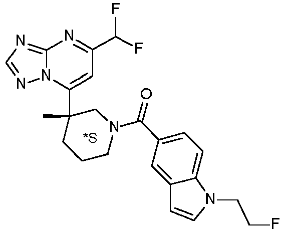 | I-30 | 54 |
| 201 | 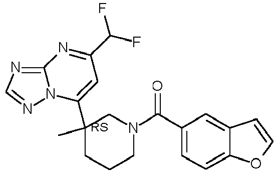 | I-30 | 30 |
| 202 | 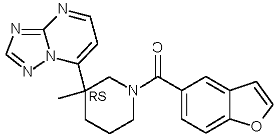 | I-42 | 31 |
| 203 | 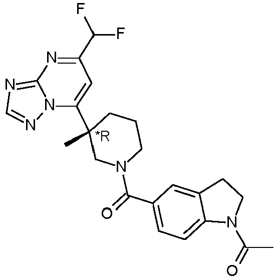 | I-30a | 87 |
| 204 | 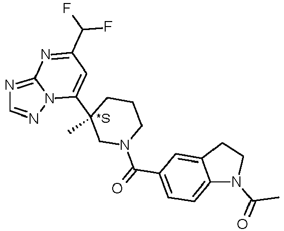 | I-30b | 89 |
| 205 | 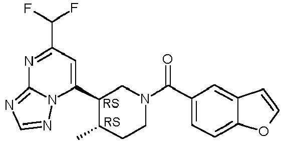 | I-12 | 22 |
| 206 | 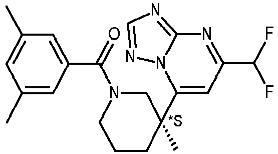 | I-30b | 60 |
| 207 | 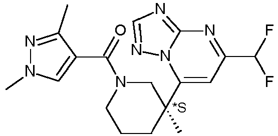 | I-30b | 40 |
| 208 | 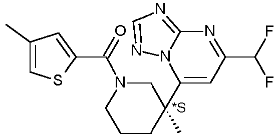 | I-30b | 75 |
| 209 | 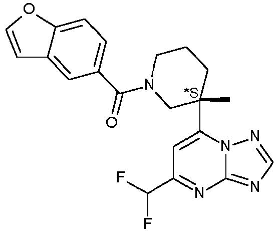 | I-30b | 57 |
| 210 | 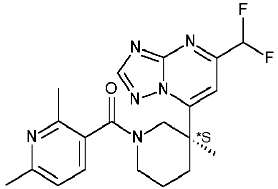 | I-30b | 65 |
| 211 | 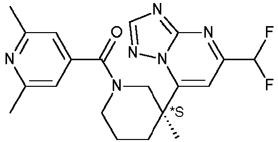 | I-30b | 25 |
| 212 | 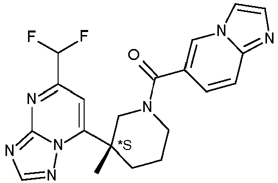 | I-12 | 14 |
| 213 | 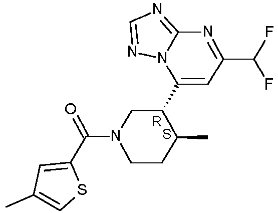 | I-9a | 61 |
| 214 | 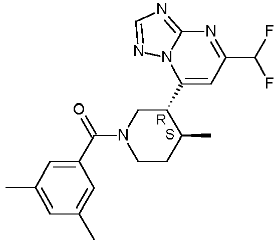 | I-9a | 55 |
| 215 | 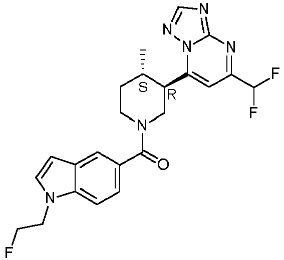 | I-9a | 54 |
| 216 | 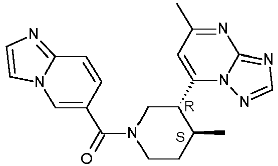 | I-9a | 29 |
| 217 | 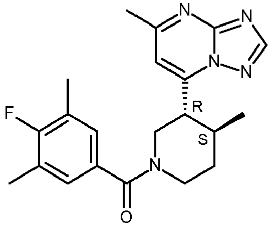 | I-9a | 25 |
| 218 | 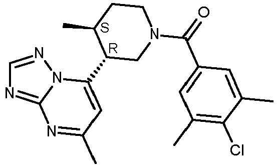 | I-9a | 26 |
| 219 | 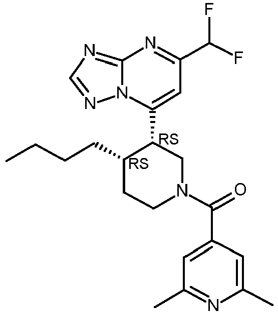 | I-28 | 6 |
| 220 | 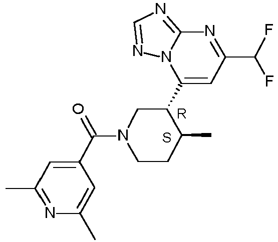 | I-9a | 42 |
| | Free base or .HCl | | |
| 221 | 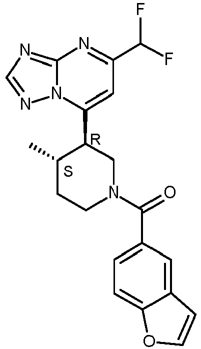 | I-9a | 58 |
| 222 | 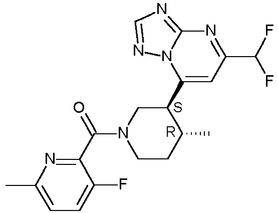 | I-9b | 24 |
| 223 | 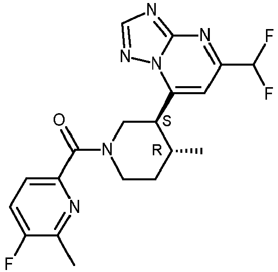 | I-9b | 37 |
| | .1.30 HCl. H₂O | | |
| 224 | 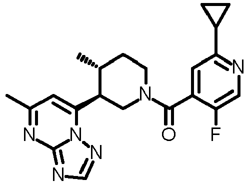 | I-12a | 55 |
| 225 | 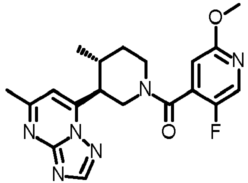 | I-12a | 58 |
| 226 | 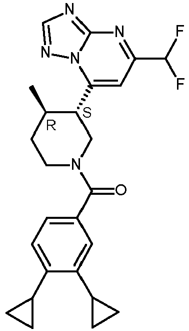 | I-9b | 14 |
| 227 | 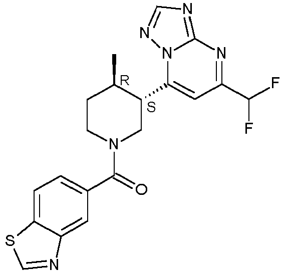 | I-9b | 49 |
| 228 | 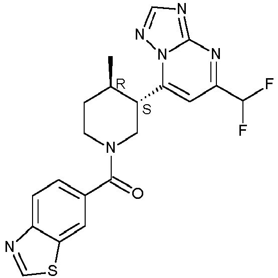 | I-9b | 50 |
| 229 | 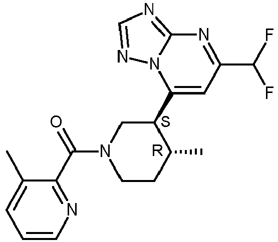 | I-9b | 83 |
| 230 | 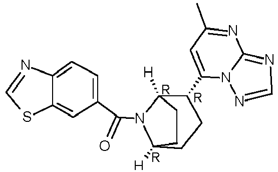 | I-25 | 32 |
| 231 | 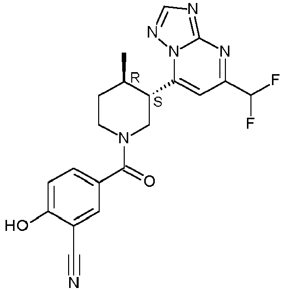 | I-9b | 29 |
| 232 | 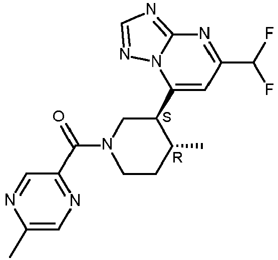 | I-9b | 65 |
| 233 | 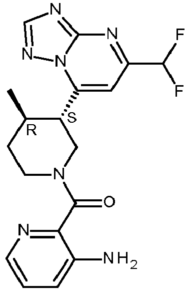 | I-9b | 57 |
| 234 | 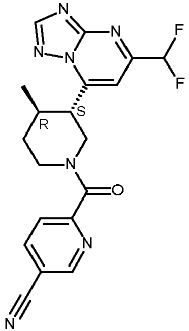 | I-9b | 63 |
| 235 | 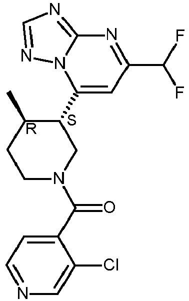 | I-9b | 62 |
| 236 | 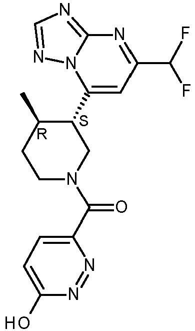 | I-9b | 2 |
| 237 | 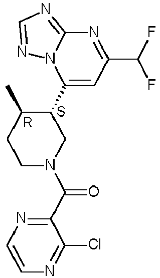 | I-9b | 54 |
| 238 | 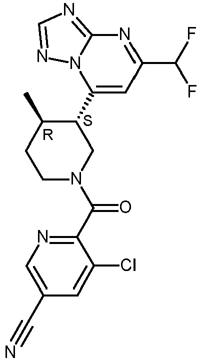 | I-9b | 49 |
| 239 | 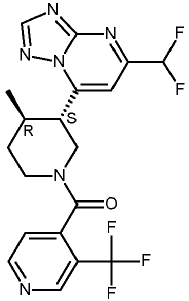 | I-9b | 45 |
| 240 | 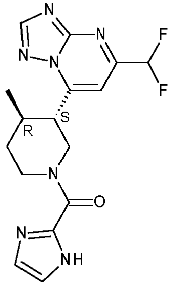 | I-9b | 59 |
| 241 | 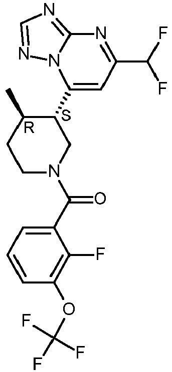 | I-9b | 66 |
| 242 | 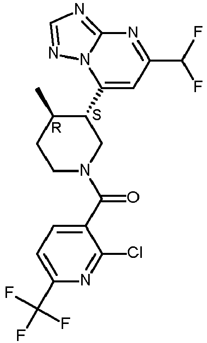 | I-9b | 67 |
| 243 | 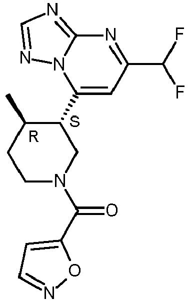 | I-9b | 44 |
| 244 | 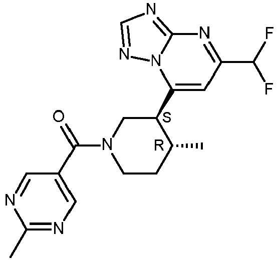 | I-9b | 53 |
| 245 | 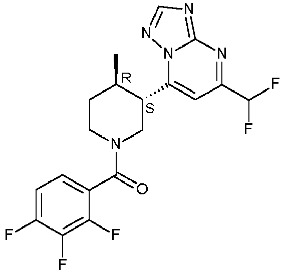 | I-9b | 62 |
| 246 | 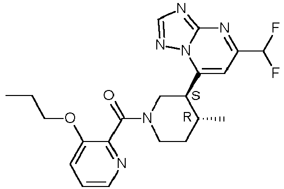 | I-9b | 58 |
| 247 | 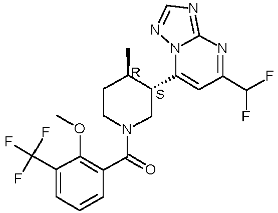 | I-9b | 53 |
| 248 | 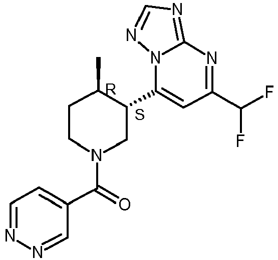 | I-9b | 63 |
| 249 | 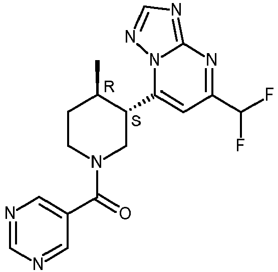 | I-9b | 53 |
| 250 | 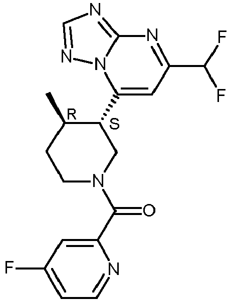 | I-9b | 66 |
| 251 | 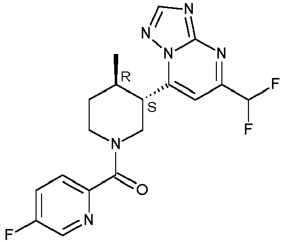 | I-9b | 70 |
| 252 | 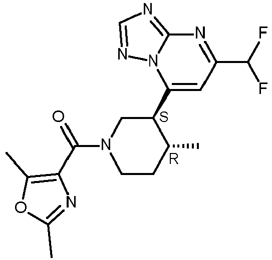 | I-9b | 62 |
| 253 | 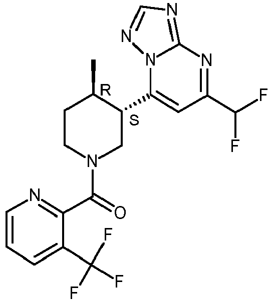 | I-9b | 62 |
| 254 | 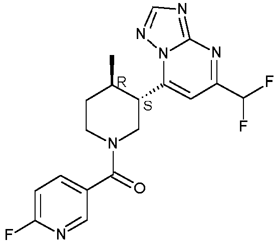 | I-9b | 50 |
| 255 | 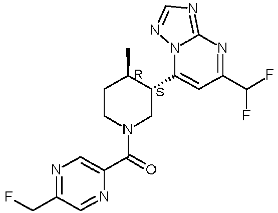 | I-9b | 64 |
| 256 | 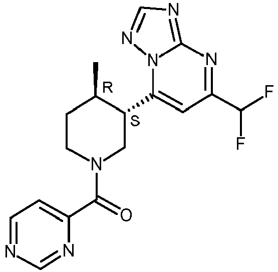 | I-9b | 63 |
| 257 | 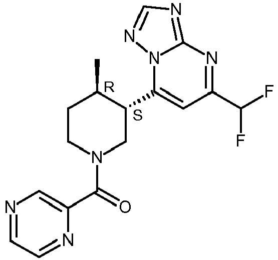 | I-9b | 59 |
| 258 | 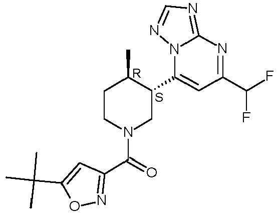 | I-9b | 53 |
| 259 | 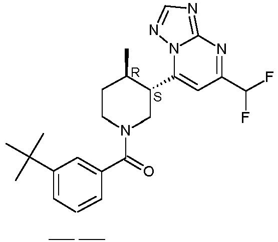 | I-9b | 63 |
| 260 | 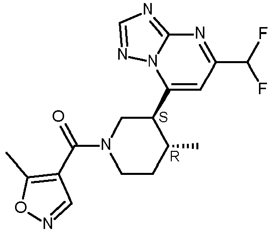 | I-9b | 40 |
| 261 | 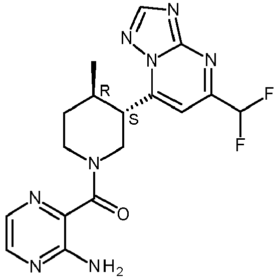 | I-9b | 64 |
| 262 | 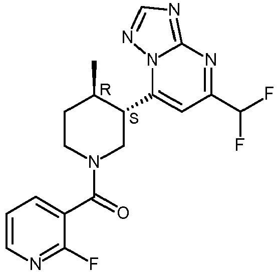 | I-9b | 68 |
| 263 | 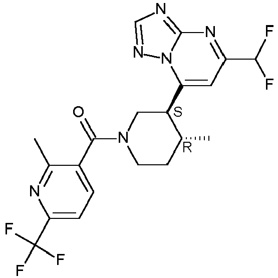 | I-9b | 27 |
| 264 | 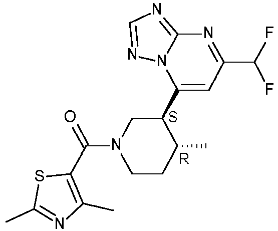 | I-9b | 58 |
| 265 | 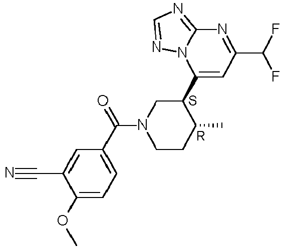 | I-9b | 62 |
| 266 | 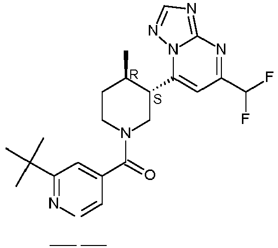 | I-9b | 72 |
| 267 | 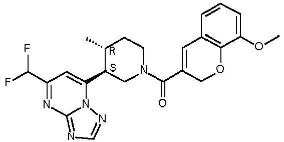 | I-9b | 57 |
| 268 | 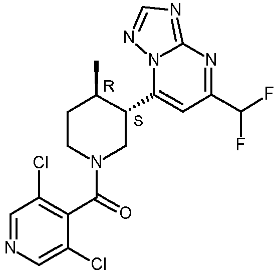 | I-9b | 61 |
| 269 | 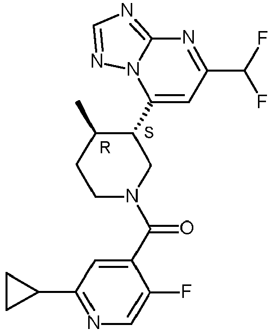 | I-9b | 61 |
| 270 | 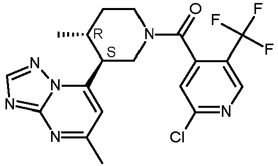 | I-9b | 45 |
| 271 | 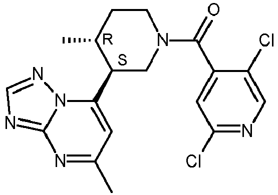 | I-9b | 32 |
| 272 | 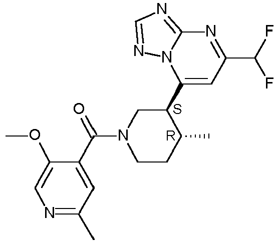 | I-9b | 30 |
| 273 | 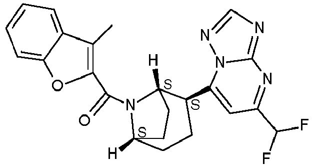 | I-25 | 37 |
| 274 | 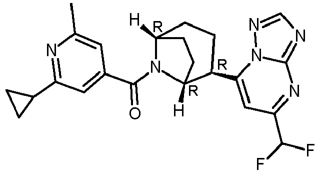 | I-25 | 35 |
| 275 | 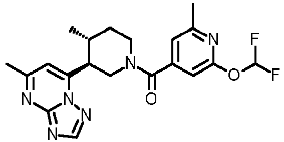 | I-12a | 31 |
| 276 | 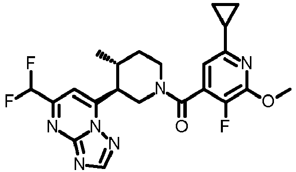 | I-9b | 3 |
| 277 | 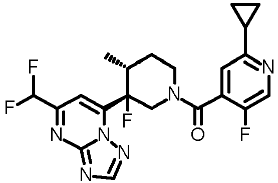 | I-19a | 71 |
| 278 | 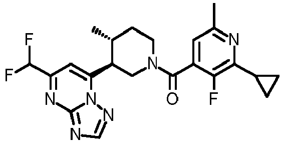 | I-9b | 14 |
| 279 | 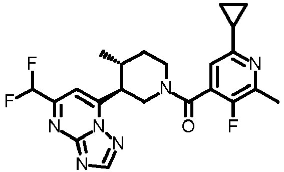 | I-9b | 11 |
| 280 | 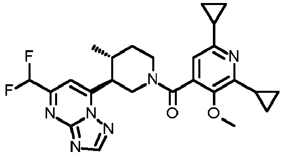 | I-9b | 3 |
| 281 | 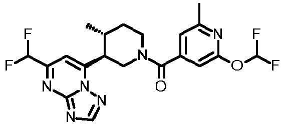 | I-9b | 30 |
| 282 | 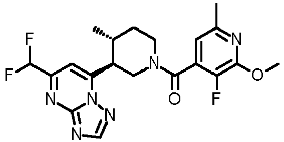 | I-9b | 24 |
| 283 | 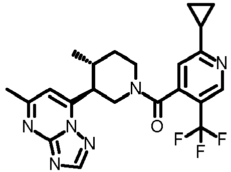 | I-12a | 48 |
| 284 | 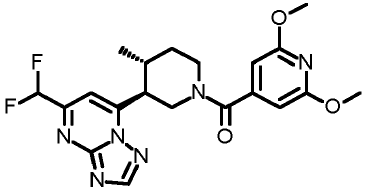 | I-9b | 62 |
| 285 | 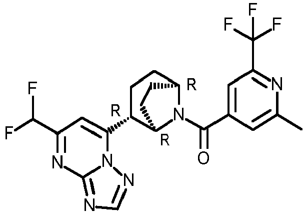 | I-25 | 7.7 |
| 286 | 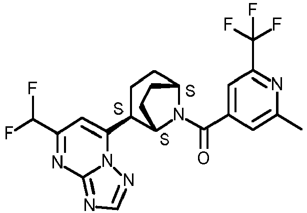 | I-25 | 9.7 |
| 287 | 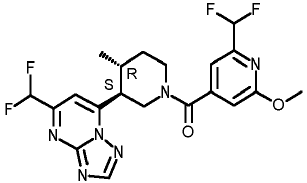 | I-9b | 62 |
| 288 | 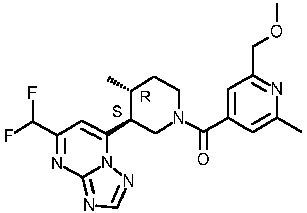 | I-9b | 26 |
| 289 | 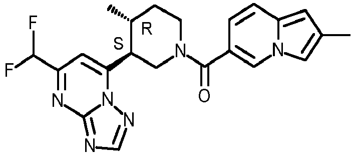 | I-9b | 53 |
| 290 | 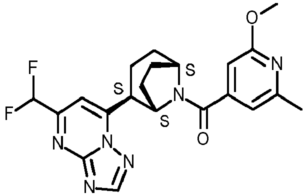 | I-25a | 40. |
| 291 | 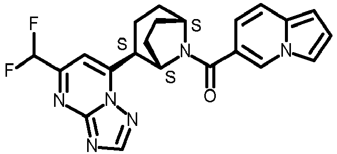 | I-25a | 65 |
| 292 | 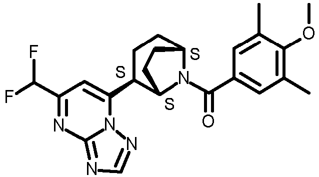 | I-25a | 59 |
| 293 | 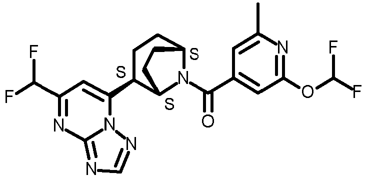 | I-25a | 65 |
| 294 | 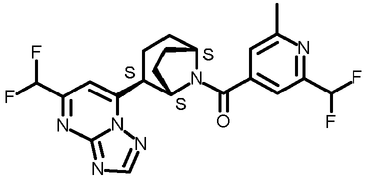 | I-25a | 60 |
| 295 | 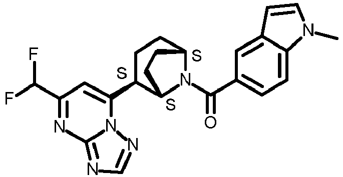 | I-25a | 94 |
| 296 | 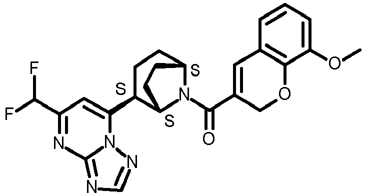 | I-25a | 39 |
| 297 | 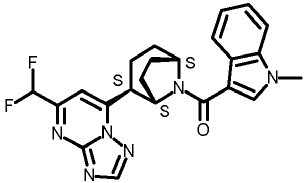 | I-25a | 61 |
| 298 | 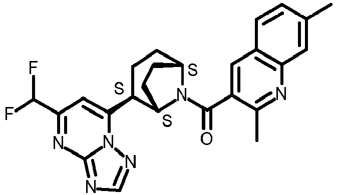 | I-25a | 56 |
| 299 | 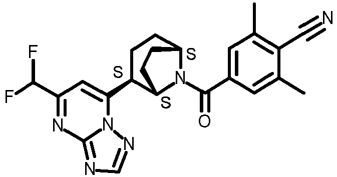 | I-25a | 54 |
| 300 | 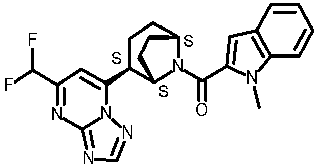 | I-25a | 22 |
| 301 | 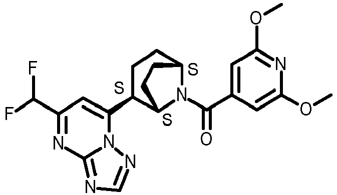 | I-25a | 60 |
| 302 | 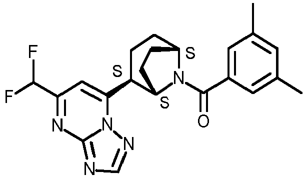 | I-25a | 61 |
| 303 | 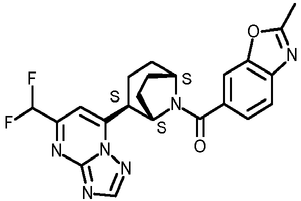 | I-25a | 41 |
| 304 | 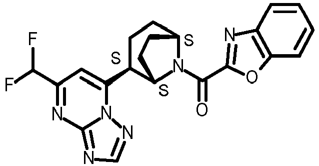 | I-25a | 56 |
| 305 | 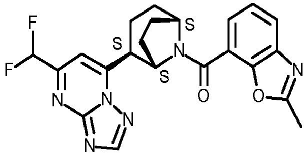 | I-25a | 64 |
| 306 | 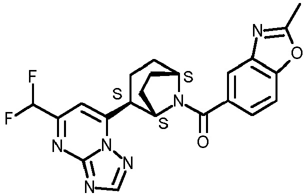 | I-25a | 68 |
| 307 | 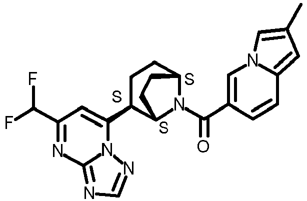 | I-25a | 55 |
| 308 | 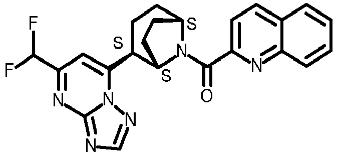 | I-25a | 48 |
| 309 | 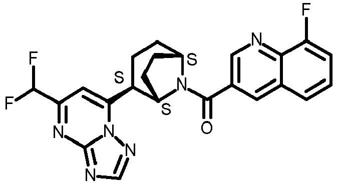 | I-25a | 65 |
| 310 | 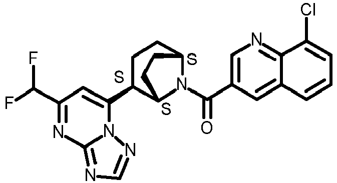 | I-25a | 52 |
| 311 | 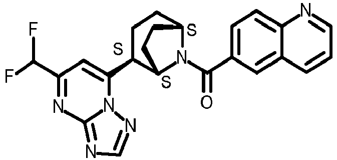 | I-25a | 62 |
| 312 | 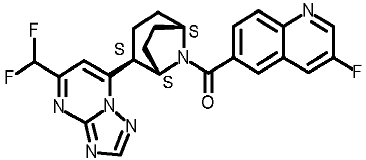 | I-25a | 63 |
| 313 | 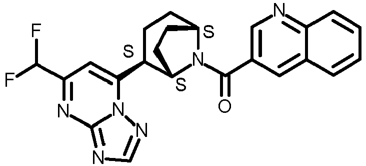 | I-25a | 63 |
| 314 | 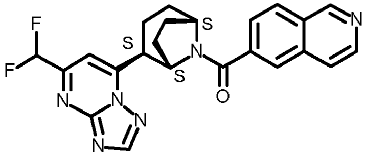 | I-25a | 64 |
| 315 | 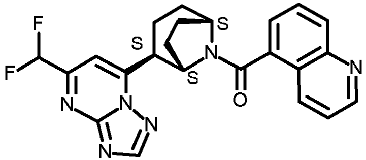 | I-25a | 63 |
| 316 | 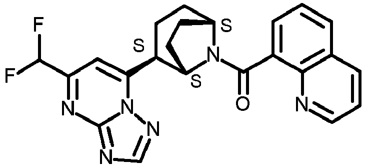 | I-25a | 100 |
| 317 | 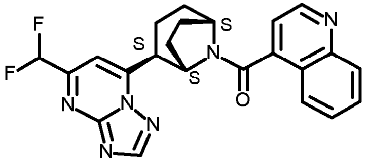 | I-25a | 61 |
| 318 | 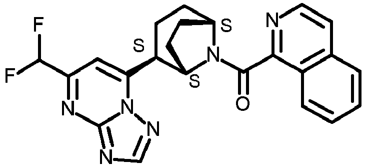 | I-25a | 86 |
| 319 | 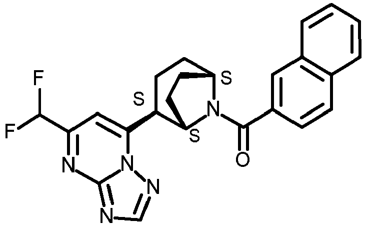 | I-25a | 57 |
| 320 | 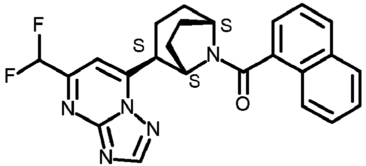 | I-25a | 50 |
| 321 | 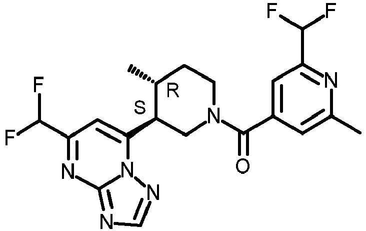 | I-9b (N.B. EDCI coupling) | 60 |
| 322 | 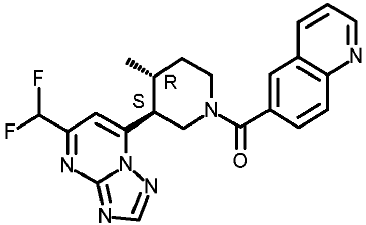 | I-9b (N.B. EDCI coupling) | 69 |
| 323 | 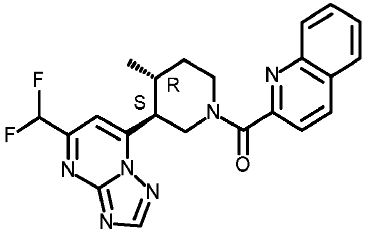 | I-9b (N.B. EDCI coupling) | 59 |
| 324 | 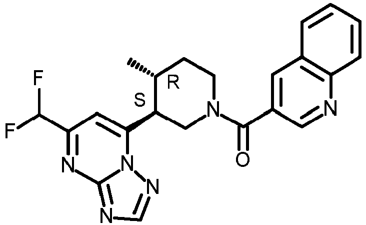 | I-9b (N.B. EDCI coupling) | 79 |
| 325 | 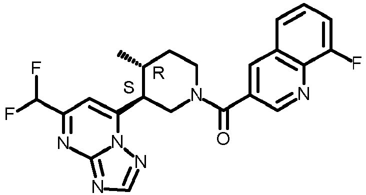 | I-9b (N.B. EDCI coupling) | 62 |
| 326 | 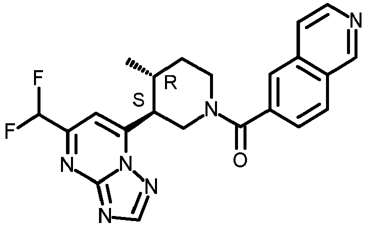 | I-9b (N.B. EDCI coupling) | 66 |
| 327 | 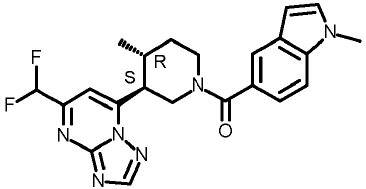 | I-9b (N.B. EDCI coupling) | 73 |
| 328 | 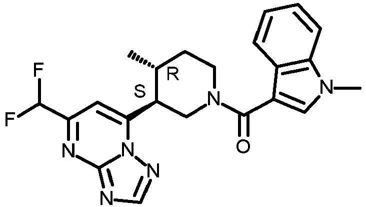 | I-9b (N.B. EDCI coupling) | 56 |
| 329 | 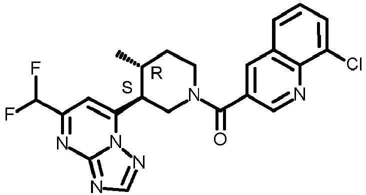 | I-9b (N.B. EDCI coupling) | 71 |
| 331 | 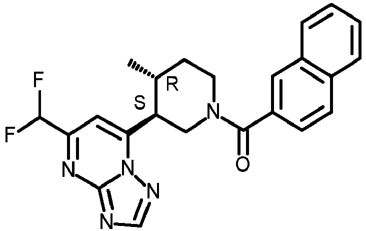 | I-9b (N.B. EDCI coupling) | 66 |
| 332 | 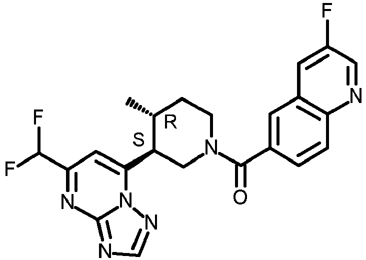 | I-9b | 32 |
| 333 | 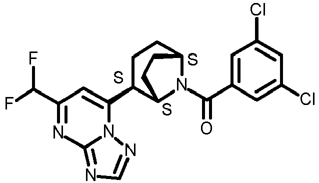 | I-25a (N.B. phosphonic acid anhydride) | 61 |
| 334 | 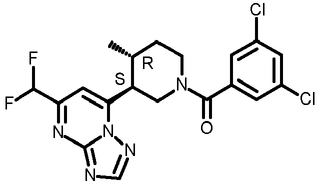 | I-9b (N.B. phosphonic acid anhydride) | 66 |
| 335 | 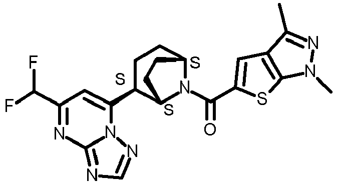 | I-25a | 71 |
| 336 | 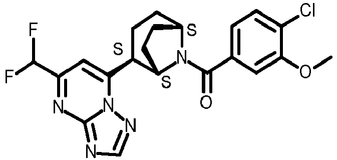 | I-25a (N.B. phosphonic acid anhydride) | 52 |
| 337 | 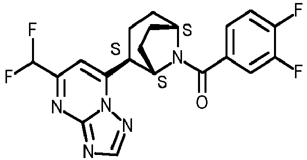 | I-25a | 62 |
| 338 | 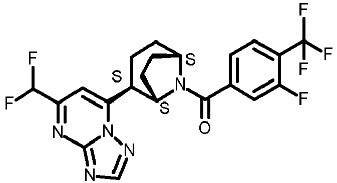 | I-25a | 33 |
| 339 | 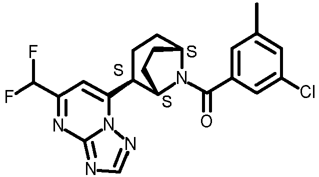 | I-25a | 69 |
| 340 | 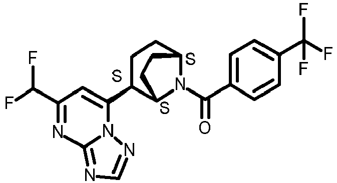 | I-25a | 38 |
| 341 | 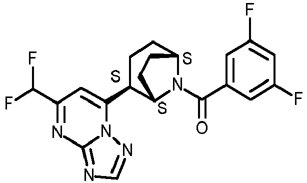 | I-25a | 72 |
| 342 | 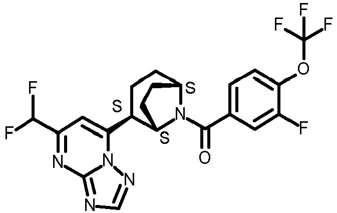 | I-25a | 42 |
| 343 | 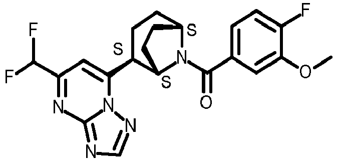 | I-25a (N.B. phosphonic acid anhydride) | 58 |
| 344 | 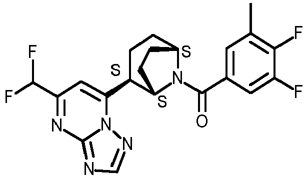 | I-25a | 64 |
| 345 | 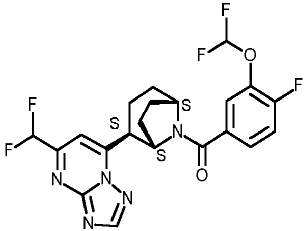 | I-25a (N.B. phosphonic acid anhydride) | 42 |
| 346 | 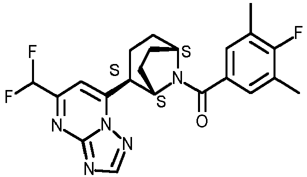 | I-25a | 41 |
| 347 | 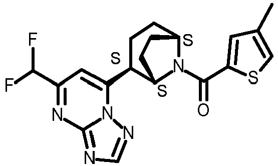 | I-25a | 52 |
| 348 | 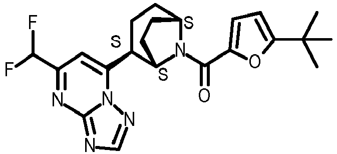 | I-25a | 51 |
| 349 | 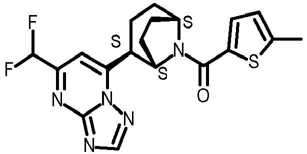 | I-25 | 46 |
| 350 | 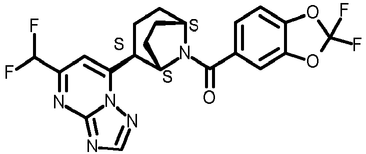 | I-25a | 55 |
| 351 | 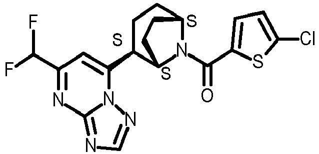 | I-25a | |
| 352 | 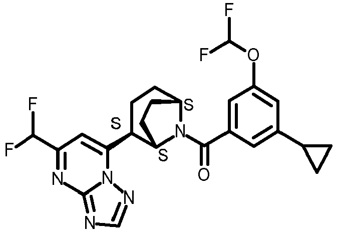 | I-25a | 67 |
| 353 | 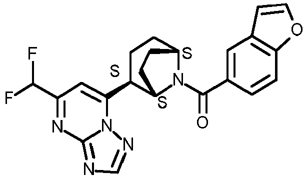 | I-25a | 66 |
| 354 | 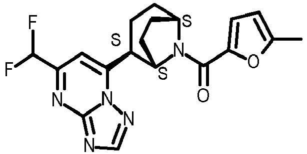 | I-25a | 59 |
| 355 | 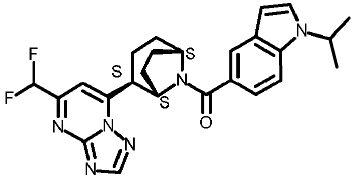 | I-25a (N.B. phosphonic acid anhydride) | 34 |
| 356 | 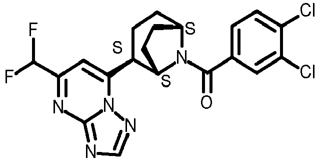 | I-25a | 78 |
| 357 | 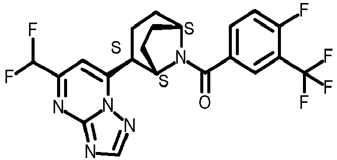 | I-25a | 65 |
| 358 | 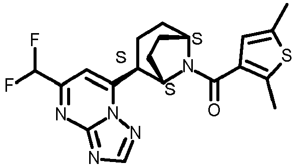 | I-25a | 17 |
| 359 | 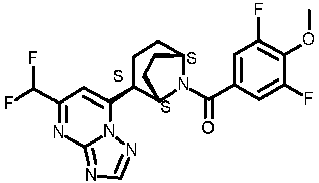 | I-25a | 58 |
| 360 | 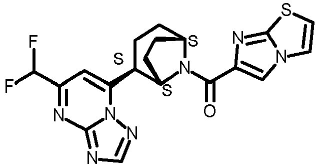 | I-25a | 43 |
| 361 | 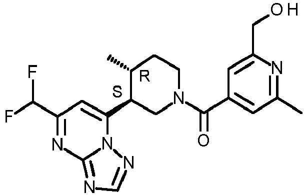 | I-9b | 15 |
| 362 | 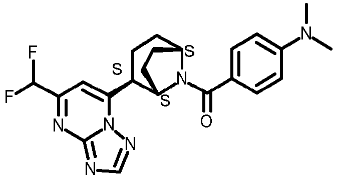 | I-25a | 47 |
| 363 | 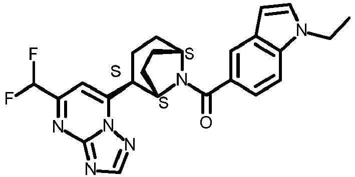 | I-25a | 49 |
| 364 | 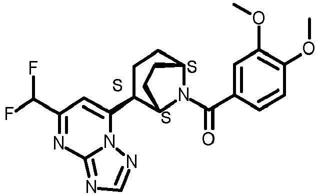 | I-25a | 68 |
| 365 | 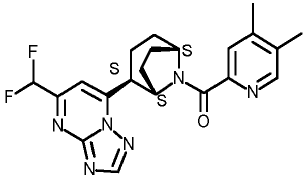 | I-25a | 13 |
| 366 | 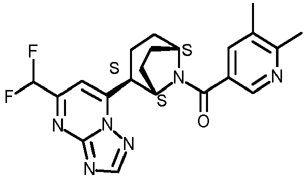 | I-25a | 59 |
| 367 | 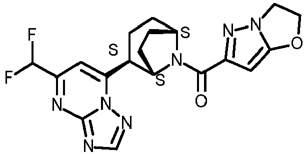 | I-25a | 68 |
| 368 | 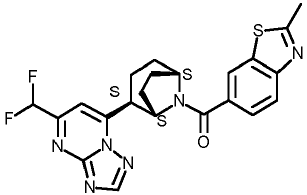 | I-25a | 50 |
| 369 | 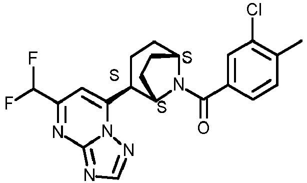 | I-25a | 26 |
| 370 | 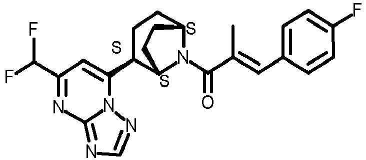 | I-25a | 60 |
| 371 | 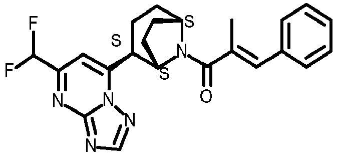 | I-25a | 34 |
| 372 | 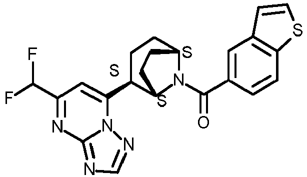 | I-25a | 37 |
| 373 | 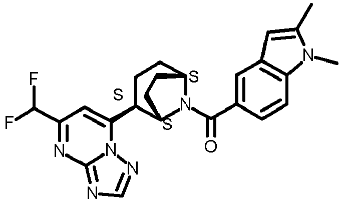 | I-25a | 63 |
| 374 | 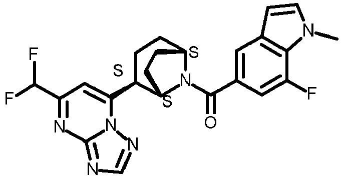 | I-25a | 53 |
| 375 | 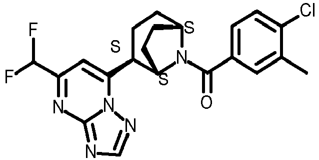 | I-25a | 71 |
| 376 | 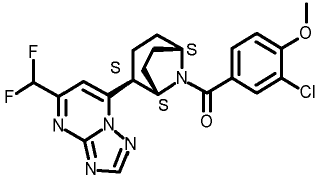 | I-25a | 57 |
| 377 | 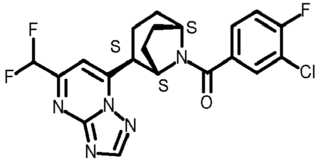 | I-25 | 41 |
| 378 | 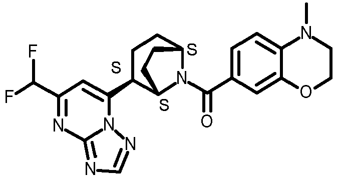 | I-25 | 58 |
| 379 | 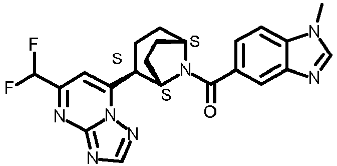 | I-25a | 63 |
| 380 | 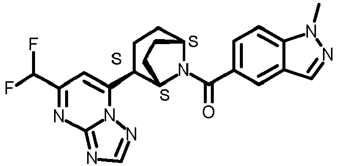 | I-25a | 68 |
| 381 | 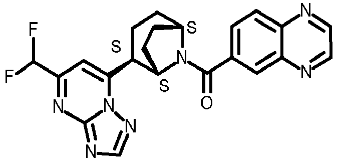 | I-25a | 59 |
| 382 | 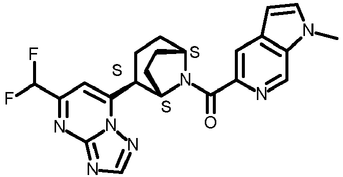 | I-25a | 54 |
| 383 | 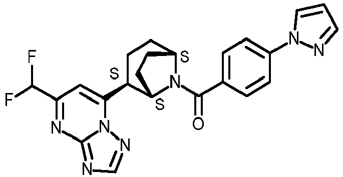 | I-25a | 55 |
| 384 | 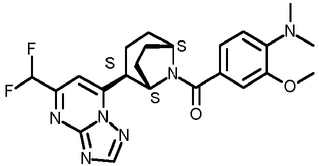 | I-25a | 22 |
| 385 | 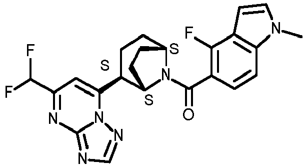 | I-25a | 72 |
| 403 | 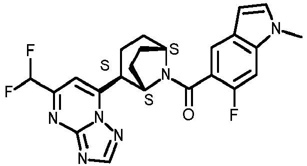 | I-25a | 57.7 |
| 404 | 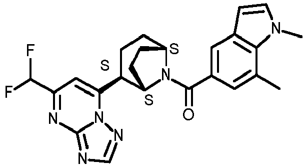 | I-25a | 67 |
| 405 | 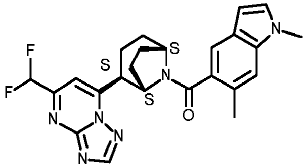 | 1-25 | 68.7 |
| 406 | 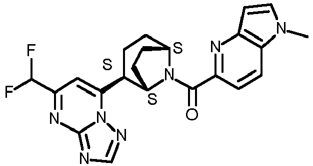 | 1-25 | 71.5 |

### CONVERSIONS AND SYNTHESIS OF FINAL COMPOUNDS BY OTHER ROUTES COMPOUND 386 AND COMPOUND 282/387

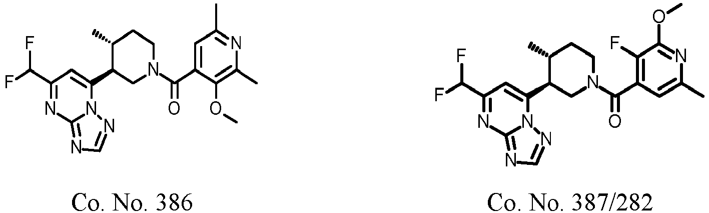

### STEP 1. COMPOUND 388 AND COMPOUND 389

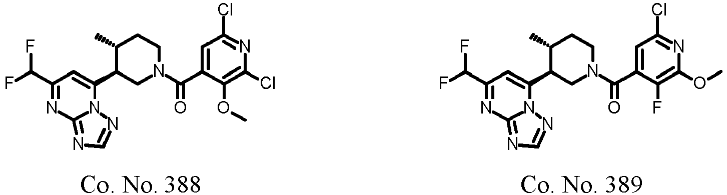

NaOMe (10.5 mL, 56.7 mmol, 30% in MeOH) was added to a solution of compound 71 (654 mg, 1.42 mmol) in MeOH (13 mL) and the resulting mixture was stirred at 0 °C for 1 h. The RM was quenched with a saturated aqueous NH₄Cl solution. The volatiles were removed under vacuo and the remaining aqueous residue partitioned with DCM. The aqueous layer was extracted with DCM (2 x 10 mL). The combined organic layers were dried over MgSO₄, filtered and evaporated in vacuo to afford a mixture of compounds 388 and 389 as a white foam (656 mg), which was used as such in the next step

### STEP 2. COMPOUND 386 AND COMPOUND 282/387

The mixture from the previous step was dissolved in 1,4-dioxane (10 mL) and the resulting solution was placed in a microwave tube and degassed for 5 minutes. K₂CO₃ (668 mg, 4.83 mmol) was then added, followed by trimethylboroxine (0.789 mL, 2.76 mmol, 3.5 M in THF) and Pd(PPh₃)₄ (159.5 mg, 0.14 mmol). The reaction mixture was then heated at 100 °C for 2.5 h under microwave irradiation. More trimethylboroxine (0.394 mL, 1.38 mmol, 3.5 M in THF) was added and the reaction mixture was heated at 100 °C under microwave irradiation for another hour. The solvent was evaporated in vacuo and the resulting residue was dissolved in a 1:1 mixture of DCM/water (40 mL). The biphasic mixture was separated and the aqueous phase was extracted with DCM (2 x 10 mL). The combined organic layers were dried over MgSO₄, filtered and concentrated under reduced pressure. The resulting residue (843 mg) was purified via flash column chromatography on silica gel, using as eluent a gradient DCM/MeOH, 100/0 to 97/3. The fractions corresponding to the title compounds were collected. One fraction was recrystallized from DIPE, to provide compound 282/387 (146 mg, 24%,). The second fraction was purified by prep HPLC using as stationary phase: RP XBridge Prep C18 OBD-10µm,30x150mm and as mobile phase: 0.25% NH₄HCO₃ solution in water, CH₃CN to provide compound 386 as a white solid (82 mg, 13.8%).

### COMPOUND 390 (AND COMPOUND 391

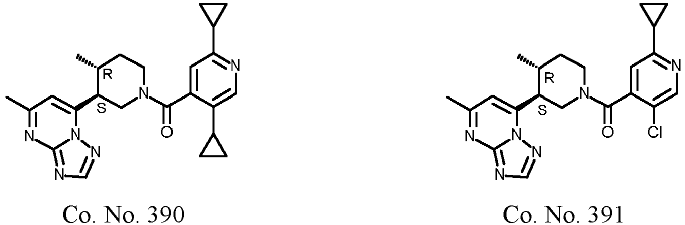

A pressure tube was charged with compound 271 (150 mg, 0.37 mmol), cyclopropylboronic acid (127.2 mg, 1.48 mmol) and toluene (3.0 mL) and this was degassed for 15 min before adding consecutively palladium(II) acetate (4.1 mg, 0.0185 mmol), tricyclohexylphosphine (10.4 mg, 0.037 mmol), distilled water (0.734 mL, 40.65 mmol) and potassium phosphate tribasic (235.7 mg, 1.11 mmol). The tube was capped and the ensuing RM was placed in an oil bath of 100 °C and stirred for 16 h. The resulting solution was cooled to RT and filtered through a Celite® pad, which was washed with toluene and EtOAc. The filtrate was evaporated to dryness under reduced pressure. The resulting residue was dissolved in EtOAc and washed with water and brine, then dried over MgSO₄, filtered and evaporated in vacuo. The resulting residue (155 mg) was purified via Prep HPLC, using as stationary phase: RP XBridge Prep C18 OBD-10µm,30x150mm and mobile phase: 0.5% NH4Ac solution in water + 10% CH₃CN, MeOH, to provide compound 390 (18 mg, 11.7%) and compound 391 (66 mg, 43.4%), after trituration with heptane.

### COMPOUND 392

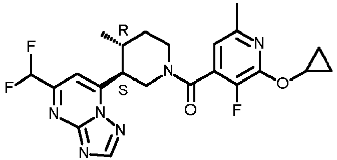

### STEP 1. COMPOUND 393

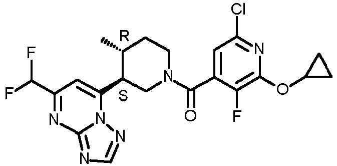

Cesium fluoride (577 mg, 3.8 mmol) was added to a solution of compound 71 (436 mg, 0.95 mmol) in DMF (7.35 mL) and the resulting mixture was stirred and degassed for 15 minutes. Cyclopropanol (0.072 mL, 1.14 mmol) was added and the RM was heated at 110 °C for 2 h under microwave irradiation. The solvent was removed in vacuo, then the resulting residue was dissolved in a 1:1 mixture of DCM/water (20 mL). The resulting biphasic mixture was separated, then the aqueous layer was extracted with DCM (2x 10 mL). The combined organic layers were dried over MgSO₄, filtered and the solvent removed under reduced pressure. The resulting residue was purified by flash column chromatography on silica gel, using as eluent a gradient DCM/MeOH, 100/0 to 99/1, to provide compound 393 (308 mg) as an impure material, which was used as such in the next step.

STEP 2. COMPOUND 392 A solution of compound 393 (308 mg, 0.64 mmol) in 1,4-dioxane (4.6 mL) was degassed for 15 minutes, then K₂CO₃ (310 mg, 2.24 mmol), trimethylboroxine (0.550 mL, 1.92 mmol, 3.5 M in THF) and Pd(PPh₃)₄ (74.015 mg, 0.064 mmol) were sequentially added and the resulting mixture was heated at 100 °C for 2.5 h in a pressure tube. The solvent was removed in vacuo, and the resulting residue was partitioned between DCM (10 mL) and water (10 mL). The resulting biphasic mixture was separated and the aqueous layer extracted with DCM (3x 10 mL). The combined organic layers were dried over MgSO₄, filtered and the solvent removed under reduced pressure. The resulting residue (340 mg) was purified via Prep HPLC, using as stationary phase: RP Vydac® Denali® C18 - 10µm, 200g, 5cm I.D and mobile phase: MeOH, to give compound 392 (55 mg, 18.6%) as white crystals after recrystallization from DIPE.

### COMPOUND 394

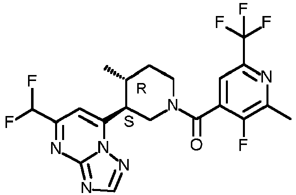

### STEP 1. COMPOUND 395

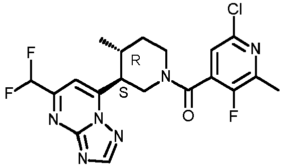

EDCI (993 mg, 5.2 mmol) and 6-chloro-3-fluoro-2-methyl-isonicotinic acid (intermediate 50, 492 mg, 2.6 mmol) were consecutively added to a stirred suspension of intermediate 9-b (750 mg, 2.47 mmol) in DCM (20 mL). DIPEA (1.78 mL, 10.4 mmol) was then added to the mixture, and the resulting yellow solution was stirred at RT for 5 h. The RM was quenched with an aqueous solution of NaOH (20 mL, 1 M). The aqueous layer was extracted with DCM (3 x 10 mL) and the combined organic layers were dried over MgSO₄, filtered and concentrated in vacuo. The resulting residue (1.5 g) was purified via flash column chromatography on silica gel, using as eluent a gradient DCM/MeOH, 100/0 to 99/1, to provide compound 395 (720 mg, 62%), as a white foam.

### STEP 2. INTERMEDIATE 56

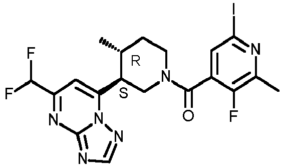

Iodotrimethylsilane (0.065 mL, 0.46 mmol) was added to a solution of compound 395 (200 mg, 0.46 mmol) in propionitrile (0.8 mL), under a nitrogen atmosphere. Sodium iodide (205 mg, 1.37 mmol) was then added and the resulting solution was heated to 80 °C. After 5 h LCMS analysis showed only 15% conversion, therefore another portion of iodotrimethylsilane (0.065 mL, 0.456 mmol) was added and the RM was stirred at 80 °C for 22 h. After this time, another portion of iodotrimethylsilane (0.065 mL, 0.456 mmol) and sodium iodide (205 mg, 1.37 mmol) were added, and the RM stirred for 12 h. The solvent was removed in vacuo, then the resulting residue was partitioned between water and DCM. The resulting biphasic mixture was separated and the aqueous layer extracted with DCM (3 x 20 mL). The combined organic layers were dried over MgSO₄, filtered and the solvent removed in vacuo. The resulting residue (1.5 g) was purified by flash column chromatography on silica gel, using as eluent a gradient DCM/MeOH, 100/0 to 97.5/2.5, to give intermediate 56 (162 mg) as an impure material, used as such in the next step.

STEP 3. COMPOUND 394 Methyl 2,2-difluoro-2-(fluorosulfonyl)acetate (0.192 mL, 1.51 mmol) was added to a mixture of intermediate 56 (160 mg, 0.3 mmol) and copper iodide (75 mg, 0.39 mmol) in anhydrous DMF (0.958 mL) in a pressure tube. The resulting mixture was heated to 80 °C for 2 h in an oil bath. The RM was cooled to RT, then quenched with a saturated aqueous NH₄Cl solution. The resulting mixture was extracted with EtOAc (3 x 15 mL). The combined organic layers were dried over MgSO₄, filtered and the solvent removed in vacuo. The resulting residue was purified via flash column chromatography, using as eluent a gradient heptane/EtOAc, 100/0 to 60/40, to provide compound 394 (17.5 mg, 12%).

### COMPOUND 396

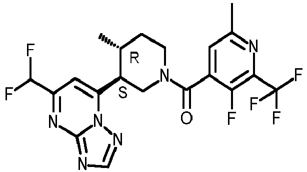

### STEP 1. COMPOUND 397

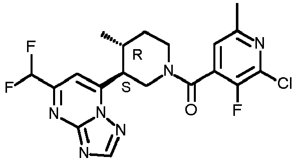

EDCI (934 mg, 4.87 mmol) and 2-chloro-3-fluoro-6-methyl isonicotinic acid (485 mg, 2.56 mmol) were added to a stirred suspension of intermediate 9-b (740.1 mg, 2.44 mmol) in DCM (18.5 mL). DIPEA (1.68 mL, 9.74 mmol) was added and the RM was stirred for 2 h at RT. The RM was quenched with an aqueous solution of NaOH (30 mL, 1 M). The aqueous layer was extracted with DCM (3 x 15 mL) and the combined organic layers were dried over MgSO₄, filtered and concentrated in vacuo. The resulting residue (1.5 g) was purified via flash column chromatography on silica gel, using as eluent a gradient DCM/MeOH, 100/0 to 99/1, to provide compound 397 (854 mg, 80%) as a white powder.

### STEP 2. INTERMEDIATE 57

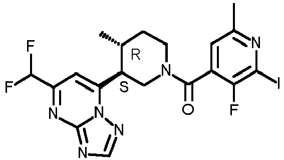

Iodotrimethylsilane (0.26 mL, 1.82 mmol) was added to a solution of compound 397 (400 mg, 0.91 mmol) in propionitrile (1.6 mL), under a nitrogen atmosphere. Sodium iodide (410 mg, 2.73 mmol) was then added and the resulting solution was heated to 80 °C for 1.5 h. The solvent was removed in vacuo, then the resulting residue was partitioned between water and EtOAc. The resulting biphasic mixture was separated and the aqueous layer extracted with EtOAc (3 x 20 mL). The combined organic layers were dried over MgSO₄, filtered and the solvent removed under reduced pressure. The resulting residue was purified by flash column chromatography on silica gel, using as eluent a gradient DCM/MeOH, 100/0 to 97.5/2.5, to give intermediate 57 (197 mg) as an impure material which was used as such in the next step.

STEP 3. COMPOUND 396 (Copper iodide (92 mg, 0.48 mmol) was added to a solution of intermediate 57 (197 mg, 0.37 mmol) in anhydrous DMF (1.18 mL) and the resulting mixture was degassed with nitrogen for 10 minutes. Methyl 2,2-difluoro-2-(fluorosulfonyl)acetate (0.236 mL, 1.86 mmol) was then added and the RM was stirred and heated at 80 °C for 2 h, after which time it was left to cool to RT overnight. The RM was quenched with a saturated aqueous NH₄Cl solution. The resulting mixture was extracted with EtOAc (6 x 20 mL). The combined organic layers were dried over MgSO₄, filtered and the solvent removed in vacuo. The resulting residue (550 mg) was purified via Prep HPLC using as stationary phase: RP XBridge Prep C18 OBD-10µm,50x150mm and as mobile phase: CH₃CN, to provide compound 396 (7.4 mg, 4%).

### COMPOUND 398

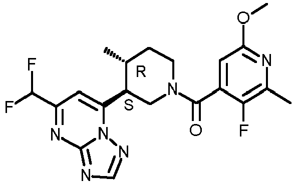

### STEP 1. COMPOUND 399

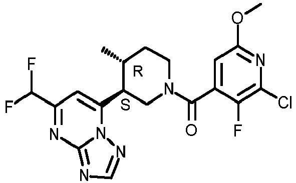

EDCI (320.5 mg, 1.67 mmol), followed by DIPEA (0.57 mL, 3.34 mmol) were added to a stirred solution of intermediate 9-b (242. mg, 0.8 mmol) and 2 -chloro-3-fluoro-6-methoxy-isonicotinic acid (intermediate 53, 172 mg, 0.84 mmol) in DCM (6.35 mL). The RM was stirred at RT overnight. Another portion of EDCI (80 mg, 0.417 mmol) and DIPEA (0.1 mL, 0.75 g/mL, 0.58 mmol) were added and the RM stirred for 12 h, then it was was quenched with an aqueous solution of NaOH (20 mL, 1 M). The aqueous layer was extracted with DCM (3 x 20 mL) and the combined organic layers were dried over MgSO₄, filtered and concentrated in vacuo. The resulting residue (600 mg) was purified via flash column chromatography on silica gel, using as eluent a gradient DCM/MeOH, 100/0 to 98.5/1.5, to provide compound 399 (136 mg, 80%) as a white foam.

### STEP 2. COMPOUND 398

A solution of compound 399 (136 mg, 0.299 mmol) in 1,4-dioxane (2.17 mL) was degassed for 15 minutes in a pressure tube. K₂CO₃ (144.6 mg, 1.05 mmol), trimethylboroxine (0.256 mL, 0.897 mmol, 3.5 M in THF) and Pd(PPh₃)₄ (34.5 mg, 0.03 mmol) were sequentially added and the resulting mixture was heated at 100 °C for 2 h. The solvent was removed in vacuo, and the resulting residue was partitioned between DCM and water. The resulting biphasic mixture was separated and the aqueous layer extracted with DCM (3x 15 mL). The combined organic layers were dried over MgSO₄, filtered and the solvent removed under reduced pressure. The resulting residue (220 mg) was purified via flash column chromatography on silica gel, using as eluent a gradient DCM/MeOH, 100/0 to 96/4 to provide compound 398 (88 mg, 68%).

### COMPOUND 400

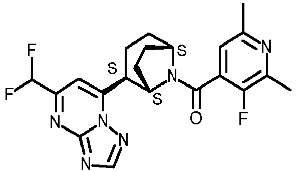

### STEP 1. COMPOUND 401

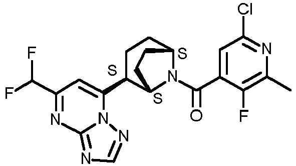

DIPEA (0.65 mL, 3.77 mmol) and HBTU (300 mg, 0.79 mmol) were added to a solution of 6-chloro-3-fluoro-2-methyl isonicotinic acid (intermediate 50, , 150 mg, 0.79 mmol) in DCM (30 mL). Intermediate 25-a (238 mg, 0.75 mmol) was then added, and the RM stirred for ∼1.5 h at RT. The RM was quenched with an aqueous solution of NaOH (1 mL, 1 M), and then filtered through an Extrelute® filter. The solvent was removed under reduced pressure. The resulting residue was purified via flash column chromatography on silica gel, using as eluent a gradient DCM/MeOH 100/0 to 98/2 to provide compound 401 (320.5 mg, 83%) as a colourless oil

### STEP 2. COMPOUND 400

A solution of compound 401 (320 mg, 0.71 mmol) in 1,4-dioxane (5.1 mL) was degassed for 15 minutes. K₂CO₃ (343.3 mg, 2.45 mmol), trimethylboroxine (0.608 mL, 2.129 mmol, 3.5 M in THF) and Pd(PPh₃)₄ (82.0 mg, 0.071 mmol) were sequentially added and the resulting mixture was heated at 100 °C for 2 h in a pressure tube. The solvent was removed in vacuo, and the resulting residue was partitioned between DCM (20 mL) and water (20 mL). The biphasic mixture was separated and the aqueous layer extracted with DCM (3x 15 mL). The combined organic layers were dried over MgSO₄, filtered and solvent removed under reduced pressure. The resulting residue was purified via flash column chromatography on silica gel, using as eluent a gradient DCM/MeOH 100/0 to 95/5. This provided a mixture of endo/exo isomers (230 mg) which were separated via prep HPLC, using as stationary phase: RP XBridge® Prep C18 OBD-10µm,50x150mm and mobile phase:MeOH. The fractions containing the product were evaporated and the resulting residue was recrystallised from Et₂O, to provide compound 400 (57 mg, 18.6%)

### COMPOUND 402

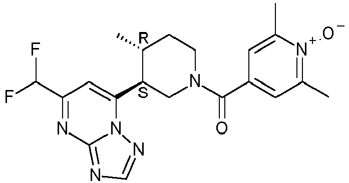

mCPBA (307.825 mg, 1.25 mmol) was added to a solution of compound 1 from WO2017/076900 (250 mg, 0.624 mmol) in DCM (1.6 mL), at 10 °C, and the resulting mixture was stirred in an Easymax® for 3 d. After this time, the RM was quenched with a saturated aqueous NaHCO₃ solution (1.5 mL). The biphasic mixture was separated and the aqueous layer was extracted with DCM (3 x 5 mL). The combined organic layers were dried over MgSO₄, filtered and concentrated in vacuo. The resulting residue was purified via flash column chromatography on silica gel, using as eluent a gradient DCM/MeOH, 100/0 to 96/4 to give compound 402 (250 mg, 96%as a white powder.

### ANALYTICAL PART

### MELTING POINTS

Values are either peak values or melt ranges, and are obtained with experimental uncertainties that are commonly associated with this analytical method.
The melting point was determined with a DSC823e (Mettler-Toledo). The melting point was measured with a temperature gradient of 10°C/min. Maximum temperature was 300°C.

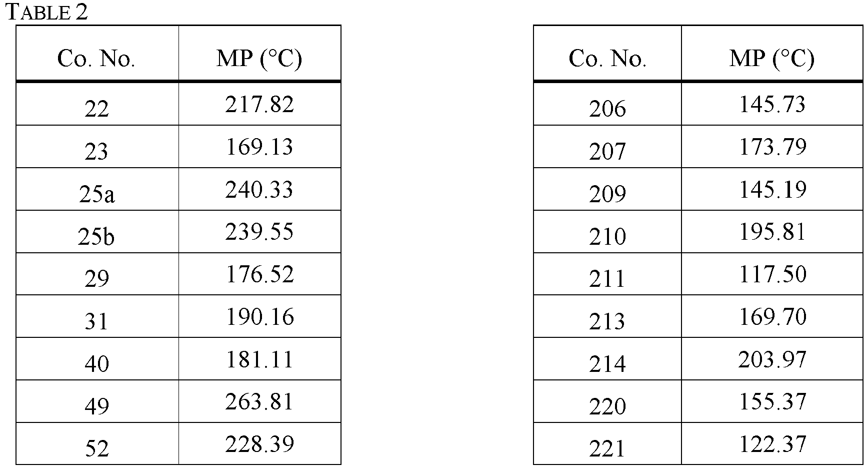

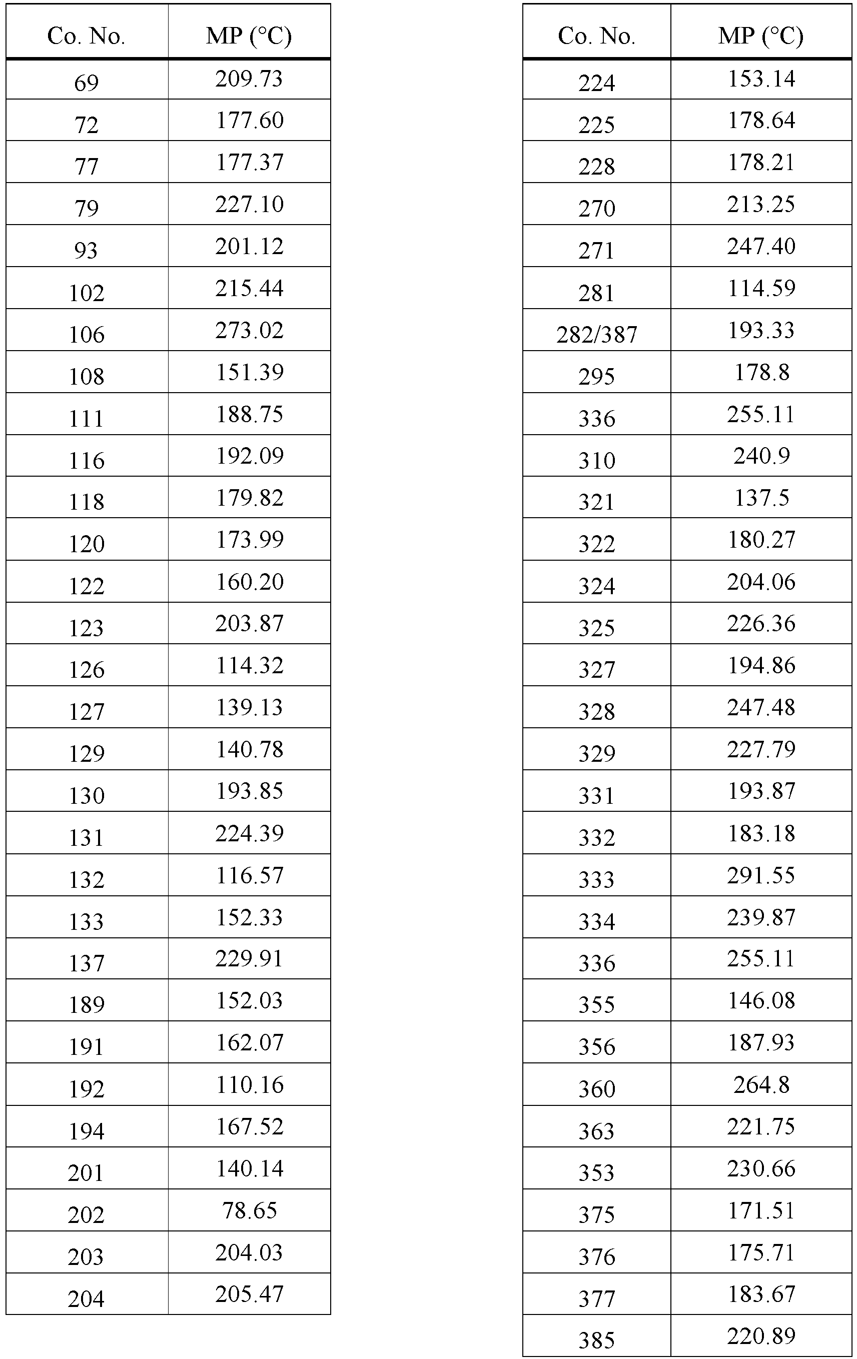

### LC/MS METHODS

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Compounds are described by their experimental retention times (Rₜ) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc...). For molecules with multiple isotopic patterns (Br, Cl), the reported value is the one obtained for the lowest isotope mass. All results were obtained with experimental uncertainties that are commonly associated with the method used. Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

**TABLE 3A. LCMS Method codes (Flow expressed in mL/min; column temperature (T) in °C; Run time in minutes)**

| METHOD CODE | INSTRUMENT | COLUMN | MOBILE PHASE | GRADIENT | $\begin{matrix}FLOW \\ COL T\end{matrix}$ | RUN TIME (MIN) |
|---|---|---|---|---|---|---|
| A | Waters: Acquity® UPLC® -DAD-SQD | Waters: BEH C18 (1.7µm, 2.1^{∗}50mm) | A: 10mM CH3COONH4 in 95% H2O + 5% CH3CN B: CH3CN | From 95% A to 5% A in 1.3 min, held for 0.7 min. | $\begin{matrix}0.8 \\ 55\end{matrix}$ | 2 |
| B | Waters: Acquity® UPLC® - DAD and SQD | Waters: BEH C18 (1. 7µm, 2.1^{∗}50mm) | A: 25 mM CH3COONH4 in 95% H2O + 5% CH3CN B: CH3CN | From 95% A to 5% A in 1.3 min, held for 0.7 min. | $\begin{matrix}0.8 \\ 55\end{matrix}$ | 2 |
| C | Waters: Acquity® UPLC® - DAD and SQD | Waters : HSS T3 (1.8µm, 2.1^{∗}100mm ) | A: 10mM CH3COONH4 in 95% H2O + 5% CH3CN B: CH3CN | From 100% A to 5% A in 2.10min, to 0% A in 0.90min, to 5% A in 0.5min | $\begin{matrix}0.7 \\ 55\end{matrix}$ | 3.5 |
| D | Waters: Alliance®-DAD - ZQ and ELSD 2000 Alltech | Xterra MS C18 column (3.5 µm, 4.6 x 100 mm) | A: 10mM CH3COONH4 in 95% H2O + 5% CH3CN B: CH3CN C: CH3OH D: (40% CH3CN and 40% CH3OH and 20% H2O with 0.25% CH3COOH | From 100% A to 1% A, 49% B and 50% C in 6.5 min, to 1% A and 99% B in 0.5 min,to 100% D in 1 min held for 1.0 min to 100% A in 0.5 min and held for 1.5min. | $\begin{matrix}1.6 \\ 40\end{matrix}$ | 11 |
| E | Waters: Acquity® UPLC® -DAD-ELSD and SQD | Waters : BEH C18 (1.7µm, 2.1^{∗}50mm) | A: 0.1% HCOOH + 5% CH3OH in H2O | From 95% A to 0% A in 2.50min, to 5% A in 0.5min | $\frac{0.7}{55}$ | 3 |
| | | | B: CH3CN | | | |

**TABLE 3B. ANALYTICAL LCMS DATA - Rₜ means retention time (in minutes), [M+H]⁺ means the protonated mass of the compound, method refers to the method used for (LC)MS analysis.**

| CO. No. | Rt (min) | [M+H]⁺ | [M+H]⁻ | METHOD |
|---|---|---|---|---|
| 1a | 1.33 | 365 | 363 | C |
| 1b | 1.33 | 365 | 363 | C |
| 2a | 1.38 | 393 | 391 | C |
| 2b | 1.38 | 393 | 391 | C |
| 3a | 1.43 | 365 | 363 | C |
| 3b | 1.43 | 365 | 363 | C |
| 4 | 0.82 | 419 | 417 | A |
| 5 | 0.87 | 390 | 388 | A |
| 6 | 0.8 | 376 | 374 | A |
| 7 | 0.94 | 390 | 388 | A |
| 8 | 0.87 | 376 | 374 | A |
| 9 | 0.96 | 382 | 380 | A |
| 10 | 0.99 | 443 | 441 | A |
| 11 | 0.76 | 389 | 387 | A |
| 12 | 0.84 | 425 | 423 | A |
| 13 | 0.7 | 456 | 514 [M+CH₃COO]⁻ | A |
| 14 | 0.66 | 339 | 337 | A |
| 15 | 0.69 | 371 | 369 | A |
| 16 | 0.85 | 370 | 428 | A |
| 17 | 0.82 | 405 | 403 | A |
| 18 | 0.72 | 385 | 383 | A |
| 19 | 0.68 | 391 | 389 | A |
| 20 | 0.84 | 444 | 442 | A |
| 21 | 0.82 | 377 | 375 | A |
| 22 | 0.84 | 401 | 459 [M+CH₃COO]⁻ | A |
| 23 | 1.57 | 391 | 389 | C |
| 24a | 1.85 | 445 | 503 [M+CH₃COO]⁻ | C |
| 24b | 1.86 | 445 | 443 | C |
| 25a | 1.54 | 441 | 439 | C |
| 25b | 1.55 | 441 | 439 | C |
| 26a | 1.46 | 376 | 374 | C |
| 26b | 1.46 | 376 | 374 | C |
| 27 | 1.65 | 421 | 419 | C |
| 28 | 1.73 | 439 | 437 | C |
| 29 | 0.72 | 413 | 411 | A |
| 30 | 0.72 | 413 | 411 | A |
| 31 | 0.72 | 413 | 411 | A |
| 32 | 0.74 | 401 | 399 | A |
| 33 | 1.66 | 447 | 445 | C |
| 34 | 1.66 | 447 | 445 | C |
| 35 | 1.59 | 492 | 490 | C |
| 36 | 0.85 | 421 | 419 | A |
| 37 | 1.68 | 435 | 433 | C |
| 38 | 1.36 | 419 | 417 | C |
| 39 | 1.84 | 475 | 473 | C |
| 40 | 0.69 | 412 | 410 | A |
| 41 | 0.85 | 455 | 453 | A |
| 42 | 1.6 | 407 | 405 | C |
| 43 | 0.9 | 441 | 439 | A |
| 44 | 0.96 | 443 | 441 | A |
| 45 | 0.98 | 443 | 441 | A |
| 46 | 0.98 | 443 | 441 | A |
| 47 | 1.79 | 413 | 411 | C |
| 48 | 1.03 | 426 | 424 | A |
| 49 | 0.88 | 401 | 399 | A |
| 50 | 1.58 | 427 | 425 | C |
| 51 | 0.81 | 421 | 419 | A |
| 52 | 0.9 | 455 | 453 | A |
| 53 | 0.82 | 417 | 415 | A |
| 54 | 0.87 | 425 | 423 | A |
| 55 | 1.7 | 425 | 423 | C |
| 56 | 1.64 | 434 | 432 | C |
| 57 | 1.45 | 376 | 374 | C |
| 58 | 1.31 | 376 | 374 | C |
| 59 | 1.8 | 480 | 478 | C |
| 60 | 1.74 | 451 | 449 | C |
| 61 | 0.74 | 404 | 402 | A |
| 62 | 1.50 | 417 | 415 | C |
| 63 | 1.88 | 452 | 450 | C |
| 64 | 1.8 | 441 | 439 | C |
| 65 | 1.84 | 406 | 404 | C |
| 66 | 1.53 | 403 | 401 | C |
| 67 | 1.53 | 375 | 373 | C |
| 68 | 0.78 | 405 | 403 | A |
| 69 | 0.96 | 412 | 410 | A |
| 70 | 0.81 | 419 | 417 | A |
| 71 | 0.99 | 459 | 457 | A |
| 72 | 2.02 | 446 | 444 | C |
| 73 | 0.95 | 443 | 441 | A |
| 74 | 1.97 | 448 | 446 | C |
| 75 | 1.97 | 448 | 506 [M+CH₃COO]⁻ | C |
| 76 | 1.1 | 434 | 432 | A |
| 77 | 0.97 | 430 | 428 | A |
| 78 | 0.94 | 425 | 423 | A |
| 79 | 0.82 | 432 | 430 | A |
| 80 | 1.6 | 415 | 413 | C |
| 81 | 1.57 | 419 | 417 | C |
| 82 | 1.58 | 419 | 417 | C |
| 83 | 0.9 | 421 | 419 | A |
| 84 | 0.78 | 405 | 403 | A |
| 85 | 1.63 | 401 | 399 | C |
| 86 | 1.5 | 417 | 415 | C |
| 87 | 0.68 | 363 | 361 | A |
| 88 | 0.71 | 444 | 442 | A |
| 89 | 0.91 | 457 | 455 | A |
| 90 | 0.9 | 411 | 409 | A |
| 91 | 1.05 | 478 | 476 | A |
| 92 | 1.85 | 426 | 424 | C |
| 93 | 1.77 | 427 | 425 | C |
| 94 | 1.99 | 479 | 477 | C |
| 95 | 1.37 | 405 | 403 | C |
| 96 | 1.77 | 428 | 426 | C |
| 97 | 0.88 | 412 | 470 [M+CH₃COO]⁻ | A |
| 98 | 1.7 | 442 | 440 | C |
| 99 | 1.2 | 376 | 374 | C |
| 100 | 1.72 | 443 | 441 | C |
| 101 | 0.99 | 429 | 427 | A |
| 102 | 1.01 | 420 | 418 | A |
| 103 | 1.03 | 430 | 428 | A |
| 104 | 1.52 | 413 | 411 | C |
| 105 | 1.86 | 442 | 440 | C |
| 106 | 1.67 | 411 | 409 | C |
| 107 | 1.04 | 445 | 443 | A |
| 108 | 0.95 | 437 | 435 | A |
| 109 | 1.9 | 398 | 396 | C |
| 110 | 0.86 | 417 | 415 | A |
| 111 | 1.04 | 440 | 438 | A |
| 112 | 1.67 | 429 | 427 | C |
| 113 | 1.03 | 443 | 441 | A |
| 114 | 1 | 425 | 423 | A |
| 115 | 1.02 | 418 | 416 | A |
| 116 | 0.76 | 413 | 411 | A |
| 117 | 1.79 | 382 | 380 | C |
| 118 | 1.01 | 442 | 440 | A |
| 119 | 1.96 | 442 | 440 | C |
| 120 | 1.78 | 431 | 429 | C |
| 121 | 1 | 472 | 470 | A |
| 122 | 2 | 445 | 443 | C |
| 123 | 1 | 400 | 398 | A |
| 124 | 0.78 | 415 | 413 | A |
| 125 | 1.78 | 453 | 451 | C |
| 126 | 0.95 | 392 | 390 | A |
| 127 | 1.61 | 427 | 425 | C |
| 128 | 1.8 | 438 | 436 | C |
| 129 | 1.95 | 438 | 436 | C |
| 130 | 0.94 | 436 | 434 | A |
| 131 | 0.88 | 411 | 409 | A |
| 132 | 0.93 | 455 | 453 | A |
| 133 | 0.93 | 468 | 466 | A |
| 134 | 1.78 | 437 | 435 | C |
| 135 | 1.03 | 430 | 428 | A |
| 136 | 1.99 | 418 | 416 | C |
| 137 | 0.93 | 401 | 399 | A |
| 138 | 0.88 | 416 | 474 [M+CH₃COO]⁻ | A |
| 139 | 1.43 | 376 | 374 | C |
| 140 | 1.98 | 438 | 436 | C |
| 141 | 0.81 | 402 | 400 | A |
| 142 | 1.32 | 388 | 386 | C |
| 143 | 0.77 | 401 | | A |
| 144 | 1.94 | 472 | 470 | C |
| 145 | 0.89 | 430 | 428 | A |
| 146 | 1.32 | 439 | 437 | C |
| 147 | 1.88 | 426 | 424 | C |
| 148 | 1.89 | 458 | 456 | C |
| 149 | 1.72 | 408 | 406 | C |
| 150 | 1.38 | 388 | 386 | C |
| 151 | 1.41 | 438 | 436 | C |
| 152 | 1.56 | 377 | 375 | C |
| 153 | 1.70 | 451 | 449 | C |
| 154 | 1.67 | 407 | 405 | C |
| 155 | 1.47 | 391 | 389 | C |
| 156 | 1.46 | 377 | 375 | C |
| 157 | 1.60 | 377 | 375 | C |
| 158 | 1.33 | 394 | 392 | C |
| 159 | 1.65 | 421 | 419 | C |
| 160 | 1.57 | 377 | 375 | C |
| 161 | 1.76 | 445 | 443 | C |
| 162 | 1.36 | 388 | 386 | C |
| 163 | 1.52 | 398 | 396 | C |
| 164 | 1.32 | 376 | 374 | C |
| 165 | 2.04 | 459 | 456 | C |
| 166 | 1.43 | 404 | 402 | C |
| 167 | 1.73 | 441 | 439 | C |
| 168 | 0.75 | 406 | 404 | A |
| 169 | 1.98 | 474 | 472 | C |
| 170 | 1.83 | 461 | 459 | C |
| 171 | 1.96 | 459 | 457 | C |
| 172 | 1.86 | 455 | 453 | C |
| 173 | 1.35 | 423 | 421 | C |
| 174 | 1.58 | 417 | 415 | C |
| 175 | 1.77 | 439 | 437 | C |
| 176 | 1.52 | 413 | 411 | C |
| 177 | 1.36 | 376 | 374 | C |
| 178 | 1.45 | 426 | 424 | C |
| 179 | 1.46 | 416 | 414 | C |
| 180 | 1.88 | 412 | 410 | C |
| 181 | 1.32 | 388 | 386 | C |
| 182 | 1.73 | 459 | 457 | C |
| 183 | 1.54 | 413 | 411 | C |
| 184 | 1.50 | 377 | 375 | C |
| 185 | 0.83 | 423 | 421 | A |
| 186 | 0.76 | 406 | 404 | A |
| 187 | 1.79 | 413 | 411 | C |
| 188 | 1.62 | 427 | 425 | C |
| 189 | 1.00 | 422 | 420 | A |
| 190 | 0.87 | 415 | 413 | A |
| 191 | 0.93 | 440 | 438 | A |
| 192 | 0.96 | 456 | 454 | A |
| 193 | 0.96 | 456 | 454 | A |
| 194 | 1.93 | 458 | 456 | C |
| 195 | 0.88 | 420 | 418 | A |
| 196 | 0.81 | 403 | 401 | A |
| 197 | 0.86 | 397 | 395 | A |
| 198 | 0.69 | 390 | 388 | A |
| 199 | 0.66 | 405 | 405 | B |
| 200 | 0.88 | 457 | | A |
| 201 | 0.92 | 412 | 470 [M+CH₃COO]⁻ | A |
| 202 | 0.76 | 362 | | A |
| 203 | 0.76 | 455 | 455 | A |
| 204 | 0.76 | 455 | | A |
| 205 | 0.89 | 412 | | A |
| 206 | 1.00 | 400 | | A |
| 207 | 0.65 | 390 | 448 [M+CH₃COO]⁻ | A |
| 208 | 0.93 | 392 | | A |
| 209 | 0.91 | 412 | | A |
| 210 | 0.75 | 401 | | A |
| 211 | 0.75 | 401 | | A |
| 212 | 0.69 | 412 | | A |
| 213 | 0.91 | 392 | 390 | A |
| 214 | 0.99 | 400 | 398 | A |
| 215 | 0.92 | 457 | | A |
| 216 | 1.20 | 376 | 374 | C |
| 217 | 1.79 | 382 | 380 | C |
| 218 | 1.90 | 398 | 396 | C |
| 219 | 0.94 | 443 | 441 | A |
| 220 (.HCl) | 0.78 | 401 | | A |
| 221 | 0.89 | 412 | 410 | A |
| 222 | 0.90 | 405 | 403 | A |
| 223 | 0.94 | 405 | 403 | A |
| 224 | 0.79 | 395 | 393 | A |
| 225 | 1.52 | 385 | 383 | C |
| 226 | 1.11 | 452 | 450 | A |
| 227 | 1.60 | 429 | 427 | C |
| 228 | 0.92 | 429 | 427 | A |
| 229 | 0.87 | 387 | 385 | A |
| 230 | 1.37 | 405 | 403 | C |
| 231 | 0.64 | 413 | 411 | A |
| 232 | 1.46 | 388 | 386 | C |
| 233 | 1.37 | 388 | 386 | C |
| 234 | 1.51 | 398 | 396 | C |
| 235 | 1.53 | 407 | 405 | C |
| 236 | 1.25 | 390 | 388 | C |
| 237 | 1.54 | 408 | 406 | C |
| 238 | 1.62 | 432 | 430 | C |
| 239 | 1.63 | 441 | 439 | C |
| 240 | 1.38 | 362 | 360 | C |
| 241 | 1.93 | 474 | 472 | C |
| 242 | 1.85 | 475 | 473 | C |
| 243 | 1.49 | 363 | 361 | C |
| 244 | 1.36 | 388 | 386 | C |
| 245 | 1.81 | 426 | 424 | C |
| 246 | 1.65 | 431 | 429 | C |
| 247 | 1.90 | 470 | 468 | C |
| 248 | 1.28 | 374 | 372 | C |
| 249 | 1.32 | 374 | 372 | C |
| 250 | 1.53 | 391 | 389 | C |
| 251 | 1.55 | 391 | 389 | C |
| 252 | 1.57 | 391 | 389 | C |
| 253 | 1.65 | 441 | 439 | C |
| 254 | 1.53 | 391 | 389 | C |
| 255 | 1.51 | 406 | 404 | C |
| 256 | 1.34 | 374 | 372 | C |
| 257 | 1.38 | 374 | 372 | C |
| 258 | 1.93 | 419 | 417 | C |
| 259 | 2.35 | 484 | 482 | C |
| 260 | 1.51 | 377 | 375 | C |
| 261 | 1.36 | 389 | 387 | C |
| 262 | 1.51 | 391 | 389 | C |
| 263 | 1.78 | 455 | 453 | C |
| 264 | 1.54 | 407 | 405 | C |
| 265 | 1.65 | 427 | 425 | C |
| 266 | 2.41 | 485 | 483 | C |
| 267 | 0.91 | 456 | 454 | A |
| 268 | 1.67 | 441 | 439 | C |
| 269 | 0.90 | 431 | 429 | A |
| 270 | 0.85 | 439 | 437 | A |
| 271 | 0.78 | 405 | 403 | A |
| 272 | 0.73 | 417 | 415 | A |
| 273 | 1.98 | 438 | 436 | C |
| 274 | 1.73 | 439 | 437 | C |
| 275 | 1.73 | 417 | 415 | C |
| 276 | 1.06 | 461 | 459 | A |
| 277 | 0.97 | 449 | 507 [M+CH₃COO]⁻ | A |
| 278 | 1.01 | 445 | 443 | A |
| 279 | 0.97 | 445 | 443 | A |
| 280 | 1.16 | 483 | 481 | A |
| 281 | 1.89 | 453 | 451 | C |
| 282/387 | 0.93 | 435 | 433 | A |
| 283 | 1.75 | 445 | 443 | C |
| 284 | 5.27 | 433 | 431 | A |
| 285 | 1.76 | 467 | 465 | C |
| 286 | 1.76 | 467 | 465 | C |
| 287 | 1.79 | 453 | 451 | C |
| 288 | 0.78 | 431 | 429 | A |
| 289 | 1.01 | 425 | 423 | A |
| 290 | 0.87 | 429 | 427 | A |
| 291 | 0.92 | 423 | 421 | A |
| 292 | 1.83 | 442 | 442 440 | C |
| 293 | 0.95 | 465 | 463 | A |
| 294 | 0.82 | 449 | 447 | A |
| 295 | 0.9 | 437 | 435 | A |
| 296 | 0.94 | 468 | 466 | A |
| 297 | 0.91 | 437 | 435 | A |
| 298 | 0.86 | 463 | 461 | A |
| 299 | 0.92 | 437 | 435 | A |
| 300 | 0.98 | 437 | 435 | A |
| 301 | 0.89 | 445 | 443 | A |
| 302 | 0.98 | 412 | 410 | A |
| 303 | 0.79 | 439 | 437 | A |
| 304 | 0.92 | 425 | 423 | A |
| 305 | 0.76 | 439 | 437 | A |
| 306 | 0.79 | 439 | 437 | A |
| 307 | 0.99 | 437 | 435 | A |
| 308 | 0.87 | 435 | 433 | A |
| 309 | 0.81 | 453 | 451 | A |
| 310 | 0.87 | 469 | 467 | A |
| 311 | 0.75 | 435 | 433 | A |
| 312 | 0.82 | 453 | 431 | A |
| 313 | 0.8 | 435 | 433 | A |
| 314 | 0.75 | 435 | 433 | A |
| 315 | 0.76 | 435 | 433 | A |
| 316 | 1.08 | 435 | - | E |
| 317 | 0.76 | 435 | 433 | A |
| 318 | 1.15 | 435 | - | E |
| 319 | 0.97 | 434 | 432 | A |
| 320 | 0.94 | 434 | 432 | A |
| 321 | 0.88 | 437 | 435 | A |
| 322 | 0.75 | 423 | 421 | A |
| 323 | 0.87 | 423 | 421 | A |
| 324 | 0.81 | 423 | 481 [M+CH₃COO⁻] | A |
| 325 | 0.82 | 441 | 439 | A |
| 326 | 0.75 | 423 | 421 | A |
| 327 | 0.9 | 425 | 423 | A |
| 328 | 0.91 | 425 | 483 [M+CH₃COO⁻] | A |
| 329 | 0.88 | 457 | 515 [M+CH₃COO⁻] | A |
| 331 | 0.98 | 422 | 480 [M+CH₃COO⁻] | A |
| 332 | 0.83 | 441 | 499 [M+CH₃COO⁻] | A |
| 333 | 1.01 | 452 | 450 | A |
| 334 | 1.03 | 440 | 438 | A |
| 335 | 1.7 | 458 | 456 | C |
| 336 | 0.93 | 448 | 446 | A |
| 337 | 0.9 | 420 | 418 | A |
| 338 | 1.00 | 470 | 468 | A |
| 339 | 0.99 | 432 | 430 | A |
| 340 | 0.98 | 452 | 450 | A |
| 341 | 0.9 | 420 | 418 | A |
| 342 | 1.02 | 486 | 484 | A |
| 343 | 1.72 | 432 | 430 | C |
| 344 | 1.87 | 434 | 432 | C |
| 345 | 1.8 | 935 [2M+1] | 466 | C |
| 346 | 1 | 430 | 428 | A |
| 347 | 0.9 | 404 | 402 | A |
| 348 | 1.04 | 430 | 428 | A |
| 349 | 0.91 | 404 | 402 | A |
| 350 | 0.98 | 464 | 462 | A |
| 351 | 0.97 | 424 | 422 | A |
| 352 | 1.02 | 490 | 488 | A |
| 353 | 1.74 | 424 | 422 | C |
| 354 | 0.83 | 388 | 386 | A |
| 355 | 1.96 | 465 | 463 | C |
| 356 | 1.01 | 452 | 450 | A |
| 357 | 1.91 | 470 | 468 | |
| 358 | 1.83 | 418 | 416 | C |
| 359 | 1.81 | 450 | 448 | C |
| 360 | 0.74 | 430 | 428 | A |
| 361 | 0.68 | 417 | 415 | A |
| 362 | 1.79 | 427 | 425 | C |
| 363 | 1.84 | 451 | 449 | C |
| 364 | 0.8 | 444 | 442 | A |
| 365 | 1.64 | 413 | 411 | C |
| 366 | 0.75 | 413 | 411 | A |
| 367 | 0.7 | 416 | 414 | A |
| 368 | 0.82 | 455 | 453 | A |
| 369 | 1 | 432 | 430 | A |
| 370 | 0.97 | 442 | 440 | A |
| 371 | 0.96 | 424 | 422 | A |
| 372 | 0.94 | 440 | 438 | A |
| 373 | 1.8 | 451 | 449 | C |
| 374 | 0.94 | 455 | 453 | A |
| 375 | 1.01 | 432 | 430 | A |
| 376 | 0.92 | 448 | 446 | A |
| 377 | 0.95 | 436 | 434 | A |
| 378 | 0.88 | 455 | 453 | A |
| 379 | 0.7 | 438 | 436 | A |
| 380 | 0.78 | 438 | 436 | A |
| 381 | 0.71 | 436 | 434 | A |
| 382 | 0.74 | 438 | 436 | A |
| 383 | 0.85 | 450 | 448 | A |
| 384 | 0.88 | 457 | 455 | A |
| 385 | 0.9 | 455 | 453 | A |
| 386 | 1.55 | 431 | 429 | C |
| 282/387 | 0.93 | 435 | 433 | A |
| 390 | 0.99 | 417 | 415 | E |
| 391 | 1.25 | 411 | 409 | E |
| 392 | 1.01 | 461 | 459 | A |
| 394 | 1.01 | 473 | 471 | A |
| 396 | 1.88 | 473 | 471 | C |
| 398 | 0.99 | 435 | 433 | A |
| 400 | 0.78 | 431 | 429 | A |
| 402 | 0.62 | 417 | 415 | A |
| 403 | 0.9 | 455 | 453 | A |
| 404 | 0.93 | 451 | 449 | A |
| 405 | 0.91 | 451 | 449 | A |
| 406 | 0.75 | 438 | 436 | A |

### SFC-MS METHODS

The SFC measurement was performed using an Analytical Supercritical fluid chromatography (SFC) system composed by a binary pump for delivering carbon dioxide (CO₂) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

**TABLE 4A. Analytical SFC-MS Methods (Flow expressed in mL/min; column temperature (T) in °C; Run time in minutes, Backpressure (BPR) in bars.**

| METHOD CODE | COLUMN | MOBILE PHASE | GRADIENT | $\begin{matrix}FLOW \\ COL T\end{matrix}$ | $\begin{matrix}RUN TIME \\ BPR\end{matrix}$ |
|---|---|---|---|---|---|
| 1 | Daicel Chiralpak® AD-H column (5.0 µm, 250 x 4.6 mm) | A:CO₂ | 40% B hold 4 min, to 50% in 1 min hold 2 min | $\begin{matrix}5 \\ 40\end{matrix}$ | $\begin{matrix}7 \\ 110\end{matrix}$ |
| | | B: EtOH+0.2% iPrNH₂ | | | |
| 2 | Daicel Chiralpak® AD-3 column (3.0 µm, 150 x 4.6 mm) | A:CO₂ | 10%-50% B in 6 min, hold 3.5 min | $\begin{matrix}2.5 \\ 40\end{matrix}$ | $\begin{matrix}9.5 \\ 110\end{matrix}$ |
| | | B: EtOH+0.2% iPrNH2 | | | |
| 3 | Daicel Chiralpak® AD-H column (5.0 µm, 250 x 4.6 mm) | A:CO₂ | 35% B hold 4 min, to 50% in 1 min hold 2 min | $\begin{matrix}5 \\ 40\end{matrix}$ | $\begin{matrix}7 \\ 110\end{matrix}$ |
| | | B: (EtOH-iPrOH)+0.2% iPrNH2 | | | |
| 4 | Daicel Chiralpak® AD-H column (5.0 µm, 250 x 4.6 mm) | A:CO₂ | 25% B hold 4 min, to 50% in 1 min hold 2 min | $\begin{matrix}5 \\ 40\end{matrix}$ | $\begin{matrix}7 \\ 110\end{matrix}$ |
| | | B: iPrOH+0.2% iPrNH₂ | | | |
| 5 | Daicel Chiralpak® AD-H column (5.0 µm, 250 x 4.6 mm) | A:CO₂ | 30% B hold 4 min, to 50% in 1 min hold 2 min | $\begin{matrix}5 \\ 40\end{matrix}$ | $\begin{matrix}7 \\ 110\end{matrix}$ |
| | | B: iPrOH+0.2% iPrNH₂ | | | |
| 6 | Daicel Chiralpak® AD-H column (5.0 µm, 250 x 4.6 mm) | A:CO₂ | 40% B hold 4 min, to 50% in 1 min hold 2 min | $\begin{matrix}5 \\ 40\end{matrix}$ | $\begin{matrix}7 \\ 110\end{matrix}$ |
| | | B: iPrOH+0.2% iPrNH₂ | | | |
| 7 | Daicel Chiralpak® AD-3 column (3.0 µm, 150 x 4.6 mm) | A:CO₂ | 10%-50% B in 6 min, hold 3.5 min | $\begin{matrix}2.5 \\ 40\end{matrix}$ | $\begin{matrix}9.5 \\ 110\end{matrix}$ |
| | | B: iPrOH+0.2% iPrNH₂ | | | |
| 8 | Daicel Chiralpak® AD-H column (5.0 µm, 250 x 4.6 mm) | A:CO₂ | 25% B hold 4 min, to 50% in 1 min hold 2 min | $\begin{matrix}5 \\ 40\end{matrix}$ | $\begin{matrix}7 \\ 110\end{matrix}$ |
| | | B: (MeOH-iPrOH)+0.2% iPrNH₂ | | | |
| 9 | Daicel Chiralpak® AD-H column (5.0 µm, 250 x 4.6 mm) | A:CO₂ | 30% B hold 4 min, to 50% in 1 min hold 2 min | $\begin{matrix}5 \\ 40\end{matrix}$ | $\begin{matrix}7 \\ 110\end{matrix}$ |
| | | B: (MeOH-iPrOH)+0.2% iPrNH₂ | | | |
| 10 | Daicel (AD, OD, OJ, AS,)-H-column (5.0 µm, 250 x 4.6 mm) | A: CO₂ | 10%-40% B in 19.4 min, 40-50% in 2.00 min hold 3.6 min | $\begin{matrix}3 \\ 30\end{matrix}$ | $\begin{matrix}25 \\ 110\end{matrix}$ |
| | | B: 3 different solvent for B used: (MeOH, EtOH, iPrOH) +0.2% iPrNH₂ | | | |
| 13 | Daicel AS-H column (5.0 µm, 500 x 4.6 mm) | A: CO₂ | 10%-40% B in 19.4 min, 40-50% in 2.00 min hold 3.6 min | $\begin{matrix}3 \\ 50\end{matrix}$ | $\begin{matrix}25 \\ 110\end{matrix}$ |
| | | B: 3 different solvent for B used: (MeOH, EtOH, iPrOH) +0.2% iPrNH₂ | | | |
| 12 | Daicel AS-H column (5.0 µm, 500 x 4.6 mm) | A: CO₂ | 20% B hold 17.5 min, 20-50% in 3.00 min hold 4.1 min | $\begin{matrix}3 \\ 50\end{matrix}$ | $\begin{matrix}25 \\ 110\end{matrix}$ |
| | | B: 3 different solvent for B used: (MeOH, EtOH, iPrOH) +0.2% iPrNH₂ | | | |
| 13 | Daicel OJ-H-column (5.0 µm, 250 x 4.6 mm) | A: CO₂ | 12% B hold 10 min | $\begin{matrix}3 \\ 30\end{matrix}$ | $\begin{matrix}10 \\ 110\end{matrix}$ |
| | | B: 3 different solvent for B used: MeOH )+0.2% iPrNH₂ | | | |
| 14 | Daicel Chiralpak® AD-H column (5.0 µm, 250 x 4.6 mm) | A:CO₂ | 20% B hold 4 min, to 50% in 1 min hold 2 min | $\begin{matrix}5 \\ 40\end{matrix}$ | $\begin{matrix}7 \\ 110\end{matrix}$ |
| | | B: MeOH+0.2% iPrNH₂ | | | |
| 15 | Daicel Chiralpak® OD-H column (5.0 µm, 250 x 4.6 mm) | A:CO₂ | 30% B hold 4 min, to 50% in 1 min hold 2 min | $\begin{matrix}5 \\ 40\end{matrix}$ | $\begin{matrix}7 \\ 110\end{matrix}$ |
| | | B: EtOH+0.2% iPrNH₂ | | | |
| 16 | Daicel Chiralpak® OD-3 column (3.0 µm, 150 x 4.6 mm) | A:CO₂ | 10%-50% B in 6 min, hold 3.5 min | $\begin{matrix}2.5 \\ 40\end{matrix}$ | $\begin{matrix}9.5 \\ 110\end{matrix}$ |
| | | B: EtOH+0.2% iPrNH₂ | | | |
| 17 | Daicel Chiralpak® AD3 column (3.0 µm, 150 x 4.6 mm) | A:CO₂ | 10%-50% B in 6 min, hold 3.5 min | $\begin{matrix}2.5 \\ 40\end{matrix}$ | $\begin{matrix}9.5 \\ 130\end{matrix}$ |
| | | B: EtOH+0.2% iPrNH₂ | | | |
| 18 | Daicel Chiralpak® IC3 column (3.0 µm, 150 x 4.6 mm) | A:CO₂ | 10%-50% B in 6 min, hold 3.5 min | $\begin{matrix}2.5 \\ 40\end{matrix}$ | $\begin{matrix}9.5 \\ 130\end{matrix}$ |
| | | B: EtOH+0.2% iPrNH2 | | | |

**TABLE 4B. ANALYTICAL SFC DATA - Rₜ means retention time (in minutes), [M+H]⁺ means the protonated mass of the compound, method refers to the method used for (SFC)MS analysis of enantiomerically pure compounds.**

| CO. No. | R_{T} (MIN) | [M+H]⁺ | METHOD | ELUTION ORDER |
|---|---|---|---|---|
| 1a | 3.66 | 365 | 14 | Enantiomer 2 |
| 1b | 2.51 | 365 | 14 | Enantiomer 1 |
| 3a | 3 | 365 | 8 | Enantiomer 1 |
| 3b | 2.11 | 365 | 8 | Enantiomer 2 |
| 24a | 1.05 | 445 | 6 | Enantiomer 1 |
| 24b | 1.52 | 445 | 6 | Enantiomer 2 |
| 25a | 4.79 | 441 | 16 | Enantiomer 1 |
| 25b | 5.48 | no response | 16 | Enantiomer 1 |
| 28 | 3.95 | 439 | 2 | Enantiomer 2 |
| 29 | 1.33 | 413 | 15 | Enantiomer 2 |
| 30 | 2.54 | 413 | 15 | Enantiomer 1 |
| 33 | 5.45 | 447 | 7 | Enantiomer 1 |
| 34 | 4.85 | 447 | 7 | Enantiomer 2 |
| 45 | 1.2 | 443 | 4 | Enantiomer 2 |
| 46 | 1.56 | 443 | 4 | Enantiomer 1 |
| 62 | 3.42 | 417 | 2 | |
| 73 | 2.47 | 448 | 5 | Enantiomer 1 |
| 75 | 1.42 | 448 | 5 | Enantiomer 2 |
| 81 | 2.81 | 419 | 2 | Enantiomer 1 |
| 82 | 3.25 | 419 | 2 | Enantiomer 2 |
| 86 | 3.09 | 417 | 2 | Enantiomer 2 |
| 95 | 6.8 | 405 | 7 | Enantiomer 2 |
| 97 | 5.22 | 412 | 13 | N/A |
| 99 | 3.33 | 376 | 3 | Enantiomer 2 |
| 104 | 1.67 | 413 | 1 | Enantiomer 2 |
| 109 | 2.54 | 398 | 1 | N/A |
| 116 | 2.12 | 382 | 9 | Enantiomer 2 |
| 123 | 5.72 | 400 | 10 | N/A |
| 140 | 4.67 | 438 | 2 | Enantiomer 2 |
| 201 | 8.79 | 412 | 11 | Enantiomer 1 |
| 203 | 9.22 | 455 | 12 | Enantiomer 1 |
| 204 | 7.53 | 455 | 12 | Enantiomer 1 |
| 216 | 2.2 | 376 | 3 | Enantiomer 1 |
| 217 | 1.29 | 382 | 9 | Enantiomer 1 |
| 218 | 1.33 | 398 | 1 | Enantiomer 1 |
| 230 | 6.35 | 405 | 7 | Enantiomer 2 |
| 273 | 4.21 | 438 | 2 | Enantiomer 2 |
| 274 | 4.5 | 439 | 2 | |
| 285 | 6.81 | 467 | 18 | Diastereomer 4 |
| 286 | 4.95 | 467 | 18 | Diastereomer 3 |
| 392 | 2.64 | 461 | 17 | Enantiomer 1 |

### NUCLEAR MAGNETIC RESONANCE (NMR)

The ¹H NMR spectrum was recorded either on Bruker DPX-400 spectrometer with standard pulse sequences, operating at 400 MHz or on a Bruker DPX-360 operating at 360 MHz, using DMSO-*d₆* (deuterated DMSO, dimethyl-d6 sulfoxide) as solvents. Chemical shifts (δ) are reported in parts per million (ppm) relative to tetramethylsilane (TMS), which was used as internal standard.
Co. No. 25a: ¹H NMR (400 MHz, DMSO-*d*_{6ON} 100 CELSIUS DEGREES) δ ppm 1.74 - 1.93 (m, 4 H) 1.97 - 2.11 (m, 3 H) 2.31 - 2.45 (m, 1 H) 4.06 - 4.23 (m, 1 H) 4.61 (br s, 1 H) 4.82 (br s, 1 H) 7.02 (t, *J*=54.2 Hz, 1 H) 7.50 (s, 1 H) 7.78 (dd, *J*=8.4, 1.8 Hz, 1 H) 8.17 (d, *J*=8.4 Hz, 1 H) 8.44 (br s, 1 H) 8.47 (d, *J*=1.1 Hz, 1 H) 9.45 (s, 1 H);
Co. No. 93: ¹H NMR (400 MHz, DMSO-*d*_{6 ON} 100 CELSIUS DEGREES) δ ppm 0.84 (d, J=6.6 Hz, 3 H) 0.88 - 0.98 (m, 4 H) 1.45 (qd, *J*=12.4, 4.4 Hz, 1 H) 1.91 (br dd, *J*=13.4, 2.6 Hz, 1 H) 2.06 - 2.14 (m, 1 H) 2.42 (s, 3 H) 2.45 - 2.49 (m, 1 H) 3.13 (br t, *J*=11.6 Hz, 1 H) 3.37 (dd, *J*=12.9, 11.1 Hz, 1 H) 3.60 (td, *J*=10.9, 4.0 Hz, 1 H) 4.17 (br s, 2 H) 6.79 - 7.23 (m, 3 H) 7.57 (s, 1 H) 8.72 (s, 1 H);
Co. No. 108: ¹H NMR (400 MHz, DMSO-*d₆*) δ ppm 0.77 (d, *J*=6.4 Hz, 3 H) 1.34 - 1.56 (m, 1 H) 1.68 - 2.00 (m, 1 H) 2.43 (br s, 1 H) 2.76 - 3.28 (m, 2 H) 3.49 - 3.74 (m, 2 H) 3.87 (s, 3 H) 4.46 - 4.76 (m, 1 H) 6.81 - 7.33 (m, 3 H) 7.65 (s, 1 H) 8.80 (s, 1 H);
Co. No. 110: ¹H NMR (400 MHz, DMSO-*d*_{6 ON} 100 CELSIUS DEGREES) δ ppm 0.83 (d, *J*=6.6 Hz, 3 H) 1.39 - 1.50 (m, 1 H) 1.85 - 1.95 (m, 1 H) 2.43 (s, 3 H) 2.44 - 2.48 (m, 1 H) 3.13 (br t, *J*=12.3 Hz, 1 H) 3.37 (dd, *J*=13.0, 11.2 Hz, 1 H) 3.58 (td, *J*=10.9, 4.0 Hz, 1 H) 3.88 (s, 3 H) 4.13 (br s, 2 H) 6.58 (s, 1 H) 6.82 (s, 1 H) 7.01 (t, *J*=54.2 Hz, 1 H) 7.56 (s, 1 H) 8.71 (s, 1 H);
Co. No. 132: ¹H NMR (360 MHz, DMSO-*d*₆) δ ppm 0.78 (d, J=6.6 Hz, 3 H) 1.38 - 1.61 (m, 1 H) 1.67 - 2.01 (m, 1 H) 2.60 (s, 3 H) 2.86 - 3.30 (m, 2 H) 3.49 (br d, *J*=14.6 Hz, 1 H) 3.59 - 3.72 (m, 2 H) 4.48 - 4.79 (m, 1 H) 6.87 - 7.35 (m, 1 H) 7.54 - 7.89 (m, 3 H) 8.79 (s, 1 H);
Co. No. 228: ¹H NMR (400 MHz, DMSO-*d*_{6 ON} 100 CELSIUS DEGREES) δ ppm 0.85 (d, *J*=6.6 Hz, 4 H) 1.43 - 1.56 (m, 1 H) 1.86 - 1.99 (m, 1 H) 3.19 (td, *J*=12.9, 2.8 Hz, 1 H) 3.42 (dd, *J*=13.0, 11.2 Hz, 1 H) 3.64 (td, *J*=10.9, 4.0 Hz, 1 H) 4.15 - 4.26 (m, 1 H) 4.28 - 4.38 (m, 1 H) 7.01 (t, *J*=54.2 Hz, 1 H) 7.58 (br s, 1 H) 7.61 (dd, *J*=8.4, 1.5 Hz, 1 H) 8.11 (d, *J*=7.9 Hz, 1 H) 8.27 (d, *J*=1.3 Hz, 1 H) 8.70 (s, 1 H) 9.40 (s, 1 H);
Co. No. 281: ¹H NMR (400 MHz, DMSO-*d*_{6 ON} 120 CESCIUS DEGREES) δ ppm 0.82 (d, *J*=6.40 Hz, 3 H) 1.46 (qd, *J*=12.47, 4.40 Hz, 1 H) 1.84 - 1.93 (m, 1 H) 2.41 - 2.53 (m, 1 H) 2.84 (s, 3 H) 3.07 - 3.18 (m, 1 H) 3.35 (dd, *J*=12.98, 11.22 Hz, 1 H) 3.58 (td, *J*=10.95, 3.85 Hz, 1 H) 3.88 - 4.33 (m, 2 H) 6.85 (s, 1 H) 6.98 (t, *J*=54.36 Hz, 1 H) 7.12 (s, 1 H) 7.53 (s, 1 H) 7.55 (t, *J*=73.07 Hz, 1 H) 7.74 (s, 1 H) 8.67 (s, 1 H);
Co. No. 295: ¹H NMR (400 MHz, DMSO-d_{6 ON} 100 CELSIUS DEGREES) δ ppm 1.67 - 1.87 (m, 4 H) 1.89 - 2.10 (m, 3 H) 2.26 - 2.38 (m, 1 H) 3.82 (s, 3 H) 4.13 (br d, *J*=12.10 Hz, 1 H) 4.56 (br s, 1 H) 4.86 (br s, 1 H) 6.55 (d, *J*=2.64 Hz, 1 H) 7.02 (t, *J*=55.00 Hz, 1 H) 7.35 (d, *J*=3.08 Hz, 1 H) 7.41 - 7.49 (m, 3 H) 7.90 (s, 1 H) 8.59 (s, 1 H);
Co. No. 321: ¹H NMR (400 MHz, DMSO-*d*_{6 ON} 100 CELSIUS DEGREES) δ ppm 0.81 (d, J=6.60 Hz, 3 H) 1.47 (qd, *J*=12.43, 4.29 Hz, 1 H) 1.85 - 1.93 (m, 1 H) 2.39 - 2.46 (m, 1 H) 2.94 (s, 3 H) 3.09 - 3.18 (m, 1 H) 3.33 - 3.40 (m, 1 H) 3.57 - 3.64 (m, 1 H) 3.83-4.52 (m, 2 H) 6.82 (m, 1 H) 7.00 (m, 1 H) 7.43 (s, 1 H) 7.46 (s, 1 H) 7.55 (s, 1 H) 8.69 (s, 1 H);
Co. No. 335: ¹H NMR (400 MHz, DMSO-d_{6 ON} 100 CELSIUS DEGREES) δ ppm 1.74 - 2.00 (m, 6 H) 2.02 - 2.08 (m, 1 H) 2.36 - 2.41 (m, 1 H) 2.42 (s, 3 H) 3.84 (s, 3 H) 4.08 - 4.14 (m, 1 H) 4.81 - 4.85 (m, 1 H) 5.13 - 5.17 (m, 1 H) 7.05 (t, *J*=54.40 Hz, 1 H) 7.56 (s, 1 H) 7.89 (s, 1 H) 8.69 (s, 1 H);
Co. No. 356: ¹H NMR (400 MHz, DMSO-d_{6 ON} 100 CELSIUS DEGREES) δ ppm 1.68 - 1.89 (m, 4 H) 1.92 - 2.04 (m, 3 H) 2.30 - 2.45 (m, 1 H) 4.01 - 4.07 (m, 1 H) 4.39 - 4.84 (m, 2 H) 7.01 (t, *J*=52.80 Hz, 1 H) 7.49 (s, 1 H) 7.58 (dd, *J*=8.25, 1.87 Hz, 1 H) 7.71 (d, *J*=8.14 Hz, 1 H) 7.83 (d, *J*=1.54 Hz, 1 H) 8.58 (br s, 1 H).

### PHARMACOLOGICAL EXAMPLES

The compounds provided in the present invention are an inhibitors of PDE2, particularly of PDE2A. The results of testing the compounds in several pharmacological assays are shown below.

### IN VITRO ASSAY PDE2A

Human recombinant PDE2A (hPDE2A) was expressed in Sf9 cells using a recombinant rPDE10A baculovirus construct. Cells were harvested after 48 h of infection and the hPDE2A protein was purified by metal chelate chromatography on Ni-sepharose 6FF . Tested compounds were dissolved and diluted in 100% DMSO to a concentration 100 fold of the final concentration in the assay. Compound dilutions (0.4 µl) were added in 384 well plates to 20 µl of incubation buffer (50 mM Tris pH 7.8, 8.3 mM MgCl₂, 1.7 mM EGTA). 10µl of hPDE2A enzyme in incubation buffer was added and the reaction was started by addition of 10 µl substrate to a final concentration of 10 µM cGMP and 0.01 µCi ³H-cGMP. The reaction was incubated for 45 minutes at room temperature. After incubation, the reaction was stopped with 20 µl of stop solution consisting of 17.8 mg/ml PDE SPA scintillation proximity assay) beads supplemented with 200 mM ZnCl₂. After sedimentation of the beads during 30 minutes the radioactivity was measured in a Perkin Elmer Topcount scintillation counter and results were expressed as cpm. For blanc values the enzyme was omitted from the reaction and replaced by incubation buffer. Control values were obtained by addition of a final concentration of 1% DMSO instead of compound. A best fit curve is fitted by a minimum sum of squares method to the plot of % of control value substracted with blanc value versus compound concentration and the half maximal inhibitory concentration (IC₅₀) value is derived from this curve.

### IN VITRO ASSAY PDE3A

Human recombinant PDE3B (hPDE3B) was supplied as a partially purified insect cell lysate by Scottish Biomedical, it was cloned from human brain and expressed in Sf9 cells. Tested compounds were dissolved and diluted in 100% DMSO to a concentration 100 fold of the final concentration in the assay. Compound dilutions (0.4 µl) were added in 384 well plates to 20 µl of incubation buffer (50 mM Tris pH 7.8, 8.3 mM MgCl₂, 1.7 mM EGTA). 10µl of hPDE3B enzyme in incubation buffer was added and the reaction was started by addition of 10 µl substrate to a final concentration of 0.4 µM cAMP and 2.4 µCi/ml [³H]-cAMP. The reaction was incubated for 60 min at room temperature. After incubation, the reaction was stopped with 20 µl of stop solution consisting of 17.8 mg/ml PDE SPA (scintillation proximity assay) beads supplemented with 200 mM ZnCl₂. After sedimentation of the beads during 30 min the radioactivity was measured in a Perkin Elmer Topcount scintillation counter and results were expressed as cpm. For blanc values the enzyme was omitted from the reaction and replaced by incubation buffer. Control values were obtained by addition of a final concentration of 1% DMSO instead of compound. A best fit curve is fitted by a minimum sum of squares method to the plot of % of control value substracted with blanc value versus compound concentration and the half maximal inhibitory concentration (IC₅₀) value is derived from this curve.

### IN VITRO ASSAY PDE10A

Rat recombinant PDE10A (rPDE10A2) was expressed in Sf9 cells using a recombinant rPDE10A baculovirus construct. Cells were harvested after 48 h of infection and the rPDE10A protein was purified by metal chelate chromatography on Ni-sepharose 6FF. Tested compounds were dissolved and diluted in 100% DMSO to a concentration 100 fold of the final concentration in the assay. Human recombinant PDE10A (hPDE2A) was expressed in Sf9 cells using a recombinant hPDE10A baculovirus that was made and amplified in house. Cells were harvested after 72 h of infection and the hPDE10A protein was purified by metal chelate chromatography on Ni-sepharose. Compound dilutions (0.4 µl) were added in 384 well plates to 20 µl of incubation buffer (50 mM Tris pH 7.8, 8.3 mM MgCl₂, 1.7 mM EGTA). 10µl of rPDE10A or hPDE10A enzyme in incubation buffer was added and the reaction was started by addition of 10 µl substrate to a final concentration of 60 nM cAMP and 0.008 µCi ³H-cAMP. The reaction was incubated for 60 minutes at room temperature. After incubation, the reaction was stopped with 20 µl of stop solution consisting of 17.8 mg/ml PDE SPA (scintillation proximity assay) beads. After sedimentation of the beads during 30 minutes the radioactivity was measured in a Perkin Elmer Topcount scintillation counter and results were expressed as cpm. For blanc values the enzyme was omitted from the reaction and replaced by incubation buffer. Control values were obtained by addition of a final concentration of 1% DMSO instead of compound. A best fit curve is fitted by a minimum sum of squares method to the plot of % of control value substracted with blanc value versus compound concentration and the half maximal inhibitory concentration (IC₅₀) value is derived from this curve.

**TABLE 5. IN VITRO DATA FOR COMPOUNDS OF THE INVENTION. By default, data on inhibition of PDE10A refers to the human clone (also indicated as (h)) unless indicated as (r), referring to the rat clone.**

| Co. No. | hPDE2A pIC₅₀ | hPDE2A Eₘₐₓ | hPDE3B pIC₅₀ | hPDE3B Eₘₐₓ | hPDE10A2 or rPDE10A2 pIC₅₀ | hPDE10A2 or rPDE10A2 Eₘₐₓ |
|---|---|---|---|---|---|---|
| 1a | 9.1 | 100 | 5.68 | 65 | 7.89 | 98 |
| 1b | 5.96 | 88 | <5 | 13 | 5.01 | 50 |
| 2a | 9.23 | 101 | 6.11 | 98 | 8.14 | 101 |
| 2b | 7.72 | 100 | <5 | 31 | 6.57 | 95 |
| 3a | 8.24 | 97 | 5.46 | 77 | 6.57 | 93 |
| 3b | 5.25 | 64 | <5 | 14 | <5 | 19 |
| 4 | 8.84 | 100 | 5.24 | 70 | 7.62 | 99 |
| 5 | 9.02 | 100 | 6.23 | 97 | 8.05 | 100 |
| 6 | 8.79 | 99 | 6.17 | 97 | 7.75 | 100 |
| 7 | 9.1 | 100 | 7.12 | 100 | 7.77 | 101 |
| 8 | 8.96 | 100 | 6.56 | 97 | 7.95 | 100 |
| 9 | 7.86 | 101 | 5.37 | 74 | 7.13 | 100 |
| 10 | 8.96 | 100 | 7.14 | 101 | 8.34 | 100 |
| 11 | 7.64 | 100 | 5.32 | 69 | 6.43 | 95 |
| 12 | 7.67 | 101 | 5.96 | 94 | 6.44 | 97 |
| 13 | 7.98 | 100 | <5 | 38 | 6.46 | 95 |
| 14 | 8.22 | 99 | 5.33 | 70 | 7.05 | 99 |
| 15 | 8.49 | 100 | 5.09 | 54 | 7.14 | 97 |
| 16 | 8.21 | 100 | 5.78 | 88 | 7.2 | 98 |
| 17 | 8.31 | 99 | 5.37 | 74 | 7.09 | 97 |
| 18 | 8.33 | 100 | 5.19 | 65 | 6.71 | 97 |
| 19 | 7.98 | 101 | 5.51 | 79 | 6.89 | 96 |
| 20 | 7.92 | 101 | 5.37 | 74 | 6.88 | 98 |
| 21 | 8.34 | 99 | 5.82 | 89 | 6.88 | 97 |
| 22 | 8.63 | 100 | 5.82 | 87 | 7.8 | 99 |
| 23 | 9.33 | 101 | 5.98 | 90 | 8.15 | 100 |
| 24a | 5.59 | 80 | <5 | 31 | <5 | 23 |
| 24b | 8.19 | 98 | <5 | 28 | 5.13 | 60 |
| 25a | 8.97 | 100 | 5.06 | 54 | 6.23 | 94 |
| 25b | 5.96 | 92 | <5 | 37 | <5 | 28 |
| 26a | <5 | 21 | <5 | 0 | <5 | 7 |
| 26b | 8.17 | 98 | <5 | 41 | 6.63 | 95 |
| 27 | 9.04 | 100 | <5 | 37 | 5.96 | 88 |
| 28 | 9.02 | 101 | <5 | 17 | 6.35 | 95 |
| 29 | 8.82 | 101 | <5 | 18 | 6.16 | 90 |
| 30 | 5.45 | 77 | <5 | 8 | <5 | 32 |
| 31 | 8.6 | 100 | <5 | 8 | 5.95 | 84 |
| 32 | 8.46 | 100 | <5 | 31 | 5.84 | 81 |
| 33 | 7.83 | 99 | <5 | 25 | 5.21 | 67 |
| 34 | 5.71 | 88 | <5 | 31 | <5 | 28 |
| 35 | 8.17 | 100 | <5 | 1 | 5.59 | 76 |
| 36 | 8.56 | 100 | 5.4 | 64 | 6.33 | 93 |
| 37 | 7.76 | 100 | 5.05 | 52 | 6.37 | 93 |
| 38 | 8.21 | 100 | <5 | 43 | 5.64 | 80 |
| 39 | 8.08 | 98 | <5 | 16 | 5.51 | 75 |
| 40 | 9.45 | 101 | 5.15 | 58 | 6.91 (h) | 97 (h) |
| | | | | | 7.1 (r) | 96 (r) |
| 41 | 7.86 | 99 | <5 | 17 | 5.38 | 72 |
| 42 | 8.94 | 100 | <5 | 48 | 6.5 | 95 |
| 43 | 8.3 | 99 | <5 | 29 | 5.88 | 88 |
| 44 | 8.02 | 100 | <5 | 39 | 5.59 | 79 |
| 45 | <5 | 37 | <5 | 1 | <5 | 24 |
| 46 | 7.82 | 100 | <5 | 5 | 5.65 | 86 |
| 47 | 8.6 | 99 | 5.47 | 80 | 6.19 | 92 |
| 48 | 9.76 | 100 | 7.06 | 99 | 7.36 | 100 |
| 49 | 8.68 | 99 | 5.24 | 42 | 6.29 | 95 |
| 50 | 8.51 | 100 | <5 | 61 | 6.14 | 93 |
| 51 | 8.26 | 101 | <5 | 44 | 5.9 | 86 |
| 52 | 7.86 | 101 | <5 | 22 | 5.52 | 76 |
| 53 | 8.25 | 100 | 5.17 | 62 | 6.11 | 89 |
| 54 | 8.38 | 100 | 5.02 | 51 | 6.18 | 89 |
| 55 | 7.9 | 101 | <5 | 41 | 5.75 | 83 |
| 56 | 7.3 | 101 | <5 | 38 | 5.25 | 63 |
| 57 | 7.56 | 100 | <5 | 21 | 5.5 | 78 |
| 58 | 7.61 | 99 | <5 | 18 | 5.35 | 69 |
| 59 | 8.43 | 98 | 5.07 | 56 | 6.23 | 95 |
| 60 | 9.07 | 100 | 5.96 | 92 | 6.82 | 97 |
| 61 | 8.36 | 101 | <5 | 45 | 6.34 | 92 |
| 62 | 5.52 | 77 | <5 | 8 | <5 | 36 |
| 63 | 8.14 | 100 | 5.16 | 60 | 6.06 | 89 |
| 64 | 8.8 | 99 | 5.17 | 62 | 6.45 | 96 |
| 65 | 8.9 | 100 | 5.17 | 70 | 6.6 | 95 |
| 66 | 7.76 | 99 | <5 | 48 | 5.64 | 79 |
| 67 | 8.59 | 99 | <5 | 49 | 6.54 | 94 |
| 68 | 8.2 | 99 | 5 | 50 | 5.86 | 85 |
| 69 | 9.39 | 102 | 6.42 | 95 | 7.34 | 100 |
| 70 | 8.71 | 102 | 5.29 | 66 | 6.48 | 93 |
| 71 | 8.73 | 98 | <6 | 32 | 6.47 | 94 |
| 72 | 8.99 | 100 | <6 | 46 | 6.98 | 92 |
| 73 | 8.71 | 101 | 5.58 | 83 | 6.66 | 98 |
| 74 | 7.52 | 101 | <5 | 30 | <5 | 36 |
| 75 | 5.53 | 81 | <5 | 22 | <5 | 27 |
| 76 | 9.64 | 99 | 5.91 | 91 | 7.5 | 101 |
| 77 | 9.34 | 101 | 6.17 | 96 | 7.08 | 98 |
| 78 | 9 | 102 | 5.8 | 87 | 6.98 | 98 |
| 79 | 8.76 | 99 | 6.04 | 93 | 6.72 | 97 |
| 80 | 9.1 | 100 | 5.38 | 71 | 7.09 | 98 |
| 81 | 5.15 | 59 | <5 | 17 | <5 | 1 |
| 82 | 7.47 | 101 | <5 | 9 | <5 | 18 |
| 83 | 9.03 | 100 | 5.1 | 58 | 7.01 | 98 |
| 84 | 8.5 | 101 | 5.13 | 30 | 6.15 | 89 |
| 85 | 8.19 | 100 | 5.11 | 37 | 5.92 | 88 |
| 86 | 7.3 | 100 | <5 | 34 | <5 | 40 |
| 87 | 6.67 | 98 | <5 | 11 | 5.34 | 69 |
| 88 | 9.68 | 101 | 6.41 | 97 | 8.4 | 99 |
| 89 | 9.64 | 104 | 6.84 | 96 | 7.68 (h) | 101 (h) |
| | | | | | 7.92 (r) | 98 (r) |
| 90 | 9.45 | 101 | 6.67 | 98 | 7.71 | 102 |
| 91 | 9.43 | 100 | 6.15 | 61 | 7.65 | 94 |
| 92 | 9.4 | 100 | 5.88 | 88 | 7.61 | 98 |
| 93 | 9.34 | 101 | 5.59 | 84 | 7.4 | 99 |
| 94 | 9.3 | 101 | 6.98 | 99 | 7.93 | 97 |
| 95 | 9.29 | 100 | 5 | 55 | 7.32 | 98 |
| 96 | 9.26 | 100 | 6.4 | 73 | 7.48 | 95 |
| 97 | 9.24 | 101 | 5.89 | 93 | 7.27 (h) | 101 (h) |
| | | | | | 7.46 (r) | 99 (r) |
| 98 | 9.22 | 100 | 6.22 | 94 | 7.4 | 98 |
| 99 | 9.22 | 100 | 5.68 | 87 | 8.02 | 100 |
| 100 | 9.18 | 101 | 6.57 | 99 | 7.79 | 100 |
| 101 | 9.18 | 101 | 5.32 | 70 | 7.24 | 98 |
| 102 | 9.17 | 100 | <6 | 44 | 7.58 | 95 |
| 103 | 9.15 | 99 | 7.06 | 100 | 7.74 | 100 |
| 104 | 9.09 | 100 | 6.29 | 92 | 7.13 | 98 |
| 105 | 9.06 | 101 | 5.87 | 90 | 7.53 | 101 |
| 106 | 9.05 | 100 | 6.41 | 98 | 7.67 | 99 |
| 107 | 9.04 | 100 | 5.38 | 61 | 7.09 | 99 |
| 108 | 9.04 | 100 | 5.57 | 80 | 7.28 | 99 |
| 109 | 9.03 | 101 | 6.34 | 98 | 8.18 | 99 |
| 110 | 8.88 | 100 | 5.47 | 76 | 6.95 | 98 |
| 111 | 9.01 | 100 | 5.98 | 92 | 7.42 | 99 |
| 112 | 9 | 101 | 5.75 | 84 | 7.32 | 99 |
| 113 | 9 | 99 | 5.95 | 88 | 7.42 | 99 |
| 114 | 8.98 | 100 | 7.05 | 98 | 7.34 | 101 |
| 115 | 8.98 | 100 | 5.71 | 87 | 7.11 | 99 |
| 117 | 8.94 | 100 | 6.07 | 97 | 8.01 | 99 |
| 116 | 8.95 | 101 | 5.66 | 82 | 7.1 | 98 |
| 118 | 8.78 | 101 | 6.02 | 92 | 7.13 | 99 |
| 119 | 8.76 | 100 | 7.21 | 101 | 7.11 | 99 |
| 120 | 8.76 | 100 | 6.15 | 95 | 6.7 | 96 |
| 121 | 8.75 | 99 | 6.29 | 95 | 7.23 | 96 |
| 122 | 8.73 | 100 | 5.74 | 86 | 7.13 | 100 |
| 123 | 8.71 | 99 | 5.79 | 87 | 7.04 | 99 |
| 124 | 8.69 | 99 | 6.38 | 98 | 7.44 | 98 |
| 125 | 8.68 | 100 | 6.23 | 96 | 6.23 | 96 |
| 126 | 8.68 | 101 | 5.22 | 65 | 6.95 | 97 |
| 127 | 8.65 | 99 | 6.36 | 94 | 7.59 | 99 |
| 128 | 8.64 | 100 | 6.03 | 90 | 6.81 | 98 |
| 129 | 8.64 | 101 | 5.71 | 85 | 7.04 | 99 |
| 130 | 8.61 | 100 | 5.76 | 87 | 6.87 | 96 |
| 131 | 8.61 | 98 | 5.11 | 61 | 7.39 | 99 |
| 132 | 8.6 | 101 | <5 | 46 | 6.7 | 98 |
| 133 | 8.53 | 100 | 5.79 | 85 | 6.87 | 98 |
| 134 | 8.5 | 101 | 5.65 | 84 | 6.8 | 100 |
| 135 | 8.48 | 100 | 5.58 | 81 | 6.72 | 97 |
| 136 | 8.47 | 101 | <5 | 51 | 6.54 | 97 |
| 137 | 7.55 | 100 | <5 | 35 | 5.87 | 84 |
| 138 | 7.55 | 93 | 5.31 | 64 | <5 | 24 |
| 139 | 7.54 | 99 | <5 | 20 | 5.62 | 78 |
| 140 | 7.53 | 100 | 5.05 | 57 | 5.51 | 83 |
| 141 | 7.53 | 100 | <5 | 12 | 5.69 | 81 |
| 142 | 7.52 | 100 | <5 | 35 | 5.59 | 79 |
| 143 | 7.48 | 97 | 5.1 | 58 | 5.62 | 77 |
| 144 | 7.46 | 100 | 5.62 | 86 | 6.33 | 96 |
| 145 | 7.44 | 99 | 5.69 | 86 | 6.19 | 93 |
| 146 | 7.43 | 100 | 5.78 | 85 | 5.48 | 76 |
| 147 | 7.42 | 100 | 5.65 | 81 | 6.06 | 90 |
| 148 | 7.41 | 101 | 5.25 | 67 | 6.04 | 89 |
| 149 | 7.41 | 99 | 5.29 | 66 | 5.74 | 83 |
| 150 | 7.4 | 100 | <5 | 22 | 5.45 | 72 |
| 151 | 7.39 | 100 | <5 | 11 | 5.86 | 85 |
| 152 | 7.38 | 99 | <5 | 30 | 5.69 | 80 |
| 153 | 7.36 | 100 | 5.51 | 76 | 5.97 | 89 |
| 154 | 7.29 | 100 | <5 | 50 | 5.56 | 79 |
| 155 | 7.27 | 98 | <5 | 37 | 5.53 | 72 |
| 156 | 7.23 | 100 | <5 | 19 | 5.7 | 81 |
| 157 | 7.22 | 99 | <5 | 33 | 5.59 | 75 |
| 158 | 7.19 | 100 | <5 | 19 | 5.67 | 78 |
| 159 | 7.14 | 100 | <5 | 42 | 5.49 | 75 |
| 160 | 7.14 | 99 | <5 | 34 | 5.99 | 88 |
| 161 | 7.13 | 98 | <5 | 11 | 5.51 | 74 |
| 162 | 7.13 | 97 | <5 | 26 | 5.37 | 72 |
| 163 | 7.05 | 98 | <5 | 12 | 5.49 | 74 |
| 164 | 7.05 | 100 | <5 | 22 | 5.71 | 82 |
| 165 | 7.03 | 100 | <5 | 36 | 5.46 | 78 |
| 166 | 7.03 | 98 | <5 | 32 | 5.65 | 81 |
| 167 | 7.01 | 97 | 5.27 | 64 | 5.7 | 81 |
| 168 | 8.4 | 98 | 5.29 | 66 | 7.27 | 99 |
| 169 | 8.43 | 98 | 5.75 | 82 | 6.75 | 98 |
| 170 | 8.34 | 100 | 5.67 | 84 | 5.89 | 84 |
| 171 | 7.93 | 99 | 5.94 | 93 | 6.45 | 97 |
| 172 | 8.34 | 101 | 6.57 | 98 | 7.49 | 100 |
| 173 | 7.86 | 101 | <5 | 31 | 6.52 | 96 |
| 174 | 8.28 | 99 | 5.88 | 85 | 6.24 | 88 |
| 175 | 8.31 | 100 | 5.22 | 71 | 6.85 | 98 |
| 176 | 8.04 | 100 | 5.06 | 56 | 6.05 | 88 |
| 177 | 8.02 | 99 | <5 | 21 | 6.34 | 93 |
| 178 | 7.87 | 101 | 5.13 | 62 | 6.1 | 92 |
| 179 | 7.67 | 100 | <5 | 32 | 6.27 | 90 |
| 180 | 8.3 | 100 | 5.29 | 66 | 6.76 | 97 |
| 181 | 8.34 | 98 | <5 | 52 | 6.51 | 94 |
| 182 | 7.6 | 100 | <5 | 44 | 6.38 | 95 |
| 183 | 7.58 | 99 | <5 | 36 | 5.64 | 79 |
| 184 | 7.55 | 100 | <5 | 17 | 5.56 | 75 |
| 185 | 7.66 | 98 | 5.48 | 79 | 5.92 | 87 |
| 186 | 7.97 | 100 | 5.22 | 69 | 6.47 | 95 |
| 187 | 7.75 | 99 | 5.46 | 74 | 6.37 | 95 |
| 188 | 8.07 | 99 | 5.81 | 87 | 6.68 | 98 |
| 189 | 8.29 | 100 | 5.56 | 80 | 6.73 | 96 |
| 190 | 7.99 | 100 | <5 | 39 | 6.8 | 96 |
| 191 | 7.89 | 100 | 5.41 | 74 | 6.65 | 97 |
| 192 | 8.03 | 99 | 6.14 | 95 | 7.14 | 99 |
| 193 | 8.23 | 100 | 5.46 | 76 | 6.86 | 98 |
| 194 | 7.73 | 99 | 5.34 | 57 | 6.58 | 97 |
| 195 | 8.35 | 98 | 5.25 | 70 | 6.43 | 93 |
| 196 | 7.63 | 99 | 5.31 | 68 | 6.25 | 92 |
| 197 | 7.99 | 98 | <5 | 42 | 6.68 | 96 |
| 198 | 8.25 | 98 | <5 | 35 | 6.38 | 91 |
| 199 | 7.27 | 92 | 5.08 | 62 | 5.23 (h) | 62 (h) |
| | | | | | 5.32 (r) | 21 (r) |
| 200 | 7.01 | 91 | <5 | 35 | <5 | 13 |
| 201 | 6.19 | 61 | <5 | 24 | 5.51 (r) | -1 (r) |
| 202 | 5.57 | 30 | <5 | 18 | <5 (h) | 38 (h) |
| | | | | | <5 (r) | 4 (r) |
| 203 | 6.02 | 48 | <5 | 41 | <5 (r) | 14 (r) |
| 204 | 6.79 | 88 | 5.13 | 48 | <5 (r) | 13 (r) |
| 205 | 8.94 | 99 | 5.65 | 83 | 7.01 | 98 |
| 206 | 5.78 | 39 | <5 | -1 | <5 | 16 |
| 207 | 5.1 | 21 | <5 | 2 | <5 | -5 |
| 208 | 6.23 | 62 | <5 | 30 | <5 | 4 |
| 209 | 6.14 | 57 | <5 | 25 | <5 | 7 |
| 210 | 6.21 | 56 | <5 | 5 | <5 | 16 |
| 211 | <5 | 3 | <5 | 13 | <5 | 10 |
| 212 | 6.24 | 61 | <5 | 7 | <5 | 8 |
| 213 | 5.63 | 28 | <5 | 11 | <5 | 23 |
| 214 | 5.2 | 9 | <5 | 9 | <5 | 30 |
| 215 | 6.58 | 82 | 5 | 45 | 5.13 | 63 |
| 216 | 6.36 | 98 | <5 | 26 | 5.35 | 67 |
| 217 | 6.25 | 99 | <5 | 37 | 5.32 | 72 |
| 218 | 6.69 | 100 | <5 | 51 | 5.65 | 85 |
| 219 | 6.86 | 100 | <5 | 18 | 5.04 | 45 |
| 220 | 6.52 | 54 | <5 | 8 | <5 | 41 |
| 220 (.HCl) | 6.52 | 54 | <5 | 8 | <5 | 41 |
| 221 | 5.92 | 45 | <5 | 4 | <5 | 26 |
| 222 | 6.55 | 98 | <5 | 13 | 5.17 | 59 |
| 223 | 6.71 | 98 | <5 | 19 | 5.46 | 71 |
| 224 | 8.52 | 100 | 5.98 | 87 | 7.15 | 99 |
| 225 | 7.94 | 99 | 5.75 | 83 | 7.03 | 98 |
| 226 | 9.06 | 100 | 6.89 | 100 | 7.09 | 100 |
| 227 | 8.66 | 101 | 6.42 | 95 | 7.34 | 98 |
| 228 | 9.65 | 100 | 5.92 | 91 | 7.77 | 100 |
| 229 | 6.25 | 95 | <5 | 18 | <5 | 33 |
| 230 | 5.99 | 93 | <5 | 40 | 5.27 | 65 |
| 231 | 8.87 | 97 | 5.04 | 57 | 7.71 | 100 |
| 232 | 6.67 | 99 | <5 | 31 | <5 | 48 |
| 233 | 6.24 | 94 | <5 | 10 | <5 | 26 |
| 234 | 6.29 | 96 | <5 | 22 | 5.06 | 59 |
| 235 | 6.91 | 98 | <5 | 32 | 5.26 | 67 |
| 236 | 6.68 | 100 | <5 | 18 | 5.11 | 58 |
| 237 | 6.73 | 97 | <5 | 20 | 5.09 | 51 |
| 238 | 6.42 | 94 | <5 | 35 | 5 | 48 |
| 239 | 6.72 | 96 | <5 | 16 | <5 | 43 |
| 240 | 5.81 | 90 | <5 | 9 | 5.04 | 51 |
| 241 | 7.68 | 97 | 5.76 | 88 | 6.57 | 94 |
| 242 | 6.87 | 97 | 5.21 | 67 | 5.09 | 56 |
| 243 | 6.88 | 99 | <5 | 13 | 5.44 | 70 |
| 244 | 6.17 | 92 | <5 | 12 | 5.03 | 54 |
| 245 | 6.91 | 99 | 5.23 | 62 | 5.66 | 79 |
| 246 | 6.9 | 98 | <5 | 25 | <5 | 44 |
| 247 | 6.81 | 98 | 5.69 | 85 | 5.54 | 77 |
| 248 | 6.46 | 95 | <5 | 2 | 4.98 | 49 |
| 249 | 6.43 | 95 | <5 | 10 | <5 | 45 |
| 250 | 6.88 | 99 | <5 | 28 | 5.31 | 64 |
| 251 | 6.55 | 98 | <5 | 17 | 5.06 | 53 |
| 252 | 6.15 | 96 | <5 | 17 | 5.17 | 66 |
| 253 | 6.34 | 95 | <5 | 17 | <5 | 39 |
| 254 | 6.97 | 99 | <5 | 25 | 5.42 | 70 |
| 255 | 6.57 | 95 | <5 | 28 | 5.11 | 58 |
| 256 | 6.49 | 97 | <5 | 12 | 5 | 48 |
| 257 | 6.58 | 95 | <5 | 15 | <5 | 45 |
| 258 | 6.82 | 99 | 5.81 | 89 | 5.9 | 90 |
| 259 | 6.37 | 99 | 5.45 | 82 | 5.77 | 92 |
| 260 | 6.86 | 97 | <5 | 23 | 5.53 | 74 |
| 261 | 6.44 | 95 | <5 | 19 | <5 | 45 |
| 262 | 6.81 | 101 | <5 | 37 | 5.49 | 74 |
| 263 | 6.89 | 99 | 5.31 | 65 | 5.29 | 64 |
| 264 | 6.98 | 100 | 4.98 | 47 | 5.48 | 74 |
| 265 | 8.62 | 102 | 5.2 | 55 | 7.43 | 98 |
| 266 | 6.38 | 100 | 5.27 | 74 | 5.58 | 89 |
| 267 | 9.4 | 102 | 7.21 | 100 | 7.95 | 99 |
| 268 | 5.72 | 82 | <5 | 13 | <5 | 39 |
| 269 | 8.93 | 99 | 5.43 | 78 | 6.25 | 93 |
| 270 | 8.34 | 99 | <5 | 29 | 6.48 | 96 |
| 271 | 8.66 | 99 | 5.13 | 61 | 6.78 | 95 |
| 272 | 8.81 | 97 | 5.05 | 54 | 6.28 | 93 |
| 273 | 8.63 | 100 | 6.09 | 96 | 6.01 | 96 |
| 274 | 6.54 | 97 | <5 | 7 | 5.22 | 63 |
| 275 | 8.55 | 100 | 6.01 | 92 | 7.54 | 100 |
| 276 | 8.65 | 100 | 5.89 | 93 | 6.78 | 98 |
| 277 | 7.12 | 99 | <5 | 14 | <5 | 27 |
| 278 | 9.03 | 99 | 6.15 | 97 | 6.84 | 99 |
| 279 | 8.65 | 99 | 5.55 | 86 | 6.5 | 96 |
| 280 | 8.6 | 100 | 6.03 | 96 | 6.61 | 99 |
| 281 | 8.78 | 100 | 5.65 | 87 | 6.96 | 99 |
| 282 | 8.54 | 100 | 5.77 | 88 | 6.56 | 98 |
| 283 | 8.07 | 100 | <5 | 38 | 6.75 | 98 |
| 284 | 8.77 | 101 | 5.9 | 89 | 7.26 | 98 |
| 285 | 5.16 | 65 | <5 | 21 | <5 | 37 |
| 286 | 8.16 | 101 | <5 | 14 | 5.65 | 79 |
| 287 | 8.42 | 100 | 5.2 | 61 | 6.76 | 98 |
| 288 | 8.57 | 101 | <5 | 44 | 6.38 | 93 |
| 289 | 8.62 | 100 | 6.75 | 98 | 7.2 | 99 |
| 290 | 8.23 | 98 | 5.16 | 53 | 5.85 | 89 |
| 291 | 8.57 | 101 | 5.58 | 86 | 6.29 | 95 |
| 292 | 8.6 | 100 | 5.33 | 73 | 6.14 | 94 |
| 293 | 7.95 | 100 | 5.46 | 80 | 5.99 | 90 |
| 294 | 7.97 | 99 | <5 | 14 | 5.68 | 81 |
| 295 | 9.31 | 101 | 6.01 | 94 | 6.61 | 98 |
| 296 | 9.34 | 99 | 6.1 | 93 | 6.92 | 100 |
| 297 | 8.63 | 97 | 5.23 | 62 | 6.23 | 96 |
| 298 | 7.73 | 99 | <5 | 25 | <5 | 47 |
| 299 | 8.11 | 99 | <5 | 40 | 5.67 | 84 |
| 300 | 8.19 | 100 | 5.7 | 88 | 5.54 | 80 |
| 301 | 8.09 | 100 | 5.2 | 62 | 5.9 | 89 |
| 302 | 8.19 | 101 | 5.1 | 59 | 6.05 | 93 |
| 303 | 8.15 | 99 | 5.18 | 57 | 6.27 | 95 |
| 304 | 7.4 | 100 | 5.04 | 48 | <5 | 45 |
| 305 | 7.06 | 100 | <5 | 7 | 5.15 | 57 |
| 306 | 7.35 | 100 | 5.35 | 71 | 5.44 | 74 |
| 307 | 8.04 | 99 | 5.83 | 88 | 6.22 | 97 |
| 308 | 7.76 | 98 | 5.34 | 72 | 5.17 | 66 |
| 309 | 8.53 | 100 | 5.12 | 53 | 5.82 | 86 |
| 310 | 8.85 | 100 | 5.41 | 78 | 6.4 | 95 |
| 311 | 8.96 | 96 | 5.13 | 62 | 6.37 | 96 |
| 312 | 7.66 | 99 | <5 | 37 | 6.21 | 93 |
| 313 | 8.87 | 97 | 5.22 | 61 | 5.91 | 86 |
| 314 | 8.24 | 100 | 5.45 | 73 | 6.03 | 91 |
| 315 | 6.95 | 98 | <5 | 20 | 5.01 | 47 |
| 316 | 6.59 | 94 | <5 | 5 | <5 | 14 |
| 317 | 7.07 | 99 | <5 | 15 | <5 | 34 |
| 318 | 5.82 | 89 | <5 | 11 | <5 | 13 |
| 319 | 8.95 | 100 | 5.99 | 95 | 6.58 | 99 |
| 320 | 7.27 | 100 | <5 | 47 | 5.15 | 62 |
| 321 | 8.66 | 101 | 5.09 | 45 | 6.51 | 97 |
| 322 | 9.3 | 100 | 6.32 | 95 | 7.73 | 99 |
| 323 | 8.41 | 100 | 6.14 | 95 | 6.65 | 97 |
| 324 | 9.07 | 100 | 6.58 | 97 | 7.18 | 100 |
| 325 | 8.94 | 100 | 6.51 | 98 | 7.19 | 98 |
| 326 | 8.67 | 100 | 6.59 | 97 | 7.22 | 99 |
| 327 | 9.38 | 101 | 7.11 | 99 | 7.69 | 100 |
| 328 | 9.2 | 100 | 5.83 | 90 | 7.21 | 100 |
| 329 | 9.26 | 100 | 6.69 | 95 | 7.67 | 100 |
| 331 | 9.32 | 98 | 7.3 | 99 | 7.84 | 100 |
| 332 | 8.32 | 99 | 5.92 | 90 | 7.51 | 98 |
| 333 | 8.09 | 99 | 5.16 | 63 | 6.15 | 93 |
| 334 | 8.61 | 100 | 5.63 | 84 | 7 | 98 |
| 335 | 9.02 | 102 | 5.14 | 52 | 7.06 | 98 |
| 336 | 7.73 | 101 | 5.49 | 78 | 5.72 | 87 |
| 337 | 6.78 | 99 | <5 | 43 | 5.34 | 71 |
| 338 | 7.04 | 99 | 5.26 | 62 | 5.51 | 70 |
| 339 | 8.18 | 101 | 5.32 | 69 | 6.21 | 95 |
| 340 | 7.15 | 100 | 5.37 | 69 | 5.48 | 72 |
| 341 | 6.85 | 97 | <5 | 33 | 5.3 | 63 |
| 342 | 7.49 | 100 | 5.35 | 69 | 5.57 | 77 |
| 343 | 7.17 | 102 | <5 | 44 | 5.34 | 68 |
| 344 | 7.53 | 99 | 5.14 | 61 | 5.59 | 80 |
| 345 | 7.12 | 99 | 5.08 | 48 | 5.43 | 72 |
| 346 | 8.28 | 100 | 5.22 | 68 | 6.14 | 92 |
| 347 | 8.27 | 101 | 5.14 | 63 | 6 | 91 |
| 348 | 7.47 | 99 | 5.35 | 70 | 5.89 | 92 |
| 349 | 7.65 | 102 | 5.32 | 64 | 5.75 | 86 |
| 350 | 7.6 | 100 | 5.31 | 74 | 5.68 | 85 |
| 351 | 7.29 | 99 | 5.2 | 69 | 5.67 | 83 |
| 352 | 8.12 | 100 | 5.74 | 89 | 6.32 | 99 |
| 353 | 8.47 | 99 | 5.32 | 69 | 6.01 | 94 |
| 354 | 7.05 | 100 | 5.01 | 45 | 5.35 | 70 |
| 355 | 9.03 | 99 | 6.14 | 98 | 6.63 | 98 |
| 356 | 8.27 | 99 | 5.23 | 65 | 6.03 | 92 |
| 357 | 7.11 | 98 | <5 | 34 | 5.47 | 73 |
| 358 | 7.57 | 102 | <5 | 40 | 5.1 | 58 |
| 359 | 7.92 | 99 | 5.4 | 71 | 5.67 | 86 |
| 360 | 7.39 | 97 | 5.07 | 50 | 5.16 | 55 |
| 361 | 8.56 | 100 | <5 | 37 | 6.45 | 92 |
| 362 | 8.77 | 99 | 6.06 | 88 | 6.1 | 93 |
| 363 | 9.56 | 99 | 6.21 | 97 | 6.75 | 98 |
| 364 | 8.41 | 101 | 5.5 | 75 | 5.74 | 85 |
| 365 | 7.2 | 99 | <5 | 24 | <5 | 33 |
| 366 | 7.99 | 99 | <5 | 22 | 5.24 | 64 |
| 367 | 6.36 | 97 | <5 | 21 | <5 | 44 |
| 368 | 8.21 | 101 | 5.19 | 61 | 6.6 | 99 |
| 369 | 8.31 | 100 | 5.43 | 72 | 6.14 | 93 |
| 370 | 7.47 | 100 | 5.84 | 87 | 6.18 | 94 |
| 371 | 7.86 | 101 | 6.41 | 96 | 6.41 | 98 |
| 372 | 8.99 | 100 | 5.87 | 90 | 6.6 | 96 |
| 373 | 8.57 | 100 | 6.27 | 97 | 6.5 | 99 |
| 374 | 9.13 | 100 | 6.13 | 97 | 6.74 | 98 |
| 375 | 8.45 | 101 | 5.35 | 73 | 6.09 | 94 |
| 376 | 8.37 | 101 | 5.47 | 79 | 6.51 | 97 |
| 377 | 7.75 | 99 | 5.01 | 47 | 5.89 | 89 |
| 378 | 8.77 | 99 | 6.12 | 96 | 6.39 | 97 |
| 379 | 7.95 | 102 | 5.28 | 67 | 5.79 | 87 |
| 380 | 8.26 | 101 | 5.4 | 72 | 6.44 | 96 |
| 381 | 8.12 | 99 | 5 | 48 | 5.88 | 88 |
| 382 | 7.99 | 99 | <5 | 37 | 5.4 | 75 |
| 386 | 8.62 | 98 | 5.6 | 79 | 6.26 | 93 |
| 387 | 8.54 | 100 | 5.77 | 88 | 6.56 | 98 |
| 390 | 8.68 | 98 | 5.59 | 84 | 7.21 | 99 |
| 391 | 8.62 | 99 | 5.65 | 81 | 7.2 | 97 |
| 392 | 8.95 | 99 | 6.35 | 97 | 6.58 | 93 |
| 394 | 8.03 | 99 | <5 | 27 | 5.65 | 76 |
| 396 | 7.82 | 98 | <5 | 56 | 5.83 | 84 |
| 398 | 8.31 | 99 | 5.28 | 67 | 6.23 | 95 |
| 400 | 7.65 | 99 | <5 | 16 | <5 | 41 |
| 402 | 7.58 | 100 | <5 | 28 | 5.83 | 87 |

### PDE2 OCCUPANCY BY TEST COMPOUNDS

### METHODS

Occupancy of PDE2A was evaluated by ex-vivo autoradiography using [³H]B-17a (described in WO2013/000924) as radioligand (compound 12 in Buijnsters et al., (2014). Structure-Based Design of a Potent, Selective, and Brain Penetrating PDE2 Inhibitor with Demonstrated Target Engagement. ACS Med Chem Lett. 5(9): 1049-53.) Male Wistar rats (200-250 g) were treated by oral administration of vehicle or increasing doses of [³H]B-17a and killed one h after. Brains were immediately removed from the skull and rapidly frozen in dry-ice cooled 2-methylbutane (-40°C). Twenty µm-thick striatal sections were cut using a Leica CM 3050 cryostat-microtome (van Hopplynus, Belgium), thaw-mounted on microscope slides (SuperFrost Plus Slides, LaboNord, France) and stored at -20°C until use.
After thawing, sections were dried under a cold stream of air and incubated for one minute with 30 nM [³H]B-17a in Tris-HCl (50 mM, pH7.4) containing 0.3% BSA. Brain sections from drug-treated and vehicle-treated animals were incubated in parallel. Non-specific binding was measured on cerebellar sections, a brain area which does not contain the PDE2A enzyme. After incubation, the excess of [³H]B-17a was washed off in ice-cold buffer 2 times 10 minutes, followed by a quick dip in distilled water. The sections were then dried under a stream of cold air.
Brain sections were loaded in a β-imager (Biospace, Paris) for 4 h and radioactivity emerging from delineated brain area was quantified using the Beta vision program (Biospace, Paris). Specific binding was determined as the difference between total binding in the striatum and non-specific binding in the cerebellum. Percentage receptor occupancy of the drug administered to the animal corresponded to 100% minus the percentage receptor labeled in the treated animal. For the determination of ED₅₀-values, the percentage of receptor occupancy was plotted against dose and the sigmoidal log dose-effect curve of best fit was calculated by non-linear regression analysis, using the GraphPad Prism program. ED₅₀s (the drug dose producing 50% receptor occupancy) with 95% confidence limits were calculated from the dose-response curves.

**TABLE 6. PO = oral; SC = subcutaneous**

| Co. No. | PDE2 Occupancy at 10 mg/kg | Route PDE2 Occupancy at 10 mg/kg | PDE Occupancy ED₅₀ | Route Occupancy ED₅₀ |
|---|---|---|---|---|
| 1a | 37 | PO | | |
| 2b | 3 | SC | | |
| 2a | 70 | SC | | |
| 3a | 0 | PO | | |
| 4 | 9 | PO | | |
| 6 | 67 | PO | | |
| 13 | 2 | PO | | |
| 18 | 4 | PO | | |
| 22 | 18 | PO | | |
| | 30 | PO | | |
| 24b | 0 | SC | | |
| 25a | 66 | SC | 4 | PO |
| 25b | -9 | PO | | |
| 27 | 34 | PO | | |
| 28 | 49 | SC | 25 | PO |
| 29 | 31 | SC | | |
| 30 | 0 | SC | | |
| 32 | -6 | PO | | |
| 33 | -7 | PO | | |
| 35 | -3 | PO | | |
| 36 | 32 | PO | 45.2 | PO |
| 37 | -13 | PO | | |
| 38 | 1 | PO | | |
| 39 | -8 | PO | | |
| 41 | 8 | PO | | |
| 42 | 54 | PO | | |
| 43 | 2 | PO | | |
| 46 | -9 | PO | | |
| 47 | 5 | PO | | |
| 48 | 96 | SC | | |
| 49 | 12 | SC | | |
| 50 | -30 | PO | | |
| 51 | 20 | PO | | |
| 52 | 2 | PO | | |
| 53 | 22 | PO | | |
| 54 | 39 | PO | | |
| 59 | 7 | PO | | |
| 60 | 32 | PO | | |
| 64 | 7 | PO | | |
| 65 | -23 | PO | | |
| | -9 | PO | | |
| 66 | -14 | PO | | |
| 68 | 31 | SC | | |
| 69 | 0 | PO | | |
| 70 | 65 | SC | 6.4 | PO |
| 71 | 49 | SC | | |
| 72 | 0 | PO | | |
| 73 | 0 | PO | | |
| 74 | 14 | SC | | |
| 76 | 17 | PO | | |
| 77 | 54 | PO | 5.67 | PO |
| | 54 | PO | | |
| 78 | 77 | SC | | |
| 79 | 15 | PO | | |
| | 0 | PO | | |
| 80 | 52 | SC | 8.63 | PO |
| 82 | 28 | PO | | |
| 83 | 82 | PO | | |
| 84 | 45 | PO | 14.13 | PO |
| 85 | 1 | SC | | |
| 89 | 88 | SC | | |
| 90 | 73 | SC | | |
| 91 | -7 | PO | | |
| 93 | 85 | PO | 7.8 | PO |
| 95 | 27 | SC | | |
| 96 | 1 | PO | | |
| 97 | 92 | PO | | |
| 99 | 1 | PO | | |
| 101 | 43 | SC | | |
| 102 | -4 | PO | | |
| 103 | 42 | SC | | |
| 106 | -0 | PO | | |
| 107 | 29 | PO | | |
| 108 | 65 | PO | 4.8 | PO |
| 109 | 43 | PO | | |
| 110 | 75 | PO | 8.3 | PO |
| 115 | 23 | PO | | |
| 117 | 8 | PO | | |
| 116 | 3 | SC | | |
| 120 | 12 | PO | | |
| 121 | -15 | PO | | |
| 122 | 0 | PO | | |
| 123 | 4 | PO | | |
| 125 | -16 | PO | | |
| 126 | 31 | PO | | |
| 127 | 34 | PO | | |
| | 53 | SC | | |
| 129 | 21 | PO | | |
| 130 | 0 | PO | | |
| 131 | -0 | PO | | |
| 132 | 76 | SC | 5 | PO |
| 133 | -19 | PO | | |
| 134 | 21 | PO | | |
| 139 | -11 | PO | | |
| 169 | 3 | PO | | |
| 170 | 1 | PO | | |
| 184 | -2 | PO | | |
| 186 | 8 | SC | | |
| 187 | 0 | SC | | |
| 188 | 2 | SC | | |
| 189 | 9 | PO | | |
| 190 | 1 | PO | | |
| 191 | -18 | PO | | |
| 192 | -13 | PO | | |
| 193 | -11 | PO | | |
| | -3 | SC | | |
| 195 | -7 | PO | | |
| | -13 | SC | | |
| 196 | 0 | PO | | |
| 197 | 8 | PO | | |
| 220 | -8 | PO | >80 | PO |
| 223 | 19 | PO | | |
| 224 | -6 | PO | | |
| 225 | -6 | PO | | |
| 227 | 18 | SC | | |
| 228 | 92 | SC | 1.5 | PO |
| 231 | -13 | SC | | |
| 267 | 59 | PO | 11 | PO |
| 269 | 22 | PO | 24.2 | PO |
| 270 | 6 | PO | | |
| 272 | 3 | PO | | |
| 273 | 65 | SC | | |
| 275 | 23 | PO | | |
| 277 | -4 | PO | | |
| 278 | 30 | PO | | |
| 281 | 57 | PO | 4.2 | PO |
| 282 | 47 | PO | 11.8 | PO |
| | 47 | PO | | |
| 283 | -12 | PO | | |
| 284 | 16 | PO | | |
| 286 | 5 | PO | | |
| 287 | 43 | PO | | |
| 290 | -3 | PO | | |
| 291 | 38 | PO | | |
| 292 | 23 | PO | | |
| 293 | 6 | PO | | |
| 294 | 17 | PO | | |
| 295 | 71 | PO | 5.63 | PO |
| 296 | 88 | PO | | |
| 297 | -2 | PO | | |
| 299 | 31 | PO | | |
| 300 | -11 | PO | | |
| 301 | -7 | PO | | |
| 302 | 1 | PO | | |
| 303 | 17 | PO | | |
| 307 | 8 | PO | | |
| 308 | 18 | PO | | |
| 309 | 12 | PO | | |
| 310 | 52 | PO | 9.6 | PO |
| 311 | -1 | PO | | |
| 312 | -16 | PO | | |
| 313 | 28 | PO | | |
| 314 | 5 | PO | | |
| 319 | 37 | PO | | |
| 321 | 73 | PO | 3.37 | PO |
| 328 | -18 | PO | | |
| 332 | 8 | PO | | |
| 334 | 17 | PO | | |
| 335 | 88 | PO | 0.985 | PO |
| 339 | -1 | PO | | |
| 346 | 19 | PO | | |
| 347 | 11 | PO | | |
| 352 | 16 | PO | | |
| 353 | 15 | PO | | |
| 356 | 51 | PO | 5.55 | PO |
| 362 | 3 | PO | | |
| 363 | -11 | PO | | |
| 364 | -5 | PO | | |
| 366 | 18 | PO | | |
| 368 | 11 | PO | | |
| 369 | 9 | PO | | |
| 386 | 23 | PO | | |
| 387 | 47 | PO | 11.8 | PO |
| | 47 | PO | | |
| 391 | 3 | PO | | |
| 392 | 46 | PO | | |
| 398 | 29 | PO | | |

### EFFECT OF COMPOUND 110 ON SYNAPTIC TRANSMISSION CRITICAL REAGENTS

Sucrose dissection buffer contained (in mM) sucrose (150), NaCl (40), KCl (4), NaH₂PO₄.H₂O (0.3), MgCl.6H₂O (7), NaHCO₃ (26), CaCl₂.2H₂O (0.5), D-glucose (10), equilibrated with 95% O₂ and 5% CO₂ gas mixture. Artificial cerebrospinal fluid (ACSF) used during equilibration and recording contained (in mM): NaCl (124), KCl (2.7), NaH₂PO₄.H₂O (1.25), MgSO₄.7H₂O (1.3), NaHCO₃ (26), CaCl₂.2H₂O (2), D-glucose (10), Ascorbic acid (2), equilibrated with 95% O₂ and 5% CO₂ gas mixture. CNQX and Kynurenic acid were prepared in ACSF at a 50 µM and 1mM concentration respectively. Test compounds were prepared fresh from stock solution (with DMSO) in ACSF and with a final DMSO concentration that did not exceed 0.1%. All reagents were from Sigma-Aldrich, unless otherwise indicated.

### ANIMALS (SPECIES, WEIGHT, AND GENDER)

Animals used were male Sprague-Dawley rats with a weight range between 145 and 200g provided by Charles River Germany.

### PREPARATION OF HIPPOCAMPAL SLICES

Horizontal brain slices (300 µm) were obtained from the mid- to ventral hippocampus of male Sprague-Dawley rats anesthetized with isofluorane according to standard protocol. Slices were cut using a vibrating tissue slicer (Leica VT1200S) in cold (4°C) sucrose dissection buffer at a speed of 0.1 mm/s. After cut, slices were placed for equilibration at 35°C for 20 min and then allowed to recover at RT for at least one hour in artificial cerebrospinal fluid (ACSF). Three to four slices were prepared from one brain.

### TEST SYSTEM

All data were recorded with a MEA set-up commercially available from MultiChannel Systems MCS GmbH (Reutlingen, Germany) composed of a 4-channel stimulus generator and a 60-channels amplifier head-stage connected to a 60-channels A/D card. Software for stimulation, recordings and analysis are the ones commercially available from Multi Channel Systems: MC Stim (II 2.0.0 release) and MC Rack (3.8.1.0 release), respectively. All of the experiments were carried out with 3-dimensional MEA (Ayanda Biosystems, S.A., CH-1015 Lausanne, Switzerland) that consist of 60 tip-shaped and 60-µm-high electrodes spaced by 100 µm. The MEA electrodes are made of platinum with 600 kΩ < impedance < 900 kΩ.

### EXPERIMENTAL DESIGN

The effect of test compounds on synaptic transmission was investigated by recording the extracellular field potentials in hippocampal slices. It is well established that synaptic transmission a can generate a deflection of the extracellular field potential that reflects the synchronized synaptic activity in the population of neurons surrounding the recording electrode.

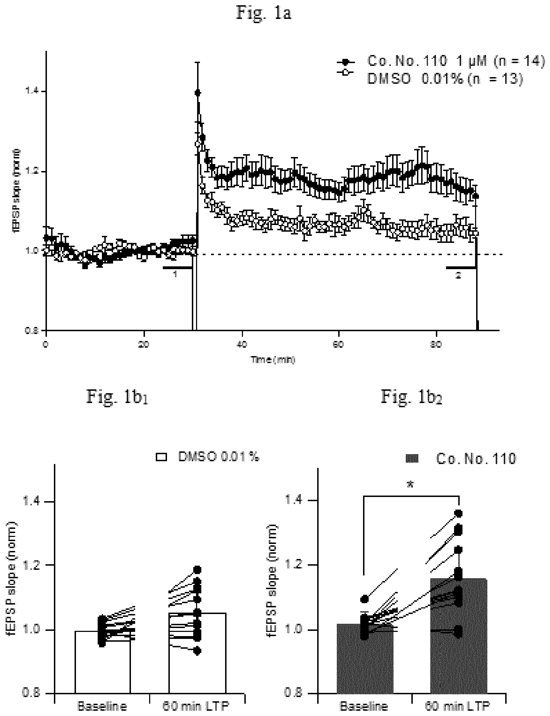

Extracellular field potential recordings. After recovery, brain slices were mounted on MEA chip under microscope and locating the 60 recording electrodes on the mossy fiber synapse (Dentate Gyrus - CA3) region of the hippocampus. ACSF solutions were continuously perfused at a rate of 2 mL/min. The temperature of the MEA chamber was maintained at 32 ± 0.1 °C with a Peltier element located in the MEA amplifier headstage. All data were recorded with a MEA set-up commercially available from MultiChannel Systems MCS GmbH (Reutlingen, Germany). Two adjacent electrodes of the chip were selected to stimulate the mossy fibres in the hilar region of the dentate gyrus and the fEPSP was recorded the terminal zone area of the CA3 region of the hippocampus. Field extracellular post-synaptic potentials (fEPSPs) were evoked by stimulation of the mossy fibre input with two consecutive electrical pulses separated by 30 ms and repeated every 60 s (pulse width 100 µs, and current stimulation strength (µA) 40% relative maximum amplitude). Control experiments were performed simultaneously from slices that were randomly assigned to be treated with vehicle (DMSO). N represents the number of slices and usually 3-4 slices were used per animal. Evoked-responses at post-synaptic neurons level (fEPSP) are recorded if they satisfy certain quality criteria including: correct location, stable baseline (fluctuation within +/- 10 % during ten consecutive minutes, amplitude >100 µV. The fEPSP from selected electrodes were sampled at 5 kHz and recorded on the hard disk of a PC for offline analysis. In parallel, fEPSP amplitudes of selected electrodes were compiled online (with MC Rack program) to monitor and to follow the quality of the experiment. Data are plotted in a spreadsheet file for off-line analysis.
Weak Long Term Potentiation (LTP) was evoked by a single high frequency stimulus (HFS) to produce a less than maximal potentiation of the fEPSP.
The results of this test are shown in Figure 1 for the effect of compound 110 a PDE2 inhibitor on the facilitation on induction of LTP with a weak Long Term Potentiation protocol.

### EFFECT OF COMPOUNDS 70, 25a AND 220 (FREE BASE) ON SYNAPTIC TRANSMISSION

### CRITICAL REAGENTS

Sucrose dissection buffer contained (in mM), NaCl (124), KCl (4.4), NaH₂PO₄.H₂O (1.2), MgCl.6H₂O (2), NaHCO₃ (26), CaCl₂.2H₂O (2), D-glucose (10), equilibrated with 95% O₂ and 5% CO₂ gas mixture. Artificial cerebrospinal fluid (ACSF) used during equilibration and recording contained (in mM): NaCl (124), KCl (4.4), NaH₂PO₄.H₂O (1.2), MgSO₄.7H₂O (2), NaHCO₃ (26), CaCl₂.2H₂O (2), D-glucose (10), Ascorbic acid (2), equilibrated with 95% O₂ and 5% CO₂ gas mixture. Compound 1, 2, and 3 were prepared fresh from stock solution (with DMSO) in ACSF and with a final DMSO concentration that did not exceed 0.1%. All reagents were from Sigma-Aldrich, unless otherwise indicated.

### ANIMALS (SPECIES, WEIGHT, AND GENDER)

Animals used were male Sprague-Dawley rats with a weight range between 145 and 200g provided by Charles River Germany.

### Preparation of hippocampal slices

Rats were anesthetized with isoflurane and decapitated acutely. The brain was placed next to an agarose block and was cut horizontally, with the blade advancing from anterior to posterior. Slices were cut at a thickness of 350 µm using a vibrating tissue slicer (Leica VT1200S) in cold (4°C) carbogenated artificial cerebrospinal fluid (ACSF) at a speed of 0.08 mm/s and 0.75 mm vibration amplitude. After cutting, slices were equilibrated at 35°C for 20 minutes and then allowed to recover at room temperature for at least one hour in ACSF. Normally, six to eight slices were prepared from each brain and three to four were used per experiment.

### TEST SYSTEM

All data were recorded with a Slicemaster set-up commercially available from Scientifica (UK) composed of a 4 recording stations. Plamtinum stimulation electrode coated with isonel was placed in the rat hippocampal CA3 area. Recording microelectrodes (resistance around 5 MΩ) were filled with ACSF and placed within rat hippocampal CA1. Placement of stimulating and recording electrodes was confirmed by applying a current stimulation every 20 s. A pre-recording of 20 min was applied to see if the responses from the slices were stabilized. The current stimulation was applied by a current isolator A365 (World Precision Instruments). Data were acquired using pClamp 10 interfaced to a Digidata 1440A data acquisition board (Molecular Devices, Sunnyvale, California, USA) at a sampling rate of 10 kHz, low-pass filtered at 1 kHz, and high-pass filtered at 3 Hz. One hundred µs stimuli ranging from 0-100 µA were used to evoke fEPSPs, and the magnitude of the fEPSP was determined by measuring the peak negative amplitude or the 20 - 80% slope of the rising phase. Data was analyzed offline using custom made algorithms in IGORpro (Wavemetrics).

### EXPERIMENTAL DESIGN

The effect of compounds 70, 25a and 220 (free base) on synaptic transmission was investigated by recording the extracellular field potentials in hippocampal slices. It is well established that synaptic transmission a can generate a deflection of the extracellular field potential that reflects the synchronized synaptic activity in the population of neurons surrounding the recording electrode.

*Extracellular field potential recordings.* After recovery, slices were continuously perfused with oxygenated ACSF (2.5 mL/min). Solution was preheated in the water bath before being pumped into the recording chamber. Recordings were performed at 32 °C. fEPSPs from four independent brain slices were recorded simultaneously. N represents the number of slices and usually 3-4 slices were used per animal. Evoked-responses at post-synaptic neurons level (fEPSP) are recorded if they satisfy certain quality criteria including: correct location, stable baseline (fluctuation within +/- 10 % during ten consecutive minutes, amplitude >100 µV. The fEPSP from selected electrodes were sampled at 5 kHz and recorded on the hard disk of a PC for offline analysis. In parallel, fEPSP amplitudes were compiled online (with pclamp) to monitor and to follow the quality of the experiment. Data are plotted in a spreadsheet file for off-line analysis.

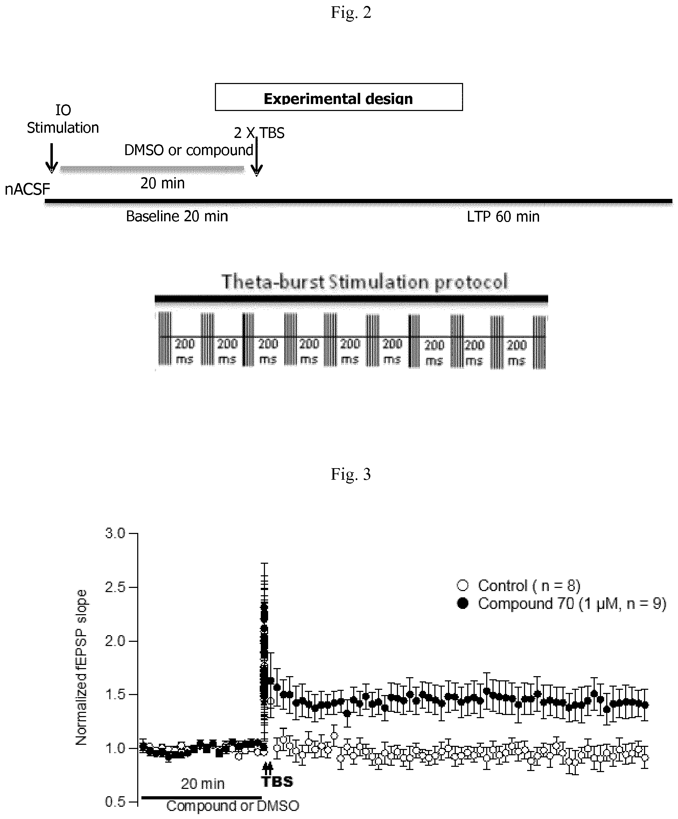

Input-output curves were generated and the stimulation strength was set to 50% of the range between the minimum and maximum fEPSP (as defined by either the stimulation strength sufficient to produce a population spike or a plateau in the amplitude of the fEPSP). For LTP experiments, slices were then stimulated every 60 s for a 20 min baseline period (vehicle with or without compound) and immediately followed by the theta-burst stimulation (shown as in Figure 2). After the theta-burst stimulation, slices were then stimulated every 60 s for 60 min to measure the level of LTP.

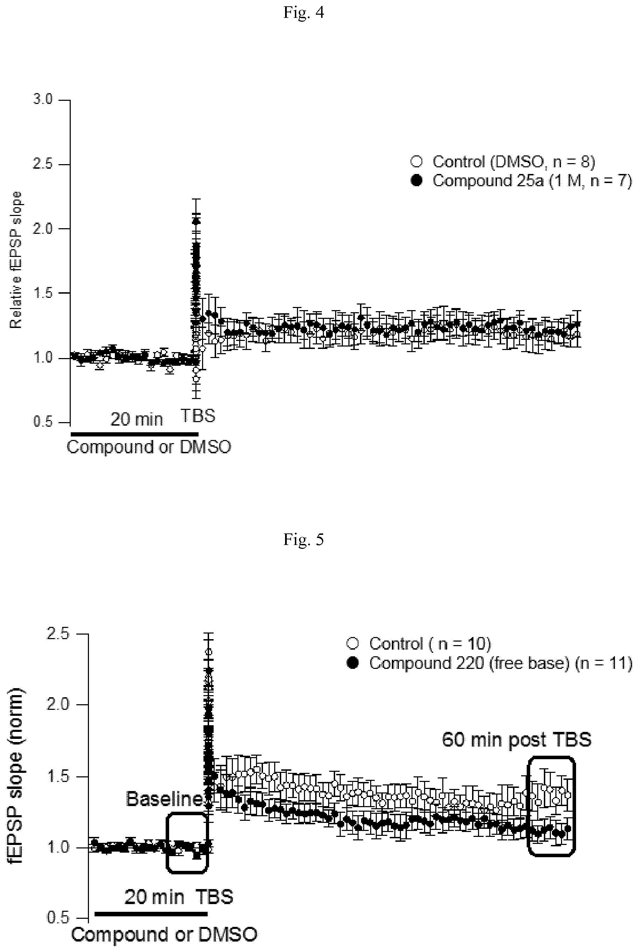

The results of this test are shown in Figure 3 for the effect of compound 70, Figure 4 for the effect of compound 25a and Figure 5 for the effect of compound 220 (free base) on the facilitation on induction of LTP with a weak Long Term Potentiation protocol.

### SINGLE DOSE PK/PD PDE2i DOG STUDY

For these studies male and female Marshall Beagle dogs (1-6y) were used: 2 males and 2 females per treatment group. Cerebrospinal fluid (CSF) was sampled from the lateral ventricle via a needle guide cannula in instrumented conscious animals.

Baseline CSF and blood samples were taken 2 to 5 days before dosing. The dogs are fasted overnight and the next morning dosed on an empty stomach (orally by gavage). At predetermined time points after dosing blood and/or CSF was collected for the measurement of compound levels and cGMP. Analysis of cGMP was done by LC-MS/MS: 25µl CSF was diluted with 125µl artificial CSF (STIL (20 ng/ml)), centrifugated and 25µl was injected. The systems used were: a Shimadzu SIL-30 UPLC-system (Hypercarb (50 mmx 1 mm (3 µm)) column, basic (10mM ammonium carbonate) aqueous-acetonitrile gradient (5% to 98% in 5.5 minutes) at a flow-rate of 250 µl/min) and an API Sciex 5500 system equiped with an ESI source (selective MRM transition (m/z 346.1->152.1 (75 msec dwelltime)). The results of this study are summarized in Figures 6-15.

### PDE2 INHIBITION ENHANCED SYNAPTIC PLASTICITY IN THE HIPPOCAMPAL SCHAFFER COLLATERAL-CA1 CIRCUIT IN ANESTHETIZED RATS: CASE STUDY WITH COMPOUND 110

### INTRODUCTION

Synaptic plasticity is a fundamental mechanism to many neurobiological functions. Long-term potentiation (LTP), a long-lasting highly localized increase in synaptic strength in the hippocampus as well as in the cortex, is a synaptic substrate for memory and learning (Cooke and Bliss, Curr Opin Investig Drugs. 2005;6(1):25-34). The increase and decrease of synaptic strength depends on the activity of presynaptic and postsynaptic neurons, on how networks in the brain operate in setting up sensory representation of multiple items in memory and producing motor response. Different features of these synaptic modifications, in intact brain, are crucial to the operation of different types of networks and the operation of several different brain circuits. Therefore, LTP is expected to be compromised in aging psychiatric and neurodegenerative disorders such as Alzheimer's disease (Bergado and Almaguer, Neural Plast. 2002;9(4):217-32; Rowan et al., Biochem Soc Trans. 2005 ;33: 563-7). In animals, the procedure carried out under anesthesia in intact highly interconnected brain regions, provides a powerful tool to investigate lasting changes in effective connectivity and plasticity in hippocampal-cortex circuits following a tetanic electrical stimulation with low and high frequency delivered in single or paired pulses (Albensi et al., Exp Neurol. 2007; 204A:1-13). The studies help expand understanding of the neural circuits underlying development of impaired synaptic strength i.e. determine the direct- circuit path and the role of specific biological target harboured by specific inter-regional network connections in mediating synaptic weakening. The procedure allows for testing pharmacological agents aimed at restoring pathological neuroplasticity e.g. reverse deficits in LTP and network connectivity by increasing synaptic efficacy, which is expected to have beneficial effects on related cognitive and learning ability (Cooke and Bliss, 2005; Albensi et al., 2007).

Phosphodiesterases (PDEs) are a class of enzymes responsible for metabolic inactivation of secondary messengers 3',5'-cyclic adenosine monophosphate (cAMP) and 3',5'-cyclic guanosine monophosphate (cGMP) (Francis et al. Physiol Rev. 2011, 9: 651-90). Up to 11 families of PDEs were categorized based on their structural, enzymatic and distribution (Omori and Kotera Circ Res. 2007;100:309-27). The role of PDEs in the augmentation of cyclic nucleotide signalling makes these enzymes attractive targets for regulating excitability and enhancing the effects of neuronal communication. In the brain, PDE2 is mainly expressed in cortex, hippocampus and striatum where it controls the hydrolysis of cAMP. Over the last few years, research groups have focused on the development of PDE2 inhibitors as a way to modify intracellular second messengers cGMP and cAMP to exert action on plasticity and cognitive processes (Duinen et al., Curr Pharm Des. 2015;21:3813-28; Gomez and Breitenbucher, Bioorg Med Chem Lett. 2013;23: 6522-7; Xu et al., Neurobiol Aging. 2015; 36:955-70; Barco et al., Expert Opin Ther Targets 2003; 7: 101-114).

In the present study, it was investigated whether PDE2 inhibition, using compound 110, leads to alterations in excitability or in the ability to express synaptic potentiation at the hippocampal Shaffer collateral-CA1 synapses in urethane-anesthetized Sprague Dawley rats.

### MATERIAL AND METHODS

### ANIMALS

The present experiments were conducted in strict accordance with the guidelines of the Association for Assessment and Accreditation of Laboratory Animal Care International (AAALAC), and with the European Communities Council Directive of 24th November 1986 (86/609/EEC) and were approved by local ethical committee. Sprague Dawley rats, weighing 170-200 g at the time of surgery, were group-housed in ventilated cages located on a 12-h light/ dark cycle (lights on at 07:00 AM) after their arrival to animal facilities maintained under controlled environmental conditions.

### SURGERY AND ELECTROPHYSIOLOGY

Rats were anesthetized with an intra-peritoneal injection of urethane 1.5 g/kg body weight. Animals were placed in a stereotactic frame for the insertion of electrodes and their body temperature was constantly monitored through a rectal probe and maintained at 37 °C with a heating pad. Supplementary administration of urethane (0.2-0.5 g/kg) was given when necessary to ensure full anaesthesia. Two small holes (1 mm diameter) were drilled in the skull at the position of left hippocampus structures for stimulating and recording electrodes. A bipolar stimulating electrode; a pair of twisted tungsten wires (75 µm) with tips horizontally separated 0.125 µm apart, were positioned at Schaffer collateral-commissural (SC) pathway (AP -3.4, ML -2.5, DV -1.9 to 2.4), and a tungsten recording electrode are positioned at the Stratum Radiatum of the *Cornu Ammonis* (CA1) area of the dorsal hippocampus (AP -4.2, ML -4.0, DV -2.5 to 3.4) (Fig 16a). The dura was pierced through both holes, and the stimulating and recording electrodes were lowered very slowly (0.2 mm/min) through the cortex and upper layers of the hippocampus into the mPP and the DG of the dorsal hippocampus. During surgery, all efforts were made to minimize animal suffering.

The field excitatory postsynaptic potential (fEPSP) slope is used as a measure of excitatory synaptic transmission. Single monophasic square 0.1 or 0.2 ms wave pulses generated by a constant current unit (MC, Germany) were applied for instance to the SC and evoked responses are generated in the CA1. Extracellular field potentials are amplified; band pass filtered between 1 Hz and 2 kHz, digitized and analyzed using custom made software. The electrodes were lowered until a negative deflecting fEPSP with the maximum response is observed. A minimum of 30 min is allowed to ensure stabilization excitability before measurements. Next, monophasic constant current pulses with stimulus intensities ranging from 1 to 12 Volts were delivered to generate Input/Output (I/O) curves and determine the maximum fEPSP slopes, and then stimulus intensity that produced 50% of the maximum response (i.e., test pulse) was used in subsequent experiments.

LTP induction: After the determination of I/O curves, test stimulation was then applied every 30 s before and after tetanic stimulation. For each time point measured during the experiments, five records of evoked responses at the frequency of 0.033 Hz were averaged. Baseline activity was measured every 5 min for at least 1 h to ensure stable baseline. The last 30 min of the baseline recording (6 time points), immediately after drug application was averaged and used as control for LTP induction. Tetanisation was induced using a high-frequency stimulation (HFS) 200-Hz protocol consisting of square pulses (0.2 ms stimulus duration, 10 bursts of 20 stimuli, 2 s inter-burst interval) at a stimulus intensity that evoked a fEPSP slope that was approximately 50% of the maximal response. fEPSP were recorded during 120 min after HFS to determine possible changes in the synaptic response of SC-CA1 neurons. LTP measurements were derived from field EPSP ratios of the normalized slope average obtained 120 min following HFS divided by the normalized slope average collected 30 min prior to HFS. Slope of putative fEPSP were measured between the end of stimulus artefact and the trough of the negative peak. In 80% interval between these points, a linear fit least square analysis was used to calculate the EPSP slope.

### HISTOLOGY

At the end of the electrophysiological study, electrical stimulation of 500 µA for 30 sec was delivered to produce a lesion at the end tip of the stimulation and recording electrodes and brains were harvested for histological verification of electrodes placement. Brain sections (20 µm) were examined using a light microscope. Animals with incorrect electrode placement were excluded from the study.

Compound 110 was dissolved in 20% Cyclodextrine (CD) + 1HCl. For subcutaneous administration, Compound 110 was dissolved in 20% CD +1HCl to achieve final concentrations of 2 and 4 mg/ml.

### STATISTIC

For each animal, the stable baseline (pre-tetanus) responses over 30 min were averaged and the mean was normalized as being 100%, and the post-tetanus response data were expressed relative by the baseline average. Comparison of the effects of vehicle and compound 110 after tetanus was performed on 30 min intervals using One-way analysis of variance (ANOVA) and least significant difference (LSD) post-hoc analysis was applied for group comparisons.

### RESULTS

No differences in the slope of the SC-CA1 path fEPSPs were found across the I/O of the study groups, suggesting that the excitability of CA1 cells was similar in all animals (Fig. 16b, c). Basal synaptic transmission was enhanced as significant changes were found between Compound 110 (20 and 40 mg/kg) and vehicle-treated control during baseline pre-tetanus (+8 and 9%, respectively) (Fig. 16d, e). During the LTP induction paradigm, subcutaneous administration of compound 110 (20 and 40 mg/kg) enhanced an enduring (>2 h) synaptic potentiation (137 ± 8% and 142 ± 5% as compared to vehicle level 119 ± 4%, respectively) (Fig. 16d, Inset plot). At 0-30 min after completion of the tetanization, fEPSP slopes were 153 ± 5% and 155 ± 4% as compared to vehicle level 139 ± 1%, p<0.05). In subsequent 30 min intervals, analysis of stimulus-response curves revealed a significant lasting increase in the fEPSP with the dose 40 mg/kg (30-60 min: 146 ± 6%, 60-90 min: 137 ± 5% and 90-120 min: 129 ± 5% as compared to vehicle levels 125 ± 6, 116 ± 9 and 106 ± 13%, p<0.05, respectively). Overall, Compound 110 facilitates basal synaptic transmission and LTP in vivo.

## Claims

1. A compound having the Formula (I) 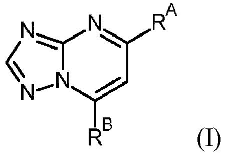 or a stereoisomeric form thereof, wherein
R^{A} is selected from the group consisting of H, CH₃, CN, and CHF₂;
R^{B} is a radical selected from the group consisting of (a), (b) and (c): 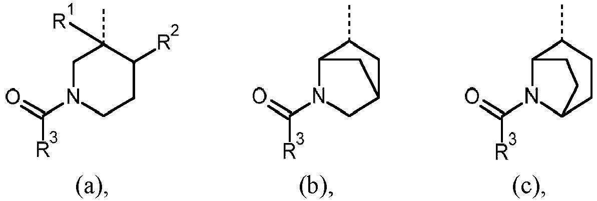 wherein
R¹ is H, F or CH₃;
R² is H or C₁₋₄alkyl; with the proviso that when R² is H, then R¹ is F or CH₃;
R³ is Ar, Het, or Ar-C₂₋₄alkenyl; wherein
Ar represents phenyl or naphthyl, each optionally substituted with 1, 2 or 3 substituents, each independently selected from the group consisting of halo; CN; NR^{2A}R^{2B} wherein R^{2A} and R^{2B} are each independently selected from H and CH₃; OH; C₁₋₆alkyl optionally substituted with 1, 2 or 3 independently selected halo substituents; C₁₋₆alkyl substituted with CN; C₃₋₆cycloalkyl; C₁₋₆alkyloxy optionally substituted with 1, 2 or 3 independently selected halo substituents; and pyrazolyl;
Het represents
(i) a 5-membered heteroaryl selected from the group consisting of 1H-pyrrolyl; thienyl; furanyl; 1H-pyrazolyl; 1H-imidazolyl; 1,2-oxazolyl; 1,3-oxazolyl; and thiazolyl; each of which may be optionally substituted with 1, 2 or 3 substituents each independently selected from the group consisting of halo; C₁₋₄alkyl optionally substituted with 1, 2, or 3 independently selected halo substituents; NR^{3A}R^{3B} wherein R^{3A} and R^{3B} are each independently selected from H and CH₃; and furan-2-yl; or
(ii) a 6-membered heteroaryl selected from the group consisting of pyridyl, pyrimidinyl, pyrazinyl, and pyridazinyl; each of which may be optionally substituted with 1, 2 or 3 substituents each independently selected from the group consisting of halo; OH; CN; NR^{4A}R^{4B} wherein R^{4A} and R^{4B} are each independently selected from H and CH₃; C₁₋₄alkyl optionally substituted with 1, 2 or 3 independently selected halo substituents; C₁₋₄alkyl substituted with OH; C₃₋₆cycloalkyl; C₃₋₆cycloalkyloxy; C₁₋₄alkyloxy optionally substituted with 1, 2 or 3 independently selected halo substituents; and C₁₋₄alkyloxyC₁₋₄alkyl; or
(iii) a 8- to 10-membered bicyclic partially unsaturated heterocyclyl selected from the group consisting of 2,3-dihydro-1-benzofuranyl; 2H-chromenyl; 3,4-dihydro-2H-chromenyl; 2,3-dihydro-1H-indolyl optionally substituted at the 1-position with C₁₋₄alkyl, methylsulfonyl, 1-acetyl, or fluoroacetyl; 2,2-difluoro-1,3-benzodioxolyl; 1,3-benzodioxolyl optionally substituted with a methyl substituent; 3,4-dihydro-2H-1,4-benzoxazinyl optionally substituted with C₁₋₄alkyl; 5,6,7,8-tetrahydroimidazo[1,2-a]pyridinyl; 5,6,7,8-tetrahydroquinolinyl optionally substituted with a halo substituent; and 2,3-dihydropyrazolo[5,1-b][1,3]oxazolyl; or
(iv) a 9- to 10-membered bicyclic heteroaryl selected from the group consisting of 1-benzofuranyl; 1-benzothiophenyl; 1H-indolyl; 1,3-benzoxazolyl; 1,3-benzothiazolyl; indolizinyl; 1H-benzimidazolyl; imidazo[1,2-a]pyridinyl; pyrazolo[1,5-a]pyridinyl; 1H-thieno[2,3-c]pyrazolyl; imidazo[2,1-b]thiazolyl; pyrrolo[2,3-c]pyridinyl; thieno[3,2-b]pyridinyl; quinolinyl; isoquinolinyl; quinoxalinyl; 1,8-naphthyridinyl; and 1,6-naphthyridinyl; each of which may be optionally substituted with 1 or 2 substituents each independently selected from the group consisting of halo; OH; NR^{5A}R^{5B} wherein R^{5A} and R^{5B} are each independently selected from H and CH₃; C₁₋₄alkyl optionally substituted with 1, 2 or 3 independently selected halo substituents; and C₁₋₄alkyloxy optionally substituted with 1, 2 or 3 independently selected halo substituents;
with the proviso that the compound is not 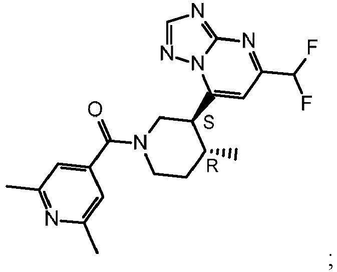 or an N-oxide, or a pharmaceutically acceptable salt or a solvate thereof.

2. The compound according to claim 1, wherein
R³ is Ar or Het; wherein
Ar represents phenyl optionally substituted with 1, 2 or 3 substituents, each independently selected from the group consisting of halo; CN; OH; C₁₋₆alkyl optionally substituted with 1, 2 or 3 independently selected halo substituents; C₁₋₆alkyl substituted with CN; C₃₋₆cycloalkyl; and C₁₋₆alkyloxy optionally substituted with 1, 2 or 3 independently selected halo substituents;
Het represents
(i) a 5-membered heteroaryl selected from the group consisting of 1H-pyrrolyl; thienyl; furanyl; 1H-pyrazolyl; 1H-imidazolyl; 1,2-oxazolyl; 1,3-oxazolyl; and thiazolyl; each of which may be optionally substituted with 1, 2 or 3 substituents each independently selected from the group consisting of halo; C₁₋₄alkyl optionally substituted with 1, 2, or 3 independently selected halo substituents; NR^{3a}R^{3b} wherein R^{3a} and R^{3b} are each independently selected from H and CH₃; and furan-2-yl; or
(ii) a 6-membered heteroaryl selected from the group consisting of pyridyl, pyrimidinyl, pyrazinyl, and pyridazinyl; each of which may be optionally substituted with 1, 2 or 3 substituents each independently selected from the group consisting of halo; OH; CN; NR^{4a}R^{4b} wherein R^{4a} and R^{4b} are each independently selected from H and CH₃; C₁₋₄alkyl optionally substituted with 1, 2 or 3 independently selected halo substituents; C₃₋₆cycloalkyl; C₃₋₆cycloalkyloxy; and C₁₋₄alkyloxy optionally substituted with 1, 2 or 3 independently selected halo substituents; or
(iii) a 8- to 10-membered bicyclic partially unsaturated heterocyclyl selected from the group consisting of 2,3-dihydro-1-benzofuranyl; 2H-chromenyl; 3,4-dihydro-2H-chromenyl; 2,3-dihydro-1H-indolyl optionally substituted at the 1-position with C₁₋₄alkyl, methylsulfonyl, 1-acetyl, or fluoroacetyl; 2,2,-difluoro-1,3-benzodioxolyl; 1,3-benzodioxolyl optionally substituted with a methyl substituent; 5,6,7,8-tetrahydroimidazo[1,2-a]pyridinyl; 5,6,7,8-tetrahydroquinolinyl optionally substituted with a halo substituent; and 2,3-dihydropyrazolo[5,1-b][1,3]oxazolyl; or
(iv) a 9- to 10-membered bicyclic heteroaryl selected from the group consisting of 1-benzofuranyl; 1-benzothiophenyl; 1H-indolyl; 1,3-benzoxazolyl; 1,3-benzothiazolyl; indolizinyl; 1H-benzimidazolyl; imidazo[1,2-a]pyridinyl; pyrazolo[1,5-a]pyridinyl; 1H-thieno[2,3-c]pyrazolyl; thieno[3,2-b]pyridinyl; quinolinyl; 1,8-naphthyridinyl; and 1,6-naphthyridinyl; each of which may be optionally substituted with 1 or 2 substituents each independently selected from the group consisting of halo; OH; NR^{3a}R^{3b} wherein R^{3a} and R^{3b} are each independently selected from H and CH₃; C₁₋₄alkyl optionally substituted with 1, 2 or 3 independently selected halo substituents; and C₁₋₄alkyloxy optionally substituted with 1, 2 or 3 independently selected halo substituents;
or a pharmaceutically acceptable salt or a solvate thereof.

3. The compound according to claim 1 or 2, wherein R^{A} is CH₃ or CHF₂.

4. The compound according to any one of claims 1 to 3, wherein R^{B} is (a) or (c).

5. The compound according to any one of claims 1 to 4, wherein R³ is Het.

6. The compound according to any one of claims 1 to 5, wherein R³ is
(i) a 6-membered heteroaryl selected from the group consisting of pyridyl, pyrimidinyl, pyrazinyl, and pyridazinyl; each of which may be optionally substituted with 1, 2 or 3 substituents each independently selected from the group consisting of halo; OH; CN; NR^{4a}R^{4b} wherein R^{4a} and R^{4b} are each independently selected from H and CH₃; C₁₋₄alkyl optionally substituted with 1, 2 or 3 independently selected halo substituents; C₃₋₆cycloalkyl; C₃₋₆cycloalkyloxy; and C₁₋₄alkyloxy optionally substituted with 1, 2 or 3 independently selected halo substituents; or
(ii) a 9- to 10-membered bicyclic heteroaryl selected from the group consisting of 1-benzofuranyl; 1-benzothiophenyl; 1H-indolyl; 1,3-benzoxazolyl; 1,3-benzothiazolyl; indolizinyl; 1H-benzimidazolyl; imidazo[1,2-a]pyridinyl; pyrazolo[1,5-a]pyridinyl; 1H-thieno[2,3-c]pyrazolyl; thieno[3,2-b]pyridinyl; quinolinyl; 1,8-naphthyridinyl; and 1,6-naphthyridinyl; each of which may be optionally substituted with 1 or 2 substituents each independently selected from the group consisting of halo; OH; NR^{3a}R^{3b} wherein R^{3a} and R^{3b} are each independently selected from H and CH₃; C₁₋₄alkyl optionally substituted with 1, 2 or 3 independently selected halo substituents; and C₁₋₄alkyloxy optionally substituted with 1, 2 or 3 independently selected halo substituents.

7. The compound according to any one of claims 1 to 6, having the Formula (I-a) or (I-b) 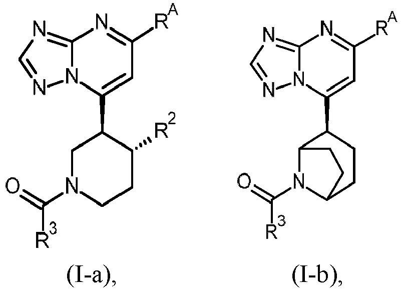 wherein R^{A}, R² and R³ are as defined in any one of claims 1 to 6.

8. The compound according to claim 1, wherein the compound is 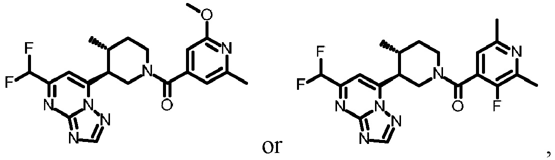 or a pharmaceutically acceptable salt or a solvate thereof.

9. A pharmaceutical composition comprising a therapeutically effective amount of a compound according to any one of claims 1 to 8 and a pharmaceutically acceptable carrier.

10. A compound according to any one of claims 1 to 8, or a pharmaceutical composition according to claim 9, for use as a medicament.

11. A compound according to any one of claims 1 to 8 or a pharmaceutical composition according to claim 9 for use in treating or preventing a central nervous system disorder selected from the group of psychotic disorders and conditions; anxiety disorders; movement disorders; drug abuse; mood disorders; neurodegenerative disorders; disorders or conditions comprising as a symptom a deficiency in attention and/or cognition; disorders related to memory acquisition and consolidation; stroke; and autistic disorder.

12. A compound or a pharmaceutical composition for use according to claim 11, wherein
the psychotic disorders are selected from the group of schizophrenia; schizophreniform disorder; schizoaffective disorder; delusional disorder; substance-induced psychotic disorder; personality disorders of the paranoid type; and personality disorder of the schizoid type;
the anxiety disorders are selected from the group of panic disorder; agoraphobia; specific phobia; social phobia; obsessive-compulsive disorder; post-traumatic stress disorder; acute stress disorder; and generalized anxiety disorder;
the movement disorders are selected from the group of Huntington's disease and dyskinesia; Parkinson's disease; restless leg syndrome and essential tremor; Tourette's syndrome and other tic disorders;
the substance-related disorders are selected from the group of alcohol abuse; alcohol dependence; alcohol withdrawal; alcohol withdrawal delirium; alcohol-induced psychotic disorder; amphetamine dependence; amphetamine withdrawal; cocaine dependence; cocaine withdrawal; nicotine dependence; nicotine withdrawal; opioid dependence and opioid withdrawal;
the mood disorders are selected from depression; mania; bipolar I disorder, bipolar II disorder; cyclothymic disorder; dysthymic disorder; major depressive disorder; treatment-resistant depression; and substance-induced mood disorder;
the neurodegenerative disorders are selected from the group of Parkinson's disease; Huntington's disease; dementia; Alzheimer's disease; multi-infarct dementia; AIDS-related dementia or frontotemperal dementia;
the disorders or conditions comprising as a symptom a deficiency in attention and/or cognition are selected from the group of dementia associated with Alzheimer's disease; multi-infarct dementia; dementia due to Lewy body disease; alcoholic dementia or substance-induced persisting dementia; dementia associated with intracranial tumours or cerebral trauma; dementia associated with Huntington's disease; dementia associated with Parkinson's disease; AIDS-related dementia; dementia due to Pick's disease; dementia due to Creutzfeldt-Jakob disease; delirium; amnestic disorder; post-traumatic stress disorder; stroke; progressive supranuclear palsy; mental retardation; a learning disorder; attention-deficit/hyperactivity disorder (ADHD); mild cognitive impairment; Asperger's syndrome; age-related cognitive impairment; and cognitive impairment related to perception, concentration, learning or memory;
the disorders related to memory acquisition and consolidation are selected from memory disorders.

13. A process for preparing a pharmaceutical composition as defined in claim 9, **characterized in that** a pharmaceutically acceptable carrier is intimately mixed with a therapeutically effective amount of a compound as defined in any one of claims 1 to 8.

14. A compound according to any one of claims 1 to 8 in combination with an additional pharmaceutical agent for use in the treatment or prevention of a condition as cited in any one of claims 11 to 12.

15. A product comprising
(a) a compound as defined in any one of claims 1 to 8; and
(b) an additional pharmaceutical agent,
as a combined preparation for simultaneous, separate or sequential use in the treatment or prevention of a condition as cited in any one of claims 11 to 12.

## Patentansprüche

1. Verbindung mit der Formel (I) 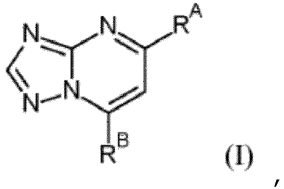 oder stereoisomere Form davon, wobei
R^{A} aus der aus H, CH₃, CN und CHF₂ bestehenden Gruppe ausgewählt ist,
R^{B} für einen aus der aus (a), (b) und (c) bestehenden Gruppe ausgewählten Rest steht: 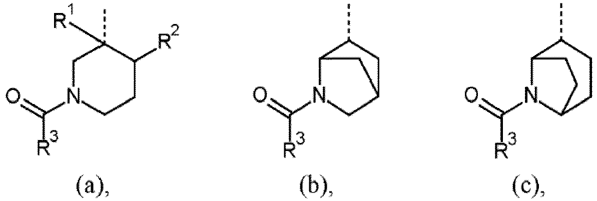 wobei
R¹ für H, F oder CH₃ steht,
R² für H oder C₁₋₄-Alkyl steht, mit der Maßgabe, dass, wenn R² für H steht, R¹ für F oder CH₃ steht,
R³ für Ar, Het oder Ar-C₂₋₄-Alkenyl steht, wobei
Ar für Phenyl oder Naphthyl steht, jeweils gegebenenfalls substituiert durch 1, 2 oder 3 Substituenten, jeweils unabhängig voneinander ausgewählt aus der Gruppe bestehend aus Halogen; CN; NR^{2A}R^{2B}, wobei R^{2A} und R^{2B} jeweils unabhängig voneinander aus H und CH₃ ausgewählt sind; OH; C₁₋₆-Alkyl, gegebenenfalls substituiert durch 1, 2 oder 3 unabhängig voneinander ausgewählte Halogensubstituenten; C₁₋₆-Alkyl, substituiert durch CN; C₃₋₆-Cycloalkyl; C₁₋₆-Alkyloxy, gegebenenfalls substituiert durch 1, 2 oder 3 unabhängig voneinander ausgewählte Halogensubstituenten; und Pyrazolyl;
Het für
(i) ein 5-gliedriges Heteroaryl ausgewählt aus der Gruppe bestehend aus 1H-Pyrrolyl, Thienyl, Furanyl, 1H-Pyrazolyl, 1H-Imidazolyl, 1,2-Oxazolyl, 1,3-Oxazolyl und Thiazolyl, jeweils gegebenenfalls substituiert durch 1, 2 oder 3 Substituenten jeweils unabhängig voneinander ausgewählt aus der Gruppe bestehend aus Halogen; C₁₋₄-Alkyl, gegebenenfalls substituiert durch 1, 2 oder 3 unabhängig voneinander ausgewählte Halogensubstituenten; NR^{3A}R^{3B}, wobei R^{3A} und R^{3B} jeweils unabhängig voneinander aus H und CH₃ ausgewählt sind; und Furan-2-yl; oder
(ii) ein 6-gliedriges Heteroaryl ausgewählt aus der Gruppe bestehend aus Pyridyl, Pyrimidinyl, Pyrazinyl und Pyridazinyl; jeweils gegebenenfalls substituiert durch 1, 2 oder 3 Substituenten jeweils unabhängig voneinander ausgewählt aus der Gruppe bestehend aus Halogen; OH; CN; NR^{4A}R^{4B}, wobei R^{4A} und R^{4B} jeweils unabhängig voneinander aus H und CH₃ ausgewählt sind; C₁₋₄-Alkyl, gegebenenfalls substituiert durch 1, 2 oder 3 unabhängig voneinander ausgewählte Halogensubstituenten; C₁₋₄-Alkyl, substituiert durch OH; C₃₋₆-Cycloalkyl; C₃₋₆-Cycloalkyloxy; C₁₋₄-Alkyloxy, gegebenenfalls substituiert durch 1, 2 oder 3 unabhängig voneinander ausgewählte Halogensubstituenten; und C₁₋₄-Alkyloxy-C₁₋₄-alkyl; oder
(iii) ein 8- bis 10-gliedriges bicyclisches teilweise ungesättigtes Heterocyclyl ausgewählt aus der Gruppe bestehend aus 2,3-Dihydro-1-benzofuranyl; 2H-Chromenyl; 3,4-Dihydro-2H-chromenyl; 2,3-Dihydro-1H-indolyl, gegebenenfalls substituiert an der 1-Position durch C₁₋₄-Alkyl, Methylsulfonyl, 1-Acetyl oder Fluoracetyl; 2,2-Difluor-1,3-benzodioxolyl; 1,3-Benzodioxolyl, gegebenenfalls substituiert durch einen Methylsubstituenten; 3,4-Dihydro-2H-1,4-benzoxazinyl, gegebenenfalls substituiert durch C₁₋₄-Alkyl; 5,6,7,8-Tetrahydroimidazo[1,2-a]pyridinyl; 5,6,7,8-Tetrahydrochinolinyl, gegebenenfalls substituiert durch einen Halogensubstituenten; und 2,3-Dihydropyrazolo[5,1-b][1,3]oxazolyl; oder
(iv) ein 9- bis 10-gliedriges bicyclisches Heteroaryl ausgewählt aus der Gruppe bestehend aus 1-Benzofuranyl, 1-Benzothiophenyl, 1H-Indolyl, 1,3-Benzoxazolyl, 1,3-Benzothiazolyl, Indolizinyl, 1H-Benzimidazolyl, Imidazo[1,2-a]pyridinyl, Pyrazolo[1,5-a]pyridinyl, 1H-Thieno[2,3-c]pyrazolyl, Imidazo[2,1-b]thiazolyl, Pyrrolo[2,3-c]pyridinyl, Thieno[3,2-b]pyridinyl, Chinolinyl, Isochinolinyl, Chinoxalinyl, 1,8-Naphthyridinyl und 1,6-Naphthyridinyl, jeweils gegebenenfalls substituiert durch 1 oder 2 Substituenten jeweils unabhängig voneinander ausgewählt aus der Gruppe bestehend aus Halogen; OH; NR^{5A}R^{5B}, wobei R^{5A} und R^{5B} jeweils unabhängig voneinander aus H und CH₃ ausgewählt sind; C₁₋₄-Alkyl, gegebenenfalls substituiert durch 1, 2 oder 3 unabhängig voneinander ausgewählte Halogensubstituenten; und C₁₋₄-Alkyloxy, gegebenenfalls substituiert durch 1, 2 oder 3 unabhängig voneinander ausgewählte Halogensubstituenten; steht, mit der Maßgabe, dass es sich bei der Verbindung nicht
um 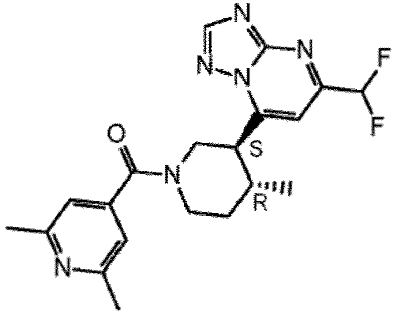 handelt, oder ein N-Oxid oder ein pharmazeutisch unbedenkliches Salz oder ein Solvat davon.

2. Verbindung nach Anspruch 1, wobei
R³ für Ar oder Het steht, wobei
Ar für Phenyl steht, gegebenenfalls substituiert durch 1, 2 oder 3 Substituenten, jeweils unabhängig voneinander ausgewählt aus der Gruppe bestehend aus Halogen; CN, OH; C₁₋₆-Alkyl, gegebenenfalls substituiert durch 1, 2 oder 3 unabhängig voneinander ausgewählte Halogensubstituenten; C₁₋₆-Alkyl, substituiert durch CN, C₃₋₆-Cycloalkyl; und C₁₋₆-Alkyloxy, gegebenenfalls substituiert durch 1, 2 oder 3 unabhängig voneinander ausgewählte Halogensubstituenten;
Het für
(i) ein 5-gliedriges Heteroaryl ausgewählt aus der Gruppe bestehend aus 1H-Pyrrolyl, Thienyl, Furanyl, 1H-Pyrazolyl, 1H-Imidazolyl, 1,2-Oxazolyl, 1,3-Oxazolyl und Thiazolyl, jeweils gegebenenfalls substituiert durch 1, 2 oder 3 Substituenten jeweils unabhängig voneinander ausgewählt aus der Gruppe bestehend aus Halogen; C₁₋₄-Alkyl, gegebenenfalls substituiert durch 1, 2 oder 3 unabhängig voneinander ausgewählte Halogensubstituenten; NR^{3a}R^{3b}, wobei R^{3a} und R^{3b} jeweils unabhängig voneinander aus H und CH₃ ausgewählt sind; und Furan-2-yl; oder
(ii) ein 6-gliedriges Heteroaryl ausgewählt aus der Gruppe bestehend aus Pyridyl, Pyrimidinyl, Pyrazinyl und Pyridazinyl, jeweils gegebenenfalls substituiert durch 1, 2 oder 3 Substituenten jeweils unabhängig voneinander ausgewählt aus der Gruppe bestehend aus Halogen; OH; CN; NR^{4a}R^{4b}, wobei R^{4a} und R^{4b} jeweils unabhängig voneinander aus H und CH₃ ausgewählt sind; C₁₋₄-Alkyl, gegebenenfalls substituiert durch 1, 2 oder 3 unabhängig voneinander ausgewählte Halogensubstituenten; C₃₋₆-Cycloalkyl; C₃₋₆-Cycloalkyloxy; und C₁₋₄-Alkyloxy, gegebenenfalls substituiert durch 1, 2 oder 3 unabhängig voneinander ausgewählte Halogensubstituenten; oder
(iii) ein 8- bis 10-gliedriges bicyclisches teilweise ungesättigtes Heterocyclyl ausgewählt aus der Gruppe bestehend aus 2,3-Dihydro-1-benzofuranyl; 2H-Chromenyl; 3,4-Dihydro-2H-chromenyl; 2,3-Dihydro-1H-indolyl, gegebenenfalls substituiert an der 1-Position durch C₁₋₄-Alkyl, Methylsulfonyl, 1-Acetyl oder Fluoracetyl; 2,2-Difluor-1,3-benzodioxolyl; 1,3-Benzodioxolyl, gegebenenfalls substituiert durch einen Methylsubstituenten; 5,6,7,8-Tetrahydroimidazo[1,2-a]pyridinyl; 5,6,7,8-Tetrahydrochinolinyl, gegebenenfalls substituiert durch einen Halogensubstituenten; und 2,3-Dihydropyrazolo[5,1-b][1,3]oxazolyl; oder
(iv) ein 9- bis 10-gliedriges bicyclisches Heteroaryl ausgewählt aus der Gruppe bestehend aus 1-Benzofuranyl, 1-Benzothiophenyl, 1H-Indolyl, 1,3-Benzoxazolyl, 1,3-Benzothiazolyl, Indolizinyl, 1H-Benzimidazolyl, Imidazo[1,2-a]pyridinyl, Pyrazolo[1,5-a]pyridinyl, 1H-Thieno[2,3-c]pyrazolyl, Thieno[3,2-b]pyridinyl, Chinolinyl, 1,8-Naphthyridinyl und 1,6-Naphthyridinyl, jeweils gegebenenfalls substituiert durch 1 oder 2 Substituenten jeweils unabhängig voneinander ausgewählt aus der Gruppe bestehend aus Halogen; OH; NR^{3a}R^{3b}, wobei R^{3a} und R^{3b} jeweils unabhängig voneinander aus H und CH₃ ausgewählt sind; C₁₋₄-Alkyl, gegebenenfalls substituiert durch 1, 2 oder 3 unabhängig voneinander ausgewählte Halogensubstituenten; und C₁₋₄-Alkyloxy, gegebenenfalls substituiert durch 1, 2 oder 3 unabhängig voneinander ausgewählte Halogensubstituenten; steht,
oder ein pharmazeutisch unbedenkliches Salz oder ein Solvat davon.

3. Verbindung nach Anspruch 1 oder 2, wobei R^{A} für CH₃ oder CHF₂ steht.

4. Verbindung nach einem der Ansprüche 1 bis 3, wobei R^{B} für (a) oder (c) steht.

5. Verbindung nach einem der Ansprüche 1 bis 4, wobei R³ für Het steht.

6. Verbindung nach einem der Ansprüche 1 bis 5, wobei R³ für
(i) ein 6-gliedriges Heteroaryl ausgewählt aus der Gruppe bestehend aus Pyridyl, Pyrimidinyl, Pyrazinyl und Pyridazinyl, jeweils gegebenenfalls substituiert durch 1, 2 oder 3 Substituenten jeweils unabhängig voneinander ausgewählt aus der Gruppe bestehend aus Halogen; OH; CN; NR^{4a}R^{4b}, wobei R^{4a} und R^{4b} jeweils unabhängig voneinander aus H und CH₃ ausgewählt sind; C₁₋₄-Alkyl, gegebenenfalls substituiert durch 1, 2 oder 3 unabhängig voneinander ausgewählte Halogensubstituenten; C₃₋₆-Cycloalkyl; C₃₋₆-Cycloalkyloxy; und C₁₋₄-Alkyloxy, gegebenenfalls substituiert durch 1, 2 oder 3 unabhängig voneinander ausgewählte Halogensubstituenten; oder
(ii) ein 9- bis 10-gliedriges bicyclisches Heteroaryl ausgewählt aus der Gruppe bestehend aus 1-Benzofuranyl, 1-Benzothiophenyl, 1H-Indolyl, 1,3-Benzoxazolyl, 1,3-Benzothiazolyl, Indolizinyl, 1H-Benzimidazolyl, Imidazo[1,2-a]pyridinyl, Pyrazolo[1,5-a]pyridinyl, 1H-Thieno[2,3-c]pyrazolyl, Thieno[3,2-b]pyridinyl, Chinolinyl, 1,8-Naphthyridinyl und 1,6-Naphthyridinyl, jeweils gegebenenfalls substituiert durch 1 oder 2 Substituenten jeweils unabhängig voneinander ausgewählt aus der Gruppe bestehend aus Halogen; OH; NR^{3a}R^{3b}, wobei R^{3a} und R^{3b} jeweils unabhängig voneinander aus H und CH₃ ausgewählt sind; C₁₋₄-Alkyl, gegebenenfalls substituiert durch 1, 2 oder 3 unabhängig voneinander ausgewählte Halogensubstituenten; und C₁₋₄-Alkyloxy, gegebenenfalls substituiert durch 1, 2 oder 3 unabhängig voneinander ausgewählte Halogensubstituenten.

7. Verbindung nach einem der Ansprüche 1 bis 6 mit der Formel (I-a) oder (I-b) 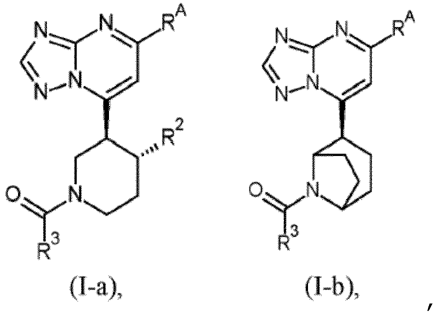 wobei R^{A}, R² und R³ wie in einem der Ansprüche 1 bis 6 definiert sind.

8. Verbindung nach Anspruch 1, wobei es sich bei der Verbindung um 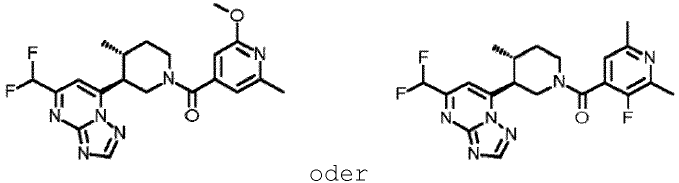 handelt, oder ein pharmazeutisch unbedenkliches Salz oder ein Solvat davon.

9. Pharmazeutische Zusammensetzung, umfassend eine therapeutisch wirksame Menge einer Verbindung nach einem der Ansprüche 1 bis 8 und einen pharmazeutisch unbedenklichen Träger.

10. Verbindung nach einem der Ansprüche 1 bis 8 oder pharmazeutische Zusammensetzung nach Anspruch 9 zur Verwendung als Medikament.

11. Verbindung nach einem der Ansprüche 1 bis 8 oder pharmazeutische Zusammensetzung nach Anspruch 9 zur Verwendung bei der Behandlung oder Prävention einer Erkrankung des zentralen Nervensystems ausgewählt aus der Gruppe der psychotischen Störungen und Leiden, Angststörungen, Bewegungsstörungen, Drogenmissbrauch, Gemütsstörungen, neurodegenerativen Erkrankungen, Erkrankungen bzw. Leiden, die als ein Symptom ein Aufmerksamkeits- und/oder Wahrnehmungsdefizit umfassen, Erkrankungen, die mit Gedächtnisspeicherung und -konsolidierung in Zusammenhang stehen, Schlaganfall und autistischer Störung.

12. Verbindung oder pharmazeutische Zusammensetzung zur Verwendung nach Anspruch 11, wobei
die psychotischen Störungen aus der Gruppe Schizophrenie, schizophreniforme Störung, schizoaffektive Störung, Wahnstörung, substanzinduzierte psychotische Störung, Persönlichkeitsstörungen des paranoiden Typs und Persönlichkeitsstörungen des schizoiden Typs ausgewählt sind,
die Angststörungen aus der Gruppe Panikstörung, Agoraphobie, spezifische Phobie, soziale Phobie, Zwangsneurose, posttraumatische Belastungsstörung, akute Stressstörung und generalisierte Angststörung ausgewählt sind,
die Bewegungsstörungen aus der Gruppe Chorea Huntington und Dyskinesie, Parkinson-Krankheit, Restless-Legs-Syndrom und essentiellem Tremor, Tourette-Syndrom und anderen Tic-Störungen ausgewählt sind,
die mit Substanzen in Zusammenhang stehenden Störungen aus der Gruppe Alkoholmissbrauch, Alkoholabhängigkeit, Alkoholentzug, Alkoholentzugsdelirium, alkoholinduzierte psychotische Störung, Amphetaminabhängigkeit, Amphetaminentzug, Kokainabhängigkeit, Kokainentzug, Nikotinabhängigkeit, Nikotinentzug, Opioidabhängigkeit und Opioidentzug ausgewählt sind,
die Gemütsstörungen aus Depression, Manie, bipolarer Störung I, bipolarer Störung II, Zyklothymia, Dysthymia, schwerer depressiver Störung, behandlungsresistenter Depression und substanzinduzierter Gemütsstörung ausgewählt sind,
die neurodegenerativen Störungen aus der Gruppe Parkinson-Krankheit, Chorea Huntington, Demenz, Alzheimer-Krankheit, Multiinfarktdemenz, AIDS-bedingte Demenz und frontotemporale Demenz ausgewählt sind,
die Erkrankungen bzw. Leiden, die als ein Symptom ein Aufmerksamkeits- und/oder Wahrnehmungsdefizit umfassen, aus der Gruppe mit Alzheimer-Krankheit assoziierte Demenz, Multiinfarktdemenz, Demenz aufgrund von Lewy-Körperchen-Krankheit, alkoholische Demenz oder substanzinduzierte persistierende Demenz, mit intrakranialen Tumoren oder zerebralem Trauma assoziierte Demenz, mit Chorea Huntington assoziierte Demenz, mit Parkinson-Krankheit assoziierte Demenz, AIDS-bedingte Demenz, Demenz aufgrund von Pick-Krankheit, Demenz aufgrund von Creutzfeldt-Jakob-Krankheit, Delirium, amnestische Störung, posttraumatische Belastungsstörung, Schlaganfall, progressive supranukleäre Blickparese, geistige Unterentwicklung, eine Lernstörung, Aufmerksamkeitsdefizit-Hyperaktivitätsstörung (Attention-Deficit/Hyperactivity Disorder, ADHD), milde kognitive Störung, Asperger-Syndrom, altersbedingte kognitive Störung und mit Wahrnehmung, Konzentration, Lernen oder Gedächtnis in Zusammenhang stehende kognitive Störung ausgewählt sind,
die mit Gedächtnisspeicherung und -konsolidierung in Zusammenhang stehenden Störungen aus Gedächtnisstörungen ausgewählt sind.

13. Verfahren zur Herstellung einer wie in Anspruch 9 definierten pharmazeutischen Zusammensetzung, **dadurch gekennzeichnet, dass** man einen pharmazeutisch unbedenklichen Träger innig mit einer therapeutisch wirksamen Menge einer wie in einem der Ansprüche 1 bis 8 definierten Verbindung mischt.

14. Verbindung nach einem der Ansprüche 1 bis 8 in Kombination mit einem zusätzlichen Pharmazeutikum zur Verwendung bei der Behandlung oder Prävention eines wie in einem der Ansprüche 11 bis 12 angeführten Leidens.

15. Produkt, umfassend
(a) eine wie in einem der Ansprüche 1 bis 8 definierte Verbindung und
(b) ein zusätzliches Pharmazeutikum,
als Kombinationspräparat zur gleichzeitigen, getrennten oder aufeinanderfolgenden Anwendung bei der Behandlung oder Prävention eines wie in einem der Ansprüche 11 bis 12 angeführten Leidens.

## Revendications

1. Composé possédant la formule (I) 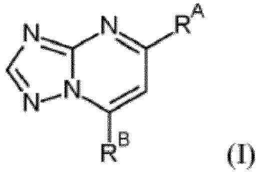 ou forme stéréoisomérique correspondante,
R^{A} étant choisi dans le groupe constitué par H, CH₃, CN, et CHF₂ ;
R^{B} étant un radical choisi dans le groupe constitué par (a), (b) et (c) : 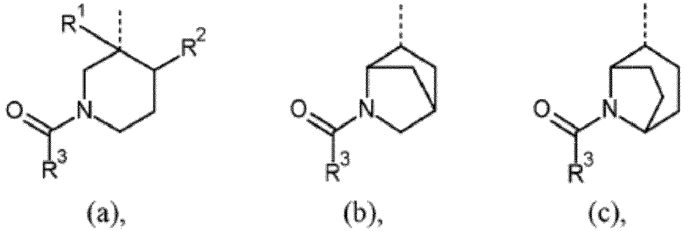
R¹ étant H, F ou CH₃ ;
R² étant H ou C₁₋₄alkyle ; étant entendu que lorsque R² est H, alors R¹ est F ou CH₃ ;
R³ étant Ar, Het ou Ar-C₂₋₄alcényle ;
Ar représentant phényle ou naphtyle, chacun éventuellement substitué par 1, 2 ou 3 substituants, chacun indépendamment choisi dans le groupe constitué par halogéno ; CN ; NR^{2A}R^{2B}, R^{2A} et R^{2B} étant chacun indépendamment choisis parmi H et CH₃; OH ; C₁₋₆alkyle éventuellement substitué par 1, 2 ou 3 substituants halogéno indépendamment choisis ; C₁₋₆alkyle substitué par CN ; C₃₋₆cycloalkyle ; C₁₋₆alkyloxy éventuellement substitué par 1, 2 ou 3 substituants halogéno indépendamment choisis ; et pyrazolyle ;
Het représentant
(i) un hétéroaryle à 5 chaînons choisi dans le groupe constitué par 1H-pyrrolyle ; thiényle ; furannyle ; 1H-pyrazolyle ; 1H-imidazolyle ; 1,2-oxazolyle ; 1,3-oxazolyle ; et thiazolyle ; chacun desquels pouvant éventuellement être substitué par 1, 2 ou 3 substituants chacun indépendamment choisis dans le groupe constitué par halogéno ; C₁₋₄alkyle éventuellement substitué par 1, 2 ou 3 substituants halogéno indépendamment choisis ; NR^{3A}R^{3B}, R^{3A} et R^{3B} étant chacun indépendamment choisis parmi H et CH₃ ; et furan-2-yle ; ou
(ii) un hétéroaryle à 6 chaînons choisi dans le groupe constitué par pyridyle, pyrimidinyle, pyrazinyle, et pyridazinyle ; chacun desquels pouvant éventuellement être substitué par 1, 2 ou 3 substituants chacun indépendamment choisis dans le groupe constitué par halogéno ; OH ; CN ; NR^{4A}R^{4B}, R^{4A} et R^{4B} étant chacun indépendamment choisis parmi H et CH₃ ; C₁₋₄alkyle éventuellement substitué par 1, 2 ou 3 substituants halogéno indépendamment choisis ; C₁₋₄alkyle substitué par OH ; C₃₋₆cycloalkyle ; C₃₋₆cycloalkyloxy ; C₁₋₄alkyloxy éventuellement substitué par 1, 2 ou 3 substituants halogéno indépendamment choisis ; et C₁₋₄alkyloxy-C₁₋₄alkyle ; ou
(iii) un hétérocyclyle bicyclique partiellement insaturé à 8 à 10 chaînons choisi dans le groupe constitué par 2,3-dihydro-1-benzofurannyle ; 2H-chroményle ; 3,4-dihydro-2H-chroményle ; 2,3-dihydro-1H-indolyle éventuellement substitué au niveau de la position 1 par C₁₋₄alkyle, méthylsulfonyle, 1-acétyle, ou fluoroacétyle ; 2,2-difluoro-1,3-benzodioxolyle ; 1,3-benzodioxolyle éventuellement substitué par un substituant méthyle ; 3,4-dihydro-2H-1,4-benzoxazinyle éventuellement substitué par C₁₋₄alkyle ; 5,6,7,8-tétrahydroimidazo[1,2-a]pyridinyle ; 5,6,7,8-tétrahydroquinoléinyle éventuellement substitué par un substituant halogéno ; et 2,3-dihydropyrazolo[5,1-b][1,3]oxazolyle ; ou
(iv) un hétéroaryle bicyclique à 9 à 10 chaînons choisi dans le groupe constitué par 1-benzofurannyle ; 1-benzothiophényle ; 1H-indolyle ; 1,3-benzoxazolyle ; 1,3-benzothiazolyle ; indolizinyle ; 1H-benzimidazolyle ; imidazo[1,2-a]pyridinyle ; pyrazolo[1,5-a]pyridinyle ; 1H-thiéno[2,3-c]pyrazolyle ; imidazo[2,1-b]thiazolyle ; pyrrolo[2,3-c]pyridinyle ; thiéno[3,2-b]pyridinyle ; quinoléinyle ; isoquinoléinyle ; quinoxalinyle ; 1,8-naphtyridinyle ; et 1,6-naphtyridinyle ; chacun desquels pouvant être éventuellement substitué par 1 ou 2 substituants chacun indépendamment choisis dans le groupe constitué par halogéno ; OH ; NR^{5A}R^{5B}, R^{5A} et R^{5B} étant chacun indépendamment choisis parmi H et CH₃ ; C₁₋₄alkyle éventuellement substitué par 1, 2 ou 3 substituants halogéno indépendamment choisis ; et C₁₋₄alkyloxy éventuellement substitué par 1, 2 ou 3 substituants halogéno indépendamment choisis ;
étant entendu que le composé n'est pas 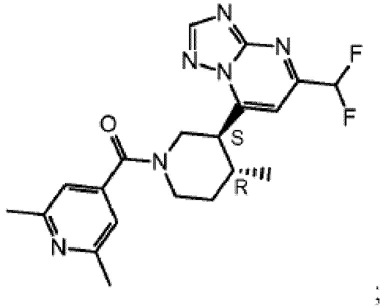 ou N-oxyde, ou sel pharmaceutiquement acceptable ou solvate correspondant.

2. Composé selon la revendication 1,
R³ étant Ar ou Het ;
Ar représentant phényle éventuellement substitué par 1, 2 ou 3 substituants, chacun indépendamment choisis dans le groupe constitué par halogéno ; CN ; OH ; C₁₋₆alkyle éventuellement substitué par 1, 2 ou 3 substituants halogéno indépendamment choisis ; C₁₋₆alkyle substitué par CN ; C₃₋₆cycloalkyle ; et C₁₋₆alkyloxy éventuellement substitué par 1, 2 ou 3 substituants halogéno indépendamment choisis ;
Het représentant
(i) un hétéroaryle à 5 chaînons choisi dans le groupe constitué par 1H-pyrrolyle ; thiényle ; furannyle ; 1H-pyrazolyle ; 1H-imidazolyle ; 1,2-oxazolyle ; 1,3-oxazolyle ; et thiazolyle ; chacun desquels pouvant éventuellement être substitué par 1, 2 ou 3 substituants chacun indépendamment choisis dans le groupe constitué par halogéno ; C₁₋₄alkyle éventuellement substitué par 1, 2, ou 3 substituants halogéno indépendamment choisis ; NR^{3a}R^{3b}, R^{3a} et R^{3b} étant chacun indépendamment choisis parmi H et CH₃ ; et furan-2-yle ; ou
(ii) un hétéroaryle à 6 chaînons choisi dans le groupe constitué par pyridyle, pyrimidinyle, pyrazinyle, et pyridazinyle ; chacun desquels pouvant éventuellement être substitué par 1, 2 ou 3 substituants chacun indépendamment choisis dans le groupe constitué par halogéno ; OH ; CN ; NR^{4a}R^{4b}, R^{4a} et R^{4b} étant chacun indépendamment choisis parmi H et CH₃ ; C₁₋₄alkyle éventuellement substitué par 1, 2 ou 3 substituants halogéno indépendamment choisis ; C₃₋₆cycloalkyle ; C₃₋₆cycloalkyloxy ; et C₁₋₄alkyloxy éventuellement substitué par 1, 2 ou 3 substituants halogéno indépendamment choisis ; ou
(iii) un hétérocyclyle bicyclique partiellement insaturé à 8 à 10 chaînons choisi dans le groupe constitué par 2,3-dihydro-1-benzofurannyle ; 2H-chroményle ; 3,4-dihydro-2H-chroményle ; 2,3-dihydro-1H-indolyle éventuellement substitué au niveau de la position 1 par C₁₋₄alkyle, méthylsulfonyle, 1-acétyle, ou fluoroacétyle ; 2,2,-difluoro-1,3-benzodioxolyle ; 1,3-benzodioxolyle éventuellement substitué par un substituant méthyle ; 5,6,7,8-tétrahydroimidazo[1,2-a]pyridinyle ; 5,6,7,8-tétrahydroquinoléinyle éventuellement substitué par un substituant halogéno ; et 2,3-dihydropyrazolo[5,1-b][1,3]oxazolyle ; ou
(iv) un hétéroaryle bicyclique à 9 à 10 chaînons choisi dans le groupe constitué par 1-benzofurannyle ; 1-benzothiophényle ; 1H-indolyle ; 1,3-benzoxazolyle ; 1,3-benzothiazolyle ; indolizinyle ; 1H-benzimidazolyle ; imidazo[1,2-a]pyridinyle ; pyrazolo[1,5-a]pyridinyle ; 1H-thiéno[2,3-c]pyrazolyle ; thiéno[3,2-b]pyridinyle ; quinoléinyle ; 1,8-naphtyridinyle ; et 1,6-naphtyridinyle ; chacun desquels pouvant éventuellement être substitué par 1 ou 2 substituants chacun indépendamment choisis dans le groupe constitué par halogéno ; OH ; NR^{3a}R^{3b}, R^{3a} et R^{3b} étant chacun indépendamment choisis parmi H et CH₃ ; C₁₋₄alkyle éventuellement substitué par 1, 2 ou 3 substituants halogéno indépendamment choisis ; et C₁₋₄alkyloxy éventuellement substitué par 1, 2 ou 3 substituants halogéno indépendamment choisis ;
ou sel pharmaceutiquement acceptable ou solvate correspondant.

3. Composé selon la revendication 1 ou 2, R^{A} étant CH₃ ou CHF₂ ;

4. Composé selon l'une quelconque des revendications 1 à 3, R^{B} étant (a) ou (c).

5. Composé selon l'une quelconque des revendications 1 à 4, R³ étant Het.

6. Composé selon l'une quelconque des revendications 1 à 5, R³ étant
(i) un hétéroaryle à 6 chaînons choisi dans le groupe constitué par pyridyle, pyrimidinyle, pyrazinyle, et pyridazinyle ; chacun desquels pouvant éventuellement être substitué par 1, 2 ou 3 substituants chacun indépendamment choisis dans le groupe constitué par halogéno ; OH ; CN ; NR^{4a}R^{4b}, R^{4a} et R^{4b} étant chacun indépendamment choisis parmi H et CH₃ ;
C₁₋₄alkyle éventuellement substitué par 1, 2 ou 3 substituants halogéno indépendamment choisis ; C₃₋₆cycloalkyle ; C₃₋₆cycloalkyloxy ; et C₁₋₄alkyloxy éventuellement substitué par 1, 2 ou 3 substituants halogéno indépendamment choisis ; ou
(ii) un hétéroaryle bicyclique à 9 à 10 chaînons choisi dans le groupe constitué par 1-benzofurannyle ; 1-benzothiophényle ; 1H-indolyle ; 1,3-benzoxazolyle ; 1,3-benzothiazolyle ; indolizinyle ; 1H-benzimidazolyle ; imidazo[1,2-a]pyridinyle ; pyrazolo[1,5-a]pyridinyle ; 1H-thiéno[2,3-c]pyrazolyle ; thiéno[3,2-b]pyridinyle ; quinoléinyle ; 1,8-naphtyridinyle ; et 1,6-naphtyridinyle ; chacun desquels pouvant éventuellement être substitué par 1 ou 2 substituants chacun indépendamment choisis dans le groupe constitué par halogéno ; OH ; NR^{3a}R^{3b}, R^{3a} et R^{3b} étant chacun indépendamment choisis parmi H et CH₃ ;
C₁₋₄alkyle éventuellement substitué par 1, 2 ou 3 substituants halogéno indépendamment choisis ; et C₁₋₄alkyloxy éventuellement substitué par 1, 2 ou 3 substituants halogéno indépendamment choisis.

7. Composé selon l'une quelconque des revendications 1 à 6 possédant la formule (I-a) ou (I-b) 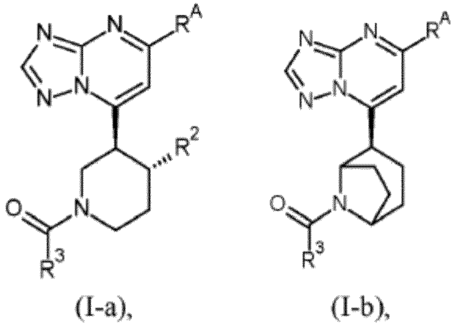 R^{A}, R² et R³ étant tels que définis dans l'une quelconque des revendications 1 à 6.

8. Composé selon la revendication 1, le composé étant 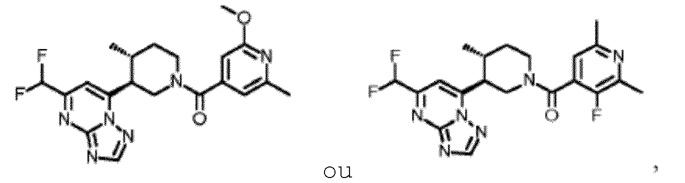 ou sel pharmaceutiquement acceptable ou solvate correspondant.

9. Composition pharmaceutique comprenant une quantité thérapeutiquement efficace d'un composé selon l'une quelconque des revendications 1 à 8 et un support pharmaceutiquement acceptable.

10. Composé selon l'une quelconque des revendications 1 à 8 ou composition pharmaceutique selon la revendication 9, pour une utilisation en tant que médicament.

11. Composé selon l'une quelconque des revendications 1 à 8 ou composition pharmaceutique selon la revendication 9 pour une utilisation dans le traitement ou la prévention d'un trouble du système nerveux central choisi dans le groupe des troubles et des affections psychotiques ; des troubles anxieux ; des troubles du mouvement ; un abus de médicament ; des troubles de l'humeur ; des troubles neurodégénératifs ; des troubles ou des affections comprenant comme symptôme une déficience de l'attention et/ou de la cognition ; des troubles liés à l'acquisition et à la consolidation de la mémoire ; un accident vasculaire cérébral ; et un trouble autistique.

12. Composé ou composition pharmaceutique pour une utilisation selon la revendication 11,
les troubles psychotiques étant choisis dans le groupe de la schizophrénie ; d'un trouble schizophréniforme ; d'un trouble schizoaffectif ; d'un trouble délirant ; d'un trouble psychotique induit par une substance ; de troubles de la personnalité du type paranoïde ; et d'un trouble de la personnalité du type schizoïde ;
les troubles anxieux étant choisis dans le groupe d'un trouble panique ; de l'agoraphobie ; de la phobie spécifique ; de la phobie sociale ; d'un trouble obsessif-compulsif ; d'un trouble de stress post-traumatique ; d'un trouble de stress aigu ; et d'un trouble anxieux généralisé ;
les troubles du mouvement étant choisis dans le groupe de la maladie de Huntington et de la dyskinésie ; de la maladie de Parkinson ; du syndrome de la jambe sans repos et d'un tremblement essentiel ; du syndrome de Gilles de la Tourette et d'autres troubles de tic ;
les troubles liés à une substance étant choisis dans le groupe de l'abus d'alcool ; de la dépendance à l'alcool ; d'un sevrage de l'alcool ; d'un délire de sevrage de l'alcool ; d'un trouble psychotique induit par l'alcool ; d'une dépendance aux amphétamines ; d'un sevrage des amphétamines ; d'une dépendance à la cocaïne ; d'un sevrage de la cocaïne ; d'une dépendance à la nicotine ; d'un sevrage de la nicotine ; d'une dépendance aux opioïdes et d'un sevrage des opioïdes ;
les troubles de l'humeur étant choisis parmi la dépression ; la manie ; un trouble bipolaire I, un trouble bipolaire II ; un trouble cyclothymique ; un trouble dysthymique ; un trouble dépressif majeur ; une dépression résistante au traitement ; et un trouble de l'humeur induit par une substance ;
les troubles neurodégénératifs étant choisis dans le groupe de la maladie de Parkinson ; de la maladie de Huntington ; de la démence ; de la maladie d'Alzheimer ; de la démence de multi-infarctus ; de la démence liée au sida ou de la démence frontotemporale ;
les troubles ou les affections comprenant comme symptôme une déficience de l'attention et/ou de la cognition étant choisi(e)s dans le groupe de la démence associée à la maladie d'Alzheimer ; de la démence de multi-infarctus ; de la démence due à une maladie à corps de Lewy ; d'une démence alcoolique ou d'une démence persistante induite par une substance ; d'une démence associée à des tumeurs intracrâniennes ou à un traumatisme cérébral ; d'une démence associée à la maladie de Huntington ; d'une démence associée à la maladie de Parkinson ; d'une démence liée au sida ; d'une démence due à la maladie de Pick ; d'une démence due à la maladie de Creutzfeld-Jacob ; d'un délire ; d'un trouble amnésique ; d'un trouble de stress post-traumatique ; d'un accident vasculaire cérébral ; d'une paralysie supranucléaire progressive ; d'un retard mental ; d'un trouble de l'apprentissage ; d'un trouble du déficit de l'attention/de l'hyperactivité (TDAH) ; d'une déficience cognitive légère ; du syndrome d'Asperger ; d'une déficience cognitive liée à l'âge ; et d'une déficience cognitive liée à la perception, à la concentration, à l'apprentissage ou à la mémoire ;
les troubles liés à l'acquisition et à la consolidation de la mémoire étant choisis parmi des troubles de la mémoire.

13. Procédé pour la préparation d'une composition pharmaceutique telle que définie dans la revendication 9, **caractérisé en ce qu'**un support pharmaceutiquement acceptable est intimement mélangé avec une quantité thérapeutiquement efficace d'un composé tel que défini selon l'une quelconque des revendications 1 à 8.

14. Composé selon l'une quelconque des revendications 1 à 8 en combinaison avec un agent pharmaceutique supplémentaire pour une utilisation dans le traitement ou la prévention d'une affection telle que citée dans l'une quelconque des revendications 11 et 12.

15. Produit comprenant
(a) un composé tel que défini dans l'une quelconque des revendications 1 à 8 ; et
(b) un agent pharmaceutique supplémentaire,
en tant que préparation combinée pour une utilisation simultanée, séparée ou séquentielle dans le traitement ou la prévention d'une affection telle que citée dans l'une quelconque des revendications 11 et 12.
